(19) **Europäisches Patentamt**
**European Patent Office**
**Office européen des brevets**

(11) **EP 3 472 358 B1**

(12) **EUROPEAN PATENT SPECIFICATION**

(45) Date of publication and mention
of the grant of the patent:
**18.08.2021   Bulletin 2021/33**

(51) Int Cl.:
*C12Q 1/68* (2018.01)      *G16B 20/00* (2019.01)
*G16B 40/00* (2019.01)

(21) Application number: **17814184.2**

(22) Date of filing: **16.06.2017**

(86) International application number:
**PCT/US2017/037900**

(87) International publication number:
**WO 2017/218908 (21.12.2017 Gazette 2017/51)**

(54) **METHODS AND SYSTEM FOR EPIGENETIC ANALYSIS**

VERFAHREN UND SYSTEM FÜR EPIGENETISCHE ANALYSE

PROCÉDÉS ET SYSTÈME D'ANALYSE ÉPIGÉNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2016   US 201662351056 P**

(43) Date of publication of application:
**24.04.2019   Bulletin 2019/17**

(73) Proprietor: **The Johns Hopkins University Baltimore, MD 21218 (US)**

(72) Inventors:
• **FEINBERG, Andrew, P.**
  **Baltimore, MD 21218 (US)**
• **GOUTSIAS, John**
  **Baltimore, MD 21218 (US)**
• **JENKINSON, William, G.**
  **Baltimore, MD 21218 (US)**
• **PUJADAS, Elisabet**
  **Baltimore, MD 21218 (US)**

(74) Representative: **Murgitroyd & Company Murgitroyd House 165-169 Scotland Street Glasgow G5 8PL (GB)**

(56) References cited:
**WO-A1-2014/043763      US-A1- 2010 143 929 US-A1- 2014 128 283**

• **M. STEVENS ET AL: "Estimating absolute methylation levels at single-CpG resolution from methylation enrichment and restriction enzyme sequencing methods", GENOME RESEARCH, vol. 23, no. 9, 26 June 2013 (2013-06-26), pages 1541-1553, XP055452303, US ISSN: 1088-9051, DOI: 10.1101/gr.152231.112**
• **STEVENS ET AL.: 'Estimating absolute methylation levels at single-CpG resolution from methylation enrichment and restriction enzyme sequencing methods' GENOME RES. vol. 23, September 2013, pages 1541 - 1553, XP055452303**

**Description**

**BACKGROUND OF THE INVENTION**

FIELD OF THE INVENTION

The invention relates generally to epigenetics and more specifically to methods and a system for analysis and classification of the epigenome in health and disease.

BACKGROUND INFORMATION

The classical definition of epigenetics by Waddington is the emergence of a phenotype that can be perturbed by the environment but whose endpoints are predetermined by genes. Waddington used the language of ordinary differential equations, including the notion of an "attractor", to describe the robustness of deterministic phenotypic endpoints to environmental perturbations, which he believed to be entirely governed by DNA sequence and genes. However, a growing appreciation for the role that stochasticity and uncertainty play in development and epigenetics has led to relatively simple probabilistic models that take into account epigenetic uncertainty by adding a "noise" term to deterministic models or probabilistically modelling methylation sites independently.

Although some authors have recognized the importance of entropy in DNA methylation, it has so far been defined in a non-model based empirical manner with limited resolution and requiring extensive cell culture expansion and even molecular tagging for its measurement. As such, there exists a need for new model-based methods of epigenetic analysis that take into account the role of stochasticity and uncertainty, while accounting for non-independent behavior among methylation sites.

M. Stevens et al., Genome Research, vol. 23, no. 9, pages 1541-1553 discloses methylCRF, a conditional random fields-based algorithm that integrates methylated DNA immunoprecipitation and methylation-sensitive restriction enzyme sequencing data to predict DNA methylation levels at single-CpG resolution.

**SUMMARY OF THE INVENTION**

In one embodiment, the invention provides a method for performing epigenetic analysis as claimed in claim 1 that includes calculating an epigenetic potential energy landscape (PEL), or the corresponding joint probability distribution, of a genomic region within one or more genomic samples. Calculating the PEL includes: a) partitioning a genome into discrete genomic regions; b) analyzing the methylation status within a genomic region by fitting a parametric statistical model (hereafter referred to as The Model) to methylation data that takes into account dependence among the methylation states at individual methylation sites, with the number of parameters of The Model growing slower than geometrically in the number of methylation sites inside the region; and c) computing and analyzing a PEL, or the corresponding joint probability distribution, within the genomic region and/or its subregions and/or merged super-regions, thereby performing epigenetic analysis.

Disclosed herein is a method for performing epigenetic analysis that includes the computation and analysis of the average methylation status of a genome. The method includes: a) partitioning the genome into discrete genomic regions; b) analyzing the methylation status within a genomic region by fitting The Model to methylation data; and c) quantifying the average methylation status of the genomic region and/or its subregions and/or merged super-regions, thereby performing epigenetic analysis.

Also disclosed is a method for performing epigenetic analysis that includes the computation and analysis of the epigenetic uncertainty of a genome. The analysis includes: a) partitioning the genome into discrete genomic regions; b) analyzing the methylation status within a genomic region by fitting The Model to methylation data; and c) quantifying methylation uncertainty of the genomic region and/or its subregions and/or merged super-regions, thereby performing epigenetic analysis.

Also disclosed is a method for performing epigenetic analysis that includes the analysis of epigenetic discordance between a first genome and a second genome (including but not limited to the analysis of epigenetic discordance between a normal and a diseased state, such as cancer, with genomes procured from one or more patients). The analysis includes: a) partitioning the first and the second genome into discrete genomic regions; b) analyzing the methylation statuses within a genomic region of the first and the second genomes by fitting The Model to methylation data in each genome; and c) quantifying a difference and/or distance between the probability distributions and/or quantities derived therefrom for the genomic region and/or its subregions and/or merged super-regions between the first and second genomes; thereby performing epigenetic analysis.

Also disclosed is a method for performing epigenetic analysis that includes detecting the skewness and/or bimodality of the probability distribution of the methylation level and classifying the average methylation status of a genomic region into discrete classes, including bistability. Detection and classification includes: a) partitioning the genome into discrete genomic regions; b) analyzing the methylation status within a genomic region by fitting The Model to methylation data; and c) detecting the skewness and/or bimodality of the probability distribution of the methylation level and classifying the average methylation status of a genomic region into discrete classes, including bistability, thereby performing epigenetic analysis.

Also disclosed is a method for performing epigenetic analysis that includes classifying methylation uncertainty within a genomic region into discrete classes. Classification includes: a) partitioning the genome into discrete genomic regions; b) analyzing the methylation status within a genomic region by fitting The Model to methylation data; and c) classifying the methylation uncertainty of a genomic region into discrete classes, thereby performing epigenetic analysis.

Also disclosed is a method for performing epigenetic analysis that includes the computation of methylation regions and methylation blocks. Computation includes: a) partitioning the genome into discrete genomic regions; b) analyzing the methylation status within a genomic region by fitting The Model to methylation data; c) classifying the methylation status of genomic regions across the entire genome; and d) grouping the classification results into methylation regions and methylation blocks, thereby performing epigenetic analysis.

Also disclosed is a method for performing epigenetic analysis that includes the computation of entropy regions and entropy blocks. Computation includes: a) partitioning the genome into discrete genomic regions; b) analyzing the methylation status within a genomic region by fitting The Model to methylation data; c) classifying the methylation uncertainty of genomic regions across the entire genome; and d) grouping the classification results into entropy regions and entropy blocks, thereby performing epigenetic analysis.

Also disclosed is a method for performing epigenetic analysis that includes the calculation of informational properties of epigenetic maintenance through methylation channels. The analysis includes: a) partitioning the genome into discrete genomic regions; b) analyzing the methylation status within a genomic region by fitting The Model to methylation data; and c) quantifying the informational properties of epigenetic maintenance (including but not limited to the capacity and relative dissipated energy of methylation channels) of a genomic region and/or its subregions and/or merged super-regions, thereby performing epigenetic analysis.

Also disclosed is a method for performing epigenetic analysis that includes computing the sensitivity to perturbations of informational/statistical properties (including but not limited to entropy) of the methylation system within a genomic region and/or its subregions and/or merged super-regions. The analysis includes: a) partitioning a genome into discrete genomic regions; b) analyzing the methylation status within a genomic region by fitting The Model to methylation data; and c) quantifying the sensitivity to perturbations of informational/statistical properties (including but not limited to entropy) of the methylation system within the genomic region and/or its subregions and/or merged super-regions, thereby performing epigenetic analysis.

Also disclosed is a method for performing epigenetic analysis that includes identifying genomic features (including but not limited to gene promoters) in a genome that exhibit high entropic sensitivity or large differences in entropic sensitivity between a first genome and a second genome (including but not limited to between a normal and a diseased state, such as cancer, with genomes procured from one or more patients). The analysis includes: a) partitioning the first and second genomes into discrete genomic regions; b) analyzing the methylation status within a genomic region by fitting The Model to methylation data; and c) identifying genomic features (including but not limited to gene promoters) in a genome that exhibit high entropic sensitivity or large differences in entropic sensitivity between a first genome and a second genome (including but not limited to between a normal and a diseased state, such as cancer, with genomes procured from one or more patients).

Also disclosed is a method for performing epigenetic analysis that identifies genomic features (including but not limited to gene promoters) with potentially important biological functions (including but not limited to regulation of normal versus diseased states, such as cancer) occult to mean-based analysis, while exhibiting higher-order statistical differences (including but not limited to entropy or information distances) in the methylation states between a first genome and a second genome. Identification includes: a) partitioning the first and second genomes into discrete genomic regions; b) analyzing the methylation status within a genomic region for the first and second genome by fitting The Model to methylation data in each genome; and c) identifying genomic features (including but not limited to gene promoters) with relatively low mean differences but relatively high epigenetic differences in higher-order statistical quantities (including but not limited to entropy or informational distances) between the first and the second genome, thereby performing epigenetic analysis.

Also disclosed is a method for performing epigenetic analysis that identifies relationships between bistability in methylation and genomic features (including but not limited to gene promoters) with potentially important biological function. The analysis includes: a) partitioning the genomes of one or more genomic samples into discrete genomic regions; b) analyzing the methylation status within a genomic region by fitting The Model to methylation data; and c) identifying genomic features (including but not limited to gene promoters) associated with high amounts of bistability in their methylation status in one or more genomic samples and relating them to potentially important biological function, thereby performing epigenetic analysis.

Also disclosed is a method for performing epigenetic analysis that detects boundaries of topologically associating domains (TADs) of the genome without performing chromatin experiments. Detection includes: a) partitioning the genomes of one or more genomic samples into discrete genomic regions; b) analyzing the methylation status within a genomic region of each genome by fitting The Model to methylation data; and c) locating TAD boundaries, thereby performing epigenetic analysis.

Also disclosed is a method for performing epigenetic analysis based on predicting euchromatin/heterochromatin domains (including but not limited to compartments A and B) from methylation data. Prediction includes: a) partitioning the genome into discrete genomic regions; b) analyzing the methylation status within a genomic region by fitting The Model to the methylation data; and c) combining results from multiple regions to estimate the euchromatin/heterochromatin domains (including but not limited to A/B compartment organization) using a regression or classification model trained on data for which A/B euchromatin/heterochromatin domain information has been previously measured or estimated, thereby performing epigenetic analysis.

Also disclosed is a method for performing epigenetic analysis that includes identifying genomic features (including but not limited to gene promoters) for which a change in euchromatin/heterochromatin structure (including but not limited to compartments A and B) is observed between a first genome and a second genome (including but not limited to between a normal and a diseased state, such as cancer, with genomes procured from one or more patients). The analysis includes: a) partitioning the first and second genomes into discrete genomic regions; b) analyzing the methylation status within a genomic region by fitting The Model to methylation data; and c) identifying genomic features (including but not limited to gene promoters) for which a change in euchromatin/heterochromatin structure (including but not limited to compartments A and B) is observed between a first genome and a second genome (including but not limited to between a normal and a diseased state, such as cancer, with genomes procured from one or more patients).

In another embodiment, the invention provides a non-transitory computer readable storage medium encoded with a computer program. The program includes instructions that, when executed by one or more processors, cause the one or more processors to perform operations that implement the method of claims 1-10.

Also disclosed is a computing system. The system includes a memory, and one or more processors coupled to the memory, with the one or more processors being configured to perform operations that implement the method of the disclosure.

## BRIEF DESCRIPTION OF THE DRAWINGS

**Figures 1A-1C** are graphical representations relating to potential energy landscapes.

**Figures 2A-2C** are graphical representations relating to the genome-wide distributions of the mean methylation level and methylation entropy in various genomic samples.

**Figures 3A-3D** are graphical representations showing changes in mean methylation level and methylation entropy between normal and cancer samples.

**Figures 4A-4B** are graphical representations showing the breakdown of mean methylation level and methylation entropy within genomic features throughout the genome in various genomic samples.

**Figures 5A-5C** are graphical representations showing that cultured fibroblasts may not be appropriate for modeling aging.

**Figure 6** is a pictorial representation showing that epigenetic distances delineate lineages.

**Figures 7A-7E** are graphical representations showing differential regulation within genomic regions of high Jensen-Shannon distance but low differential mean methylation level near promoters of some genes.

**Figure 8** is a graphical representation showing the relationship between methylation entropy and bistable genomic subregions.

**Figures 9A-9E** are pictorial and graphical representations relating to methylation bistability and imprinting.

**Figures 10A-10B** are pictorial and graphical representations showing that the location of TAD boundaries is associated with boundaries of entropic blocks.

**Figure 11** is a pictorial representation relating entropy blocks to TAD boundaries.

**Figure 12** is a graphical representation showing the accuracy of locating TAD boundaries within boundaries of entropic blocks.

**Figure 13** is a graphical representation showing the genome-wide distribution of information-theoretic properties of methylation channels in various genomic samples.

**Figures 14A-14B** is a graphical representation showing the breakdown of information-theoretic properties of methylation channels within genomic features throughout the genome in various genomic samples.

**Figures 15A-15C** is a graphical representation showing that information-theoretic properties of methylation channels can be used to predict large-scale chromatin organization.

**Figure 16** is a graphical representation showing switching of compartments A and B in cancer.

**Figure 17** is a graphical representation relating compartment A/B switching with clustering of genomic samples.

**Figures 18A-18B** are graphical representations showing that compartment B overlaps with hypomethylated blocks, lamina associate domains and large organized chromatin K9-modifications, and is enriched for larger epigenetic differences between normal and cancer .

**Figures 19A-19D** are graphical representations showing A/B compartmental relocation of genes in cancer.

**Figures 20A-20C** are graphical representations relating to the computation and comparison of entropic sensitivity across the genome.

**Figure 21** is a graphical representation showing the breakdown of entropic sensitivity within genomic features throughout the genome in various genomic samples.

**Figures 22A-22E** are graphical representations showing a wide behavior of entropic sensitivity in the genome.

**Figure 23** is a graphical representation showing the breakdown of entropic sensitivity within compartments A and B in various genomic samples.

## DETAILED DESCRIPTION OF THE INVENTION

The present invention is based on innovative computational methods for epigenomic analysis. Epigenetics is defined as genomic modifications carrying information independent of DNA sequence heritable through cell division. In 1940, Waddington coined the term "epigenetic landscape" as a metaphor for pluripotency and differentiation, but epigenetic potential energy landscapes have not yet been rigorously defined. Using well-grounded biological assumptions and principles of statistical physics and information theory, the present disclosure describes derivation of potential energy landscapes from whole genome bisulfite sequencing data, or other data sources of methylation status, which allow quantification of genome-wide methylation stochasticity and epigenetic differences using Shannon's entropy and the Jensen-Shannon distance. The present disclosure further discusses discovery of important developmental genes occult to previous mean-based methylation analysis and the exploration of a relationship between entropy and chromatin structure. Viewing methylation maintenance as a communications system, methylation channels are introduced into the analytical methods and show that higher-order chromatin organization can be predicted from their informational properties. The results herein provide a fundamental understanding of the information-theoretic nature of the epigenome and a powerful methodology for studying its role in disease and aging.

Before the present compositions and methods are described, it is to be understood that this invention is not limited to particular methods and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

A foundational approach has been taken to understanding the nature of epigenetic information by using principles of statistical physics and information theory to organically incorporate stochasticity into the mathematical framework and applying it on primary whole genome bisulfite sequencing (WGBS) datasets. The results allow one to combine "hardwired" mechanistic principles of epigenetic biology with the Ising model of statistical physics and rigorously derive epigenetic potential energy landscapes that can be computed genome-wide, in contrast to metaphorical "Waddingtonian" landscapes. These landscapes encapsulate the higher-order statistical behavior of methylation in a biologically relevant manner, and not just its mean as it has been customary.

Methylation uncertainty is quantified genome-wide using Shannon's entropy. Moreover, a powerful information-theoretic methodology for distinguishing epigenomes using the Jensen-Shannon distance between sample-specific potential energy landscapes associated with stem cells, tissue lineages and cancer is provided, which is used to discover important developmental genes previously occult to mean-based analysis that exhibit higher-order statistical differences in the methylation states between two genomes. A relationship between entropy and topologically associating domains (TADs) is also established, which allows one to efficiently predict their boundaries from individual WGBS samples.

Methylation channels are also introduced as models of DNA methylation maintenance and show that their informational properties can be effectively used to predict higher-order chromatin organization using machine learning. Lastly, a sensitivity index is introduced that quantifies the rate by which environmental or external perturbations influence methylation uncertainty along the genome, suggesting that genomic loci associated with high sensitivity are those most affected by such perturbations.

This merger of epigenetic biology, statistical physics and information theory yields many fundamental insights into the relationship between information-theoretic properties of the epigenome and nuclear organization in normal development and disease, and demonstrates that the inventors can precisely identify informational properties of individual WGBS samples and their chromatin structure, as well as their differences among tissue lineages, aging, and cancer.

COMPUTATIONAL METHODS

The present invention provides methods of epigenetic analysis that take into account the role of stochasticity and uncertainty.

*Potential Energy Landscapes*

In an embodiment, the invention provides a method for performing epigenetic analysis that includes calculating an epigenetic potential energy landscape (PEL), or the corresponding joint probability distribution, of a genomic region within one or more genomic samples. Calculating the PEL includes: a) partitioning a genome into discrete genomic regions; b) analyzing the methylation status within a genomic region by fitting a parametric statistical model (hereafter referred to as The Model) to methylation data that takes into account dependence among the methylation states at individual methylation sites, with the number of parameters of The Model growing slower than geometrically in the number of methylation sites inside the region; and c) computing and analyzing a PEL, or the corresponding joint probability distribution, within the genomic region and/or its subregions and/or merged super-regions, thereby performing epigenetic analysis.

Despite it being known that stochastic variation is a fundamental property of the DNA methylome, genome-wide modeling and analysis of the methylation state continues to focus on individual CpG dinucleotides and ignores statistical dependence among these sites. However, DNA methylation is correlated, at least over small distances, due to the processivity of the DNMT enzymes. Therefore, one cannot adequately analyze methylation with methods that do not take into account such correlation. To this end, and to better understand the relationship between stochastic epigenetic fluctuation and phenotypic variability, a general path to methylation modeling and analysis is taken herein by developing an information-theoretic approach based on the Ising model of statistical physics. This approach leads to a rigorous definition of a potential energy landscape, which associates each methylation state with a potential that quantifies the information content of that state. The Ising model provides a natural way of modeling statistically dependent binary methylation data that is consistent with observed means and pairwise correlations.

Here, DNA methylation is viewed as a process that reliably transmits linear strings of binary (0-1) data from a cell to its progeny in a manner that is robust to intrinsic and extrinsic stochastic biochemical fluctuations. First, the methylation state within a given genomic region containing $N$ CpG sites is modeled by an $N$-dimensional binary-valued random vector $\mathbf{X}$ whose $n$-th element $X_n$ takes value 0 or 1 depending on whether or not the $n$-th CpG site is unmethylated or methylated, respectively. Then, the potential energy landscape (PEL) of methylation is defined by

$$V_{\mathbf{X}}(\mathbf{x}) = \phi_0 - \log P_{\mathbf{X}}(\mathbf{x}), \qquad (1)$$

for some constant $\phi_0$, where $P_{\mathbf{x}}(\mathbf{x})$ is the joint probability of a methylation state $\mathbf{x}$ within the genomic region. As a consequence, $P_{\mathbf{x}}(\mathbf{x})$ is the Boltzmann-Gibbs distribution of statistical physics, given by

$$P_{\mathbf{X}}(\mathbf{x}) = \frac{1}{Z} \exp\{-V_{\mathbf{X}}(\mathbf{x})\}, \qquad (2)$$

with state energy $V_{\mathbf{x}}(\mathbf{x})$ and partition function

$$Z = \sum_{\mathbf{x}} \exp\{-V_{\mathbf{X}}(\mathbf{x})\}. \qquad (3)$$

The potential $V_{\mathbf{x}}(\mathbf{x})$ - $\phi_0$ quantifies the amount of information associated with the methylation state $\mathbf{x}$, which is given by - $\log P_{\mathbf{x}}(\mathbf{x})$.

By using the well-known maximum-entropy principle, it is determined that the PEL which maximizes uncertainty about the particular choice of the Boltzmann-Gibbs distribution that is consistent with the methylation means and pairwise correlations is given by

$$V_{\mathbf{X}}(\mathbf{x}) = -\sum_{n=1}^{N} a_n (2x_n - 1) - \sum_{n=2}^{N} c_n (2x_n - 1)(2x_{n-1} - 1), \tag{4}$$

for some parameters $\{a_1,...,a_N\}$ and $\{c_2,...,c_N\}$. This leads to a methylation probability $P_{\mathbf{X}}(\mathbf{x})$ that is modeled by the one-dimensional nearest-neighbor Ising model. Parameter $a_n$ influences the propensity of the $n$-th CpG site to be methylated due to non-cooperative factors, with positive $a_n$ promoting methylation and negative $a_n$ inhibiting methylation, whereas parameter $c_n$ influences the correlation between the methylation states of two consecutive CpG sites $n$ and $n$ - 1 due to cooperative factors, with positive $c_n$ promoting positive correlation and negative $c_n$ promoting negative correlation (anti-correlation).

Computing the PEL requires estimating values for the parameters $\{a_1,...,a_N\}$ and $\{c_2,...,c_N\}$ from methylation data. For a given chromosome containing a large number $N$ of CpG sites, one must estimate $2N$ - 1 parameters, which is prohibitive for reliable estimation in low to moderate coverage sequencing data. To address this problem, a chromosome is partitioned into relatively small and equally sized non-overlapping regions (hereafter referred to as genomic regions) whose lengths are taken to be 3000 base pairs each, a length that has been determined by striking a balance between estimation and computational performance. Moreover, the parameters $a_n$ and $c_n$ are taken to satisfy

$$a_n = \alpha + \beta \rho_n \quad \text{and} \quad c_n = \gamma / d_n, \tag{5}$$

where $\rho_n$ is the CpG density within a symmetric neighborhood of 1000 nucleotides centered at a CpG site $n$, given by

$$\rho_n = \frac{1}{1,000}[\# \text{ of CpG sites within } \pm 500 \text{ nucleotides downstream and upstream of } n], \tag{6}$$

and $d_n$ is the distance of CpG site $n$ from its "nearest-neighbor" CpG site $n$-1, given by

$$d_n = [\# \text{ of base-pair steps between the cytosines of CpG sites } n \text{ and } n-1]. \tag{7}$$

Parameter $\alpha$ accounts for intrinsic factors that uniformly affect CpG methylation over a genomic region, whereas parameter $\beta$ modulates the influence of the CpG density on methylation. The previous expression for $c_n$ accounts for the expectation that correlation between the methylation of two consecutive CpG sites decays as the distance between these two sites increases, since the longer a DNMT enzyme must move along the DNA the higher is the probability of dissociating from the DNA before reaching the next CpG site. It can be shown that, in this case, the PEL within a genomic region is given by

$$V_{\mathbf{X}}(\mathbf{x}) = -\alpha'(2x_1 - 1) - \alpha \sum_{n=2}^{N-1}(2x_n - 1) - \alpha''(2x_n - 1) - \beta \sum_{n=2}^{N-1}(2x_n - 1)\rho_n$$

$$- \gamma \sum_{n=2}^{N}(2x_n - 1)(2x_{n-1} - 1)/d_n, \tag{8}$$

where $N$ is the number of CpG sites within the genomic region and the parameters $\alpha'$ and $\alpha''$ account for boundary effects that occur when restricting the PEL associated with the entire chromosome to the individual PELs associated with the genomic regions within the chromosome.

The PEL encapsulates the view that methylation within a genomic region depends on two distinct factors: the underlying CpG architecture of the genome at that location, quantified by the CpG density $\rho_n$, defined by Equation (6) and the distance $d_n$, given by Equation (7), whose values can be readily determined from the DNA sequence itself, as well as by the current biochemical environment in the nucleus provided by the methylation machinery, quantified by the parameters of the Ising model whose values must be estimated from available methylation data.

Computing the PEL within a genomic region requires estimating values for only five parameters $\theta = [\alpha' \; \alpha \; \alpha'' \; \beta \; \gamma]$ from methylation data within the genomic region. This estimation is performed by a maximum-likelihood approach, which computes the value of $\theta$ that maximizes the average log-likelihood function $(1/M)\sum_{m=1}^{M}\log P_{\mathbf{X}}(\mathbf{x}_m \mid \boldsymbol{\theta})$, where $\mathbf{x}_1,\mathbf{x}_2,...,\mathbf{x}_M$ are $M$ independent observations of the methylation state within the genomic region. To take into account partially observable methylation states measured by current experimental methods, the methylation probability $P_{\mathbf{x}}(\mathbf{x}_m \mid \theta)$ is replaced by the joint probability distribution over only those sites at which methylation information is measured. Moreover, to avoid statistical overfitting, regions with less than 10 CpG sites are not modeled, and the same applies for regions with not enough data for which the methylation state of less than 2/3 of the CpG sites is measured or for which the average depth of coverage is less than 2.5 observations per CpG sites. In addition, likelihood maximization is performed by multilevel coordinated search (MCS), a general-purpose global non-convex and derivative-free optimization algorithm.

Evaluating the joint probability of a methylation state $\mathbf{x}$, requires calculating the partition function $Z$ of the Boltzmann-Gibbs distribution, which cannot be computed directly from Equation (3), since $Z$ is expressed as a sum over a large number of distinct states that grows geometrically (as $2^N$) in the number $N$ of CpG sites within the genomic region. However, it can be shown that

$$Z = Z_1(0) + Z_1(1), \qquad (9)$$

where $Z_1$ is computed using the following recursion:

$$
\begin{aligned}
Z_N(0) &= Z_N(1) = 1 \\
Z_n(0) &= \phi_n(0,0)Z_{n+1}(0) + \phi_n(0,1)Z_{n+1}(1) \\
Z_n(1) &= \phi_n(1,0)Z_{n+1}(0) + \phi_n(1,1)Z_{n+1}(1), \\
& \quad n = N-1, N-2, ..., 1,
\end{aligned}
\qquad (10)
$$

with

$$
\begin{aligned}
\phi_1(x_1, x_2) &= \exp\{a_1(2x_1 - 1) + a_2(2x_2 - 1) + c_2(2x_1 - 1)(2x_2 - 1)\} \\
\phi_n(x_n, x_{n+1}) &= \exp\{a_{n+1}(2x_{n+1} - 1) + c_{n+1}(2x_n - 1)(2x_{n+1} - 1)\}, \\
& \quad n = 2, 3, ..., N-1,
\end{aligned}
\qquad (11)
$$

which provides a fast method for calculating the partition function. Knowledge of the partition function allows evaluation of the probability of any methylation state $\mathbf{x}$ using

$$P_X(x_1, ..., x_N) = \frac{1}{Z}\prod_{n=1}^{N-1}\phi_n(x_n, x_{n+1}). \qquad (12)$$

Since the Ising model depends on the CpG density and distance, its statistical properties may vary within a genomic region suggesting that a smaller region of the genome must be used for high-resolution methylation analysis. Consistent with the length of DNA within a nucleosome, each genomic region is further partitioned into small and equally sized non-overlapping regions (hereafter referred to as genomic subregions) of 150 base pairs each and methylation analysis is performed at a resolution of one genomic subregion.

Within a genomic subregion, epigenetic regulation is most likely controlled by the number of methylated sites and not by the particular configuration of methylation within the genomic subregion. For this reason, methylation within a genomic subregion is quantified by the methylation level $L$ (the fraction of methylated CpG sites within a genomic subregion), given by

$$L = \frac{1}{N}\sum_{n=1}^{N}X_n, \qquad (13)$$

where $N$ is the number of CpG sites within the genomic subregion and $X_n$ is a binary random variable that takes value 0 or 1 depending on whether or not the $n$-th CpG site in the genomic subregion is unmethylated or methylated, respectively.

The methylation level within a genomic subregion with $N$ CpG sites is statistically characterized by the probability distribution $P_L(l) = \Pr[L = l]$, $l = 0,1/N,...,1$, which is computed from the probability distribution $\Pr[\mathbf{X} = \mathbf{x}]$ of the methylation state within the genomic subregion by

$$P_L(l) = \sum_{x \in S(Nl)} \Pr[\mathbf{X} = \mathbf{x}], \qquad (14)$$

where $S(Nl)$ is the number of methylation states within the genomic subregion with exactly $N \times l$ CpG sites being methylated and the methylation probabilities $\Pr[\mathbf{X} = \mathbf{x}]$ are computed my marginalizing the Ising model.

Computing a marginalized form $P_X(x_r,...,x_{r+s})$, $1 \le r \le r + s \le N$, of the Ising probability distribution $P_X(x_1,...,x_N)$ is done in a computationally efficient manner by means of

$$P_X(x_r,...,x_{r+s}) = \frac{1}{Z} Z_{r+s}(x_{r+s}) Q_r(x_r) \prod_{n=r}^{r+s-1} \phi_n(x_n, x_{n+1}), \qquad (15)$$

where $Z$ and $Z_n(x_n)$ are computed using Equations (9) and (10), $\phi_n(x_n,x_{n+1})$ is computed using Equation (11), and $Q_r(x_r)$ is computed by means of the following recursion:

$$\begin{aligned}
Q_1(0) &= Q_1(1) = 1 \\
Q_n(0) &= \phi_{n-1}(0,0)Q_{n-1}(0) + \phi_{n-1}(1,0)Q_{n-1}(1) \\
Q_n(1) &= \phi_{n-1}(0,1)Q_{n-1}(0) + \phi_{n-1}(1,1)Q_{n-1}(1), \\
&\qquad n = 2,3,...,r.
\end{aligned} \qquad (16)$$

*Mean Methylation Level*

Also disclosed is a method for performing epigenetic analysis that includes the computation and analysis of the average methylation status of a genome. The method includes: a) partitioning the genome into discrete genomic regions; b) analyzing the methylation status within a genomic region by fitting The Model to methylation data; and c) quantifying the average methylation status of the genomic region and/or its subregions and/or merged super-regions, thereby performing epigenetic analysis.

The average methylation status within a genomic subregion is quantified by the mean value of the methylation level, which is referred to as the mean methylation level (MML), given by

$$E[L] = \frac{1}{N} \sum_{n=1}^{N} P_n(1), \qquad (17)$$

where $N$ is the number of CpG sites within the genomic subregion, and $P_n(1)$ is the probability that the $n$-th CpG site within the genomic subregion is methylated. The probability $P_n(1)$ is computed from the probability distribution $P_X(\mathbf{x})$ of the methylation state within the genomic subregion by marginalization.

The MML is an effective measure of methylation status that can be reliably computed genome-wide from low coverage methylation data using the Ising model. Moreover, distributions of MML values can be computed over selected genomic features (e.g., CpG islands, island shores, shelves, open sea, exons, introns, gene promoters, and the like), thus providing a genome-wide breakdown of methylation uncertainty showing lower or higher levels of methylation within said genomic features of a first genome as compared to a second genome.

*Epigenetic Uncertainty*

Also disclosed is a method for performing epigenetic analysis that includes the computation and analysis of the epigenetic uncertainty of a genome. The analysis includes: a) partitioning the genome into discrete genomic regions; b) analyzing the methylation status within a genomic region by fitting The Model to methylation data; and c) quantifying methylation uncertainty of the genomic region and/or its subregions and/or merged super-regions, thereby performing epigenetic analysis.

Due to their first-order marginal nature, means and variances provide a narrow view of methylation and its uncertainty. Previous methods of methylation analysis have attempted to provide a more comprehensive view by using the notions of epipolymorphism and combinatorial (Boltzmann) entropy. However, these methods rely on empirically estimating probabilities of specific methylation patterns (epialleles). It has been demonstrated that, in contrast to the model-based estimation of joint probabilities and Shannon entropy employed here, empirical estimation of epiallelic probabilities, epipolymorphisms and combinatorial entropies, requires much higher coverage than routinely available from WGBS data. With regards to a previous study, it has been often found that the 95% confidence intervals of empirically estimated epipolymorphisms will not include the true values resulting in potentially large errors.

Methylation uncertainty within a genomic subregion that contains $N$ CpG sites is quantified by the normalized methylation entropy (NME)

$$h = \frac{H}{\log_2(N+1)}, \qquad (18)$$

where

$$H = -\sum_l P_L(l)\log_2 P_L(l) \qquad (19)$$

is the informational (Shannon) entropy of the methylation level within the genomic subregion that provides an average assessment of the amount of epigenetic information conveyed by any given genomic subregion. When all methylation levels are equally likely (fully disordered state), the NME takes its maximum value of 1 regardless of the number of CpG sites in the genomic subregion, whereas it achieves its minimum value of 0 only when a single methylation level is observed (perfectly ordered state).

The NME is an effective measure of methylation uncertainty that can be reliably computed genome-wide from low coverage methylation data using the Ising model. Moreover, distributions of NME values can be computed over selected genomic features (e.g., CpG islands, island shores, shelves, open sea, exons, introns, gene promoters, and the like), thus providing a genome-wide breakdown of methylation uncertainty showing lower or higher levels of methylation uncertainty within said genomic features of a first genome as compared to a second genome.

*Epigenetic Distances*

Also disclosed is a method for performing epigenetic analysis that includes the analysis of epigenetic discordance between a first genome and a second genome (including but not limited to the analysis of epigenetic discordance between a normal and a diseased state, such as cancer, with genomes produced from one or more patients). The analysis includes: a) partitioning the first and the second genome into discrete genomic regions; b) analyzing the methylation statuses within a genomic region of the first and the second genomes by fitting The Model to methylation data in each genome; and c) quantifying a difference and/or distance between the probability distributions and/or quantities derived therefrom for the genomic region and/or its subregions and/or merged super-regions between the first and second genomes; thereby performing epigenetic analysis.

To understand the relationship between epigenetic information and phenotypic variation, it is possible to precisely quantify epigenetic discordance between pairs of genomic samples using the Jensen-Shannon distance (JSD), which measures the dissimilarity between the probability distributions of the methylation level within a genomic subregion across two genomic samples. This distance is used to distinguish between genomic samples from normal tissue and genomic samples from tumors, and more generally to distinguish between genomic samples from diverse tissue types.

The JSD is given by

$$D_{JS} = \sqrt{\frac{1}{2}\left[D_{KL}(P_L^{(1)}, \overline{P}_L) + D_{KL}(P_L^{(2)}, \overline{P}_L)\right]}, \qquad (20)$$

where $P_L^{(1)}$ and $P_L^{(2)}$ are the probability distributions of the methylation level within a genomic subregion in the two genomes, $\overline{P}_L = [P_L^{(1)} + P_L^{(2)}] / 2$ is the average distribution of the methylation level, and

$$D_{KL}(P,Q) = \sum_l P(l) \log_2 \left[ \frac{P(l)}{Q(l)} \right] \qquad (21)$$

is the relative entropy or Kullback-Leibler divergence. The JSD is a normalized distance metric that takes values between 0 and 1, whereas the square JSD is the average information a value of the methylation level drawn from one of the two probability distributions $P$ or $Q$ provides about the identity of the distribution. The JSD equals 0 only when the two distributions are identical and reaches its maximum value of 1 if the two distributions do not overlap and can, therefore, be perfectly distinguished from a single genomic sample.

To quantify the epigenetic distance between two genomic samples, the JSD values between all corresponding pairs of genomic subregions are computed genome-wide, the values are ordered in increasing order, and the smallest value in the list is determined such that 90% of the distances is less than or equal to that value (90-th percentile).

To visualize epigenetic similarities or dissimilarities between genomic samples, the epigenetic distances between pairs of genomic samples are computed, the distances are used to construct a dissimilarity matrix, and a two-dimensional representation is employed using multidimensional scaling (MDS) based on Kruskal's non-metric method, which finds a two-dimensional configuration of points whose inter-point distances correspond to the epigenetic dissimilarities among the genomic samples.

*Classification of Methylation Status*

Also disclosed is a method for performing epigenetic analysis that includes detecting the skewness and/or bimodality of the probability distribution of the methylation level and classifying the average methylation status of a genomic region into discrete classes, including bistability. Detection and classification includes: a) partitioning the genome into discrete genomic regions; b) analyzing the methylation status within a genomic region by fitting The Model to methylation data; and c) detecting the skewness and/or bimodality of the probability distribution of the methylation level and classifying the average methylation status of a genomic region into discrete classes, including bistability, thereby performing epigenetic analysis.

Classifying the methylation status of a genome is an important part of methylation analysis. The methylation status within a genomic subregion is effectively summarized by classifying the genomic subregion into one of seven discrete classes: highly unmethylated, partially unmethylated, partially methylated, highly methylated, mixed, highly mixed, and bistable. Classification is based on calculating the probability distribution of methylation level within the genomic subregion and on classifying the genomic subregion into one of the seven classes by analyzing the shape of this distribution and detecting its skewness and/or bimodality. Analysis comprises computing the probabilities

$$
\begin{aligned}
p_1 &= \Pr[0 \le L \le 0.25] \\
p_2 &= \Pr[0.25 < L < 0.5] + 0.5 \times \Pr[L = 0.5] \\
p_3 &= 0.5 \times \Pr[L = 0.5] + \Pr[0.5 < L < 0.75] \\
p_4 &= \Pr[0.75 \le L \le 1]
\end{aligned}
\qquad (22)
$$

from the probability distribution $P_L(l)$ of the methylation level, and classifying the genomic subregion using the following scheme:

- highly unmethylated: if $0.6 < p_1 + p_2 \le 1$ & $p_1 > 0.6$
- partially unmethylated: if $0.6 < p_1 + p_2 \le 1$ & $0 \le p_1 \le 0.6$
- partially methylated: if $0 \le p_1 + p_2 < 0.4$ & $0 \le p_4 \le 0.6$
- highly methylated: if $0 \le p_1 + p_2 < 0.4$ & $p_4 > 0.6$
- mixed: if $0.4 \le p_1 + p_2 < 0.6$ & $0 \le p_1 / (p_1 + p_2) \le 0.4$ & $0 \le p_4 / (p_3 + p_4) \le 0.4$
- highly mixed: if $0.4 \le p_1 + p_2 < 0.6$ & $0.4 < p_1 / (p_1 + p_2) < 0.6$ & $0.4 < p_4 / (p_3 + p_4) < 0.6$
- bistable: if $0.4 \le p_1 + p_2 < 0.6$ & $0.6 \le p_1 / (p_1 + p_2) \le 1$ & $0.6 \le p_4 / (p_3 + p_4) \le 1$

It turns out that a small number of genomic subregions will not be classified by this scheme, and these genomic subregions are ignored as far as classification of methylation status is concerned.

*Classification of Methylation Uncertainty*

Also disclosed is a method for performing epigenetic analysis that includes classifying methylation uncertainty within a genomic region into discrete classes. Classification includes: a) partitioning the genome into discrete genomic regions; b) analyzing the methylation status within a genomic region by fitting The Model to methylation data; and c) classifying the methylation uncertainty of a genomic region into discrete classes, thereby performing epigenetic analysis.

Classifying methylation uncertainty in a genome is another important part of methylation analysis. Methylation uncertainty within a genomic subregion is effectively summarized by classifying the genomic subregion into one of five discrete classes: highly ordered, moderately ordered, weakly ordered/disordered, moderately disordered, highly disordered. This classification is based on calculating the NME $h$ within the genomic subregion and on classifying the genomic subregion and using the following scheme:

- highly ordered: if $0 \leq h \leq 0.28$
- moderately ordered: if $0.28 < h \leq 0.44$
- weakly ordered/disordered: if $0.44 < h < 0.92$
- moderately disordered: if $0.92 \leq h < 0.99$
- highly disordered: if $0.99 \leq h \leq 1$

*Methylation Regions and Blocks*

Also disclosed is a method for performing epigenetic analysis that includes the computation of methylation regions and methylation blocks. Computation includes: a) partitioning the genome into discrete genomic regions; b) analyzing the methylation status within a genomic region by fitting The Model to methylation data; c) classifying the methylation status of genomic regions across the entire genome; and d) grouping the classification results into methylation regions and methylation blocks, thereby performing epigenetic analysis.

In addition to methylation analysis at the level of genomic units, it is of great interest to analyze the methylation status of a genome at the level of genomic features, such as gene promoters, enhancers and the like, as well as at the level of chromatin organization, such as lamina associated domains (LADs), large organized chromatin K9-modifications (LOCKs), and the like. This is accomplished by generating coarser versions of classification of the methylation status than at the level of genomic subregions.

For analysis at the level of genomic features, a window of 5 genomic subregions (5 times 150 = 750 base pairs in length) is slid along a genome. At each location, the window is labeled as being methylated if at least 75% of the genomic subregions intersecting the window are respectively classified as being partially/highly methylated, whereas the window is labeled as being unmethylated if at least 75% of the genomic subregions touching the window are respectively classified as being partially/highly unmethylated. All methylated windows are then grouped together using the operation of union followed by removal of regions overlapping with unmethylated windows, and the same is done for all unmethylated windows. This process generates methylation regions (MRs), classified as methylated or unmethylated, along the entire genome.

For analysis at the level of chromatin organization, a window of 500 genomic subregions (500 times 150 = 75,000 base pairs in length) is slid along a genome. At each location, the window is labeled as being methylated if at least 75% of the genomic subregions intersecting the window are respectively classified as being partially/highly methylated, whereas the window is labeled as being unmethylated if at least 75% of the genomic subregions touching the window are respectively classified as being partially/highly unmethylated. All methylated windows are then grouped together using the operation of union followed by removal of regions overlapping unmethylated windows, and the same is done for all unmethylated windows. This process generates methylation blocks (MBs), classified as methylated or unmethylated, along the entire genome.

*Entropy Regions and Blocks*

Also disclosed is a method for performing epigenetic analysis that includes the computation of entropy regions and entropy blocks. Computation includes: a) partitioning the genome into discrete genomic regions; b) analyzing the methylation status within a genomic region by fitting The Model to methylation data; c) classifying the methylation uncertainty of genomic regions across the entire genome; and d) grouping the classification results into entropy regions and entropy blocks, thereby performing epigenetic analysis.

In addition to methylation analysis at the level of genomic units, it is of great interest to analyze methylation uncertainty of a genome at the level of genomic features, such as gene promoters, enhancers and the like, as well as at the level of chromatin organization, such as lamina associated domains (LADs), large organized chromatin K9-modifications (LOCKs), and the like. This is accomplished by generating coarser versions of classification of the methylation uncertainty than at the level of genomic subregions.

For analysis at the level of genomic features, a window of 5 genomic subregions (5 times 150 = 750 base pairs in length) is slided along a genome. At each location, the window is labeled as being ordered if at least 75% of the genomic subregions intersecting the window are respectively classified as being moderately/highly ordered, whereas the window is labeled as being disordered if at least 75% of the genomic subregions touching the window are respectively classified as being moderately/highly disordered. All ordered windows are then grouped together using the operation of union followed by removal of regions overlapping disordered windows, and the same is done for all disordered windows. This process generates entropy regions (ERs), classified as ordered or disordered, along the entire genome.

For analysis at the level of genomic features, a window of 500 genomic subregions (500 times 150 = 75,000 base pairs in length) is slided along a genome. At each location, the window is labeled as being ordered if at least 75% of the genomic subregions intersecting the window are respectively classified as being moderately/highly ordered, whereas the window is labeled as being disordered if at least 75% of the genomic subregions touching the window are respectively classified as being moderately/highly disordered. All ordered windows are then grouped together using the operation of union followed by removal of regions overlapping disordered windows, and the same is done for all disordered windows. This process generates entropy blocks (EBs), classified as ordered or disordered, along the entire genome.

*Informational Properties of Epigenetic Maintenance*

Also disclosed is a method for performing epigenetic analysis that includes the calculation of informational properties of epigenetic maintenance through methylation channels. The analysis includes: a) partitioning the genome into discrete genomic regions; b) analyzing the methylation status within a genomic region by fitting The Model to methylation data; and c) quantifying the informational properties of epigenetic maintenance (including but not limited to the capacity and relative dissipated energy of methylation channels) of a genomic region and/or its subregions and/or merged super-regions, thereby performing epigenetic analysis.

Stable conservation of the DNA methylation state is essential for epigenetic memory maintenance. To quantify this process, a noisy binary communication channel is employed as a model, which dynamically updates the methylation state at a CpG site and leads to an information-theoretic perspective that enables a fundamental understanding of the relationship between reliability of methylation maintenance, energy availability, and methylation uncertainty.

Transmission of methylation information at the *n*-th CpG site of a genome is modeled by a Markov chain $X_n(0) \to X_n(1) \to ... \to X_n(k-1) \to X_n(k) \to ...$, where $X_n(0)$ is the initial methylation state before any maintenance steps and $X_n(k)$ is the methylation state after $k$ maintenance steps. In this case,

$$\begin{aligned} \Pr[X_n(k) = 0] = [1 - \nu_n(k)]\Pr[X_n(k-1) = 0] + \mu_n(k)\Pr[X_n(k-1) = 1] \\ \Pr[X_n(k) = 1] = \nu_n(k)\Pr[X_n(k-1) = 0] + [1 - \mu_n(k)]\Pr[X_n(k-1) = 1] \end{aligned}, \quad (23)$$

where $\mu_n(k)$ is the probability of demethylation associated with the *n*-th CpG site during the *k*-th maintenance step, $\nu_n(k)$ is the probability of *de novo* methylation, $1-\mu_n(k)$ is the probability of maintenance methylation, and $1-\nu_n(k)$ is the probability of lack of *de novo* methylation. The MC can be specified by the probabilities $\{\mu_n(k), \nu_n(k)\}$ of demethylation and *de novo* methylation. These probabilities are thought to be regulated by the maintenance and *de novo* methyltransferases (DNMT1, DNMT3A, and DNMT3B), by active (TET) and passive demethylation processes, as well as by other potential mechanisms, which are anticipated to be constrained by the free energy available for methylation maintenance.

To characterize a MC from methylation data, appropriate values for the probabilities $\{\mu_n(k), \nu_n(k)\}$ must be specified. Transmission of methylation information during maintenance is in general a dynamic process during which these probabilities may vary. To address this problem, it is assumed that subject to relatively invariant conditions, the biochemical properties of methylation transmission change slowly during successive maintenance steps so that the values of the parameters of the Ising model and the probabilities $\{\mu_n(k), \nu_n(k)\}$ do not change appreciably. As a consequence, Equations (23) approximately become

$$\begin{aligned} P_n(0) = (1 - \nu_n)P_n(0) + \mu_n P_n(1) \\ P_n(1) = \nu_n P_n(0) + (1 - \mu_n)P_n(1) \end{aligned}, \quad (24)$$

where $P_n(0)$ is the probability that the *n*-th CpG site is unmethylated and $P_n(1)$ is the probability that the site is methylated. This is based on the assumption that methylation information is transmitted in a stable manner through maintenance and that this process can be modeled by a stationary stochastic process operating near equilibrium. One can then show from Equations (24) that

$$\frac{\nu_n}{\mu_n} = \frac{P_n(1)}{1 - P_n(1)}. \tag{25}$$

The ratio $\lambda_n = \nu_n / \mu_n$ between the probability of *de novo* methylation and the probability of demethylation is referred to as the turnover ratio. This ratio is calculated directly from methylation data using Equation (25) with the probability $P_n(1)$ of the *n*-th CpG site to be methylated being computed from the Ising model using marginalization.

The amount of methylation uncertainty associated with the input or output of a MC at a particular CpG site *n* is given by the CG entropy (CGE)

$$S_n = -\left[1 - P_n(1)\right]\log_2\left[1 - P_n(1)\right] - P_n(1)\log_2 P_n(1), \tag{26}$$

where $P_n(1)$ is the probability that the CpG site is methylated. The CGE is calculated directly from methylation data using Equation (26) with the probability $P_n(1)$ of the *n*-th CpG site to be methylated being computed from the Ising model using marginalization.

Only a certain amount of methylation information can be transmitted by a MC at a CpG site *n* of a genome, with the maximum possible amount given by the information capacity (IC) of the MC, given by

$$C_n = \max\nolimits_{P_n(1)} I_n(X'; X), \tag{27}$$

where $I_n(X';X)$ is the mutual information between the input and the output $X'$ of the MC, and $P_n(1)$ is the probability that the CpG site is methylated. Although an exact formula can be derived for $C_n$, implementation of this formula requires that the probabilities $\{\mu_n, \nu_n\}$ of demethylation and *de novo* methylation are known or estimated at each CpG site of a genome, which is not possible using currently available technologies. However, it can be shown that the IC of a MC can be approximately calculated by:

$$C_n = \begin{cases} 1 - 0.52\left[\psi\left(\lambda_n / (1+\lambda_n)\right)\right]^{-1}[\lambda_n / (1+\lambda_n)], & \text{when } \lambda_n \leq 1 \\ 1 - 0.52\left[\psi\left(\lambda_n / (1+\lambda_n)\right)\right]^{-1}[1 / (1+\lambda_n)], & \text{when } \lambda_n > 1 \end{cases}, \tag{28}$$

where $\lambda_n$ is the turnover ratio at the *n*-th CpG site and $\psi(x)$ is the function $\psi(x) = -x\log_2(x) - (1-x)\log_2(1-x)$. The IC is calculated by computing the turnover ratio $\lambda_n$ directly from methylation data and using Equation (28).

Information processing by a MC and, as a matter of fact, by any biological system, requires consumption of free energy. An amount of work is needed to correctly transmit the methylation state during maintenance and this consumes energy that is dissipated to the surroundings in the form of heat. Due to stochastic fluctuations in the underlying biochemistry, the methylation system always drifts towards imperfect transmission of information, characterized by a non-negligible probability of error.

Consistent with general engineering principles, it is postulated in this disclosure that the (minimum) energy $E_n$ dissipated during maintenance of the methylation state at the n-th CpG site of a genome is approximately related to the probability of transmission error $\pi_n$ by

$$E_n \sim -k_B T_n \log \pi_n, \tag{29}$$

where $k_B$ is Boltzmann's constant and $T_n$ is the absolute temperature at the CpG site. Since the proportionality factor is not known in this relationship, the relative dissipated energy (RDE)

$$\varepsilon_n = \frac{E_n}{E_n^{\min}} = -\frac{\log \pi_n}{\log 2} = -\log_2 \pi_n \tag{30}$$

is used as a measure of reliability in methylation transmission, where $E_n^{\min} \sim -k_B T_n \log 2$ is the least possible energy dissipation. This implies that higher reliability (lower probability of error) can only be achieved by increasing the amount of free energy available for methylation maintenance, whereas reduction in free energy can lead to lower reliability (higher probability of error). Notably, it is not physically possible for a MC to achieve exact transmission of the methylation state (zero probability of error) since this would require an unlimited amount of available free energy.

Although an exact formula can be derived for $\varepsilon_n$, implementation of this formula requires that the probabilities $\{\mu_n, \nu_n\}$ of demethylation and *de novo* methylation are known or estimated at each CpG site of a genome, which is not possible using currently available technologies. However, it can be shown that the RDE of a MC can be approximately calculated by:

$$\varepsilon_n = \begin{cases} 4.76 + \log_2[(1+\lambda_n)/(2\lambda_n)], & \text{when } \lambda_n \leq 1 \\ 4.76 + \log_2[(1+\lambda_n)/2], & \text{when } \lambda_n > 1 \end{cases}, \qquad (31)$$

where $\lambda_n$ is the turnover ratio at the n-th methylation site. The RDE is calculated by computing the turnover ratio $\lambda_n$ directly from methylation data and using Equation (31).

ICs, RDEs, and CGEs are effective measures of the informational behavior of epigenetic maintenance that can be reliably computed genome-wide from low coverage methylation data using the Ising model. Moreover, distributions of IC, RDE, and CGE values can be computed over selected genomic features (e.g., CpG islands, island shores, shelves, open sea, exons, introns, gene promoters, and the like), thus providing a genome-wide breakdown of methylation uncertainty showing different aspects of the informational properties of epigenetic maintenance within said genomic features of a first genome as compared to a second genome.

*Epigenetic Sensitivity*

Also disclosed is a method for performing epigenetic analysis that includes computing the sensitivity to perturbations of informational/statistical properties (including but not limited to entropy) of the methylation system within a genomic region and/or its subregions and/or merged super-regions. The analysis includes: a) partitioning a genome into discrete genomic regions; b) analyzing the methylation status within a genomic region by fitting The Model to methylation data; and c) quantifying the sensitivity to perturbations of informational/statistical properties (including but not limited to entropy) of the methylation system within the genomic region and/or its subregions and/or merged super-regions, thereby performing epigenetic analysis.

Methylation stochasticity, as quantified by the Ising model used in this disclosure, is influenced by the values of the parameters $\theta = [\alpha' \ \alpha \ \alpha'' \ \beta \ \gamma]$ within each genomic subregion. Environmental and biochemical conditions may influence these values and thus regulate the level of methylation stochasticity, for example, by increasing or decreasing the entropy of methylation. An important aspect of methylation analysis is to determine the sensitivity of informational/statistical properties of the methylation system to perturbations of methylation parameters.

In this disclosure, a measure is used to quantify the effect of variations in parameters $\theta$ on the NME within a genomic subregion of a genome. It is assumed that, within a genomic subregion, the Ising parameters fluctuate around their estimated values $\theta$ by a random amount $G \times \theta$, where $G$ is a random variable that follows a zero-mean Gaussian distribution with small standard deviation $\sigma$. In this case, it can be shown that the standard deviation $\sigma_h$ of the NME within the genomic subregion is approximately related to the standard deviation $\sigma$ of the Ising parameters by $\sigma_h = \eta \times \sigma$, where

$$\eta = \frac{\sigma_h}{\sigma} = \left| \frac{\partial h(g)}{\partial g} \right|_{g=0}, \qquad (32)$$

with $h(g)$ being the NME within the genomic subregion when the values of the Ising parameters are given by $(1 + g) \times \theta$. Clearly, a small value of $\eta$ implies that small variations in parameter values result in a small variation in the NME, whereas a large value of $\eta$ implies that small variations in parameter values result in a large variation in NME. For this reason, $\eta$ is used to quantify the sensitivity of NME within a genomic subregion to perturbations. This measure is referred to as the entropic sensitivity index (ESI).

Calculating the ESI requires approximating the derivative in Equation (32). This is accomplished by using a finite-difference derivative approximation, in which case $\eta$ is approximated by

$$\eta = \frac{|h(w) - h(0)|}{w}, \qquad\qquad (33)$$

where w is a small number, which can be set equal to 0.01. Equation (33) is implemented by computing the NME $h(0)$ within a genomic subregion with parameter values $\theta$, obtained by estimation from methylation data, as well as the NME $h(\grave{o})$ within the genomic subregion with perturbed parameter values $(1+w)\times\theta$.

*Discovering Important Genomic Features Occult to Mean Methylation Analysis*

Also disclosed is a method for performing epigenetic analysis that identifies important genomic features (including but not limited to gene promoters) with potentially important biological functions (including but not limited to regulation of normal versus diseased states, such as cancer) occult to mean-based analysis, while exhibiting higher-order statistical differences (including but not limited to entropy or information distances) in the methylation states between a first genome and a second genome. Identification includes: a) partitioning the first and second genomes into discrete genomic regions; b) analyzing the methylation status within a genomic region for the first and second genome by fitting The Model to methylation data in each genome; and c) identifying genomic features (including but not limited to gene promoters) with relatively low mean differences but relatively high epigenetic differences in higher-order statistical quantities (including but not limited to entropy or informational distances) between the first and the second genome, thereby performing epigenetic analysis.

Current methods for the analysis of methylation are based on identifying genomic features for which differences in mean methylation are observed between a first and a second genome. However, identifying higher-order statistical differences in methylation between a first and a second genome can result in discovering genomic features with potentially important function that have not been previously found using mean-based methylation analysis.

To this end, a master ranked list of genomic features is constructed, with genomic features located higher in the master rank list being associated with relatively low mean-based differences in methylation but relatively high epigenetic differences between a first and a second genome. To form the master list, a mean-based score is calculated for each genomic feature and this score is then used to form a first rank list of genomic features, with genomic features associated with larger mean-based scores being located higher in the first rank list. Subsequently, a higher-order statistical score based on the JSD is calculated for each genomic feature and this score is then used to form a second rank list of genomic features, with genomic features associated with larger JSD-based scores being located higher in the second rank list.

To score a genomic feature in terms of mean methylation, the absolute difference between the MMLs observed for the first and the second genome are calculated for each genomic subregion that intersects the genomic feature, and a score is formed by averaging all such absolute differences, where missing data are accounted for setting the MML value equal to 0. To score a genomic feature using the JSD, the JSD is calculated for each genomic subregion that intersects the genomic feature, and a score is formed by averaging all such JSD values, where missing data are accounted for setting the JSD value equal to 0.

Using the first and the second rank lists, each genomic feature is further scored using the ratio of its ranking in the second rank list to its ranking in the first rank list. These scores are then used to form the master rank list with genomic features associated with higher scores being located lower in the master rank list. Genomic features located near the top of the master rank list are characterized by high JSD values but little difference in mean methylation level, indicating that the probability distributions of methylation level within these genomic features are different between a first and a second genome, although these probability distributions have similar means.

*Bistability and Biological Function*

Also disclosed is a method for performing epigenetic analysis that identifies relationships between bistability in methylation and genomic features (including but not limited to gene promoters) with potentially important biological function. The analysis includes: a) partitioning the genomes of one or more genomic samples into discrete genomic regions; b) analyzing the methylation status within a genomic region by fitting The Model to methylation data; and c) identifying genomic features (including but not limited to gene promoters) associated with high amounts of bistability in their methylation status in one or more genomic samples and relating them to genomic features of potentially important biological function, thereby performing epigenetic analysis.

As a direct consequence of known results of statistical physics that relate the magnetization and covariance of the one-dimensional Ising model with its underlying parameters, it was postulated that methylation within any given genomic subregion of a genome can be subject to a form of phase transition. To this end, it was found that DNA methylation can be subject to a bistable behavior that manifests itself as a coexistence of two distinct epigenetic phases:

a fully methylated and a fully unmethylated phase. This result was attributed to a reallocation of the ground states (the states of lowest potential) of the PEL $V_L(l)$ of the methylation level within the genomic subregion, given by

$$V_L(l) = \log[\max_u\{P_L(u)\}] - \log P_L(l), \qquad (34)$$

caused by a biochemically-induced deformation of its topographic surface, which results in a bimodal probability distribution for the methylation level over the fully methylated and the fully unmethylated states.

To investigate whether bistability in methylation might be associated with important biological function, its possible enrichment in selected genomic features (e.g., CpG islands, island shores, shelves, open sea, exons, introns, gene promoters, and the like) is examined. To evaluate enrichment of bistability in a particular genomic feature, two binary (0-1) random variables $R$ and $B$ are defined for each genomic subregion of a genome, such that $R = 1$, if the subregion overlaps the genomic feature, and $B = 1$, if the genomic subregion is bistable. The null hypothesis that $R$ and $B$ are statistically independent is then tested by applying the $\chi^2$-test on the $2\times2$ contingency table for $R$ and $B$ and the odds ratio (OR) is calculated as a measure of enrichment.

To evaluate possible association between bistability and genomic features associated with a specific biological phenomenon, a reference set of genomic features is considered (e.g., all gene promoters in the genome) and one or more genomic samples are employed. For each genomic sample, a score is computed for a genomic feature in the reference set, by calculating the fraction of base pairs within the genomic feature that are inside genomic subregions being classified as bistable in the genomic sample by the method used to classify the methylation status of a genome. For each genomic feature in the reference set, a bistability score is then calculated by averaging all scores obtained for the genomic feature using one or more genomic samples. The bistability scores are then used to form a rank list of the genomic features in the reference set in order of decreasing bistability. Subsequently, a test set of genomic features associated with a specific biological phenomenon is considered and a $p$-value is then calculated for the test set to be ranked higher in the bistability rank list of the reference set just by chance.

To do so, a $p$-value is first computed for each genomic feature in the test set to be ranked higher in the bistability rank list of the reference set just by chance by testing against the null hypothesis that the genomic feature appears at a random location in the bistability rank list. The rank of the genomic feature is used as the test statistic which, under the null hypothesis, follows a uniform distribution. This implies that the $p$-value of the genomic feature in the test set can be calculated by dividing the ranking of the genomic feature in the bistability rank list by the total number of genomic features in the list. The $p$-value for the test set to be ranked higher in the bistability rank list of the reference set just by chance is finally calculated by combining the individual $p$-values associated with the genomic features in the test set using Fisher's meta-analysis method.

*TAD Boundary Detection*

Also disclosed is a method for performing epigenetic analysis that detects boundaries of topologically associating domains (TADs) of the genome without performing chromatin experiments. Detection includes: a) partitioning the genomes of one or more genomic samples into discrete genomic regions; b) analyzing the methylation status within a genomic region of each genome by fitting The Model to methylation data; and c) locating TAD boundaries, thereby performing epigenetic analysis.

Topologically associating domains (TADs) are structural features of the chromatin that are highly conserved across tissue types and species. Their importance stems from the fact that loci within these domains tend to frequently interact with each other, with much less frequent interactions being observed between loci within adjacent domains. Genome-wide detection of TAD boundaries is an essential but experimentally challenging task.

The NME can be effectively used to computationally locate TAD boundaries from one or more genomic samples.

For genomic sample, ordered and disordered entropy blocks (EBs) are computed genome-wide from WGBS data by employing the method for calculating entropy regions and blocks. Regions of the genome predictive of the location of TAD boundaries are identified by detecting the unclassified genomic space between successive ordered and disordered EBs or between successive disordered and ordered EBs. For example, if an ordered EB located at chrl: 1-1000 were followed by a disordered EB at chrl: 1501-2500, then chrl: 1001-1500 is deemed to be a "predictive region". To reduce false identification of predictive regions, successive EBs of the same type are not considered, since the genomic space between two such EBs may be due to missing data or other unpredictable factors. To control the resolution of locating a TAD boundary, only unclassified genomic spaces smaller than 50,000 base pairs are considered. This results in a resolution of an order of magnitude smaller than the mean TAD size (~900-kb).

"Predictive regions" obtained from methylation analysis of more than one genomic sample are subsequently combined. The "predictive coverage" of each base pair is calculated by counting the number of "predictive regions" containing the base pair. "Predictive regions" are then combined by grouping consecutive base pairs whose predictive coverage is at least 4.

*Prediction of Euchromatin and Heterochromatin Domains*

Also disclosed is a method for performing epigenetic analysis that predicts euchromatin/heterochromatin domains (including but not limited to compartments A and B of the three-dimensional organization of a genome) from methylation data. Prediction includes: a) partitioning the genome into discrete genomic regions; b) analyzing the methylation status within a genomic region by fitting The Model to the methylation data; and c) combining results from multiple regions to estimate euchromatin/heterochromatin domains (including but not limited to A/B compartment organization) using a regression or classification model trained on data for which euchromatin/heterochromatin domain information has been previously measured or estimated, thereby performing epigenetic analysis.

The three-dimensional spatial organization of the genome allows for regions that are linearly located far from each other to come into proximity and reside in the same regulatory environment. Recent work seeking to understand this organization has demonstrated the existence of cell-type specific compartments A and B, which are known to be associated with gene-rich transcriptionally active open chromatin and gene-poor transcriptionally inactive closed chromatin, respectively.

Despite the fact that identifying compartments A/B is becoming an increasingly important aspect of fully characterizing the epigenome of a given genomic sample, the availability of such data is limited by cost, technical difficulties, and the need for sizable amounts of input material with intact nuclei required by conformation capture technologies such as Hi-C. Furthermore, conformation capture measurements are not possible on frozen tissue or DNA. This is not a limitation of the method discussed in this disclosure, since methylation data is readily captured from frozen samples using methods known in the art.

Computational prediction methods using data obtained by more routine experimental methods show promise in addressing this problem. Local information-theoretic properties of the methylome can be effectively used to computationally predict compartments A/B in the genome of any given genomic sample by a machine learning approach based on a random forest regression model applied directly to models built from WGBS data.

To do so, the entire genome is partitioned into discrete genomic bins of 100,000 base pairs each (to match training data) and 8 information-theoretic features of methylation maintenance are computed within each genomic bin from WGBS data, which include the median values and interquartile ranges of IC, RDE, NME and MML.

A random forest model with 1000 trees is trained on data consisting of input WGBS data that are matched to output chromosome conformational capture data, such as Hi-C, and/or measured or estimated compartment A/B data for one or more genomic samples. Values of the regression/classification feature vector are computed from the input WGBS data and all feature/output pairs are then used to learn a binary discriminant function that maps input feature vector values to known output compartment A/B classification.

The trained random forest model is subsequently applied on a genomic sample. The genomic sample is first partitioned into discrete genomic bins. The value of the feature vector is then calculated from WGBS data for each genomic bin, and the genomic bin is classified as being in compartment A or B by using the binary discriminant function learned during training. Since regression takes into account only information within a 100,000 base pair bin, predicted A/B values are averaged using a three-bin smoothing window and the genome-wide median value is removed from the overall A/B signal.

The accuracy of the method depends on the training step. Availability of more chromosome conformational capture and high quality measured or estimated compartment A/B data is expected to result in better training, thus increasing classification performance.

SAMPLES

In various embodiments, a genome is present in a biological sample taken from a subject. The biological sample can be virtually any biological sample, particularly a sample that contains DNA from the subject. The biological sample can be a germline, stem cell, reprogrammed cell, cultured cell, or tissue sample which contains 1000 to about 10,000,000 cells. However, it is possible to obtain samples that contain smaller numbers of cells, even a single cell, in embodiments that utilize an amplification protocol such as PCR. The sample need not contain any intact cells, so long as it contains sufficient biological material (e.g., DNA) to assess methylation status within one or more regions of the genome. The sample might also contain chromatin for analysis of euchromatin and heterochromatin by ATAC-seq or similar methods.

In some embodiments, a biological or tissue sample can be drawn from any tissue that includes cells with DNA. A biological or tissue sample may be obtained by surgery, biopsy, swab, stool, or other collection method. In some embodiments, the sample is derived from blood, plasma, serum, lymph, nerve-cell containing tissue, cerebrospinal fluid, biopsy material, tumor tissue, bone marrow, nervous tissue, skin, hair, tears, fetal material, amniocentesis material, uterine tissue, saliva, feces, or sperm. Methods for isolating PBLs from whole blood are well known in the art.

As disclosed above, the biological sample can be a blood sample. The blood sample can be obtained using methods known in the art, such as finger prick or phlebotomy. Suitably, the blood sample is approximately 0.1 to 20 ml, or alternatively approximately 1 to 15 ml with the volume of blood being approximately 10 ml. Smaller amounts may also be used, as well as circulating free DNA in blood. Microsampling and sampling by needle biopsy, catheter, excretion or production of bodily fluids containing DNA are also potential biological sample sources.

In the present invention, the subject is typically a human but also can be any species with methylation marks on its genome, including, but not limited to, a dog, cat, rabbit, cow, bird, rat, horse, pig, or monkey.

METHYLATION STATUS

While the present invention exemplifies use of WGBS for methylation analysis, in fact many other methods for performing nucleic acid sequencing or analyzing methylation status or chromatin status may be utilized including nucleic acid amplification, polymerase chain reaction (PCR), bisulfite pyrosequencing, nanopore sequencing, 454 sequencing, insertion tagged sequencing. In embodiments, the methodology of the disclosure utilizes systems such as those provided by Illumina, Inc, (HiSeq™ X10, HiSeq™ 1000, HiSeq™ 2000, HiSeq™ 2500, Genome Analyzers™, MiSeq™ systems), Applied Biosystems Life Technologies (ABI PRISM™ Sequence detection systems, SOLiD™ System, Ion PGM™ Sequencer, ion Proton™ Sequencer). Nucleic acid analysis can also be carried out by systems provided by Oxford Nanopore Technologies (GridiON™, MiniON™) or Pacific Biosciences (Pacbio™ RS II). Sequencing can also be carried out by standard Sanger dideoxy terminator sequencing methods and devices, or on other sequencing instruments, further as those described in, for example, United States patents and patent applications U.S. Pat. Nos. 5,888,737, 6,175,002, 5,695,934, 6,140,489, 5,863,722, 2007/007991, 2009/0247414, 2010/0111768 and PCT application WO2007/123744. Importantly, in embodiments, sequencing may be performed using any of the methods described herein with, or without, bisulfite conversion.

Chromatin can be analyzed using similar analytical methodology after ATAC sequencing and related methods. As illustrated in the Examples herein, analysis of methylation can be performed by bisulfite genomic sequencing. Bisulfite treatment modifies DNA converting unmethylated, but not methylated, cytosines to uracil. Bisulfite treatment can be carried out using the METHYLEASY™ bisulfite modification kit (Human Genetic Signatures).

In some embodiments, bisulfite pyrosequencing, which is a sequencing-based analysis of DNA methylation that quantitatively measures multiple, consecutive CpG sites individually with high accuracy and reproducibility may be used. This can be done by whole genome bisulfite sequencing or by MiSeq™ using primers for such analysis.

For bisulfite sequencing, 1% unmethylated Lambda DNA (Promega, cat # D1521) can be spiked-in to monitor bisulfite conversion efficiency. Genomic DNA was fragmented to an average size of 350bp using a Covaris S2 sonicator (Woburn, MA). Bisulfite sequencing libraries can be constructed using the Illumina TruSeq™ DNA Library Preparation kit protocol (primers included) or NEBNext™ Ultra (NEBNext™ Multiplex Oligos for Illumina module, New England BioLabs, cat # E7535L) according to the manufacturer's instructions. Both protocols use a Kapa HiFi Uracil+ PCR system (Kapa Biosystems, cat # KK2801).

For Illumina TruSeq™ DNA libraries, gel-based size selection can be performed to enrich for fragments in the 300-400bp range. For NEBNext™ libraries, size selection can be performed using modified AMPure XP™ bead ratios of 0.4x and 0.2x, aiming also for an insert size of 300-400bp. After size-selection, the samples can be bisulfite converted and purified using the EZ DNA™ Methylation Gold Kit (Zymo Research, cat # D5005). PCR-enriched products can be cleaned up using 0.9X AMPure XP™ beads (Beckman Coulter, cat # A63881).

Final libraries can be run on the 2100 Bioanalyzer™ (Agilent, Santa Clare, CA, USA) using the High-Sensitivity DNA assay for quality control purposes. Libraries can be quantified by qPCR using the Library Quantification Kit for Illumina sequencing platforms (cat # KK4824, KAPA Biosystems, Boston, USA), using 7900HT Real Time PCR System™ (Applied Biosystems) and sequenced on the Illumina HiSeq2000 (2x100bp read length, v3 chemistry according to the manufacturer's protocol with lOx PhiX spike-in) and HiSeq2500™ (2x125bp read length, v4 chemistry according to the manufacturer's protocol with lOx PhiX spike-in).

Altered methylation can be determined by identifying a detectable difference in methylation. For example, hypomethylation can be determined by identifying whether after bisulfite treatment a uracil or a cytosine is present a particular location. If uracil is present after bisulfite treatment, then the residue is unmethylated. Hypomethylation is present when there is a measurable decrease in methylation.

For WGBS, methylation calling can be performed using FASTQ files processed using Trim Galore! v0.3.6 (Babraham Institute) to perform single-pass adapter- and quality-trimming of reads, as well as running FastQC v0.11.2 for general quality check of sequencing data. Reads can then aligned be aligned to the hgl9/GRCh37 or other human or other species builds using Bismark v0.12.3 and Bowtie2 v2.1.0 or comparable and/or updated software. Separate mbias plots for read 1 and read 2 can be generated by running the Bismark methylation extractor using the "mbias_only" flag. These plots can be used to determine how many bases to remove from the 5' end of reads. BAM files can subsequently be processed with Samtools v0.1.19 for sorting, merging, duplicate removal and indexing, as well as for methylation base calling.

In an alternative embodiment, the method for analyzing methylation status can include amplification after oligonucleotide capture, MiSeq™ sequencing, or MinION™ long read sequencing without bisulfite conversion.

DIAGNOSTICS

The methods described herein may be used in a variety of ways to predict, diagnose and/or monitor diseases, such as cancer. Further, the methods may be utilized to distinguish various cell types from one another as well as determine cellular age. These aspects may be accomplished by performing the respective epigenetic analysis method for a test genome and comparing the obtained epigenetic measure to a corresponding known measure for a reference genome; i.e., a measure for a known cell type or disease.

COMPUTER SYSTEMS

The present invention is described partly in terms of functional components and various processing steps. Such functional components and processing steps may be realized by any number of components, operations and techniques configured to perform the specified functions and achieve the various results. For example, the present invention may employ various biological samples, biomarkers, elements, materials, computers, data sources, storage systems and media, information gathering techniques and processes, data processing criteria, statistical analyses, regression analyses and the like, which may carry out a variety of functions. In addition, although the invention is described in the medical diagnosis context, the present invention may be practiced in conjunction with any number of applications, environments and data analyses; the systems described herein are merely exemplary applications for the invention.

Methods for epigenetic analysis according to various aspects of the present invention are implemented using a computer program operating on a computer system. An exemplary epigenetic analysis system, according to various aspects of the present invention, may be implemented in conjunction with a computer system, for example a conventional computer system comprising a processor and a random access memory, such as a remotely-accessible application server, network server, personal computer or workstation. The computer system also suitably includes additional memory devices or information storage systems, such as a mass storage system and a user interface, for example a conventional monitor, keyboard and tracking device. The computer system may, however, comprise any suitable computer system and associated equipment and may be configured in any suitable manner. In one embodiment, the computer system comprises a stand-alone system. In another embodiment, the computer system is part of a network of computers including a server and a database.

The software required for receiving, processing, and analyzing biomarker information may be implemented in a single device or implemented in a plurality of devices. The software may be accessible via a network such that storage and processing of information takes place remotely with respect to users. The epigenetic analysis system according to various aspects of the present invention and its various elements provide functions and operations to facilitate biomarker analysis, such as data gathering, processing, analysis, reporting and/or diagnosis. The present epigenetic analysis system maintains information relating to methylation and samples and facilitates analysis and/or diagnosis, For example, in the present embodiment, the computer system executes the computer program, which may receive, store, search, analyze, and report information relating to the epigenome. The computer program may comprise multiple modules performing various functions or operations, such as a processing module for processing raw data and generating supplemental data and an analysis module for analyzing raw data and supplemental data to generate a disease status model and/or diagnosis information.

The procedures performed by the epigenetic analysis system may comprise any suitable processes to facilitate epigenetic analysis and/or disease diagnosis. In one embodiment, the epigenetic analysis system is configured to establish a disease status model and/or determine disease status in a patient. Determining or identifying disease status may comprise generating any useful information regarding the condition of the patient relative to the disease, such as performing a diagnosis, providing information helpful to a diagnosis, assessing the stage or progress of a disease, identifying a condition that may indicate a susceptibility to the disease, identify whether further tests may be recommended, predicting and/or assessing the efficacy of one or more treatment programs, or otherwise assessing the disease status, likelihood of disease, or other health aspect of the patient.

The epigenetic analysis system may also provide various additional modules and/or individual functions. For example, the epigenetic analysis system may also include a reporting function, for example to provide information relating to the processing and analysis functions. The epigenetic analysis system may also provide various administrative and management functions, such as controlling access and performing other administrative functions.

The epigenetic analysis system suitably generates a disease status model and/or provides a diagnosis for a patient based on raw biomarker data and/or additional subject data relating to the subjects. The epigenetic data may be acquired from any suitable biological samples.

The following example is provided to further illustrate the advantages and features of the present invention, but it is not intended to limit the scope of the invention. While this example is typical of those that might be used, other procedures, methodologies, or techniques known to those skilled in the art may alternatively be used.

## EXAMPLE

### *EPIGENOME ANALYSIS USING POTENTIAL ENERGY LANDSCAPES TO REVEAL THE INFORMATION-THEORETIC NATURE OF THE EPIGENOME*

In this example, using well-grounded biological assumptions and principles of statistical physics and information theory, potential energy landscapes are derived from whole genome bisulfite sequencing data that allow quantification of genome-wide methylation stochasticity and epigenetic differences using Shannon's entropy and the Jensen-Shannon distance. This example details the discovery of a "developmental wheel" of germ cell lineages and the identification of developmentally critical genes characterized by low differential mean methylation but high epigenetic differences, a relationship between bistability in methylation level and imprinting, the relationship between entropy and information-theoretic properties of methylation channels and chromatin structure, and the importance of quantifying environmental influences on epigenetic stochasticity using entropic sensitivity analysis. The example illustrates the main capabilities of the invention, which can be used to achieve a fundamental understanding of the information-theoretic nature of the epigenome by provided a powerful computational methodology and a computing system for the analysis and classification of epigenetic information in health and disease.

EXPERIMENTAL MATERIALS AND METHODS

*Whole Genome Bisulfite Sequencing Samples*

Previously published WGBS data corresponding to 10 genomic samples are used, which include HI human embryonic stem cells, normal and matched cancer cells from colon normal and cancer, cells from liver, keratinocytes from skin biopsies of sun protected sites from younger and older individuals, and EBV-immortalized lymphoblasts (Supplementary Table 1 below). Additional WGBS data corresponding to 25 genomic samples were also generated that include normal and matched cancer cells from liver and lung, pre-frontal cortex, cultured HNF fibroblasts at 5 passage numbers, and sorted $CD4^+$ T-cells from younger and older individuals, all with IRB approval (Supplementary Table 1 below). Pre-frontal cortex samples were obtained from the University of Maryland Brain and Tissue Bank, which is a Brain and Tissue Repository of the NIH NeuroBioBank. Peripheral blood mononuclear cells (PBMCs) were isolated from peripheral blood collected from healthy subjects and separated by using a Ficoll density gradient separation method (Sigma-Aldrich). $CD4^+$ T-cells were subsequently isolated from PBMCs by positive selection with MACS magnetic bead technology (Miltenyi). Post-separation flow cytometry assessed the purity of $CD4^+$ T-cells to be at 97%. Primary neonatal dermal fibroblasts were acquired from Lonza and cultured in Gibco's DMEM supplemented with 15% FBS (Gemini BioProducts).

*DNA Isolation*

Genomic DNA was extracted from samples using the Masterpure™ DNA Purification Kit (Epicentre). High molecular weight of the extracted DNA was verified by running a 1% agarose gel and by assessing the 260/280 and 260/230 ratios of samples on Nanodrop. Concentration was quantified using Qubit 2.0 Fluorometer™ (Invitrogen).

*Generation of WGBS Libraries*

For every sample, 1% unmethylated Lambda DNA (Promega, cat # D1521) was spiked-in to monitor bisulfite conversion efficiency. Genomic DNA was fragmented to an average size of 350 base pairs using a Covaris S2™ sonicator (Woburn, MA). Bisulfite sequencing libraries were constructed using the Illumina TruSeq™ DNA Library Preparation kit protocol (primers included) or NEBNext Ultra™ (NEBNext Multiplex Oligos for Illumina module, New England BioLabs, cat # E7535L) according to the manufacturer's instructions. Both protocols use a Kapa HiFi Uracil+ PCR system (Kapa Biosystems, cat # KK2801).

For Illumina TruSeq™ DNA libraries, gel-based size selection was performed to enrich for fragments in the 300-400 base pair range. For NEBNext™ libraries, size selection was performed using modified AMPure XP™ bead ratios of $0.4\times$ and $0.2\times$, aiming also for an insert size of 300-400 base pairs. After size-selection, the samples were bisulfite converted and purified using the EZ DNA™ Methylation Gold Kit (Zymo Research, cat # D5005). PCR-enriched products were cleaned up using $0.9\times$AMPure XP™ beads (Beckman Coulter, cat # A63881).

Final libraries were run on the 2100 Bioanalyzer™ (Agilent, Santa Clare, CA, USA) using the High-Sensitivity DNA assay for quality control purposes. Libraries were then quantified by qPCR using the Library Quantification Kit™ for Illumina sequencing platforms (cat # KK4824, KAPA Biosystems, Boston, USA), using 7900HT Real Time PCR System™ (Applied Biosystems) and sequenced on the Illumina HiSeq2000™ (2× 100 base pair read length, v3 chemistry according to the manufacturer's protocol with 10×PhiX spike-in) and HiSeq2500™ (2×125 base pair read length, v4 chemistry according to the manufacturer's protocol with 10×PhiX spike-in).

*Quality Control and Alignment*

FASTQ files were processed using Trim Galore! ™ v0.3.6 (Babraham Institute) to perform single-pass adapter- and quality-trimming of reads, as well as running FastQC™ v0.11.2 for general quality check of sequencing data. Reads were then aligned to the hgl9/GRCh37 genome using Bismark™ v0.12.3 and Bowtie2™ v2.1.0. Separate mbias plots for read 1 and read 2 were generated by running the Bismark methylation extractor using the "mbias_only" flag. These plots were used to determine how many bases to remove from the 5' end of reads. The number was generally higher for read 2, which is known to have poorer quality. The amount of 5' trimming ranged from 4 to 25 base pairs, with most common values being around 10 base pairs. BAM files were subsequently processed with Samtools™ v0.1.19 for sorting, merging, duplicate removal, and indexing.

FASTQ files associated with the EBV sample were processed using the same pipeline described for the in-house samples. BAM files associated with some colon and liver normal samples, obtained from [Ziller, M. J. et al. Nature 500, 477-481 (2013)], could not be assessed using the Bismark™ methylation extractor due to incompatibility of the original alignment tool (MAQ) used on these samples. Therefore, the advice of Ziller et al. was followed and 4 base pairs were trimmed from all reads in those files.

*Genomic Features and Annotations*

Files and tracks bear genomic coordinates for hgl9. CpG islands (CGIs) were obtained from [Wu, H. et al. Biostatistics 11, 499-514 (2010)]. CGI shores were defined as sequences flanking 2000 base pairs on either side of islands, shelves as sequences flanking 2000 base pairs on either side of shores, and open seas as everything else. The R Bioconductor™ package "TxDb.Hsapiens.UCSC.hgl9.knownGene" was used for defining exons, introns and transcription start sites (TSSs). Promoter regions were defined as sequences flanking 2000 base pairs on either side of TSSs. A curated list of enhancers was obtained from the VISTA™ Enhancer Browser (http://enhancer.lbl.gov) by downloading all human (hg19) positive enhancers that show reproducible expression in at least three independent transgenic embryos. Hypomethylated blocks (colon and lung cancer) were obtained from [Timp, W. et al. Genome Med. 6, 61 (2014)]. HI stem cell LOCKs and Human Pulmonary Fibroblast (HPF) LOCKs were obtained from [Wen, B. et al. BMC Genomics 13, 566 (2012)]. LAD tracks associated with Tig3 cells derived from embryonic lung fibroblasts were obtained from [Guelen, L. et al. Nature 453, 948-951 (2008)]. Gene bodies were obtained from the UCSC genome browser. HI and IMR90 TAD boundaries were obtained from http://chromosome.sdsc.edu/mouse/hi-c/download.html. BED files for Hi-C data processed into compartments A and B were provided by Fortin and Hansen (https://github.com/Jfortin1/HiC_AB_Compartments). CTCF and EZH2/SUZ12 binding data were obtained from the UCSC Genome Browser [Transcription Factor ChIP-seq track (161 factors) from ENCODE].

*Data Access*

Raw files have been deposited to NCBI's Sequencing Read Archive (SRA) under Accessions SRP072078, SRP072071, SRP072075, and SRP072141.

RESULTS

*Stochastic Epigenetic Variation and Potential Energy Landscapes*

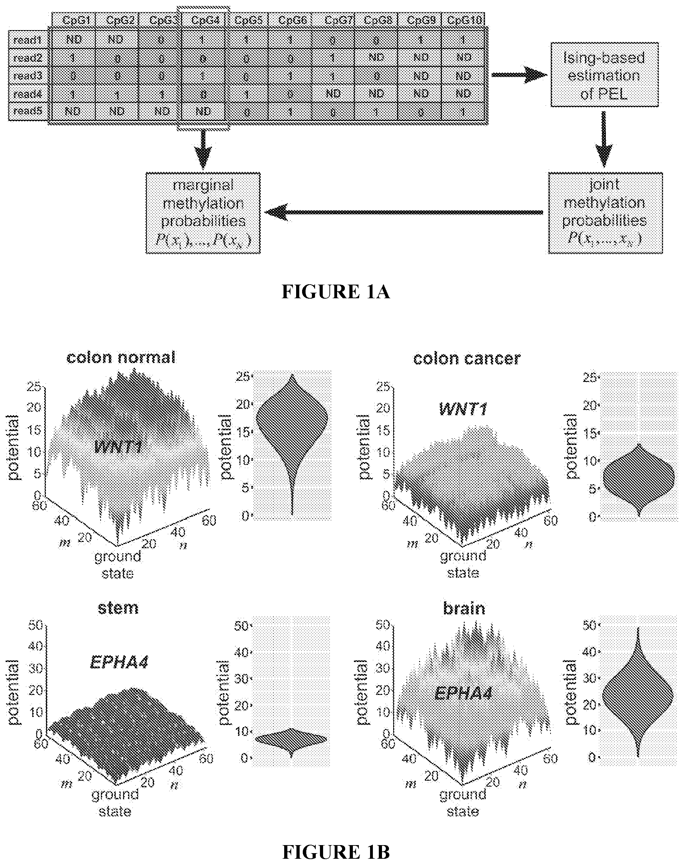

The methylation PEL $V_X(x)$ was estimated from WGBS data corresponding to 35 genomic samples, including stem cells, normal cells from colon, liver, lung, and brain tissues, *matched* cancers from three of these tissues, cultured fibroblasts at 5 passage numbers, CD4$^+$ lymphocytes and skin keratinocytes from younger and older individuals, and EBV-immortalized lymphoblasts (Supplementary Table 1 below). To this end, the genome was partitioned into consecutive non-overlapping genomic regions of 3000 base pairs in length each, and the maximum-likelihood estimation method introduced earlier was used to estimate the PEL parameters within each genomic region. The strategy capitalizes on appropriately combining the full information available in multiple methylation reads, especially the correlation between methylation at CpG sites, as opposed to the customary approach of estimating marginal probabilities at each individual CpG site (Fig. 1A).

Due to its dependence on a small number of parameters, one can estimate the joint probability distribution of methylation from low coverage WGBS data (as low as $7\times$ in the data used in this example). In turn, this allows reliable calculation of marginal probabilities at individual CpG sites, computation of PELs, evaluation of correlations, and computation of a number of new methylation measures that have not been considered before.

Since the size of the methylation state-space within a genomic region with $N$ CpG sites grows geometrically ($2^N$) in terms of $N$, visualization of the PEL is chosen to be performed within a region of a CpG island (CGI) near the promoter of a gene containing 12 CpG sites. To plot a PEL, the $2^{12}$ computed values are distributed over a $64\times64$ square grid using a two-dimensional version of Gray's code, so that methylation states located adjacent to each other in the east/west and north/south directions differ in only one bit.

Computed PELs demonstrate that most methylation states associated with the CGI of *WNT1*, an important signaling gene, in colon normal exhibit high potential (Fig. 1B, three-dimensional and violin plots), implying that significant energy is required to leave the fully unmethylated state, which is the state of lowest potential (ground state). Any deviation from this state will rapidly be "funneled" back, leading to low uncertainty in methylation. Notably, the methylation states of *WNT1* in colon cancer demonstrate low potential (Fig. 1B, three-dimensional and violin plots), implying that relatively little energy is required to leave the fully unmethylated ground state. In this case, deviations from this state will be frequent and long lasting, leading to uncertainty in methylation.

Similarly, the methylation states associated with the CGI of *EPHA4*, a key developmental gene, exhibit low potential in stem cells (Fig. 1B, three-dimensional and violin plots), suggesting that low energy is needed to leave the fully unmethylated ground state, thus leading to uncertainty in methylation. In contrast, *EPHA4* shows high potential in the brain (Fig. 1B, three-dimensional and violin plots), implying that appreciable energy is required to leave the fully unmethylated ground state, thus leading to low uncertainty in methylation.

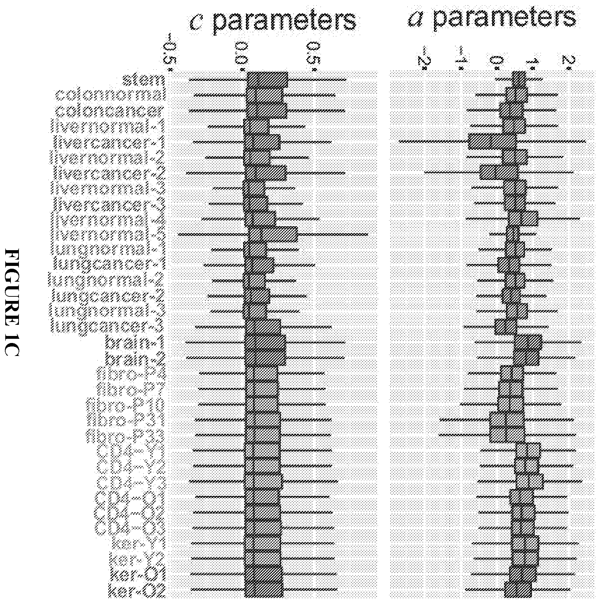

Global distributions of the PEL parameters $a_n$ and $c_n$ (Fig. 1C) show that the motivation for using the Ising model is well founded. Specifically, more than 75% of the $c_n$ parameters along the genome are positive, showing extensive cooperativity in methylation (Fig. 1C). Interestingly, a global increase in the values of the $c_n$ parameters is consistently observed in cancer, implying an overall increase in methylation cooperativity in tumors. In addition, most genomic samples demonstrate positive median $a_n$ values, indicating that methylation is more common than non-methylation, except in two liver cancer samples that were subject to extended extreme hypomethylation. Even in those cases, however, $c_n$ is increased in the tumors.

*Epigenetic Entropy Quantifies Methylation Uncertainty in Biological States*

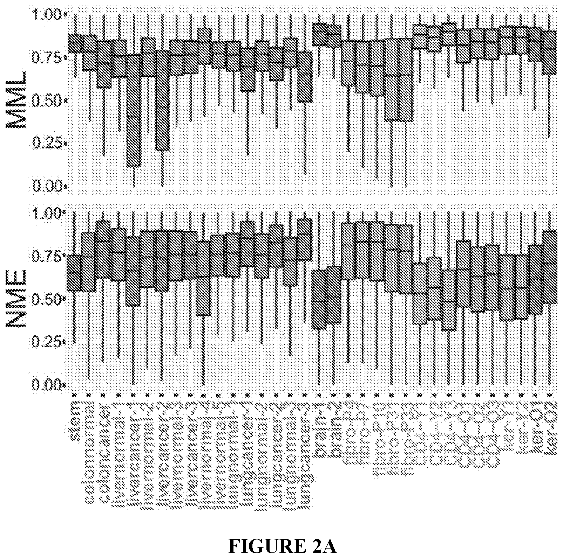

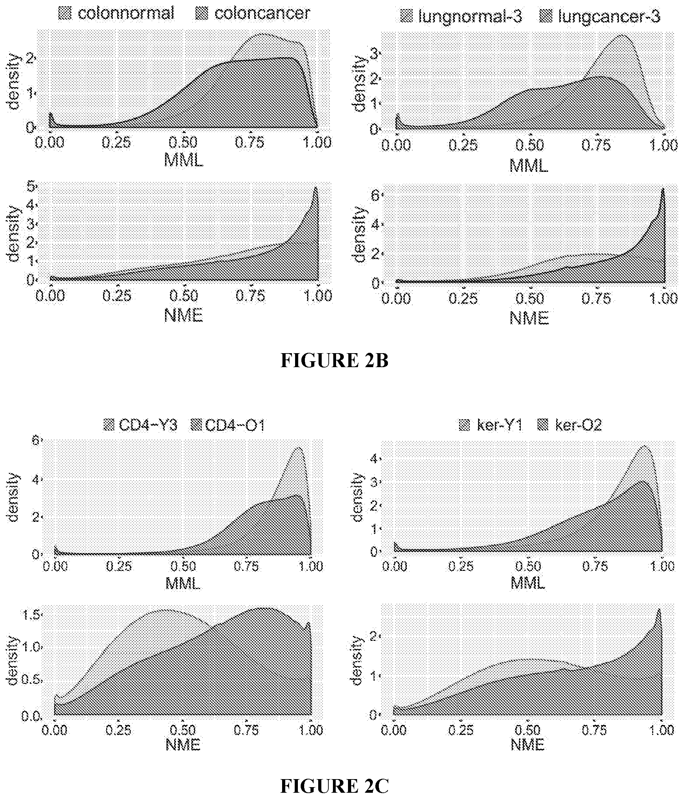

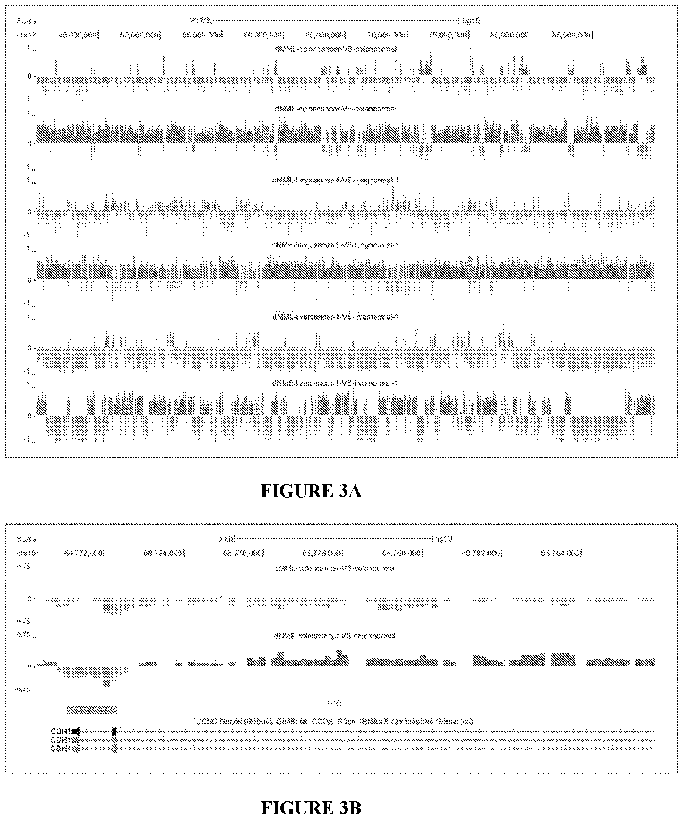

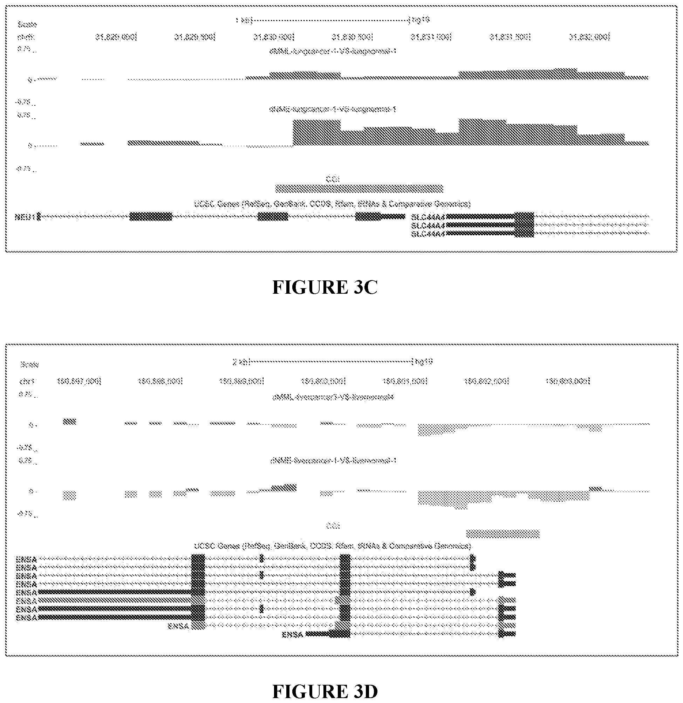

The NME is an effective measure of methylation uncertainty that can be reliably computed genome-wide from low coverage WGBS data using the Ising model, together with the mean methylation level (MML), which is the average of the methylation means at individual CpG sites within a genomic subregion. The genome-wide distributions of MML and NME values were calculated and compared among genomic samples. Consistent with previous reports, the MML in stem cells and brain tissues was globally higher than in normal colon, liver, and lung and that the same was true for CD4[+] lymphocytes and skin keratinocytes (Fig. 2A). Moreover, the MML was reduced in all seven cancers studied compared to their matched normal tissue (Fig. 2A,B), and was also progressively lost in cultured fibroblasts (Fig. 2A). Low NME was also observed in stem and brain cells, as well as in CD4[+] lymphocytes and skin keratinocytes associated with young subjects, and a global increase of NME in most cancers except for liver cancer, which exhibited profound hypomethylation leading to a less entropic methylation state (Figs. 2 & 3). While changes of NME in cancer were often associated with changes in MML (Fig. 3A), this was often not the case (Fig. 3B,C,D), indicating that changes in stochasticity are not necessarily related to changes in mean methylation, and demanding that both be assessed when interrogating biological samples.

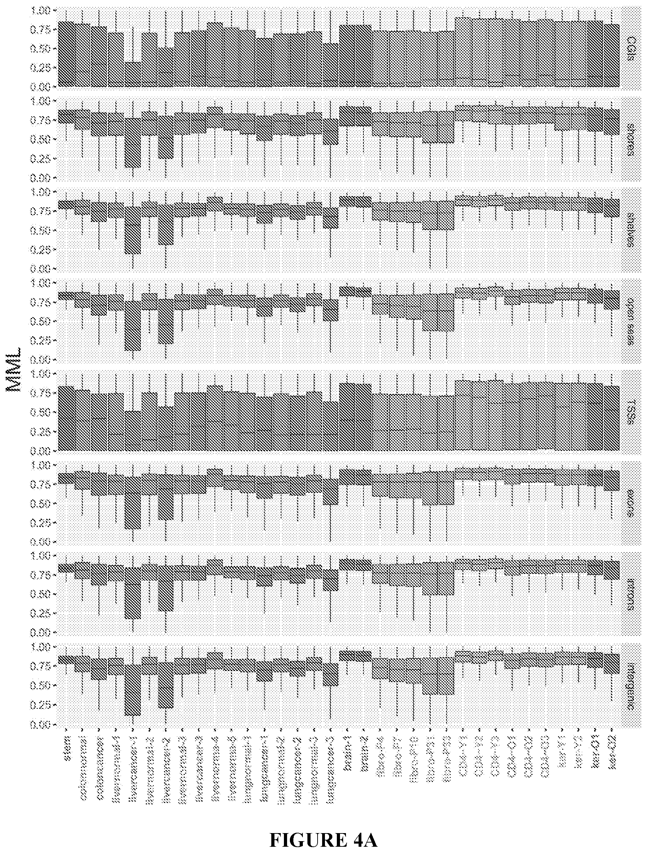

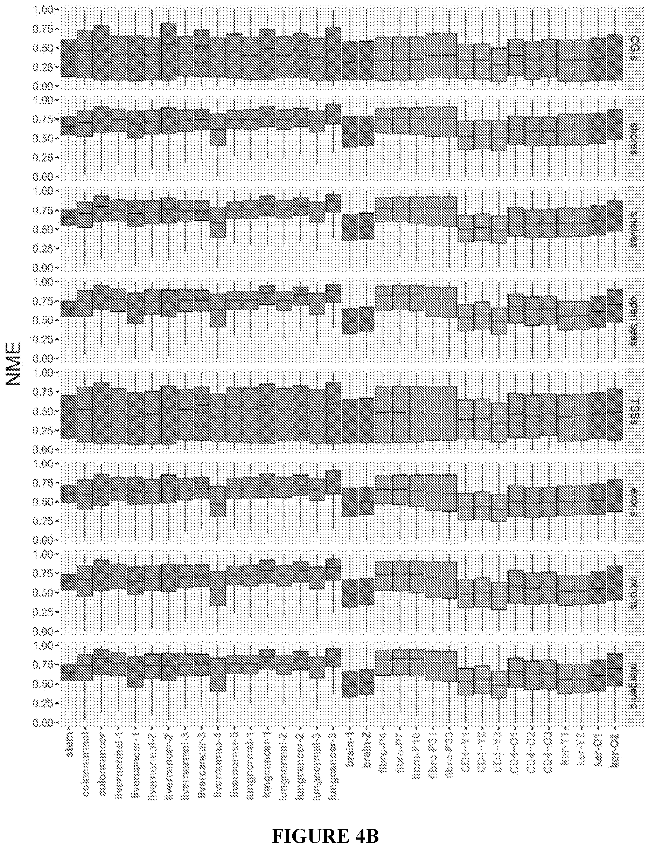

MML and NME distributions were also computed over selected genomic features and provided a genome-wide breakdown showing lower and more variable methylation levels and entropy values within CGIs and TSSs compared to other genomic features, such as shores, exons, introns and the like (Fig. 4A,B).

Global hypomethylation and gain in entropy was found in all three CD4[+] lymphocyte samples from older people compared to three from younger individuals, as well as in both skin keratinocyte samples compared to younger samples (Fig. 2A,C), with the percentage change in entropy being more pronounced. For example, an average 23% increase (11%-38% range) in median NME genome-wide was found between young and old CD4 samples but only an average 5.6% decrease (3.2%-8.5% range) in median MML

To account for biological and statistical variability, using the three young CD4 samples, the absolute NME differences (dNMEs) was first computed at each genomic subregion associated with all three pairwise comparisons and, by pooling these values, an empirical null distribution was constructed that accounted for biological and statistical variability of differential entropy in the young samples. Subsequently, he absolute dNME values corresponding to a young-old pair (CD4-Y3, CD4-O1) were computed and multiple hypotheses testing was performed to reject the null hypothesis that the observed NME difference is due to biological or statistical variability. By using the "qvalue" package of Bioconductor™ with default parameters, false discovery rate (FDR) analysis was performed and the probability that the null hypothesis is rejected at a randomly chosen genomic subregion was estimated. This resulted in approximately computing the fraction of genomic subregions found to be differentially entropic for reasons other than biological or statistical variability among the young samples.

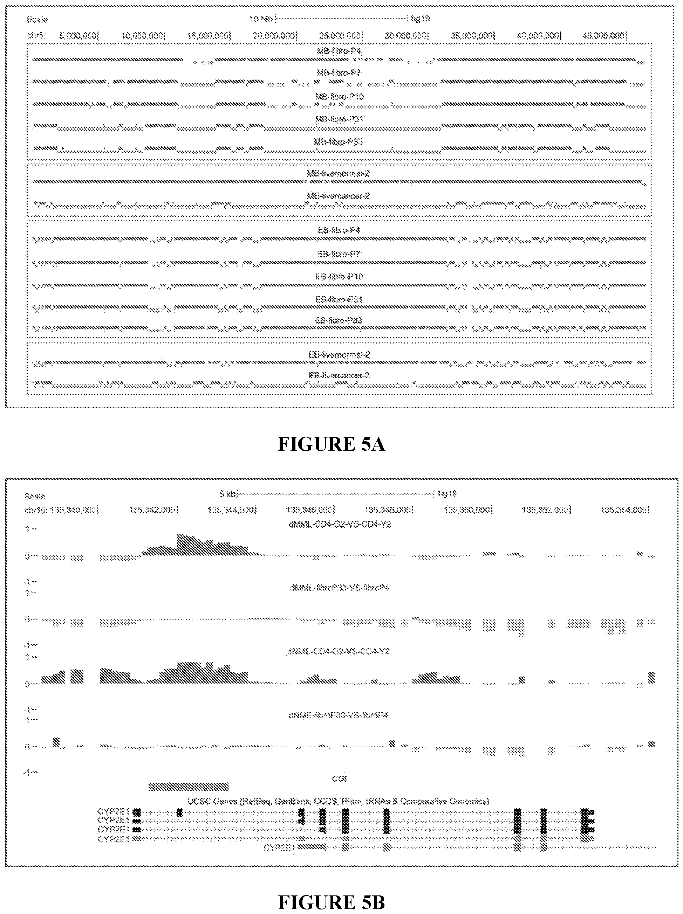

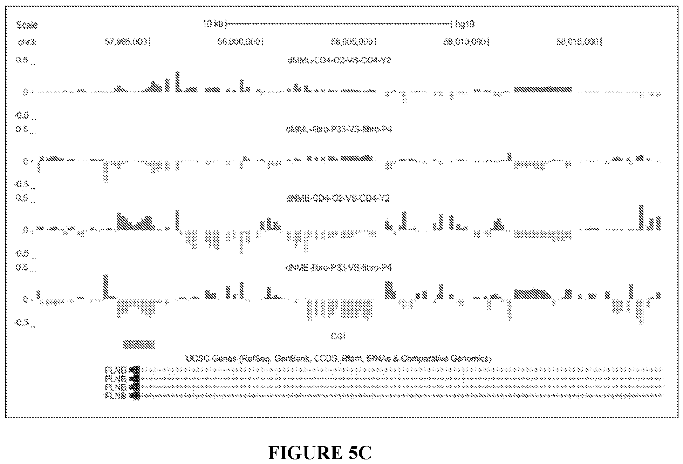

It was statistically estimated that up to 34% of the genomic subregions were differentially entropic, demonstrating that profound changes in entropy can result in old individuals. Notably, striking differences were observed between true aging and cultured fibroblasts. Although passage number in fibroblasts was also associated with progressive global hypomethylation, the entropy distribution was relatively stable (Figs. 2A & 5A). For example, the promoters of *CYP2E1* and *FLNB,* two genes which are known to be downregulated with age, exhibited noticeable gain in methylation level and entropy in old CD4$^+$ lymphocytes. This was in stark contrast to the lack of changes with passage in *CYP2E1* and the noticeable loss of entropy in *FLNB* (Fig. 5B,C) in cultured fibroblasts. Therefore, age-related PELs in multiple tissues are not well characterized by increasing fibroblast passage number, and aging appears to be associated with a gain in entropy.

*Informational Distances Delineate Lineages and Identify Developmentally Critical Genes*

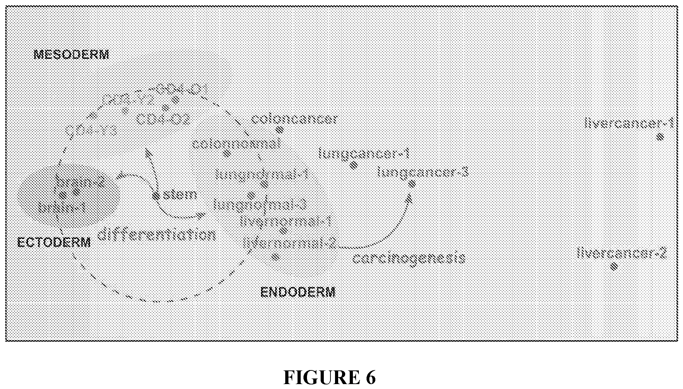

To understand the relationship between epigenetic information and phenotypic variation, it was sought to precisely quantify epigenetic discordance between pairs of genomic samples using the Jensen-Shannon distance (JSD). It was then asked if this distance could be used to distinguish colon, lung, and liver from each other and from matched cancers, as well as from stem, brain, and CD4$^+$ lymphocytes. For computational feasibility, the study was limited to 17 representative cell and tissue samples and computed all 136 pairwise epigenetic distances genome-wide. The results were visualized by performing multidimensional scaling. The samples fell into clear categories based on developmental germ layers (Fig. 6), with clusters of ectoderm (brain), mesoderm (CD4), and endoderm (normal colon, lung, and liver) derived tissues located roughly equidistant from stem cells. On the other hand, cancerous tissues were far removed from their normal matched tissues as well as from the stem cells (Fig. 6).

Given the interesting relationship between the stem cell sample and the three germ layers, genes that exhibited appreciable differential methylation level (dMML) and/or JSD in stem cells compared to differential tissues were examined. To this end, genes were ranked based on the absolute value of the dMML as well as on the JSD within their promoters (Supplementary Data 1 described below and attached) and it was surprising to find that many genes known to be involved in development and differentiation showed relatively small changes in dMML yet very high JSD, indicating that the probability distributions of methylation level within their promoters were appreciably different, despite little difference in mean methylation level.

To explore this further, it was investigated whether non-mean related methylation differences could identify genes between sample groups that would have been previously occult to mean-based analyses by employing a relative JSD-based ranking scheme (RJSD) that assigned a higher score to genes with higher JSD but smaller dMML. Many key genes were found at the top of the RJSD list, such as *IGF2BP1, FOXD3, NKX6-2, SALL1, EPHA4,* and *OTX1,* with RJSD-based GO annotation ranking analysis revealing key categories associated with stem cell maintenance and brain cell development (Supplementary Data 1 & 2 described below and attached). Notably, similar results were obtained when stem cells were compared to normal lung, with RJSD-based GO annotation analysis revealing key developmental categories and genes in both mesodermal and stem cell categories (Supplementary Data 1 & 2 described below and attached). Comparing stem cells to CD4$^+$ lymphocytes, showed enrichment for immune-related functions driven by dMML and many developmental and morphogenesis categories driven by RJSD (Supplementary Data 2 described below and attached). In contrast, when differentiated tissues were compared, it was noticed that dMML-based GO annotation analysis resulted in a higher number of significant categories than RJSD-based analysis, and these were closely related to differentiated functions, such as immune regulation and neuronal signaling in the case of brain and CD4 (Supplementary Data 2 described below and attached). Interestingly, when lung normal was compared to cancer, it was noticed that RJSD-based GO annotation analysis produced a higher number of significant categories than dMML-based analysis, and these were again related to developmental morphogenesis categories.

These previous results show that PEL computation can reveal major changes in the probability distributions of DNA methylation associated with developmentally critical genes, and that the shape of these distributions, rather than their means per se, may often be closely related to pluripotency and fate lineage determination in development and cancer.

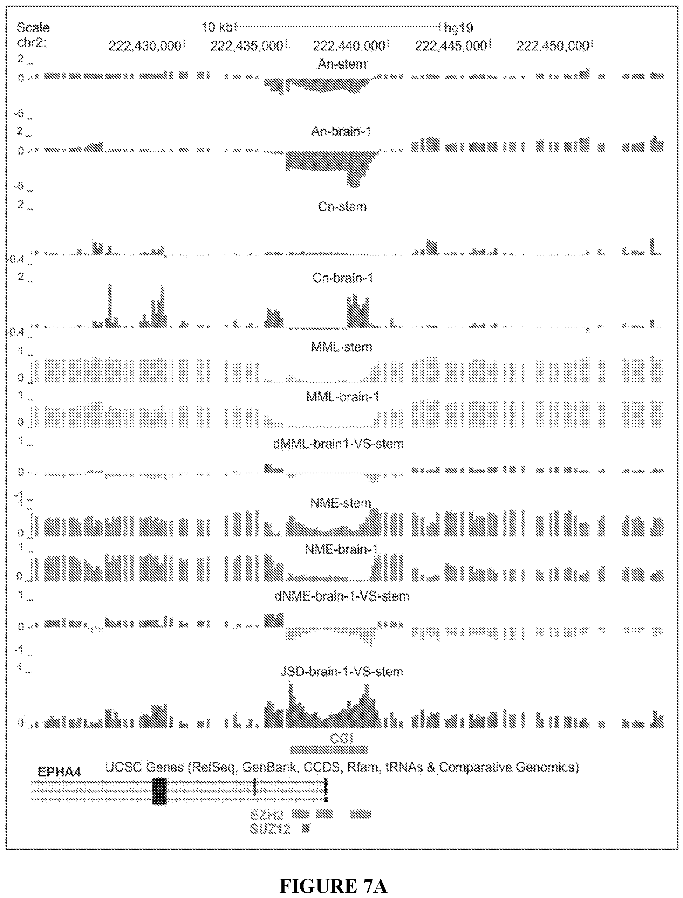

Next the link between changes in the probability state, as reflected by the JSD and the values of the PEL parameters $a_n$ and $c_n$, was explored. For example, a CGI near the promoter of *EPH4A* showed high JSD when comparing stem cells with brain (Fig. 7A). Although this region exhibited comparable mean methylation levels, it displayed high JSD over the entire CGI and especially over its shores. Notably, the JSD is not driven by methylation propensity, since the PEL parameters $a_n$ are strongly negative in both stem and brain, in which case the fully unmethylated state is the PEL's ground state (Fig. 1B, lower panel), resulting in low methylation level within the CGI. However, it is driven by methylation cooperativity at the CGI shores in brain, since the PEL parameters $c_n$ are strongly positive, compared to low methylation cooperativity in stem (almost zero $c_n$'s) that flattens the PEL (Fig. 1B, lower panel) and results in higher entropy than in brain (Fig. 7A). Intriguingly, the region shows binding of EZH2 and SUZ12, functional enzymatic components of the polycomb repressive complex 2 (PRC2), which regulates heterochromatin formation.

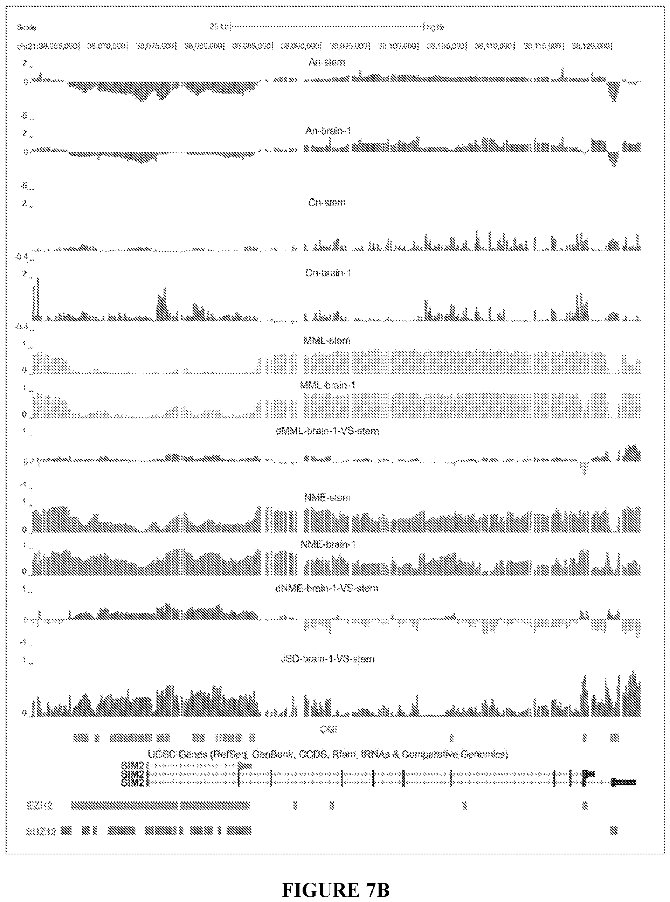

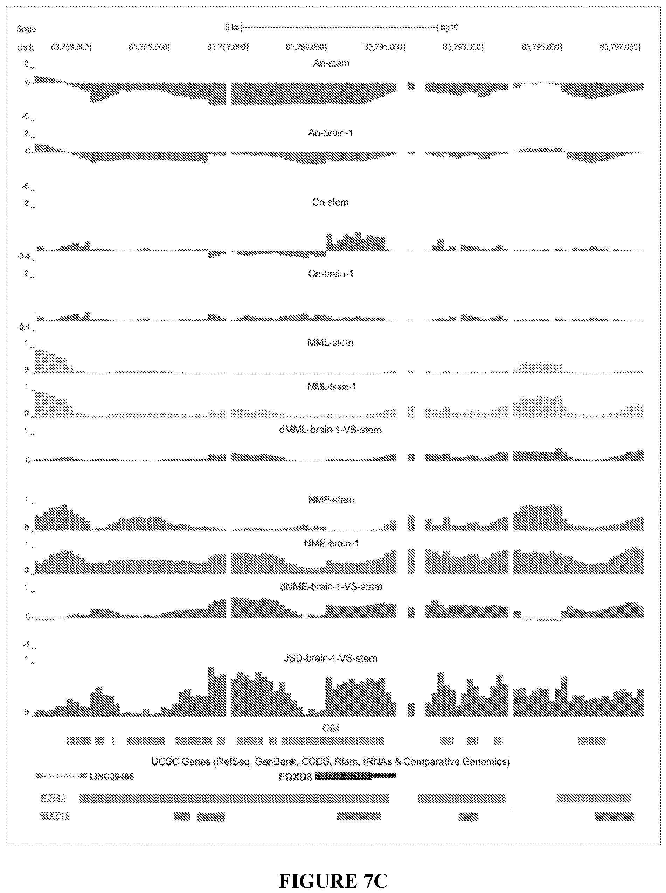

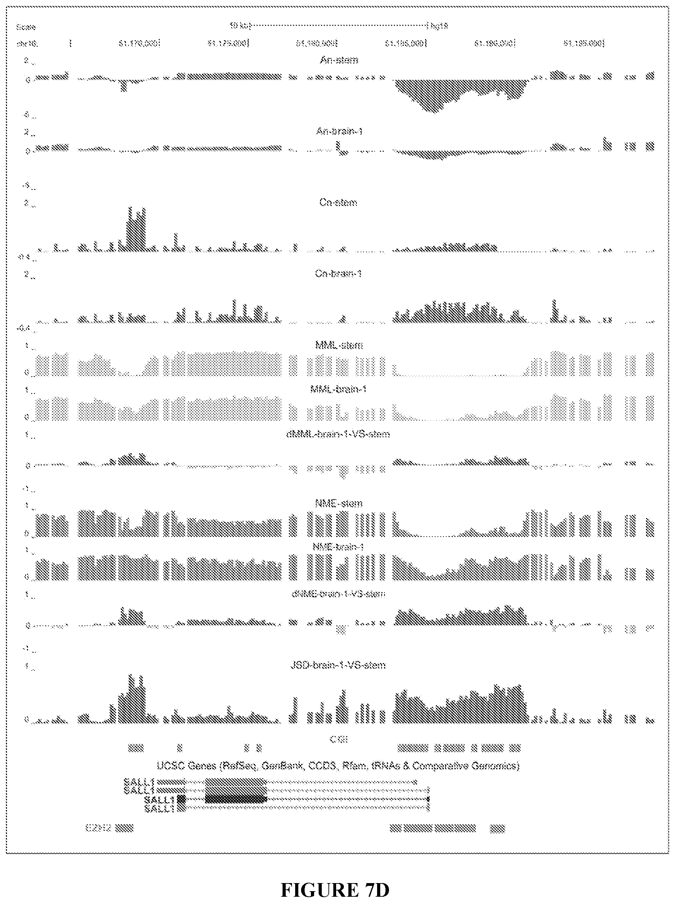

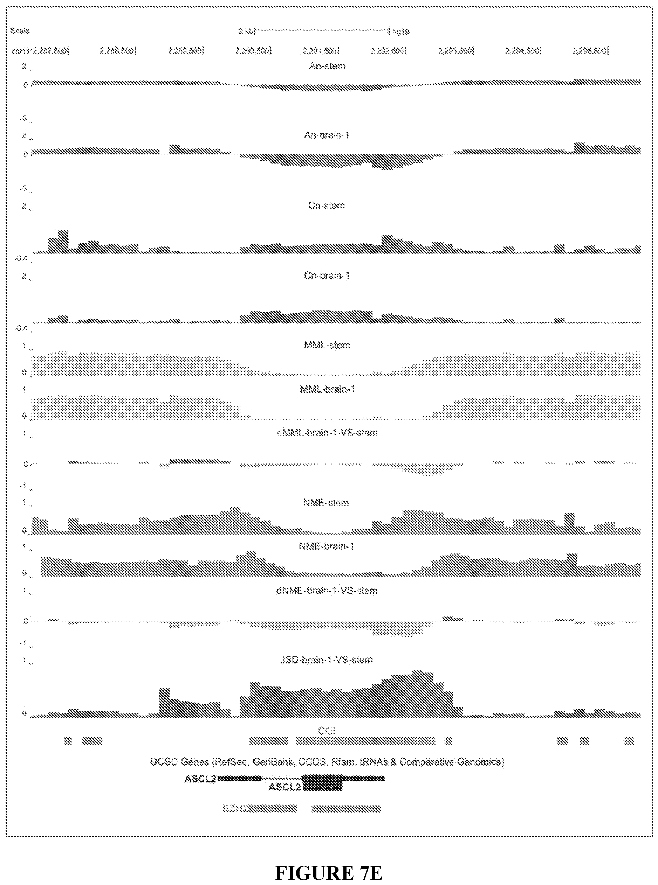

Likewise, *SIM2*, a master regulator of neurogenesis, is associated with high JSD regions with similar EZH2/SUZ12 binding, which span several CGIs located near its promoter (Fig. 7B). In this case, a gain of entropy is observed in brain, corresponding to a simultaneous loss in methylation propensity (through reduced $a_n$'s) and a gain in methylation cooperativity (through increased $c_n$'s). Similar remarks hold for other developmental genes, such as *ASCL2, SALL1,* and *FOXD3* (Fig. 7C,D,E).

The presence of EZH2 and SUZ12 binding sites was repeatedly observed in areas of high JSD, suggesting that they may play a critical role in generating increased entropy with minimal change in mean methylation. To determine whether this association was significant, the Fisher's exact test was used and promoters and enhancers with high dMML were compared to those with low dMML as well as promoters and enhancers with high JSD to those with low JSD. Several-fold greater enrichments for both EZH2 and SUZ12 binding sites at promoters and enhancers with high JSD vs. low JSD were observed, which provided further evidence of JSD's importance (Supplementary Table 2 below). Binomial logistic regression of EZH2/SUZ12 binding data on JSD scores at promoters and enhancers was then performed and significant positive association (EZH2: score = 5.6 for promoters & 18.1 for enhancers, *p*-value < $2.2 \times 10^{-16}$; SUZ12: score = 6.2 for promoters & 23 for enhancers, *p*-value < $2.2 \times 10^{-16}$; see Supplementary Table 2 below) was found.

The previous results show a significant association of EZH2 and SUZ12 with promoters and enhancers at high JSD regions of the genome, suggesting the intriguing possibility that the PRC2 complex controls stochastic variability in DNA methylation at selected genomic loci by regulating the methylation PEL.

*Methylation PELs Uncover Bistable Behavior Associated to Imprinting*

To investigate whether bistability in methylation might be associated with important biological functions, its possible enrichment was examined in several genomic features.

To identify bistable genomic subregions in a given WGBS sample, bimodality was detected in the probability distribution $P_L(l)$ of the methylation level within a genomic subregion. To evaluate enrichment of bistability in a particular genomic feature, two binary (0-1) random variables $R$ and $B$ were defined for each genomic subregion, such that $R = 1$, if the genomic subregion overlaps the genomic feature, and $B = 1$, if the genomic subregion is bistable. It was then tested against the null hypothesis that $R$ and $B$ are statistically independent by applying the $\chi^2$-test on the $2 \times 2$ contingency table for $R$ and $B$ and calculated the odds ratio (OR) as a measure of enrichment.

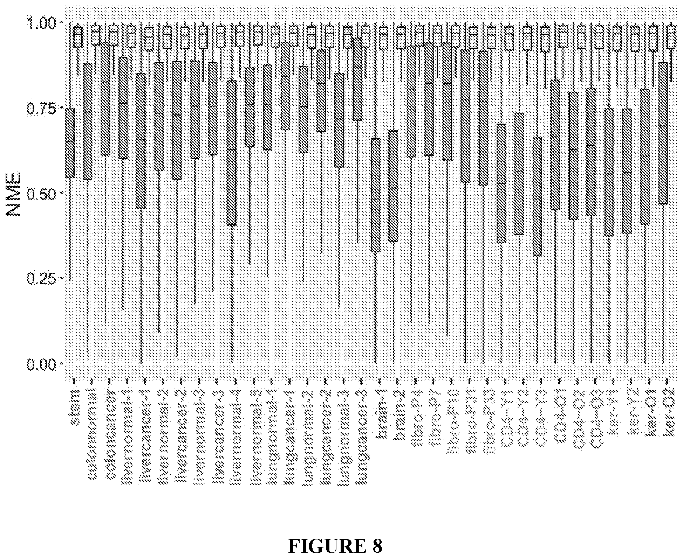

Bistability enrichment was evaluated within CGIs, shores, promoters, and gene bodies. It was found (Supplementary Table 3 below) that bistable genomic subregions were in general enriched in CpG island shores (ORs > 1 in 29/34 phenotypes, *p*-values < $2.2 \times 10^{-16}$) and promoters (ORs > 1 in 26/34 phenotypes, *p*-values $\leq 1.68 \times 10^{-9}$), but depleted in CGIs (ORs < 1 in 26/34 phenotypes, *p*-values < $2.2 \times 10^{-16}$) and gene bodies (ORs < 1 in 29/34 phenotypes, *p*-values $\leq 3.06 \times 10^{-14}$). Moreover, it was noticed that bistable genomic subregions were associated with appreciably higher NME than the rest of the genome [Fig. 8; comparing the bistable regions (yellow) to the rest of the genome (purple)].

To investigate whether methylation bistability is associated with specific genes, each gene was rank-ordered in the genome using a bistability score, which was calculated as the average frequency of methylation bistability within the gene's promoter in 17 normal genomic samples. Surprisingly, a substantial number of genes that have been known to be imprinted were highly ranked (Supplementary Data 3 described below and attached), which was attributed to the fact that full methylation on one chromosome and complete unmethylation on the other would give rise to bistable methylation. In fact, 82 curated imprinted genes from the Catalogue of Parent of Origin Effect (CPOE) were much more highly ranked in the list than would be expected by chance (*p*-value $2.89 \times 10^{-16}$), with notable overrepresentation of imprinted genes near the top of the list. Interestingly, more than 8% of imprinted genes in CPOE appeared in the top 25 bistable genes *(SNRPN, SNURF, MEST, MESTIT1, ZIM2, PEG3, MIMT1)*, raising the possibility that imprinting of these genes may be associated with allele-specific methylation of selective loci near their promoters.

The possibility that genes subject to monoallelic expression (MAE) are associated with bistability was also investigated. By using a recently created data set of 4227 MAE genes, only a slight enrichment of bistability in these genes was detected, likely because MAE is not a result of silenced expression from one of the two alleles. It was noticed, however, that 10 MAE genes, not classified in CPOE as being imprinted, exhibited methylation bistability (score > 0.1), raising the possibility that these genes might be imprinted, and one of these, *C11ORF21,* is known to lie within the Beckwith-Wiedemann syndrome (BWS) domain but is not known to be imprinted.

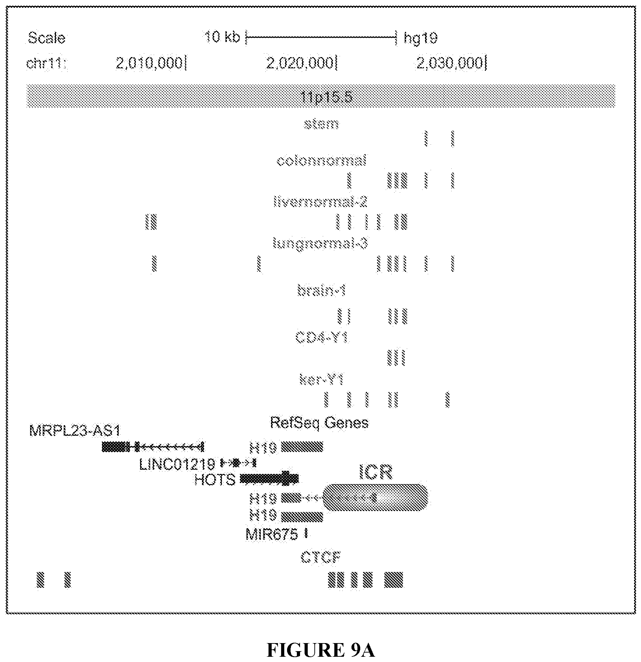

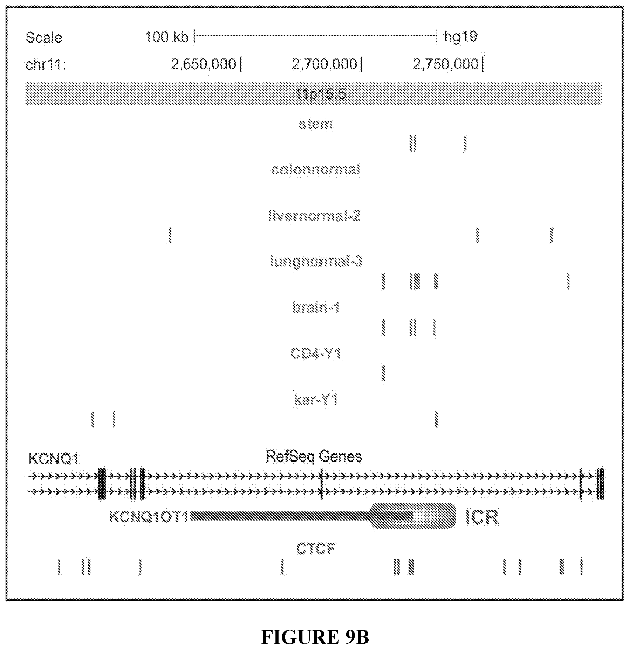

Considerable effort was previously expended to identify imprinted genes in the 11p15.5 chromosomal region related to Beckwith-Wiedemann syndrome (BWS) and loss of imprinting in cancer. The position of bistable marks in this well-studied imprinted locus was therefore assessed and revealed a correspondence with known imprinting control regions (ICRs) and CTCF binding sites just upstream of *H19,* as well as near the promoter of *KCNQ1OT1* (Fig. 9A,B).

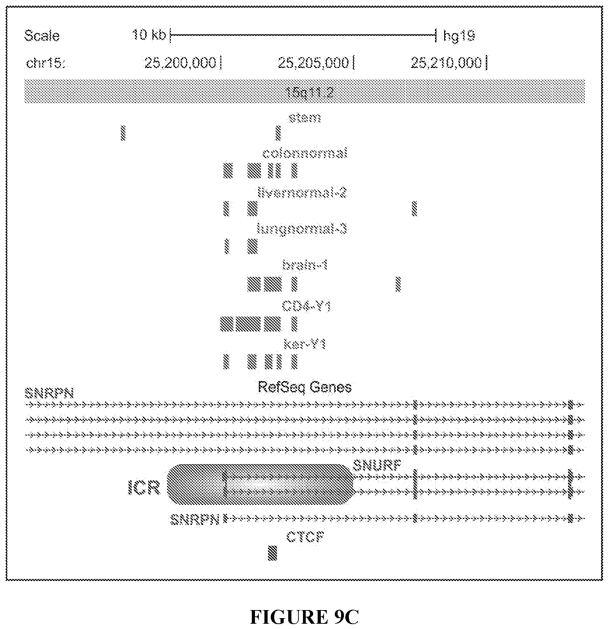

Bistable marks were also found near the *SNURF/SNRPN* promoter, which matched the location of a known ICR (Fig. 9C), as well as near the *PEG3/ZIM2* and *MEST/MESTIT1* promoter regions (9D,E).

*Entropy Blocks Predict TAD Boundaries*

It was also investigated whether the NME can be effectively used to computationally locate TAD boundaries.

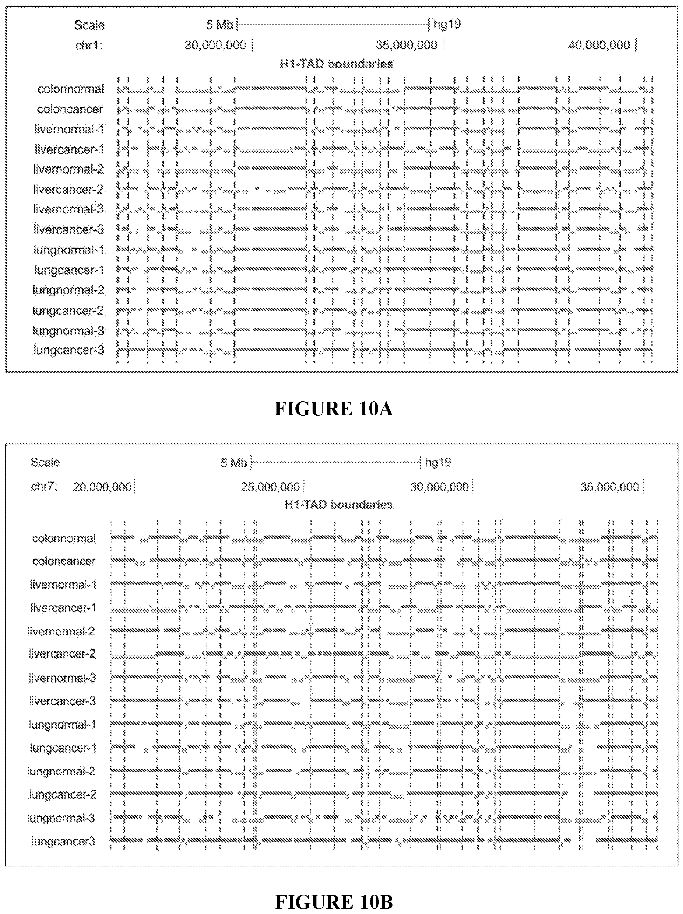

It was observed that, in many genomic samples, known TAD boundary annotations were visually proximal to boundaries of entropy blocks (EBs), i.e., genomic blocks of consistently low or high NME values (Fig. 10). This suggested that TAD boundaries may be located within genomic regions that separate successive EBs.

To determine whether this is true, EBs were computed in the WGBS stem data and 404 regions were generated to predict the location of TAD boundaries. It was then found, using "GenometriCorr", a statistical package for evaluating the correlation of genome-wide data with given genomic features, that the 5862 annotated TAD boundaries in HI stem cells were located within these predictive regions or were close in a statistically significant manner. These EB-based predictive regions correctly identified 6% of the annotated TAD boundaries (362 out of 5862) derived from 90% of computed predictive regions.

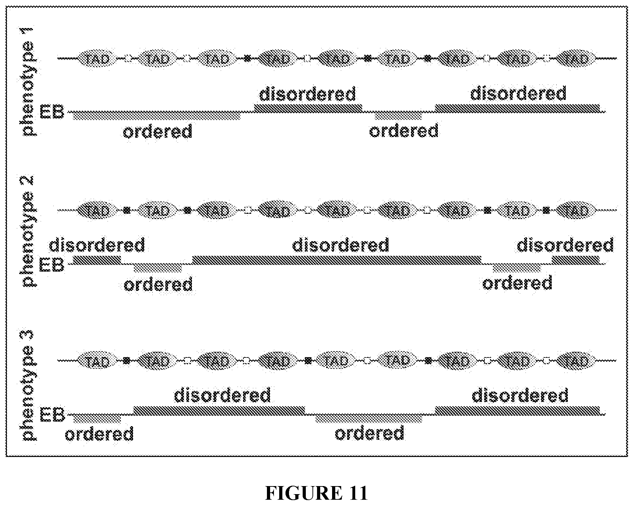

Subsequently, the analysis was extended by combining the TAD boundary annotations for HI stem cells with available annotations for IMR90 lung fibroblasts (a total of 10,276 annotations). Since TADs are largely thought to be cell-type invariant, it was realized that it is possible to predict the location of more TAD boundaries by combining information from EBs derived from additional phenotypes (Fig. 11). Therefore, WGBS data from 17 different cell types (stem, colonnormal, coloncancer, livernormal-1, livercancer-1, livernormal-2, livercancer-2, livernormal-3, livercancer-3, lungnormal-1, lungcancer-1, lungnormal-2, lungcancer-2, lungnormal-3, lungcancer-3, brain-1, brain-2) was employed, the corresponding EBs computed, predictive regions for each cell type determined, and these regions were appropriately combined to form a single list encompassing information (6632 predictive regions) from all genomic samples. Analysis using "GenometriCorr" produced results similar to those obtained in the case of stem cells and demonstrated that TAD boundaries that fell within identified predictive regions did so significantly more often than expected by chance, resulting in 62% correct identification of the annotated TAD boundaries (6408 out of 10,276) derived from 97% of computed predictive regions. This performance can be further improved by considering additional phenotypes.

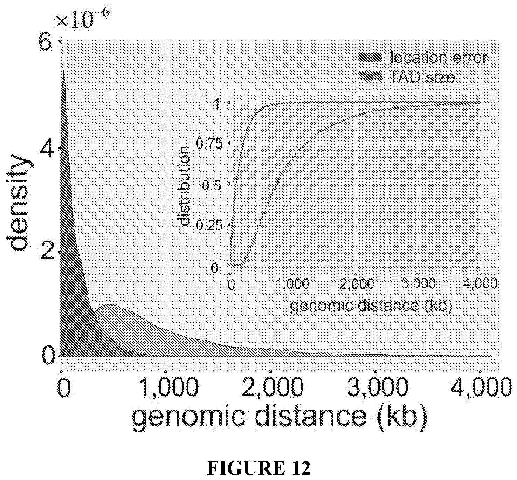

To further assess TAD boundary predictions, it was noted that it is natural to locate a TAD boundary at the center of the associated predictive region in the absence of prior information. The errors of locating TAD boundaries were small when compared to the TAD sizes as demonstrated by estimating the probability density and the corresponding cumulative probability distribution of the location errors as well as of the TAD sizes using a kernel density estimator (Fig. 12). Computed cumulative probability distributions implied that the probability of the location error being smaller than $N$ base pairs was larger than the probability of the TAD size being smaller than $N$, for every $N$. It was therefore concluded that the location error was smaller than the TAD size in a well-defined statistical sense (stochastic ordering). It was also observed that the median location error was an order of magnitude smaller than the median TAD size (94,000 vs. 760,000 base pairs). Finally, a boundary prediction was considered to be "correct" when the distance of a "true" TAD boundary from the center of a predictive region was less than the first quartile of the "true" TAD width distribution (Fig. 12 insert - green).

Taken together, the previous observations provide strong statistical evidence that there is an underlying relationship between EBs and TADs, and that this relationship can be easily harnessed to effectively predict TAD boundaries from WGBS data.

*Information-Theoretic Properties of Methylation Channels*

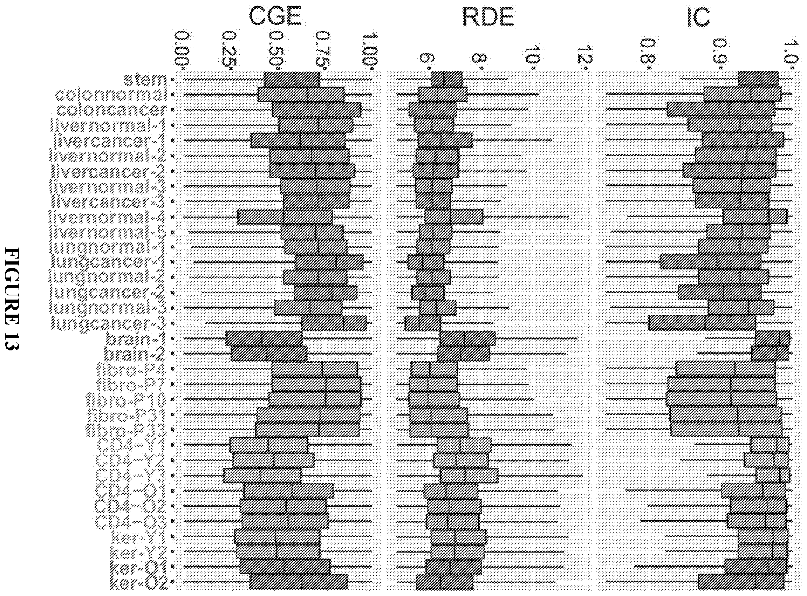

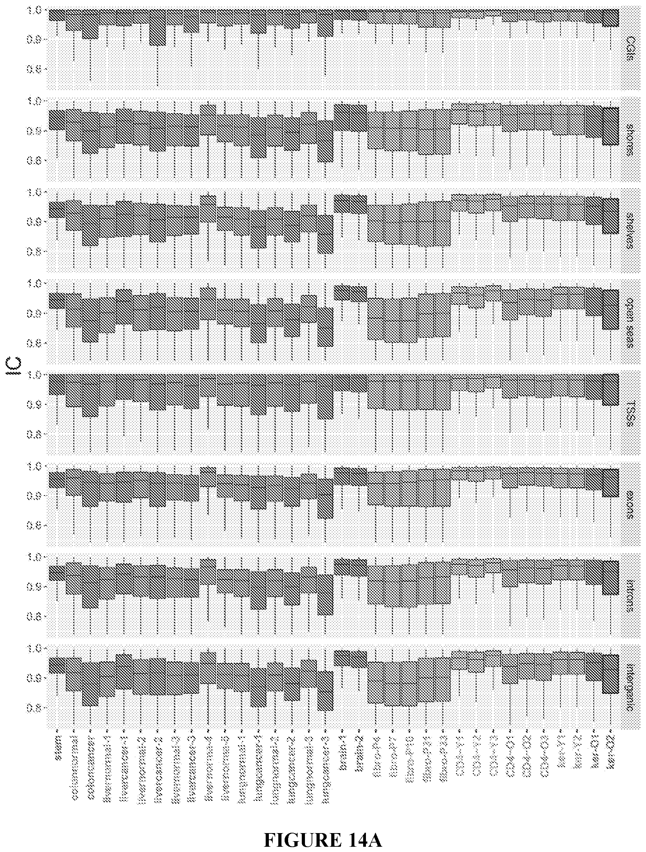

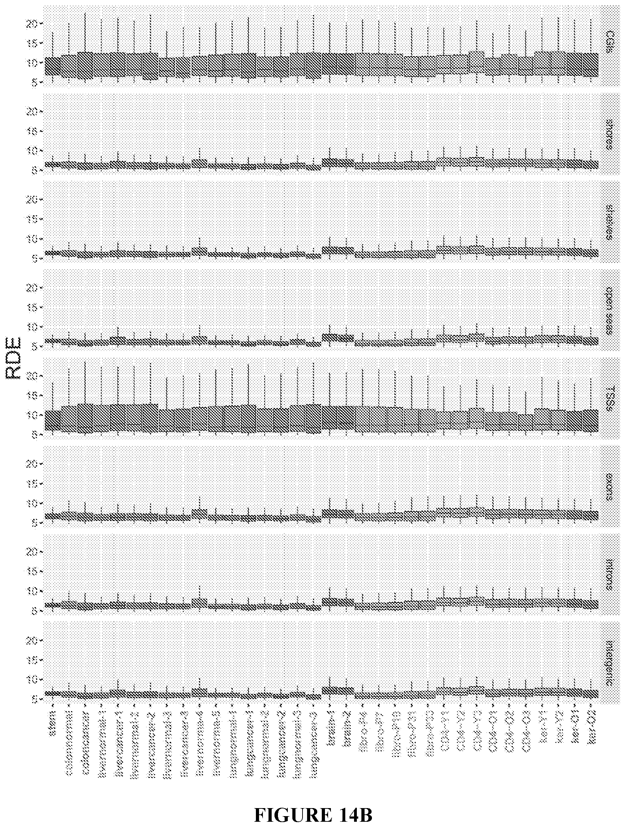

Information capacities (ICs), relative dissipated energies (RDEs), and CpG entropies (CGEs) of methylations channels (MCs) were computed in individual genomic samples and comparative studies were performed genome-wide (Fig. 13). A global trend of IC and RDE loss was observed in colon and lung cancer, accompanied by a global gain in CGE, although this was not true in liver cancer. Moreover, stem cells demonstrated a narrow range of relatively high IC and RDE values, whereas brain cells, CD4$^+$ lymphocytes, and skin keratinocytes exhibited high levels of IC and RDE, with noticeable loss in old individuals. Notably, the methylation state within CpG islands (CGIs) and transcription start sites (TSSs) is maintained by MCs whose capacities are appreciably higher overall than within shores, shelves, open seas, exons, introns and intergenic regions, and this is accomplished by significantly higher energy consumption (Fig. 14A,B).

These results reveal an information-theoretic view of genome organization, according to which methylation within certain regions of the genome is reliably transmitted by high capacity MCs leading to low uncertainty in the methylation state at the expense of high energy consumption, while methylation within other regions of the genome is transmitted by low capacity MCs that consume less energy but leading to high uncertainty in the methylation state.

*Information-theoretic Prediction of Chromatin Changes*

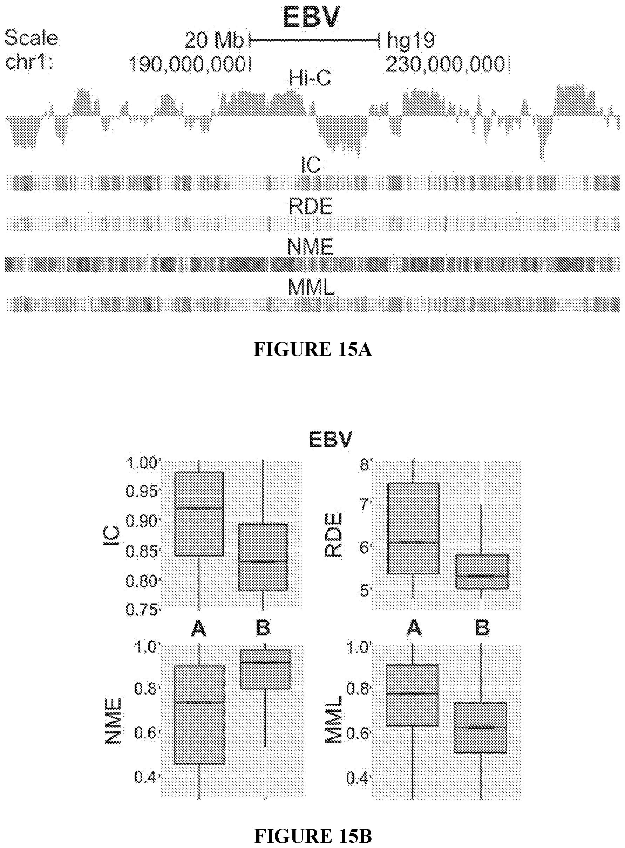

Calculating methylation channels (MCs) from WGBS data and comparing results to available A/B compartment tracks for EBV cells derived from Hi-C experiments, revealed enrichment of low IC, high NME, and low RDE within compartment B, and the opposite was globally observed for compartment A (Fig. 15A,B). These results led to the hypothesis that information-theoretic properties of methylation maintenance can be effectively used to predict the locations of compartments A and B. To test this prediction, a random forest regression model was employed to learn the informational structure of compartments A/B from available "ground-truth" data. That included a small number of available Hi-C data associated with EBV and IMR90 samples, obtained from [Dixon, J. R. et al. Nature 518, 331-336, (2015)], as well as A/B tracks produce using a method developed by Fortin and Hansen (FH) [Fortin, J. P. & Hansen, K. D. Genome Biol. 16, 180, (2015)] based on long-range correlations computed from pooled 450k array data associated with colon cancer, liver cancer and lung cancer samples. Due to the paucity of currently available Hi-C data, the FH data were included in order to increase the number of training samples and improve the accuracy of performance evaluation.

First, the Hi-C and FH data were paired with WGBS EBV, fibro-P10, and colon cancer samples, as well as with samples obtained by pooling WGBS liver cancer (livercancer-1, livercancer-2, livercancer-3) and lung cancer (lungcancer-1, luncancer-2, lungcancer-3) data. Subsequently, the entire genome was partitioned into 100,000 base pair bins (to match the available Hi-C and FH data) and 8 information-theoretic features of methylation maintenance were computed within each bin (median values and interquartile ranges of IC, RDE, NME and MML). By using all feature/output pairs, a random forest model was trained using the R package "randomForest" with its default settings, except that the number of trees was increased to 1,000. Then, the trained random forest model was applied on each WGBS sample and A/B tracks were produced that approximately identified A/B compartments associated with the samples. Since regression takes into account only information within a 100-kb bin, the predicted A/B values were averaged using a three-bin smoothing window and the genome-wide median value was removed from the overall A/B signal, as suggested by Fortin and Hansen [Fortin, J. P. & Hansen, K. D. Genome Biol. 16, 180, (2015)].

To test the accuracy of the resulting predictions, a 5-fold cross validation was employed, which involved training using four sample pairs and testing on the remaining pair for all five combinations. Performance was evaluated by computing the average correlation as well as the average percentage agreement between the predicted and each of the "ground-truth" A/B signals within 100-kb bins, where the absolute values of the predicted and "ground-truth" signals were both greater than a calling margin. A non-zero calling margin can be used to remove unreliable predictions. Finally, agreement was calculated by testing whether the predicted and the "ground-truth" A/B values within a 100-kb bin had the same sign.

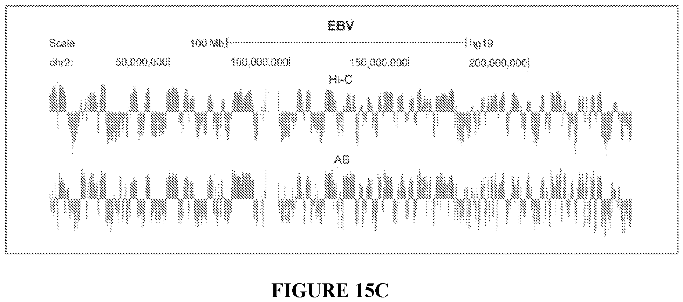

Random forest regression was capable of reliably predicting A/B compartments from single WGBS samples (see Fig. 15C for an example), resulting in cross-validated average correlation of 0.74 and an average agreement of 81% between predicted and true A/B signals when using a calling margin of zero, which increased to 0.82 and 91% when the calling margin was set equal to 0.2.

These results suggest that a small number of local information-theoretic properties of methylation maintenance can be highly predictive of large-scale chromatin organization, such as compartments A and B. Once properly trained, the random forest A/B predictor can be applied robustly on any WGBS sample.

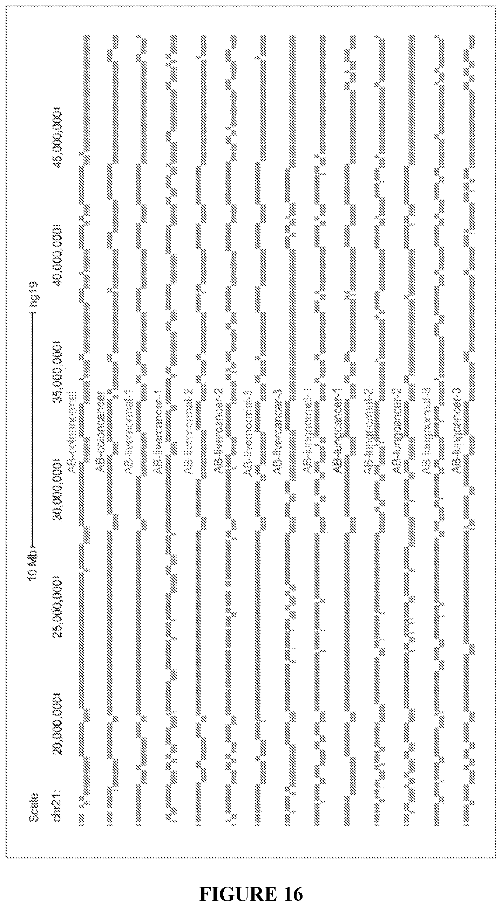

Consistent with the fact that compartments A and B are cell-type specific, and in agreement with results of a previous study that demonstrated extensive A/B compartment reorganization during early stages of development, many differences between predicted compartments A/B were observed (see Fig. 16 for an example). In order to comprehensively quantify observed differences in compartments A and B, percentages of A to B and B to A switching were computed in all sample pairs (Supplementary Data 4 described below and attached).

For each pair of WGBS samples, the percentage of A to B compartment switching was computed by dividing the number of 100-kb bin pairs for which an A prediction was made in the first sample and a B prediction made in the second sample by the total number of bins for which A/B predictions were available in both samples, and similarly for the case of B to A switching.

High levels ($\geq 20\%$) of A to B and B to A compartment switching were observed between stem and most of the remaining genomic samples, at least 10% switching between brain and most of the remaining samples, and low levels (< 10%) of switching between most normal colon, liver and lung samples. Also, at least 10% compartment B to A switching was noticed between colon, liver and lung normal and most cancer samples.

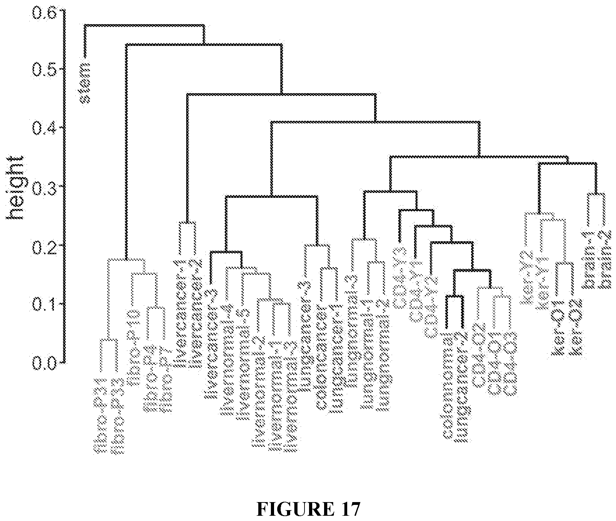

It was subsequently noticed that the net percentage of A/B compartment switching can be employed as a dissimilarity measure between two genomic samples, and used this measure to cluster samples (Fig. 17). These percentages were summed and the sums were employed to form a matrix of dissimilarity measures, which was then used as an input to a Ward error sum of squares hierarchical clustering scheme that was implemented using the R package "hclust" by setting the method variable to ward.D2. The clustering results provided evidence that stem cell differentiation is associated with high levels of chromatin reorganization. In particular, differentiated lineages and cancer were clustered together but they were distinguished from each other, while the brain was clustered closest to stem cells, as has been suggested by recent biochemical studies. Notably, young CD4 samples formed one cluster, whereas old CD4 samples formed another, and the same was true for skin.

Intriguingly, normal lung showed strikingly different chromatin organization from lung cancer, as did colon normal from colon cancer (Fig. 17). For this reason, it was attempted to relate these changes to known chromatin or methylation structures.

Previous studies have demonstrated the presence of large hypomethylated blocks in cancer that are remarkably consistent across tumor types. These blocks have been shown to correspond closely to large-scale regions of chromatin organization, such as lamin-associated domains (LADs) and large organized chromatin K9-modifications (LOCKs). Consistent with observations on the information-theoretic properties of compartment B and of carcinogenesis (Figs. 13 & 15A,B), it was asked whether hypomethylated blocks are associated mainly with compartment B.

To test this hypothesis, available hypomethylated blocks, LOCKs, and LADs were matched to their most closely related random-forest-predicted compartment B data, which came from the lungnormal-1, lungnormal-2, and lungnormal-3 samples. To evaluate enrichment of hypomethylated blocks (and similarly for LADs and LOCKs) within compartment B, two binary (0-1) random variables $R$ and $B$ were defined for each genomic subregion, such that $R = 1$ if the genomic subregion overlapped a block, and $B = 1$ if the genomic subregion overlapped compartment B. Then, a test was performed against the null hypothesis that $R$ and $B$ are statistically independent by applying the $\chi^2$-test on the $2 \times 2$ contingency table for $R$ and $B$ and the odds ratio (OR) was calculated as a measure of enrichment.

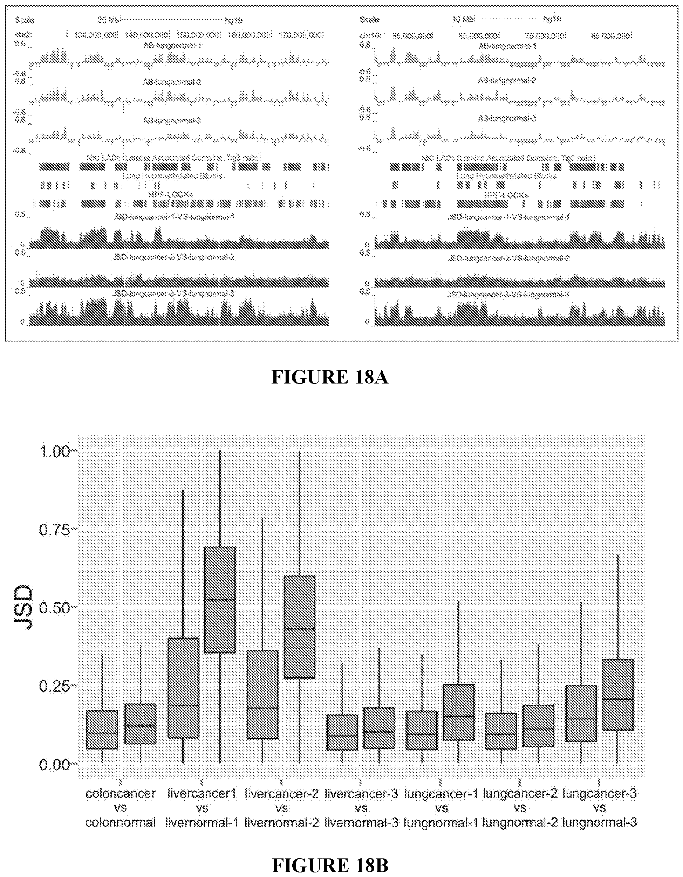

Significant overlap (Fig. 18) with compartment B in normal lung was found with the hypomethylated blocks (OR $\approx 3.3$, $p$-value < $2.2 \times 10^{-16}$), and the same was true for LADs (OR $\approx 4$, $p$-value < $2.2 \times 10^{-16}$) and LOCKs (OR $\approx 5.3$, $p$-value < $2.2 \times 10^{-16}$).

Interestingly, compartment B in normal tissue may exhibit regions of large JSD values between normal and cancer (Fig. 18A), suggesting that considerable epigenetic changes may occur within this compartment during carcinogenesis. This observation was further supported by the observed differences in the genome-wide distributions of JSD values between normal and cancer within compartments A and B in normal (Fig. 18B).

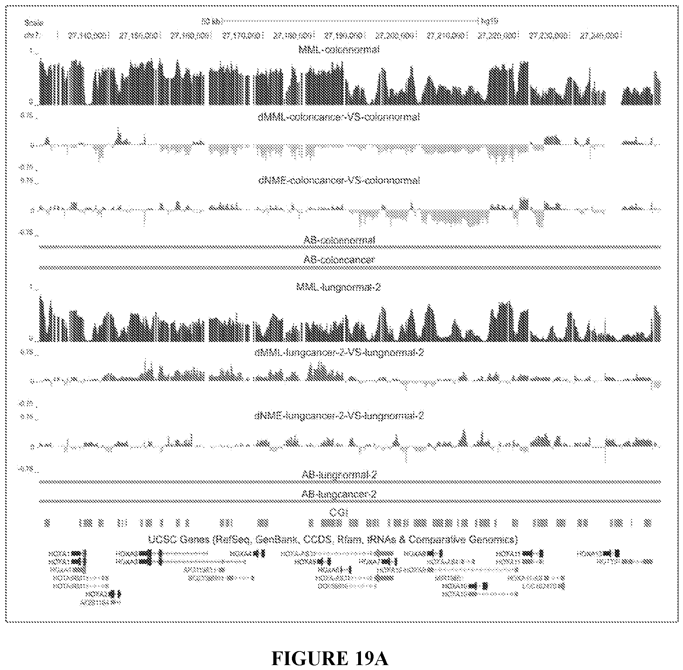

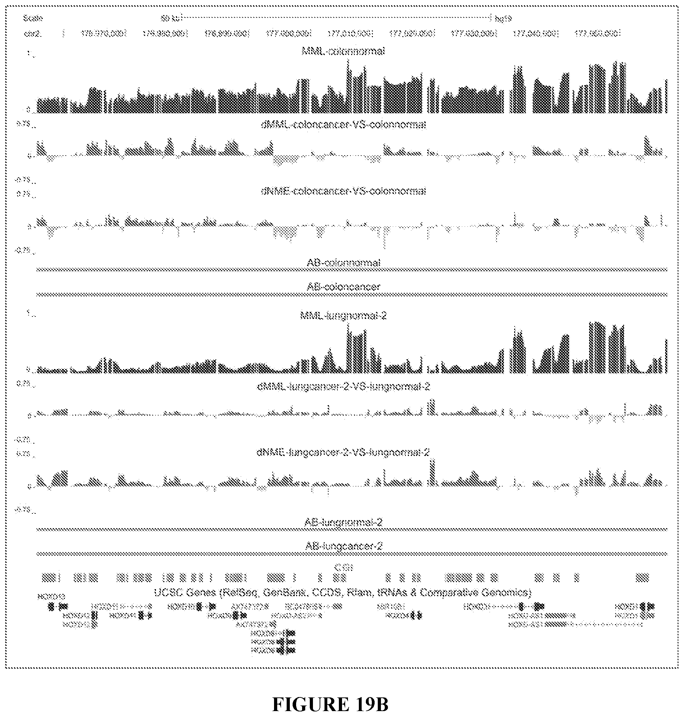

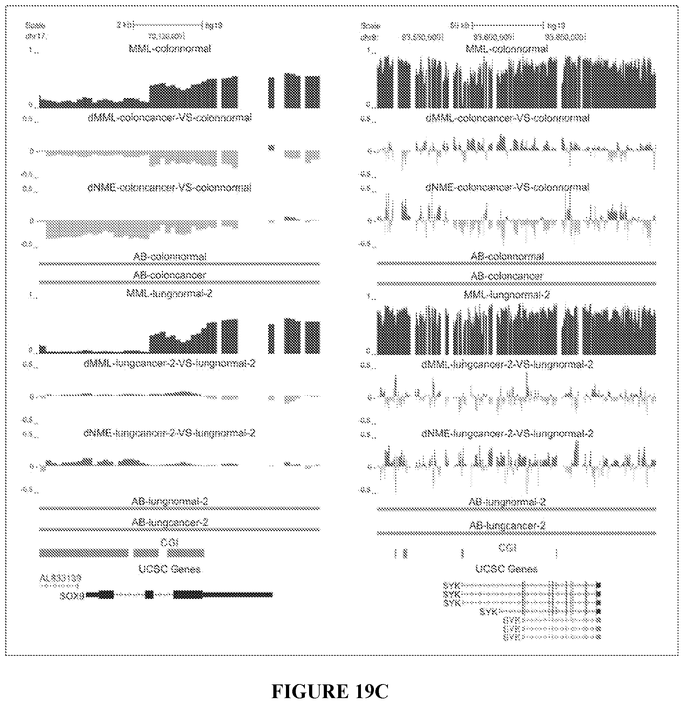

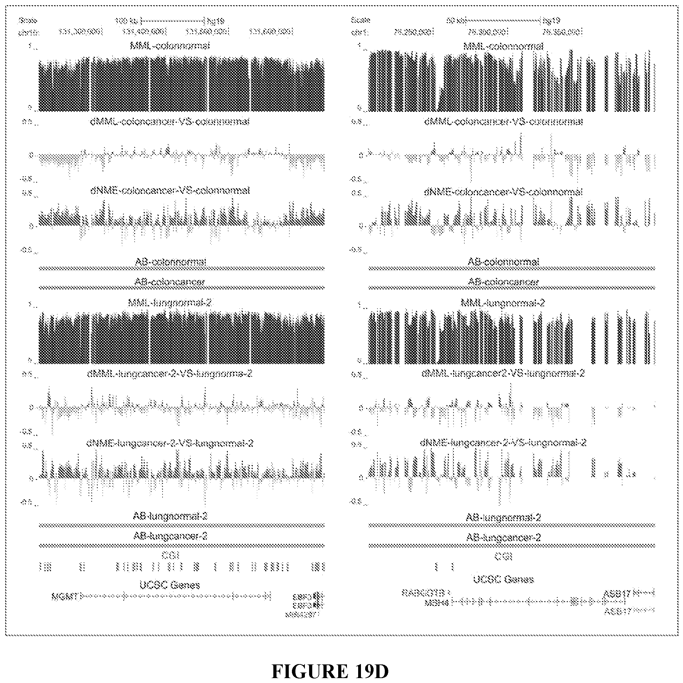

Compartment B to A switching in colon cancer included the *HOXA* and *HOXD* gene clusters, whereas, in lung cancer, it included the *HOXD* gene cluster but not *HOXA* (Fig. 19A,B). It also included *SOX9* in colon cancer and the tyrosine kinase *SYK* in both colon and lung cancer (Fig. 19C). Fewer regions showed compartment A to B switching in cancer, consistent with the directionality of LAD and LOCKs changes in cancer. Interestingly, this included *MGMT* in colon but not lung, a gene implicated in the repair of alkylation DNA damage that is known to be methylated and silenced in colorectal cancer, as well as the mismatch repair *gene MSH4* (Fig. 19D).

Together with the previous observation of significant compartment B to A switching between normal/cancer samples, these results suggest that compartment B demarcates genomic regions in which it is more likely for methylation information to be degraded during carcinogenesis.

*Entropic Sensitivity Quantifies Environmental Influences on Epigenetic Stochasticity*

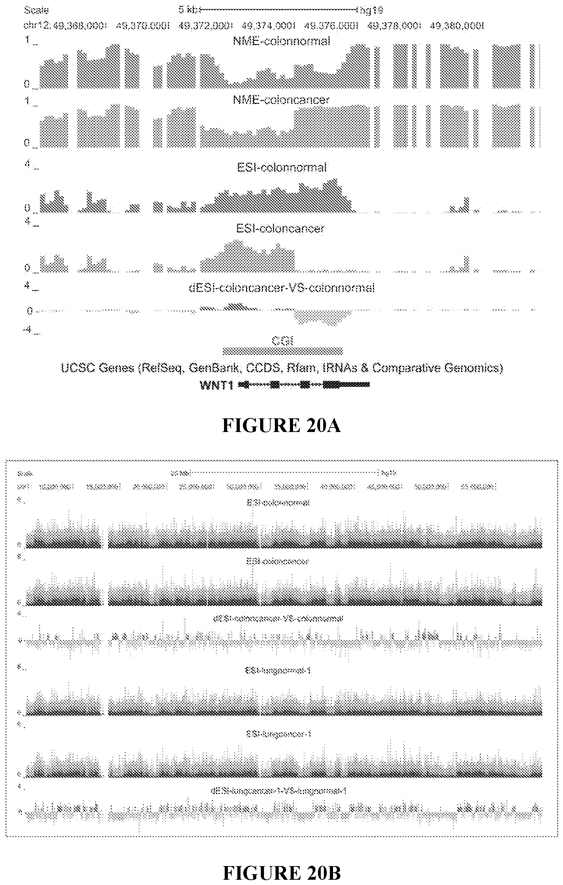

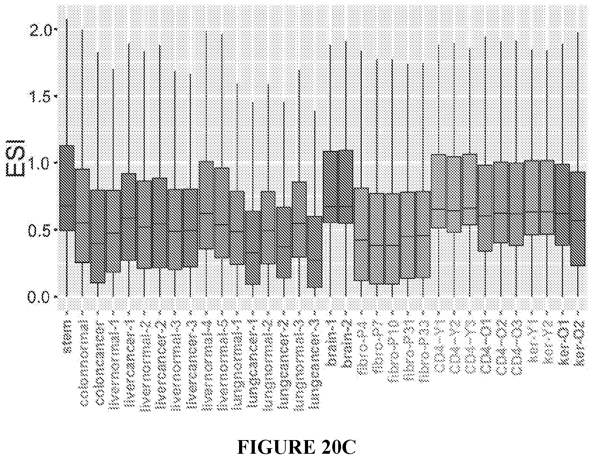

Epigenetic changes, such as altered DNA methylation and post-translational modifications of chromatin, integrate external and internal environmental signals with genetic variation to modulate phenotype. In this regard, it was sought to investigate the influence of environmental exposure on methylation stochasticity by following a sensitivity analysis approach that enables quantification of the effect of environmental variability on methylation entropy. To this end, environmental variability was viewed as a process that directly influences the methylation PEL parameters and a stochastic approach was developed that allowed use of the entropic sensitivity index (ESI) as a relative measure of NME to parameter variability. Calculation of the ESI values genome-wide from single WGBS data allowed quantification of the influence of environmental fluctuations on epigenetic uncertainty in individual genomic samples as well as comparative studies (Figs. 20, 21 & 22). For example, in colon normal, appreciable entropic sensitivity was observed within the CGI associated with *WNT1,* with part of the CGI exhibiting a gain in entropy and loss of sensitivity in colon cancer (Fig. 20A).

Globally, differences in ESI among tissues were observed (Fig. 20B,C), with stem and brain cells exhibiting higher levels of entropic sensitivity than the rest of the genomic samples. Together with the fact that brain cells are highly methylated (Fig. 2A), high levels of entropic sensitivity would predict that brain can show high rates of demethylation in response to environmental stimuli, consistent with recent data showing that the DNA demethylase *Tet3* acts as a synaptic activity sensor that epigenetically regulates neural plasticity by active demethylation, and a similar observation could be true for stem cells and CD4[+] lymphocytes. Colon and lung cancer exhibited global loss of entropic sensitivity, whereas gain was noted in liver cancer. Moreover, CD4[+] lymphocytes and skin keratinocytes exhibited global loss of entropic sensitivity in older individuals (Fig. 20C), while cultured fibroblasts showed noticeably lower ESI without any downward trend in passage number.

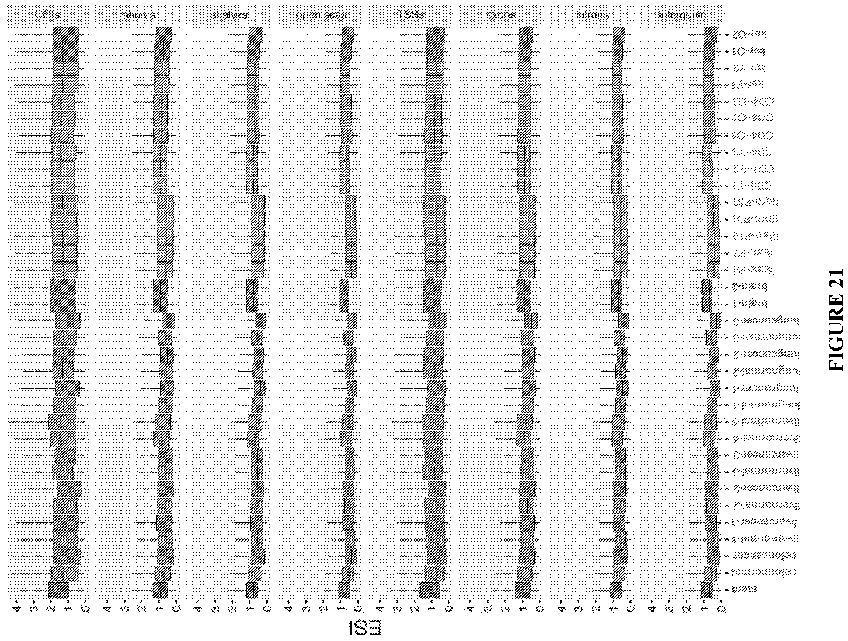

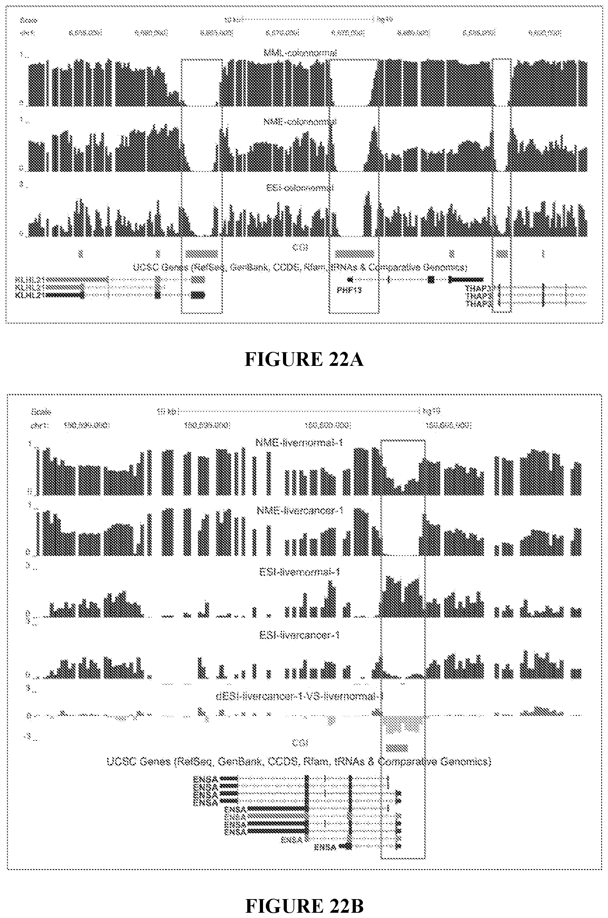

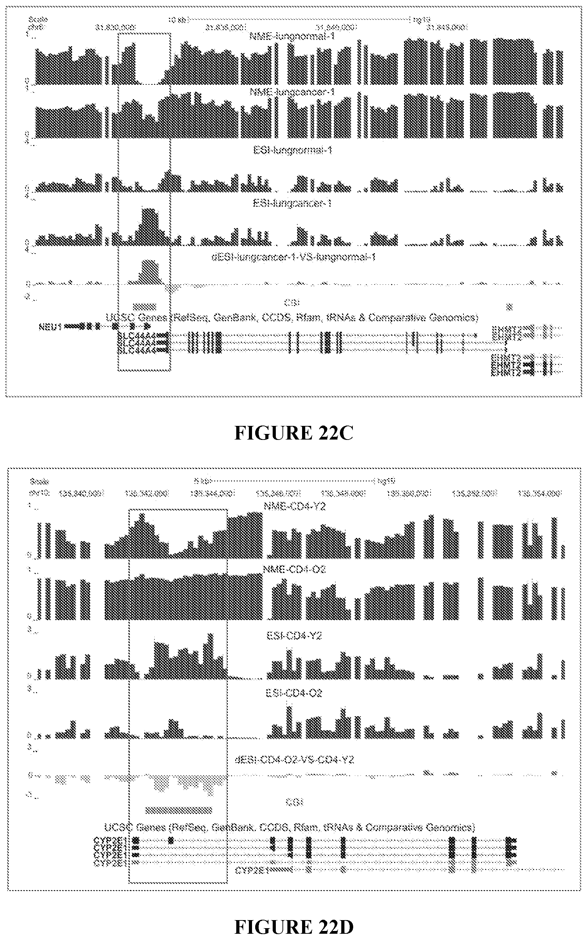

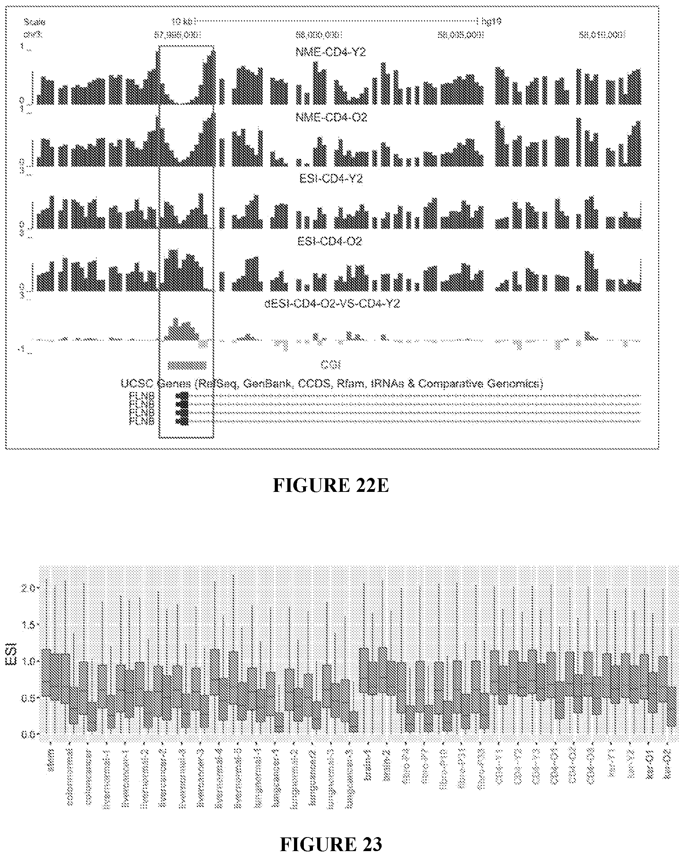

Higher and more variable ESI values were observed within CGIs and at TSSs, compared to other genomic features, such as shores, exons, and introns (Fig. 21). However, some unmethylated CGIs exhibited low entropic sensitivity (Fig. 22A), whereas gain or loss of entropic sensitivity within CGIs was observed between normal and cancer (Fig. 22B,C), as well as in older individuals (Fig. 22D,E). Notably, differences in ESI were not simply due to entropy itself, as many regions of low entropy showed small ESI values (Fig. 22A,B,C), while other such regions exhibited noticeable ESI values (Fig. 22B,D,E), indicating substantial sensitivity to environmental perturbations.

The relationship of entropic sensitivity to higher-order chromatin structure was also examined. It was found that entropic sensitivity within compartment A was appreciably higher than in compartment B in all genomic samples except stem cells (Fig. 23), consistent with the notion that the transcriptionally active compartment A would be more responsive to stimuli. Moreover, observed differences among normal tissues and between normal and cancer were largely confined to compartment B (Fig. 23). One could notice substantial loss of entropic sensitivity in compartment B in older CD4$^+$ lymphocytes and skin keratinocytes, but not in compartment A. This is in contrast to cell culture that showed a sensitivity gain in compartment B (Fig. 23).

To further investigate entropic sensitivity changes between tissues, genes were ranked according to their differential ESI (dESI) within their promoters between colon normal and colon cancer (Supplementary Data 5 described below and attached). Colon cancer showed several LIM-domain proteins, including *LIMD2* (ranked 4th), which transduce environmental signals regulating cell motility and tumor progression, as well as genes implicated in colon and other types of cancer, such as *QK1* (ranked 1st), a critical regulator of colon epithelial differentiation and suppressor of colon cancer that was recently discovered to be a fusion partner with *MYB* in glioma leading to an auto-regulatory feedback loop, *HOXA9* (ranked 8th), a canonical rearranged homeobox gene that is dysregulated in cancer, and *FOXQ1* (ranked 9th), which is overexpressed and enhances tumorigenicity of colorectal cancer.

Together, the previous results suggest that environmental exposure can influence epigenetic uncertainty in cells with a level of sensitivity that varies along the genome and between compartments in a cell-type specific manner, and present the intriguing possibility that disease, environmental exposure, and aging are associated with substantial loss or gain of entropic sensitivity that could compromise the integration of environmental cues regulating cell growth and function.

## DISCUSSION

In this document, the Ising model of statistical physics was employed to derive, from whole genome bisulfite sequencing, epigenetic potential energy landscapes (PELs) representing intrinsic epigenetic stochasticity. Rather than epigenetic landscapes with external "noise" terms, biologically sound principles of methylation processivity, distance-dependent cooperativity, and CpG density were employed to build a rigorous approach to modeling DNA methylation landscapes. This approach was not only capable of modeling stochasticity in DNA methylation from low coverage data, but also allowed genome-wide analysis of Shannon entropy at high resolution. By incorporating fundamental principles of information theory into a framework of methylation channels, it was also possible to predict in detail, high-order chromatin organization from single WGBS samples without performing Hi-C experiments.

Several significant insights ensued from this analysis. It was found that Shannon entropy varies markedly among tissues, across the genome and across features of the genome. Loss of methylation and entropy gain in cells from older individuals was consistently observed, in contrast to cell culture, which exhibited large losses of methylation level and a relatively stable entropy distribution with passage. Genes associated with entropy gain appeared to be highly relevant to aging, although the full implications of this observation requires further investigation. In some instances, it was observed that high entropy is due to the coexistence of a fully methylated and a fully unmethylated state, which is termed bistability. Bistability in methylation level was found to be associated with many known imprinted regions, presumably because of allele-specific methylation.

Rather than identifying differentially methylated regions (DMRs) among compared genomic samples using marginal statistics, the Jensen-Shannon distance (JSD) was employed to compute information-theoretic epigenetic differences genome-wide. This approach allows one to determine epigenetic differences between individual genomic samples with the potential clinical advantage of identifying specific epigenetic differences, which are unique to that genomic sample compared to a matched normal tissue. Analysis of a panel of tissues of diverse origins revealed a "developmental wheel" of the three germ cell lineages around a stem cell hub. Consistently, cancers are extremely divergent and most importantly not intermediate in their methylation properties between stem cells and normal tissue.

It was investigated whether the JSD simply embodies mean differences that have been exhaustively characterized in the past, or if it reveals new insights independent of the mean. To address this question, genomic regions with high JSD but low mean differences between sample pairs were identified, with greater enrichment for many categories of stem cell maintenance or lineage development than found for regions with mean differences per se, suggesting a key role of stochasticity in development. In turn, this type of stochasticity appears to be driven by localized regions of high cooperativity, which tends to flatten the PEL with little change in mean methylation. Regions with high JSD and low mean methylation differences were found to be enriched in Polycomb repressive complex (PRC2) binding sites, suggesting a possible role for PRC2 in stochastic switching during development. Intriguingly, PRC2 components are critical for stochastic epigenetic silencing in an early area of the field of epigenetics, position effect variegation, which also involves stochasticity. It is suggested that PRC2 is important not only for gene silencing but also for regulating epigenetic stochasticity in general.

A new insight was achieved by discovering a relationship between TAD boundaries and entropy blocks. It was demonstrated that TAD boundaries can be located within transition domains between high and low entropy in one or more genomic samples. This suggests a model in which TAD boundaries, which are relatively invariant across cell types and are associated with CTCF binding sites, are potential transition points at which high and low entropy blocks can be demarcated in the genome, and the particular combination of TAD boundaries that transition between high and low entropy define, in large part, the A/B compartments distinguishing tissue types.

An information-theoretic approach to epigenetics was also introduced by means of methylation channels, which allows one to estimate the information capacity of the methylation machinery to reliably maintain the methylation state. A close relationship was found between information capacity, CG entropy, and relative dissipated energy, as well as between regional localization of high information capacity and attendant high energy consumption (e.g., within CpG island shores and compartment A). It was realized that informational properties of methylation channels can be used to predict A/B compartments and a machine learning algorithm was designed to perform such predictions on widely available WGBS samples from individual tissues and cell culture. This algorithm can be used to predict large-scale chromatin organization from DNA methylation data on individual genomic samples. Single paired WGBS data sets of normal and cancer were used to predict A/B compartment transitions. Both colon and lung cancers showed marked compartment switching, most often from B to A, with regions of B to A switching corresponding closely to LADs and LOCKs. Domains of B to A and A to B switching include many genes that are activated or silenced in cancer, suggesting that compartment switching could contribute to cancer.

Lastly, by viewing environmental variability as a process that directly influences the methylation PEL parameters, the concept of entropic sensitivity was introduced, identifying genomic loci where external factors are likely to influence the methylation PEL. While the inventors have only begun to explore the epigenetic implications of entropic sensitivity, it appears that aging and some cancers are associated with global loss of entropic sensitivity and thus to less responsive PELs. If this observation holds true on further study, it could be related to the well-known reduced physiological plasticity of aging, as well as to the autonomous nature of tumor cells.

This study demonstrates a potential relationship between epigenetic information, entropy and energy that may maximize efficiency in information storage in the nucleus. Pluripotent stem cells require a high degree of energy to maintain methylation channels, with certain regions of the genome containing highly deformable PELs corresponding to differentiation branch points, as suggested metaphorically by Waddington, which can now be identified and their parameters responsible for plasticity be mapped. In differentiated cells, large portions of the genome (compartment B, LADs, LOCKs) need not maintain high information capacity and attendant high energy consumption, with their relative sequestration thus providing increased efficiency. However, when domains within compartment B switch to compartment A, previously accumulated epigenetic errors become deleterious and, compounded with reduced entropic sensitivity, may decrease the chance for homeostatic correction.

Finally, the stochastic nature of DNA methylation and the close relationship between methylation entropy, channel capacity, dissipated energy and chromatin structure demonstrated herein raises the intriguing possibility that DNA methylation in a given tissue may carry information about both the current state and the possibility of stochastic switching. This information could then be propagated in part through methylation channels over many cycles of DNA replication, even for higher order chromatin organization where the chromatin post-translational modifications themselves may be lost during cell division. This could imply that epigenetic information is carried by a population of cells as a whole, and that this information not only helps to maintain a differentiated state but to also help mediate developmental plasticity throughout the life of an organism.

## FIGURE LEGENDS

Figure 1 relates to potential energy landscapes. 1A: Multiple WGBS reads of the methylation state within a genomic locus are used to form a methylation matrix whose entries represent the methylation status of each CpG site (1: methylated, 0: unmethylated, ND: no data). Most methods for methylation analysis estimate marginal methylation probabilities and means at individual CpG sites by using the methylation information only within each column associated with a CpG site. The statistical physics approach presented in this disclosure computes the most likely PEL by determining the likelihood of each row of the methylation matrix, combining this information across rows into an average likelihood, and maximizing this likelihood with respect to the PEL parameters. 1B: PELs associated with the CpG islands (CGIs) of *WNT1* in colon normal and colon cancer and *EPHA4* in stem and brain. Point *(m,n)* marks a methylation state, with (0,0) indicating the fully unmethylated state, which is also the ground state (i.e., the state of lowest potential) in both examples. 1C: Boxplots of the Ising PEL parameter distributions for all genomic samples used in this study. The boxes show the 25% quantile, the median, and the 75% quantile, whereas each whisker has a length of $1.5 \times$ the interquartile range.

Figure 2 relates to the mean methylation level (MML) and the normalized methylation entropy (NME). 2A: Boxplots of MML and NME distributions for all genomic samples used in this study. The boxes show the 25% quantile, the median, and the 75% quantile, whereas each whisker has a length of $1.5 \times$ the interquartile range. 2B: Genome-wide MML and NME densities associated with two normal/cancer samples show global MML loss in colon and lung cancer, accompanied by a gain in entropy. 2C: Genome-wide MML and NME densities associated with young/old CD4$^+$ lymphocytes and skin keratinocytes show global MML loss in old individuals, accompanied by a gain in entropy.

Figure 3 relates to changes in mean methylation level and methylation entropy in cancer. 3A: Genome browser image showing significant loss in mean methylation level (dMML) in colon and lung cancer, accompanied by gain in methylation entropy (dNME). Liver cancer exhibits loss of methylation entropy within large regions of the genome due to profound hypomethylation. 3B: The CGI near the promoter of *CDH1,* a tumor suppressor gene, exhibits entropy loss in colon cancer. 3C: The CGI near the promoter of *NEU1* shows gain of methylation entropy in lung cancer. *NEU1* sialidase is required for normal lung development and function, whereas its expression has been implicated in tumorigenesis and metastatic potential. 3D: Noticeable loss of methylation entropy is observed in liver cancer at the shores of the CGI near the promoter of *ENSA,* a gene that is known to be hypomethylated in liver cancer.

Figure 4 pertains to the breakdown of mean methylation level (MML) and normalized methylation entropy (NME) within genomic features throughout the genome in various genomic samples. Boxplots of genome-wide distributions of methylation measures for all genomic samples used in this study within CGIs, shores, shelves, open seas, TSSs, exons, introns, and intergenic regions. 4A: Mean methylation level (MML). 4B: Normalized methylation entropy (NME). The boxes show the 25% quantile, the median, and the 75% quantile, whereas each whisker has a length of $1.5 \times$ the interquartile range.

Figure 5 shows that cultured fibroblasts may not be appropriate for modeling aging. 5A: Unmethylated blocks (MB-green) progressively form with passage in HNF fibroblasts and this process is similar to the one observed during carcinogenesis in liver cells. However, entropic blocks (EB-red) remain relatively stable. 5B: An example of the potentially misleading nature of HNF fibroblasts as a model for aging is *CYP2E1,* a gene that has been found to be downregulated with age. The differential mean methylation level (dMML) track shows methylation gain in old CD4$^+$ lymphocytes near the promoter of this gene, whereas no appreciable change in methylation level is observed with passage. Similarly, the *CYP2E1* promoter demonstrates large entropy differential (dNME) in old CD4$^+$ lymphocytes, but virtually no entropy change with passage in HNF fibroblasts. 5C: Noticeable gain in methylation entropy is also observed near the promoter of *FLNB* in old CD4$^+$ lymphocytes, a gene found to be downregulated with age. However, the *FLNB* promoter exhibits loss of entropy with passage in fibroblasts.

Figure 6 shows that epigenetic distances delineate lineages. Multidimensional scaling (MDS) visualization of genomic dissimilarity between 17 diverse cell and tissue samples, evaluated using the Jensen-Shannon distance (JSD), reveals grouping of genomic samples into clear categories based on lineage.

Figure 7 shows differential regulation within genomic regions of high Jensen-Shannon distance (JSD) but low differential mean methylation level (dMML) near promoters of some genes. 7A: The promoter of *EPHA4* shows binding of EZH2 and SUZ12, key components of the histone methyltransferase PRC2, and demonstrates negligible differential methylation between stem cells and brain but high JSD, driven by the PEL parameters, which leads to gain of entropy in brain. 7B: The promoter of *SIM2,* a master regulation of neurogenesis, exhibits low level of dMML but high JSD between stem cells and brain, demonstrating large epigenetic distance. Regulation of the PEL parameters results in low methylation level in both stem and brain but in gain of entropy in brain. This region also shows binding of EZH2 and SUZ12. 7C: A similar behavior is observed within a 14,000 base pair region that contains *FOXD3,* a transcription factor associated with pluripotency. 7D: The promoter of *SALL1,* a key developmental gene, exhibits differential behavior between stem and brain that is similar to the one exhibited by *SIM2.* 7E: The promoter of *ASCL2,* a developmental gene involved in the determination of the neuronal precursors in the peripheral and central nervous systems, exhibits a similar behavior as the promoters of *SIM2* and *SALL1* but shows entropy loss in brain.

Figure 8 relates to methylation bistability and entropy. Boxplots of NME distributions within bistable genomic subregions (yellow) as compared to the rest of the genome (purple). The boxes show the 25% quantile, the median, and the 75% quantile, whereas each whisker has a length of $1.5 \times$ the interquartile range.

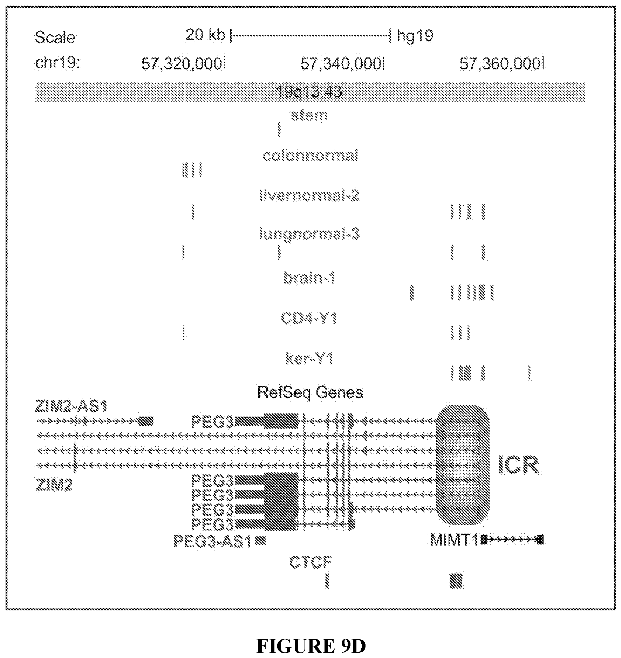

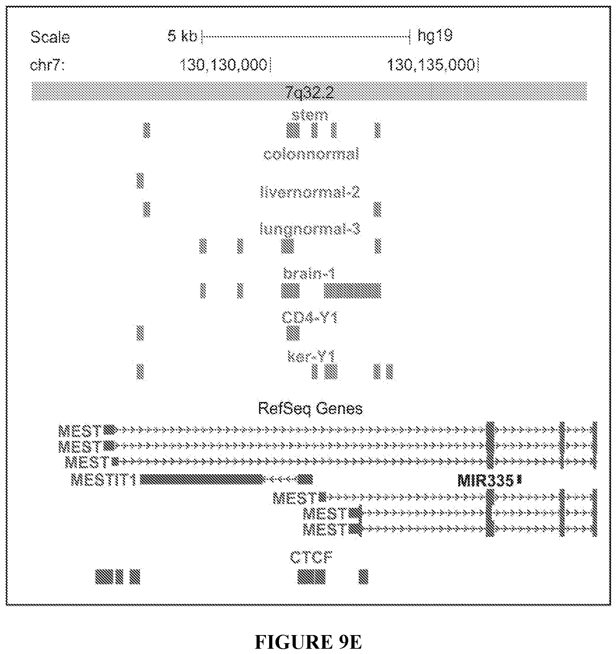

Figure 9 relates to bistability in methylation level and imprinting. 9A: Genome browser image displaying part of the 11p15.5 chromosomal region associated with *H19.* 9B: A portion of the 11p15.5 chromosomal region associated with *KCNQ1OT1.* 9C: The 15q11.2 chromosomal region near the *SNURF* promoter. 9D: Genome browser image displaying part of the 19q13.43 chromosomal region around the *PEG3/ZIM2* promoter. Bistable methylation marks, shown for a number of normal tissues, coincide with the location of the *PEG3/ZIM2* ICR that exhibits CTCF binding. Note that the ICR also includes the transcriptional start site of the imprinted gene *MIMT1.* 9E: Genome browser image displaying part of the 7q32.2 chromosomal region around the *MEST/MESTIT1* promoter. Bistable methylation marks, shown for a number of normal tissues, coincide with areas rich in CTCF binding sites.

Figure 10 relates to entropy blocks and TAD boundaries. 10A: In the normal/cancer panel, a subset of known TAD boundary annotations in H1 stem cells appeared to be associated with boundaries of entropic blocks (green:

ordered, red: disordered), suggesting that TADs may maintain a consistent level of methylation entropy within themselves. 10B: Another example showing that the location of TAD boundaries may associate with boundaries of ordered (green) or disordered (red) blocks.

Figure 11 relates to entropy blocks and TAD boundaries. Regions of entopic transitions can be effectively used to identify the location of some TAD boundaries (black squares). Since TADs are cell-type invariant, the location of more TAD boundaries can be identified by using additional WGBS data corresponding to distinct phenotypes.

Figure 12 relates to entropy blocks and TAD boundaries. Probability densities and cumulative probability distributions (insert) of TAD boundary location error and TAD sizes.

Figure 13 relates to information-theoretic properties of methylation channels (MCs). Boxplots of genome-wide ICs, RDEs and CGEs at individual CpG sites show global differences among genomic samples. The boxes show the 25% quantile, the median, and the 75% quantile, whereas each whisker has a length of $1.5 \times$ the interquartile range.

Figure 14 pertains to the breakdown of information-theoretic properties of methylation channels (MCs) within genomic features throughout the genome in various genomic samples. Boxplots of information-theoretic properties of MCs for all genomic samples used in this study within CGIs, shores, shelves, open seas, TSSs, exons, introns, and intergenic regions. 14A: Information capacity (IC). 14B: Relative dissipated energy (RDE). The boxes show the 25% quantile, the median, and the 75% quantile, whereas each whisker has a length of $1.5 \times$ the interquartile range.

Figure 15 shows that information-theoretic properties of methylation channels (MCs) can be used to predict large-scale chromatin organization. 15A: Analysis of Hi-C and WGBS data reveals that maintenance of the methylation state within compartment B (blue) in EBV cells is mainly performed by MCs with low information capacity (IC) that dissipate low amounts of energy (RDE) resulting in a relatively disordered (NME) and less methylated (MML) state than in compartment A (brown). 15B: Boxplots of genome-wide distributions of IC, RDE, NME and MML demonstrate their attractiveness as features for predicting compartments A/B using WGBS data from single genomic samples. The boxes show the 25% quantile, the median, and the 75% quantile, whereas each whisker has a length of $1.5 \times$ the interquartile range. 15C: An example of random forest based prediction of A/B compartments (AB) in EBV cells using information-theoretic properties of methylation maintenance.

Figure 16 relates to A/B compartment switching. An example of switching between predicted compartments A (brown) and B (blue) observed in cancer, with B to A compartment switching being more frequent than A to B switching.

Figure 17 relates to A/B compartment switching and clustering of genomic samples. Net percentage of A/B compartment switching was used as a dissimilarity measure in hierarchical agglomerative clustering. At a given height, a cluster is characterized by lower overall compartment switching than an alternative grouping of genomic samples.

Figure 18 relates to compartment B overlapping hypomethylated blocks, LADs, and LOCKs, as well as its enrichment in high epigenetic distances. 18A: Genome browser images of two chromosomal regions show significant overlap of compartment B in normal lung (blue) with hypomethylated blocks, LADs, and LOCKs. Gain in JSD is observed within compartment B (blue) in normal lung during carcinogenesis. 18B: Boxplots of genome-wide JSD distributions within compartments A (brown) and B (blue) in normal colon, liver and lung demonstrate gain in JSD within compartment B in cancer. The boxes show the 25% quantile, the median, and the 75% quantile, whereas each whisker has a length of $1.5 \times$ the interquartile range.

Figure 19 relates to the relocation of compartments A and B in cancer. 19A: The *HOXA* cluster of developmental genes is within compartment B in normal colon, liver and lung. It is however relocated to compartment A in colon and liver cancer but not in lung cancer. Compartmental reorganization of the *HOXA* genes is accompanied by marked hypomethylation and entropy loss within selected loci, implicating a role of chromatin reorganization in altered *HOXA* gene expression within tumors. 19B: The *HOXD* genes are within compartment B in normal colon, liver and lung and are relocated to compartment A in all three cancers. 19C: *SOX9* is within compartment B in colon and lung normal and is relocated to compartment B only in colon cancer. This is accompanied by marked hypomethylation and entropy loss. *SYK* is within compartment B in colon and lung normal and it is relocated to compartment B both in colon and lung cancer. 19D: *MGMT* and *MSH4* are within compartment A in colon and lung normal and they are relocated to compartment B only in colon cancer. Compartmental reorganization is accompanied mostly by hypomethylation and a marked gain in entropy.

Figure 20 relates to computing and comparing entropic sensitivity. 20A: Gain of entropy and loss in the entropic sensitivity index (ESI) is observed within a portion of the CGI associated with *WNT1*. 20B: Large differences in entropic sensitivity (dESI) may be observed genome-wide between normal and cancer tissues (visualized here for a large section of chromosome 1), exhibiting alternate bands of hyposensitivity and hypersensitivity. 20C: Boxplots of genome-wide ESI distributions corresponding to the genomic samples used in this study reveal global differences in entropic sensitivity across genomic samples. The boxes show the 25% quantile, the median, and the 75% quantile, whereas each whisker has a length of $1.5 \times$ the interquartile range.

Figure 21 pertains to the breakdown of entropic sensitivity within various genomic features throughout the genome in various genomic samples. Boxplots of genome-wide distributions of the entropic sensitivity index (ESI) for all genomic samples used in this study within CGIs, shores, shelves, open seas, TSSs, exons, introns, and intergenic regions. The boxes show the 25% quantile, the median, and the 75% quantile, whereas each whisker has a length of 1.5 × the interquartile range.

Figure 22 shows a wide behavior of entropic sensitivity in the genome. 22A: An example of ESI values in colon normal tissue shows wide-spread entropic sensitivity along the genome. However, unmethylated CGIs may exhibit low entropic sensitivity. *KLHL21* is a substrate-specific adapter of a BCR (BTB-CUL3-RBX1) E3 ubiquitin-protein ligase complex required for efficient chromosome alignment and cytokinesis. *PHF13* regulates chromatin structure. *THAP3* is required for regulation of *RRM1* that may play a role in malignancies and disease. 22B: In liver normal cells, substantial entropic sensitivity is observed within the CGI near the promoter of the polycomb target gene *ENSA,* which is significantly reduced in liver cancer. *ENSA* is known to be hypomethylated in liver cancer. 22C: In lung normal cells, the CGI near the promoter of *NEU1* exhibits low entropic sensitivity, which is significantly increased in lung cancer. *NEU1* sialidase is required for normal lung development and function, whereas its expression has been implicated in tumorigenesis and metastatic potential. 22D: In young CD4$^+$ lymphocytes, substantial entropic sensitivity is observed within the CGI near the promoter of *CYP2E1,* which is lost in old individuals. CYP2E1 is known to be downregulated with age. 22E: The CGI near the promoter of *FLNB* exhibits gain in entropic sensitivity in old CD4$^+$ lymphocytes. *FLNB* is known to be downregulated with age.

Figure 23 pertains to the breakdown of entropic sensitivity within compartments A and B in various genomic samples. Boxplots of genome-wide ESI distributions within compartment A (brown) and compartment B (blue) show that entropic sensitivity is higher within compartment A than within compartment B. The boxes show the 25% quantile, the median, and the 75% quantile, whereas each whisker has a length of 1. 5× the interquartile range.

**REFERENCES**

Bandopadhayay, P. et al. MYB-QKI rearrangements in angiocentric glioma drive tumorigenicity through a tripartite mechanism. Nat. Genet. 48, 273-282, doi:10.1038/ng.3500 (2016).

Baxter, R. J. Exactly Solved Models in Statistical Mechanics. Academic Press, doi: 10.1142/9789814415255_0002 (1982).

Bennet, C. H. The thermodynamics of computation - a review. Int. J. Theor. Phys. 21, 905-940, doi:10.1007/BF02084158 (1982).

Bergman, Y. & Cedar, H. DNA methylation dynamics in health and disease. Nat. Struct. Mol. Biol. 20, 274-281, doi:10.1038/nsmb.2518 (2013).

Berman, B. P. et al. Regions of focal DNA hypermethylation and long-range hypomethylation in colorectal cancer coincide with nuclear lamina-associated domains. Nat. Genet. 44, 40-46, doi:10.1038/ng.969 (2012).

Bickel, P. J. & Doksum, K. A. Mathematical Statistics: Basic Ideas and Selected Topics, Volume I. Prentice-Hall, doi: 10.2307/2286373 (2007).

Boyes, J. & Bird, A. Repression of genes by DNA methylation depends on CpG density and promoter strength: evidence for involvement of a methyl-CpG binding protein. EMBO J. 11, 327-333 (1992).

Cover, T. M. & Thomas, J.A. Elements of Information Theory. John Wiley & Sons, 10.1002/047174882X (2006).

de la Cruz, C. C. et al. The polycomb group protein SUZ12 regulates histone H3 lysine 9 methylation and HP1 alpha distribution. Chromosome Res. 15, 299-314, doi: 10.1007/s 10577-007-1126-1 (2007).

DeBaun, M. R. et al. Epigenetic alterations of H19 and LIT1 distinguish patients with Beckwith-Wiedemann syndrome with cancer and birth defects. Am. J. Hum. Genet. 70, 604-611, doi:10.1086/338934 (2002).

Dekker, J., Marti-Renom, M. A. & Mirny, L. A. Exploring the three-dimensional organization of genomes: interpreting chromatin interaction data. Nat. Rev. Genet. 14, 390-403, doi:10.1038/nrg3454 (2013).

Dixon, J. R. et al. Topological domains in mammalian genomes identified by analysis of chromatin interactions. Nature 485, 376-380, doi:10.1038/nature11082 (2012).

Dixon, J. R. et al. Chromatin architecture reorganization during stem cell differentiation. Nature 518, 331-336, doi:10.1038/nature14222 (2015).

Eden, E. et al. GOrilla: a tool for discovery and visualization of enriched GO terms in ranked gene lists. BMC Bioinformatics 10, 48, doi:10.1186/1471-2105-10-48 (2009).

Feng, F. et al. Genomic landscape of human allele-specific DNA methylation. Proc. Natl. Acad. Sci. USA, 109, 7332-7337 (2012).

Fashami, M. S., Atulasimha, J. & Bandyopadhyay, S. Energy dissipation and error probability in fault-tolerant binary switching. Sci. Rep. 3, 3204, doi:10.1038/srep03204 (2013).

Favorov, A. et al. Exploring massive, genome scale datasets with the GenometriCorr package. PLoS Comput. Biol. 8, e1002529, doi:10.1371/journal.pcbi. 1002529 (2012).

Fortin, J. P. & Hansen, K. D. Reconstructing A/B compartments as revealed by Hi-C using long-range correlations in epigenetic data. Genome Biol. 16, 180, doi:10.1186/sl3059-015-0741-y (2015).

Friel, N. & Rue, H. Recursive computing and simulation-free inference for general factorizable models. Biometrika, 94, 661-672, doi: 10.1093/biomet/asm052 (2007).

Fu, A. Q. et al. Statistical inference of transmission fidelity of DNA methylation patterns over somatic cell divisions in mammals. Ann. Appl. Stat. 4, 871-892, doi: 10.1214/09-AOAS297 (2010).

Fu, A. Q. et al. Statistical inference of in vivo properties of human DNA methyltransferases from double-stranded methylation patterns, PLoS One, 7, e32225, doi:10.1371/journal.pone.0032225 (2012).

Genereux, D. P. et al. A population-epigenetic model to infer site-specific methylation rates from double-stranded DNA methylation patterns, P. Natl. Acad. Sci. USA, 102, 16, 5802-5807, 10.1073/pnas.0502036102 (2005).

Gibcus, J. H. & Dekker, J. The hierarchy of the 3D genome. Mol. Cell 49, 773-782, doi:10.1016/j.molcel.2013.02.011 (2013).

Guelen, L. et al. Domain organization of human chromosomes revealed by mapping of nuclear lamina interactions. Nature 453, 948-951, doi:10.1038/nature06947 (2008).

Hansen, K. D. et al. Increased methylation variation in epigenetic domains across cancer types. Nat. Genet. 43, 768-775, doi:10.1038/ng.865 (2011).

Hansen, K. D. et al. Large-scale hypomethylated blocks associated with Epstein-Barr virus-induced B-cell immortalization. Genome Res. 24, 177-184, doi:10.1101/gr.157743.113 (2014).

Huang, J., Marco, E., Pinello, L. & Yuan, G. C. Predicting chromatin organization using histone marks. Genome Biol. 16, 162, doi:10.1186/s13059-O15-0740-z (2015).

Huyer, W. & Neumaier, A. Global optimization by multilevel coordinate search. J. Global Optim. 14, 331-355 (1999).

Illingworth, R. S. & Bird, A. P. CpG islands - 'A rough guide', FEBS Lett., 583, 1713-1720, doi 10.1016/j.febslet.2009.04.012 (2009).

Kaneda, H. et al. FOXQ1 is overexpressed in colorectal cancer and enhances tumorigenicity and tumor growth. Cancer Res. 70, 2053-2063, doi:10.1158/0008-5472.CAN-09-2161 (2010).

Kohli, R. M. & Zhang, Y. TET enzymes, TDG and the dynamics of DNA demethylation, Nature, 502, 472-479, doi:10.1038/nature12750 (2013).

Lacey, M. R. & Ehrlich, M. Modeling dependence in methylation patterns with application to ovarian carcinomas, Stat. Appl. Genet. M. B. 8, 40, doi:10.2202/1544-6115.1489 (2009).

Landan, G. et al. Epigenetic polymorphism and the stochastic formation of differentially methylated regions in normal and cancerous tissues. Nat. Genet. 44, 1207-1214, doi:10.1038/ng.2442 (2012).

Landauer, R. Uncertainty principle and minimal energy dissipation in the computer. Int. J. Theor. Phys. 21, 283-297, doi:10.1007/BF01857731 (1982).

Lewis, A. & Murrell, A. Genomic imprinting: CTCF protects the boundaries. Curr. Biol. 14, R284-286, doi:10.1016/j.cub.2004.03.026 (2004).

Li, S. et al. Dynamic evolution of clonal epialleles revealed by methclone. Genome Biol. 15, 472, doi:10.1186/s13059-014-0472-5 (2014).

Lin, J. Divergence measures based on the Shannon entropy. IEEE Trans. Inform. Theory 37, 145-151, doi: 10.1109/18.61115 (1991).

Mannens, M. et al. Positional cloning of genes involved in the Beckwith-Wiedemann syndrome, hemihypertrophy, and associated childhood tumors. Med. Pediatr. Oncol. 27, 490-494, doi:10.1002/(SICI)1096-911X(199611)27:5<490::AID-MPO17>3.0.CO;2-E (1996).

Margueron, R. & Reinberg, D. The Polycomb complex PRC2 and its mark in life. Nature 469, 343-349, doi:10.1038/nature09784 (2011).

Marvan, M. The energy dissipation, the error probability and the time of duration of a logical operation. Kybernetika, 18, 345-355, doi: 10.1038/srep03204 (1982).

Murtagh, F. & Legendre, P. Ward's hierarchical agglomerative clustering method: Which algorithms implement Ward's criterion? J. Classif. 31, 274-295, doi: 10.1007/s00357-014-9161-z (2014).

Nakamura, T. et al. Fusion of the nucleoporin gene NUP98 to HOXA9 by the chromosome translocation t(7;11)(p15;p15) in human myeloid leukaemia. Nat. Genet. 12, 154-158, doi:10.1038/ng0296-154 (1996).

Nora, E. P. et al. Spatial partitioning of the regulatory landscape of the X-inactivation centre. Nature 485, 381-385, doi:10.1038/nature11049 (2012).

Ogawa, O. et al. Relaxation of insulin-like growth factor II gene imprinting implicated in Wilms' tumour. Nature 362, 749-751, doi:10.1038/362749a0 (1993).

Peng, H. et al. LIMD2 is a small LIM-only protein overexpressed in metastatic lesions that regulates cell motility and tumor progression by directly binding to and activating the integrin-linked kinase. Cancer Res. 74, 1390-1403, doi:10.1158/0008-5472.CAN-13-1275 (2014).

Peters, M. J. et al. The transcriptional landscape of age in human peripheral blood. Nat Commun 6, 8570, doi:10.1038/ncomms9570 (2015).

Pfeifer, G. P. et al. Polymerase chain reaction-aided genomic sequencing of an X chromosome-linked CpG island: methylation patterns suggest clonal inheritance, CpG site autonomy, and an explanation of activity state stability, Proc. Natl. Acad. Sci. USA, 87, 8252-8256 (1990).

Press, W. H., Teukolsky, S.A., Vetterling, W.T. & Flannery, B.P. Numerical Recipes. The Art of Scientific Computing. Cambridge University Press, doi: 10.1145/1874391.187410 (2007).

Pujadas, E. & Feinberg, A. P. Regulated noise in the epigenetic landscape of development and disease. Cell 148, 1123-1131, doi:10.1016/j.cell.2012.02.045 (2012).

Rao, S. S. et al. A 3D map of the human genome at kilobase resolution reveals principles of chromatin looping. Cell 159, 1665-1680, doi:10.1016/j.cell.2014.11.021 (2014).

Reeves, R. & Pettit, A. N. Efficient recursions for general factorisable models. Biometrika, 91, 751-757, doi:10.1093/biomet/91.3.751 (2004).

Schlaeger, T. M. et al. A comparison of non-integrating reprogramming methods. Nat. Biotechnol. 33, 58-63, doi:10.1038/nbt.3070 (2015).

Shipony, Z. et al. Dynamic and static maintenance of epigenetic memory in pluripotent and somatic cells. Nature 513, 115-119, doi:10.1038/nature13458 (2014).

Sontag, L. B., Lorincz, M. C. & Luebeck, E. G. Dynamics, stability and inheritance of somatic DNA methylation imprints, J. Theor. Biol. 242, 890-899, doi:10.1016/j.jtbi.2006.05.012 (2006).

Stöger, R. et al. Epigenetic variation illustrated by DNA methylation patterns of the fragile-X gene FMR1, Hum. Mol. Genet., 6, 1791-1801, doi:10.1093/hmg/6.11.1791 (1997).

Storey, J. D. & Tibshirani, R. Statistical significance for genomewide studies. Proc. Natl. Acad. Sci. U. S. A. 100, 9440-9445, doi:10.1073/pnas.1530509100 (2003).

Timp, W. & Feinberg, A. P. Cancer as a dysregulated epigenome allowing cellular growth advantage at the expense of the host. Nat. Rev. Cancer 13, 497-510, doi:10.1038/nrc3486 (2013).

Timp, W. et al. Large hypomethylated blocks as a universal defining epigenetic alteration in human solid tumors. Genome Med. 6, 61, doi:10.1186/s13073-014-0061-y (2014).

Vandiver, A. R. et al. Age and sun exposure-related widespread genomic blocks of hypomethylation in nonmalignant skin. Genome Biol. 16, 80, doi:10.1186/s13059-015-0644-y (2015).

Visel, A., Minovitsky, S., Dubchak, I. & Pennacchio, L. A. VISTA Enhancer Browser - a database of tissue-specific human enhancers. Nucleic Acids Res. 35, D88-92, doi:10.1093/nar/gkl822 (2007).

Waddington, C. H. The strategy of genes. Allen and Unwin (1957).

Wen, B. et al. Large histone H3 lysine 9 dimethylated chromatin blocks distinguish differentiated from embryonic stem cells. Nat. Genet. 41, 246-250, doi:10.1038/ng.297 (2009).

Wen, B. et al. Euchromatin islands in large heterochromatin domains are enriched for CTCF binding and differentially DNA-methylated regions. BMC Genomics 13, 566, doi:10.1186/1471-2164-13-566 (2012).

Wu, H. et al. Redefining CpG islands using hidden Markov models. Biostatistics 11, 499-514, doi:10.1093/biostatistics/kxq005 (2010).

Yamamoto, K. et al. Polycomb group suppressor of zeste 12 links heterochromatin protein 1 alpha and enhancer of zeste 2. J. Biol. Chem. 279, 401-406, doi:10.1074/jbc.M307344200 (2004).

Yang, G. et al. RNA-binding protein quaking, a critical regulator of colon epithelial differentiation and a suppressor of colon cancer. Gastroenterology 138, 231-240 e231-235, doi:10.1053/j.gastro.2009.08.001 (2010).

Yu, H. et al. Tet3 regulates synaptic transmission and homeostatic plasticity via DNA oxidation and repair. Nat. Neurosci. 18, 836-843, doi:10.1038/nn.4008 (2015).

Ziller, M. J. et al. Charting a dynamic DNA methylation landscape of the human genome. Nature 500, 477-481, doi:10.1038/nature12433 (2013).

## SUPPLEMENTARY TABLES

SUPPLEMENTARY TABLE 1

Supplementary Table 1 provides a list of all WGBS genomic samples used in this disclosure.

| NICKNAME | MATCHED | SAMPLE TYPE | SOURCE[1] | COVERAGE |
|---|---|---|---|---|
| **Stem Cells** | | | | |
| stem | | H1 human embryonic stem cell line | [1], SRP072141[2] | 24 |
| **Normal / Cancer** | | | | |
| colonnormal | 1 | colon normal | [2] | 30 |
| coloncancer | 1 | colon cancer | [2] | 30 |
| livernormal-1 | 2 | liver normal | SRP072078 | 9 |
| livercancer-1 | 2 | liver cancer | SRP072078 | 8 |
| livernormal-2 | 3 | liver normal | SRP072078 | 7 |
| livercancer-2 | 3 | liver cancer | SRP072078 | 8 |
| livernormal-3 | 4 | liver normal | SRP072078 | 18 |
| livercancer-3 | 4 | liver cancer | SRP072078 | 18 |
| livernormal-4 | | liver normal | [2] | 60 |
| livernormal-5 | | liver normal | [2] | 41 |
| lungnormal-1 | 5 | lung normal | SRP072078 | 14 |
| lungcancer-1 | 5 | lung cancer | SRP072078 | 15 |
| lungnormal-2 | 6 | lung normal | SRP072078 | 10 |
| lungcancer-2 | 6 | lung cancer | SRP072078 | 10 |
| lungnormal-3 | 7 | lung normal | SRP072078 | 19 |
| lungcancer-3 | 7 | lung cancer | SRP072078 | 18 |
| brain-1 | | post-mortem brain, pre-frontal cortex, normal | SRP072071 | 11 |
| brain-2 | | post-mortem brain, pre-frontal cortex, normal | SRP072071 | 12 |
| **HNF Fibroblasts** | | | | |
| fibro-P4 | | human neonatal fibroblasts, passage 4 | SRP072075 | 12 |
| fibro-P7 | | human neonatal fibroblasts, passage 7 | SRP072075 | 11 |
| fibro-P10 | | human neonatal fibroblasts, passage 10 | SRP072075 | 11 |
| fibro-P31 | | human neonatal fibroblasts, passage 31 | SRP072075 | 11 |
| fibro-P33 | | human neonatal fibroblasts, passage 33, senescent | SRP072075 | 11 |
| **CD4 T-Cells** | | | | |
| CD4-Y1 | | flow-sorted peripheral CD4 T-cells from an 18 year old female | SRP072075 | 8 |
| CD4-Y2 | | flow-sorted peripheral CD4 T-cells from a 25 year old female | SRP072075 | 8 |
| CD4-Y3 | | flow-sorted peripheral CD4 T-cells from a 25 year old female | SRP072075 | 7 |
| CD4-O1 | | flow-sorted peripheral CD4 T-cells from an 82 year old female | SRP072075 | 7 |
| CD4-O2 | | flow-sorted peripheral CD4 T-cells from an 82 year old female | SRP072075 | 8 |
| CD4-O3 | | flow-sorted peripheral CD4 T-cells from an 86 year old female | SRP072075 | 7 |
| **Keratinocytes** | | | | |
| ker-Y1 | | keratinocytes from a skin biopsy of a sun-protected site on a young individual | [3] | 8 |
| ker-Y2 | | keratinocytes from a skin biopsy of a sun-protected site on a young individual | [3] | 8 |
| ker-O1 | | keratinocytes from a skin biopsy of a sun-exposed site on an older individual | [3] | 7 |
| ker-O2 | | keratinocytes from a skin biopsy of a sun-exposed site on an older individual | [3] | 7 |
| **EBV** | | | | |
| EBV | | EBV-immortalized lymphoblasts | [4] | 9 |

[1]SRP accessions correspond to NCBI Sequencing Read Archive (SRA).

[2]Original sequence along with additional coverage have been deposited in the reference SRP accession.

REFERENCES
[1] Schlaeger TM, Daheron L, Brickler TR, et al. A comparison of non-integrating reprogramming methods. Nat Biotechnol. 33(1):58-63 (2015).
[2] Ziller MJ, Gu H, Müller F, et al. Charting a dynamic DNA methylation landscape of the human genome. Nature 500(7463):477-81 (2013).
[3] Vandiver AR, Irizarry RA, Hansen KD, et al. Age and sun exposure-related widespread genomic blocks of hypomethylation in nonmalignant skin. Genome Biol. 16:80 (2015).
[4] Hansen KD, Sabunciyan S, Langmead B, et al. Large-scale hypomethylated blocks associated with Epstein-Barr virus-induced B-cell immortalization. Genome Res. 24(2):177-84 (2014).

SUPPLEMENTARY TABLE 2

Supplementary Table 2 provides the results of statistical analysis for EZH2/SUZ 12 binding association with promoters and enhancers at genomic loci characterized by high Jensen-Shannon distance (JSD).

| FISHER'S EXACT TEST FOR COUNT DATA | | | | | | | | | | | |
|---|---|---|---|---|---|---|---|---|---|---|---|
| PROMOTERS | | | | | | | | | | | |
| | | EZH2 | | | | | SUZ12 | | | | |
| criterion | # genes | present | absent | frequency | P value | odds ratio | present | absent | frequency | P value | odds ratio |
| dMML | top 1000 | 305 | 695 | 31% | < 2.2E-16 | 2.69 | 94 | 906 | 9% | 2.05E-05 | 2.20 |
| | bottom 1000 | 140 | 860 | 14% | | | 45 | 955 | 5% | | |
| JSD | top 1000 | 457 | 543 | 46% | < 2.2E-16 | 7.57 | 191 | 809 | 19% | < 2.2E-16 | 8.84 |
| | bottom 1000 | 100 | 900 | 10% | | | 26 | 974 | 3% | | |
| ENHANCERS | | | | | | | | | | | |
| | | EZH2 | | | | | SUZ12 | | | | |
| criterion | # genes | present | absent | frequency | P value | odds ratio | present | absent | frequency | P value | odds ratio |
| dMML | top 100 | 42 | 58 | 42% | 7.24E-13 | 34.95 | 29 | 71 | 29% | 6.20E-09 | 39.92 |
| | bottom 100 | 2 | 98 | 2% | | | 1 | 99 | 1% | | |
| JSD | top 100 | 53 | 47 | 53% | < 2.2E-16 | 109.49 | 40 | 60 | 40% | 1.34E-14 | infinite |
| | bottom 100 | 1 | 99 | 1% | | | 0 | 100 | 0% | | |

| BINOMIAL LOGISTIC REGRESSION | | | | | | | | | |
|---|---|---|---|---|---|---|---|---|---|
| PROMOTERS | | | | | | | | | |
| | | EZH2 | | | | SUZ12 | | | |
| | | coefficient | std error | P value | holdout accuracy* | coefficient | std error | P value | holdout accuracy* |
| JSD | intercept | -2.4030 | 0.0395 | < 2.2E-16 | 82% | -3.9217 | 0.0638 | < 2.2E-16 | 95% |
| | score | 5.5511 | 0.1991 | < 2.2E-16 | | 6.1825 | 0.2760 | < 2.2E-16 | |
| ENHANCERS | | | | | | | | | |
| | | EZH2 | | | | SUZ12 | | | |
| | | coefficient | std error | P value | holdout accuracy* | coefficient | std error | P value | holdout accuracy* |
| JSD | intercept | -4.3962 | 0.2914 | < 2.2E-16 | 88% | -6.4587 | 0.5133 | < 2.2E-16 | 93% |
| | score | 18.1070 | 1.7861 | < 2.2E-16 | | 23.0143 | 2.4591 | < 2.2E-16 | |

*90% of data was randomly selected for training, while the remaining was used for estimating performance.

SUPPLEMENTARY TABLE 3

Supplementary Table 3 provides the results of odds ratio (OR) analysis of bistability enrichment in CGIs, shores, promoters, and gene bodies.

| SAMPLE | CGIs | | SHORES | | PROMOTERS | | GENE BODIES | |
|---|---|---|---|---|---|---|---|---|
| | OR | P value | OR | P value | OR | P value | OR | P value |
| stem | 1.03 | 5.19E-01 | 4.34 | 0.00E+00 | 4.22 | 0.00E+00 | 0.90 | 3.06E-14 |
| colonnormal | 0.41 | 4.26E-190 | 1.54 | 0.00E+00 | 1.69 | 0.00E+00 | 0.72 | 0.00E+00 |
| coloncancer | 0.26 | 0.00E+00 | 0.94 | 1.21E-21 | 0.90 | 9.45E-42 | 0.63 | 0.00E+00 |
| livernormal-1 | 0.25 | 0.00E+00 | 1.19 | 1.22E-78 | 1.17 | 3.74E-51 | 0.67 | 0.00E+00 |
| livercancer-1 | 0.23 | 0.00E+00 | 1.30 | 4.20E-166 | 1.21 | 1.34E-62 | 0.84 | 1.43E-158 |
| livernormal-2 | 0.24 | 0.00E+00 | 1.17 | 2.12E-58 | 1.11 | 5.08E-21 | 0.68 | 0.00E+00 |
| livercancer-2 | 0.30 | 0.00E+00 | 1.28 | 1.01E-214 | 1.06 | 1.68E-09 | 0.74 | 0.00E+00 |
| livernormal-3 | 0.26 | 0.00E+00 | 1.28 | 1.73E-143 | 1.24 | 1.66E-83 | 0.71 | 0.00E+00 |
| livercancer-3 | 0.38 | 1.03E-249 | 1.42 | 1.58E-306 | 1.43 | 1.57E-253 | 0.76 | 0.00E+00 |
| livernormal-4 | 0.44 | 1.25E-145 | 1.64 | 0.00E+00 | 1.92 | 0.00E+00 | 0.81 | 9.69E-172 |
| livernormal-5 | 0.49 | 3.51E-120 | 2.01 | 0.00E+00 | 2.24 | 0.00E+00 | 0.89 | 1.46E-59 |
| lungnormal-1 | 0.35 | 9.42E-219 | 1.77 | 0.00E+00 | 1.70 | 0.00E+00 | 0.83 | 3.26E-153 |
| lungcancer-1 | 0.25 | 0.00E+00 | 1.10 | 5.33E-50 | 0.78 | 2.70E-189 | 0.60 | 0.00E+00 |
| lungnormal-2 | 0.34 | 1.47E-219 | 1.68 | 0.00E+00 | 1.64 | 0.00E+00 | 0.84 | 2.50E-125 |
| lungcancer-2 | 0.21 | 0.00E+00 | 1.15 | 3.64E-57 | 1.10 | 2.17E-19 | 0.70 | 0.00E+00 |
| lungnormal-3 | 0.39 | 2.38E-176 | 1.80 | 0.00E+00 | 1.73 | 0.00E+00 | 0.89 | 2.47E-54 |
| lungcancer-3 | 0.23 | 0.00E+00 | 0.97 | 9.14E-07 | 0.70 | 0.00E+00 | 0.62 | 0.00E+00 |
| brain-1 | 1.06 | 7.62E-02 | 3.46 | 0.00E+00 | 3.27 | 0.00E+00 | 1.45 | 6.95E-293 |
| brain-1 | 1.07 | 3.36E-02 | 3.48 | 0.00E+00 | 3.39 | 0.00E+00 | 1.38 | 7.61E-217 |
| fibro-P4 | 0.20 | 0.00E+00 | 0.89 | 3.23E-41 | 0.84 | 6.04E-67 | 0.59 | 0.00E+00 |
| fibro-P7 | 0.19 | 0.00E+00 | 0.81 | 1.15E-147 | 0.76 | 2.39E-184 | 0.57 | 0.00E+00 |
| fibro-P10 | 0.18 | 0.00E+00 | 0.81 | 2.02E-151 | 0.74 | 9.99E-218 | 0.57 | 0.00E+00 |
| fibro-P31 | 0.27 | 0.00E+00 | 1.15 | 3.15E-93 | 0.89 | 1.19E-39 | 0.68 | 0.00E+00 |
| fibro-P33 | 0.27 | 0.00E+00 | 1.18 | 1.46E-114 | 0.91 | 3.21E-24 | 0.68 | 0.00E+00 |
| CD4-Y1 | 1.26 | 6.01E-10 | 2.84 | 0.00E+00 | 2.93 | 0.00E+00 | 1.04 | 1.43E-03 |
| CD4-Y2 | 1.17 | 2.62E-05 | 2.71 | 0.00E+00 | 2.74 | 0.00E+00 | 1.00 | 9.26E-01 |
| CD4-Y3 | 0.89 | 1.50E-03 | 2.50 | 0.00E+00 | 2.52 | 0.00E+00 | 1.11 | 2.82E-27 |
| CD4-O1 | 0.68 | 1.46E-25 | 1.68 | 0.00E+00 | 1.83 | 0.00E+00 | 0.77 | 4.72E-200 |
| CD4-O2 | 0.94 | 1.41E-01 | 2.18 | 0.00E+00 | 2.25 | 0.00E+00 | 0.85 | 4.23E-61 |
| CD4-O3 | 0.93 | 8.54E-02 | 2.01 | 0.00E+00 | 2.11 | 0.00E+00 | 0.84 | 1.76E-76 |
| ker-Y1 | 0.63 | 3.54E-48 | 2.04 | 0.00E+00 | 1.93 | 0.00E+00 | 0.94 | 1.90E-15 |
| ker-Y2 | 0.66 | 4.17E-36 | 2.05 | 0.00E+00 | 1.90 | 0.00E+00 | 0.94 | 3.53E-16 |
| ker-O1 | 0.61 | 6.39E-53 | 1.82 | 0.00E+00 | 1.65 | 0.00E+00 | 0.86 | 2.62E-112 |
| ker-O2 | 0.40 | 1.92E-212 | 1.39 | 0.00E+00 | 1.22 | 5.98E-84 | 0.72 | 0.00E+00 |

| |
|---|
| depletion: OR < 1 |
| enrichment: OR > 1 |

## SUPPLEMENTARY DATA

SUPPLEMENTARY DATA 1

Supplementary Data 1 provides gene rankings for some genomic sample pairs based on the magnitude of the differential methylation level (dMML), the Jensen-Shannon distance (JSD), and the relative Jensen-Shannon distance (RJSD). Supplementary Data 1 as attached hereto includes a portion of the collective data set as a representative sample.

Supplementary Data 1

stem-VS-brain-1

| dMML MAGNITUDE RANKING | |
|---|---|
| GENE | SCORE |
| CBLN2 | 0.6661 |
| HIST1H2BB | 0.6359 |
| PRR34 | 0.6052 |
| POU5F1 | 0.5891 |
| PRR34-AS1 | 0.5816 |
| MIRLET7BHG | 0.5816 |
| SCNN1A | 0.5735 |
| LTBR | 0.5609 |
| HIST1H3C | 0.5573 |
| CBLN4 | 0.5326 |
| ESRG | 0.5209 |
| IFFO1 | 0.5172 |
| TDGF1 | 0.5155 |
| DPPA4 | 0.5151 |
| NR4A2 | 0.5143 |
| DMRT2 | 0.4915 |
| VRTN | 0.4826 |
| VMO1 | 0.4629 |
| EDNRB | 0.4626 |
| NBAT1 | 0.4593 |
| ANKRD20A8P | 0.4542 |
| NCOR2 | 0.4536 |
| MIR663B | 0.4517 |
| LINC00678 | 0.4459 |
| RNF219-AS1 | 0.4456 |
| MIR3619 | 0.4376 |
| PCDHGA12 | 0.4328 |
| PCDHGA7 | 0.4319 |
| ANKRD30BL | 0.4316 |
| PLAGL1 | 0.4313 |
| VPS37B | 0.4306 |
| LNX1 | 0.4233 |
| PCDHGA5 | 0.4204 |
| LRBA | 0.4202 |
| CFLAR | 0.4152 |
| CASC15 | 0.4146 |
| MAB21L2 | 0.4143 |
| MIR302B | 0.4128 |
| NR1D1 | 0.4095 |
| USP44 | 0.4086 |
| LNX1-AS2 | 0.4023 |
| CCK | 0.4014 |
| PCDHGA6 | 0.4008 |
| TCF4 | 0.4000 |
| MCF2L | 0.3973 |
| PCDHGA11 | 0.3947 |
| NANOG | 0.3947 |
| NBEA | 0.3933 |
| BRSK2 | 0.3921 |
| MEG3 | 0.3915 |
| MT1H | 0.3905 |
| CYP2E1 | 0.3859 |
| MIR99AHG | 0.3823 |
| MT1G | 0.3823 |
| PRKCZ | 0.3808 |
| ZIC4 | 0.3796 |
| ZFHX3 | 0.3775 |
| HSPA2 | 0.3723 |
| GPM6A | 0.3713 |
| LOC100132215 | 0.3708 |
| PCDHGB3 | 0.3702 |
| MAL | 0.3654 |
| CYP26C1 | 0.3654 |
| MIR4321 | 0.3638 |
| IFITM1 | 0.3627 |

| JSD RANKING | |
|---|---|
| GENE | SCORE |
| CBLN2 | 0.8195 |
| DMRT2 | 0.7720 |
| HIST1H2BB | 0.7265 |
| LRBA | 0.7213 |
| PRR34 | 0.7209 |
| ZIC4 | 0.7144 |
| MAB21L2 | 0.7131 |
| MIRLET7BHG | 0.6975 |
| PRR34-AS1 | 0.6940 |
| POU5F1 | 0.6893 |
| RNF157-AS1 | 0.6775 |
| LOC100132215 | 0.6775 |
| CBLN4 | 0.6667 |
| NR4A2 | 0.6659 |
| LINC00273 | 0.6567 |
| ESRRG | 0.6565 |
| DPPA4 | 0.6555 |
| HIST1H3C | 0.6540 |
| MAL | 0.6529 |
| SCNN1A | 0.6502 |
| MIR663B | 0.6439 |
| LTBR | 0.6405 |
| HMGA1 | 0.6399 |
| FAM182B | 0.6390 |
| VMO1 | 0.6382 |
| NBEA | 0.6310 |
| TDGF1 | 0.6301 |
| IFFO1 | 0.6228 |
| PCDHGA11 | 0.6207 |
| MIR3619 | 0.6192 |
| PCDHGA6 | 0.6189 |
| GRHL2 | 0.6177 |
| MIR4321 | 0.6126 |
| PCDHGA12 | 0.6105 |
| NCOR2 | 0.6087 |
| ESRG | 0.6030 |
| EVA1B | 0.5956 |
| LINGO3 | 0.5946 |
| MEG3 | 0.5917 |
| FBP1 | 0.5915 |
| MT1G | 0.5913 |
| MT1H | 0.5887 |
| BRSK2 | 0.5879 |
| ANKRD20A8P | 0.5843 |
| CFLAR | 0.5838 |
| ANKRD30BL | 0.5831 |
| NR2F1-AS1 | 0.5822 |
| PCDHGA5 | 0.5819 |
| MOB3A | 0.5807 |
| WNT3 | 0.5786 |
| CCDC8 | 0.5786 |
| CCK | 0.5780 |
| MCF2L | 0.5766 |
| HSPA2 | 0.5762 |
| NKX6-2 | 0.5755 |
| PCDHGA7 | 0.5729 |
| IGF2BP3 | 0.5718 |
| LOC146880 | 0.5717 |
| CYP26C1 | 0.5707 |
| PRKCDBP | 0.5674 |
| PRKCZ | 0.5653 |
| VPS37B | 0.5636 |
| FOXJ1 | 0.5635 |
| OLIG2 | 0.5616 |
| REREP3 | 0.5607 |

| RJSD RANKING | | | |
|---|---|---|---|
| GENE | SCORE | JSD RANK | dMMLRANK |
| IGF2BP1 | 51.2500 | 76 | 3895 |
| FOXD3 | 48.6782 | 87 | 4235 |
| NKX6-2 | 44.9091 | 55 | 2470 |
| IRX1 | 38.0657 | 213 | 8108 |
| SALL1 | 26.3215 | 339 | 8923 |
| TMEM200B | 25.1618 | 198 | 4986 |
| SP9 | 22.1877 | 261 | 5791 |
| MAPT-IT1 | 21.6115 | 659 | 14242 |
| EPHA4 | 21.1444 | 630 | 13321 |
| MAPT-AS1 | 20.4378 | 699 | 14286 |
| NOTUM | 19.4537 | 335 | 6517 |
| ASCL2 | 18.8623 | 167 | 3150 |
| SIM2 | 17.1703 | 822 | 14114 |
| EMX1 | 14.9250 | 1080 | 16119 |
| IGF2BP3 | 14.8772 | 57 | 848 |
| SPHK1 | 14.5865 | 416 | 6068 |
| GAD2 | 14.4898 | 737 | 10679 |
| RHEB | 14.4653 | 144 | 2083 |
| PRDM14 | 14.4394 | 66 | 953 |
| SFMBT2 | 14.3053 | 819 | 11716 |
| GATA3 | 13.8601 | 193 | 2675 |
| HMGA1 | 13.7391 | 23 | 316 |
| FEZF1 | 13.6605 | 1128 | 15409 |
| OTX1 | 13.5261 | 211 | 2854 |
| IFT140 | 13.1738 | 604 | 7957 |
| TBX3 | 12.7792 | 308 | 3936 |
| MAPT | 12.6327 | 972 | 12279 |
| GATA3-AS1 | 12.4153 | 850 | 10553 |
| TFAP2A-AS1 | 11.9510 | 817 | 9764 |
| SOX11 | 11.6239 | 1396 | 16227 |
| SP5 | 11.5669 | 628 | 7264 |
| TYMP | 11.1955 | 220 | 2463 |
| NRN1 | 11.0378 | 687 | 7583 |
| ADM | 10.9767 | 473 | 5192 |
| SCAF11 | 10.9328 | 119 | 1301 |
| STK3 | 10.9103 | 1159 | 12645 |
| KCTD1 | 10.8750 | 224 | 2436 |
| LHX1 | 10.3254 | 1755 | 18121 |
| EP400NL | 10.2000 | 450 | 4590 |
| BTBD6 | 10.1629 | 706 | 7175 |
| FZD2 | 9.9634 | 629 | 6267 |
| TRIM71 | 9.9630 | 81 | 807 |
| GCGR | 9.7845 | 348 | 3405 |
| LINC01124 | 9.4290 | 345 | 3253 |
| ZIC4 | 9.3333 | 6 | 56 |
| CCDC85C | 9.3300 | 1009 | 9414 |
| WNT3A | 9.0162 | 1299 | 11712 |
| ZNF503-AS2 | 8.7905 | 210 | 1846 |
| DPYSL4 | 8.6608 | 171 | 1481 |
| OLIG2 | 8.5156 | 64 | 545 |
| LRBA | 8.5000 | 4 | 34 |
| HAND1 | 8.4222 | 2044 | 17215 |
| IRX5 | 8.2521 | 2313 | 19087 |
| RTN4RL1 | 8.1222 | 1857 | 15083 |
| DMRT2 | 8.0000 | 2 | 16 |
| ZNF580 | 7.9720 | 107 | 853 |
| NR2E1 | 7.9583 | 575 | 4576 |
| FOXB1 | 7.9190 | 2197 | 17398 |
| RNF157-AS1 | 7.9091 | 11 | 87 |
| BHLHE22 | 7.8863 | 255 | 2011 |
| EVA1B | 7.8378 | 37 | 290 |
| BCL2L11 | 7.6738 | 2109 | 16184 |
| RFX2 | 7.6473 | 1219 | 9322 |
| ZBTB21 | 7.6318 | 1043 | 7960 |
| DNAJB6 | 7.4612 | 644 | 4805 |

| | | | | | | | |
|---|---|---|---|---|---|---|---|
| TNK2 | 0.3627 | PRDM14 | 0.5573 | ESRRG | 7.4375 | 16 | 119 |
| PCDHA7 | 0.3620 | SFRP2 | 0.5571 | OLIG3 | 7.4286 | 658 | 4888 |
| MIR219A2 | 0.3568 | PCDHA3 | 0.5534 | ID4 | 7.3987 | 1555 | 11505 |
| PCDHA3 | 0.3558 | EDNRB | 0.5519 | SHOX2 | 7.3598 | 895 | 6587 |
| PCDHA9 | 0.3558 | ZNF667-AS1 | 0.5509 | FEZF1-AS1 | 7.3144 | 1937 | 14168 |
| PCDHGA3 | 0.3557 | ZFP42 | 0.5508 | TFAP2A | 7.3066 | 212 | 1549 |
| WNK4 | 0.3543 | MIR1225 | 0.5506 | SNTG2 | 7.2771 | 249 | 1812 |
| MIR219B | 0.3538 | PAX8 | 0.5483 | MDFI | 7.2674 | 172 | 1250 |
| TOLLIP | 0.3537 | MIR219A2 | 0.5447 | HIST1H2BI | 7.2548 | 1711 | 12413 |
| FAM182B | 0.3534 | MIR219B | 0.5414 | RGS20 | 7.2424 | 1415 | 10248 |
| ELAVL4 | 0.3534 | IGF2BP1 | 0.5401 | CXXC5 | 7.2031 | 586 | 4221 |
| PUF60 | 0.3531 | LBX2-AS1 | 0.5381 | MIR3621 | 7.1991 | 221 | 1591 |
| RGS12 | 0.3530 | VRTN | 0.5369 | ADRB1 | 7.1416 | 1695 | 12105 |
| SNORA63 | 0.3504 | PCDHA7 | 0.5349 | TWIST2 | 7.1124 | 169 | 1202 |
| MT1JP | 0.3504 | RNF219-AS1 | 0.5338 | BARHL2 | 7.0523 | 2140 | 15092 |
| NLRP6 | 0.3501 | TRIM71 | 0.5323 | SIX5 | 7.0361 | 1633 | 11490 |
| SEPT7P9 | 0.3480 | PAX6 | 0.5299 | NAAA | 7.0200 | 1551 | 10888 |
| MIR135B | 0.3479 | ZNF667 | 0.5286 | CALY | 7.0198 | 252 | 1769 |
| LINC00273 | 0.3467 | FSTL3 | 0.5279 | FAM84B | 6.9475 | 1143 | 7941 |
| GRAMD1B | 0.3428 | WNT5A | 0.5238 | EBF3 | 6.7637 | 986 | 6669 |
| ZNF257 | 0.3425 | RXFP3 | 0.5235 | ODF3B | 6.7338 | 710 | 4781 |
| RNF157-AS1 | 0.3417 | FOXD3 | 0.5233 | FENDRR | 6.6986 | 2492 | 16693 |
| CD14 | 0.3401 | C9orf172 | 0.5233 | KDM2B | 6.6380 | 489 | 3246 |
| MIR200C | 0.3401 | ACTB | 0.5228 | EOMES | 6.5745 | 1692 | 11124 |
| KRT8 | 0.3372 | KRT18 | 0.5219 | SP8 | 6.5655 | 267 | 1753 |
| FSTL3 | 0.3368 | WNK4 | 0.5205 | CEP131 | 6.5401 | 548 | 3584 |
| ZNF492 | 0.3365 | LBX2 | 0.5188 | ALX1 | 6.4452 | 1406 | 9062 |
| LINGO3 | 0.3363 | NBAT1 | 0.5188 | ACTG1 | 6.4416 | 1909 | 12297 |
| TEF | 0.3362 | PCDHGB3 | 0.5183 | BHLHE23 | 6.4252 | 1933 | 12420 |
| PRKCDBP | 0.3360 | CACNA1B | 0.5182 | RAP1B | 6.3833 | 1054 | 6728 |
| PCDHGB5 | 0.3338 | NR1D1 | 0.5170 | BCL7A | 6.3144 | 792 | 5001 |
| NR2F1-AS1 | 0.3331 | ABHD14A | 0.5168 | CBFA2T3 | 6.2939 | 296 | 1863 |
| ERF | 0.3326 | ABHD14A-ACY1 | 0.5168 | PTTG1IP | 6.2000 | 960 | 5952 |
| ACTB | 0.3325 | LNX1 | 0.5153 | SH3RF3 | 6.1944 | 540 | 3345 |
| MIR4726 | 0.3318 | OTX2 | 0.5144 | GRHL2 | 6.1875 | 32 | 198 |
| SNORA81 | 0.3304 | TNK2 | 0.5139 | CLDN7 | 6.1673 | 1470 | 9066 |
| TREX1 | 0.3298 | APC2 | 0.5134 | UAP1L1 | 6.1600 | 125 | 770 |
| MLLT6 | 0.3288 | GRIN1 | 0.5089 | KCNA4 | 6.1531 | 2573 | 15832 |
| PCDHGA9 | 0.3288 | ABCA3 | 0.5080 | DMRTA2 | 6.1395 | 215 | 1320 |
| CRYAB | 0.3288 | ABCA17P | 0.5070 | NKX3-2 | 6.0846 | 1064 | 6474 |
| ZFP42 | 0.3275 | MT1JP | 0.5055 | RBM38 | 6.0547 | 128 | 775 |
| KRT18 | 0.3274 | ZNF580 | 0.5051 | NAGS | 6.0380 | 1132 | 6835 |
| CMAHP | 0.3273 | ABHD14B | 0.5042 | HIC1 | 6.0327 | 2905 | 17525 |
| SNORA4 | 0.3272 | SIX3 | 0.5039 | MTA3 | 5.9624 | 1570 | 9361 |
| ZNF729 | 0.3268 | RXRA | 0.5039 | ADRA2A | 5.9586 | 145 | 864 |
| ACAP3 | 0.3265 | MIR124-2 | 0.5028 | SSBP4 | 5.9551 | 847 | 5044 |
| RXFP3 | 0.3262 | HLX | 0.5011 | MRGBP | 5.9332 | 2397 | 14222 |
| HTR2A | 0.3261 | LTBP4 | 0.5008 | COL26A1 | 5.9108 | 975 | 5763 |
| LHX8 | 0.3260 | MT1L | 0.5007 | POU3F1 | 5.9000 | 2000 | 11800 |
| ZNF454 | 0.3258 | MT1M | 0.5007 | ST3GAL1 | 5.8986 | 2565 | 15130 |
| APC2 | 0.3239 | ZFHX3 | 0.4991 | TGFBR3L | 5.8760 | 484 | 2844 |
| MT1L | 0.3237 | ZNF596 | 0.4986 | GATA6-AS1 | 5.8753 | 2774 | 16298 |
| MT1M | 0.3237 | KLHDC7B | 0.4982 | EPCAM | 5.8364 | 330 | 1926 |
| ESRRG | 0.3237 | SCAF11 | 0.4972 | TMEM132E | 5.7947 | 1807 | 10471 |
| PLD6 | 0.3224 | CD70 | 0.4968 | DKK2 | 5.7726 | 853 | 4924 |
| MIR141 | 0.3203 | PCDHGB5 | 0.4965 | TET1 | 5.7606 | 2318 | 13353 |
| IRF2BP2 | 0.3199 | LNX1-AS2 | 0.4951 | MSC-AS1 | 5.7479 | 238 | 1368 |
| LOC440040 | 0.3199 | ZADH2 | 0.4939 | MFSD10 | 5.7232 | 625 | 3577 |
| PCDHA1 | 0.3187 | SRCIN1 | 0.4939 | LINC00577 | 5.6996 | 2550 | 14534 |
| RNF216 | 0.3172 | UAP1L1 | 0.4936 | CELSR3 | 5.6743 | 1888 | 10713 |
| ZNF439 | 0.3172 | ACAP3 | 0.4936 | WNT7B | 5.6715 | 627 | 3556 |
| TTYH1 | 0.3170 | ZNF454 | 0.4927 | PCDH8 | 5.6320 | 2701 | 15212 |
| SFRP2 | 0.3153 | RBM38 | 0.4916 | LINC00273 | 5.6000 | 15 | 84 |
| MIR1225 | 0.3147 | RPL23AP53 | 0.4912 | ABHD14B | 5.5926 | 108 | 604 |
| ZNF667-AS1 | 0.3146 | SEPT7P9 | 0.4904 | SLC1A4 | 5.5869 | 1973 | 11023 |
| PCDHGA8 | 0.3146 | PCDHA1 | 0.4900 | HIST1H2AG | 5.5788 | 3305 | 18438 |
| LINC01132 | 0.3139 | TOLLIP | 0.4888 | FGF19 | 5.5758 | 679 | 3786 |
| PHACTR3 | 0.3136 | CCDC166 | 0.4887 | TBCD | 5.5609 | 665 | 3698 |
| HIST2H2BA | 0.3133 | TFAP2E | 0.4875 | FOXJ1 | 5.5556 | 63 | 350 |

| | | | | | | | |
|---|---|---|---|---|---|---|---|
| TBC1D16 | 0.3125 | NANOG | 0.4866 | SOX2 | 5.5398 | 3592 | 19899 |
| CPEB4 | 0.3122 | PCDHA6 | 0.4864 | RTKN | 5.5380 | 303 | 1678 |
| PCDHB18P | 0.3114 | LTBP3 | 0.4860 | PHPT1 | 5.5288 | 1303 | 7204 |
| MOS | 0.3108 | PPP1R3B | 0.4858 | NR2F6 | 5.5174 | 1720 | 9490 |
| LRRC4C | 0.3106 | PCDHGA3 | 0.4854 | ABHD14A | 5.4948 | 97 | 533 |
| REM1 | 0.3105 | RGS14 | 0.4852 | LOC100505666 | 5.4925 | 201 | 1104 |
| ZNF596 | 0.3095 | ADGRA1 | 0.4828 | WDR34 | 5.4829 | 1226 | 6722 |
| LOC441666 | 0.3095 | ESRP2 | 0.4825 | OBSCN | 5.4555 | 584 | 3186 |
| ABR | 0.3093 | TMEM121 | 0.4824 | ABHD14A-ACY1 | 5.4490 | 98 | 534 |
| TUBBP5 | 0.3091 | RHEB | 0.4812 | OSR2 | 5.4456 | 1122 | 6110 |
| MAP2K3 | 0.3086 | ADRA2A | 0.4805 | RUNX3 | 5.4178 | 943 | 5109 |
| RXRA | 0.3084 | MAFK | 0.4805 | TRIM67 | 5.4164 | 353 | 1912 |
| LOC100287846 | 0.3082 | PCDHA9 | 0.4801 | C7orf50 | 5.3852 | 283 | 1524 |
| NR3C1 | 0.3081 | NDUFA4L2 | 0.4799 | KIAA0753 | 5.3514 | 1201 | 6427 |
| PCDHGB1 | 0.3076 | CYP2E1 | 0.4794 | OTX2-AS1 | 5.3464 | 153 | 818 |
| TTC34 | 0.3071 | LINC00678 | 0.4789 | TXNDC17 | 5.3111 | 1215 | 6453 |
| RPL23AP53 | 0.3068 | PLD6 | 0.4789 | HIST1H2AM | 5.2990 | 1117 | 5919 |
| PCDHGB2 | 0.3068 | KRT8 | 0.4780 | EFNA4 | 5.2971 | 175 | 927 |
| OTX2 | 0.3067 | OTX2-AS1 | 0.4776 | MAB21L2 | 5.2857 | 7 | 37 |
| TRIM4 | 0.3067 | CLDN3 | 0.4761 | SETX | 5.2796 | 1259 | 6647 |
| YJEFN3 | 0.3065 | ERF | 0.4759 | EFNA3 | 5.2727 | 176 | 928 |
| PARD3 | 0.3060 | MIR200C | 0.4757 | ANKLE1 | 5.2308 | 195 | 1020 |
| C5orf52 | 0.3059 | ABR | 0.4756 | AHNAK | 5.2246 | 806 | 4211 |
| MYT1 | 0.3057 | CDX1 | 0.4745 | GRINA | 5.2065 | 3041 | 15833 |
| LOC146880 | 0.3053 | MLLT6 | 0.4734 | DRAXIN | 5.1888 | 466 | 2418 |
| SOX30 | 0.3048 | CASC15 | 0.4733 | UTF1 | 5.1799 | 1645 | 8521 |
| DNMBP | 0.3040 | WI2-2373I1.2 | 0.4728 | NCR3LG1 | 5.1778 | 2221 | 11500 |
| HSPB2 | 0.3039 | YJEFN3 | 0.4722 | ZNF37A | 5.1549 | 297 | 1531 |
| HSPB2-C11orf52 | 0.3039 | TMEM68 | 0.4712 | HELT | 5.1481 | 2903 | 14945 |
| MEIS1 | 0.3034 | TUBBP5 | 0.4711 | SEH1L | 5.1303 | 3046 | 15627 |
| DDR1 | 0.3020 | IZUMO4 | 0.4709 | KDELC1 | 5.0988 | 3593 | 18320 |
| PCDHA6 | 0.3016 | PUF60 | 0.4708 | DNASE1L2 | 5.0980 | 204 | 1040 |
| ZIM2 | 0.3013 | ASCL2 | 0.4703 | HIST1H3G | 5.0569 | 1282 | 6483 |
| PEG3 | 0.3013 | L1TD1 | 0.4700 | MIR663AHG | 5.0409 | 2052 | 10344 |
| WNT3 | 0.3013 | TWIST2 | 0.4697 | MEPCE | 5.0219 | 961 | 4826 |
| ABLIM1 | 0.3013 | ZNF257 | 0.4695 | CRAMP1L | 5.0114 | 1141 | 5718 |
| NAV2 | 0.3002 | DPYSL4 | 0.4693 | DACT3 | 5.0104 | 2511 | 12581 |
| GRM1 | 0.2998 | MDFI | 0.4690 | LOC100132215 | 5.0000 | 12 | 60 |
| FAM131A | 0.2998 | ZNF398 | 0.4684 | GAS1 | 4.9769 | 3468 | 17260 |
| ELMO1 | 0.2993 | PCDHB6 | 0.4684 | MRPS31P5 | 4.9288 | 281 | 1385 |
| FBP1 | 0.2991 | EFNA4 | 0.4677 | PFN1 | 4.9273 | 3110 | 15324 |
| ZNF560 | 0.2988 | EFNA3 | 0.4677 | LAPTM4B | 4.9264 | 652 | 3212 |
| RBM47 | 0.2980 | GALNT9 | 0.4668 | GRTP1 | 4.9135 | 1388 | 6820 |
| RAPGEF2 | 0.2979 | ATP2B2 | 0.4666 | DPP7 | 4.8628 | 911 | 4430 |
| KIAA1324L | 0.2978 | MEIS1 | 0.4663 | GSC2 | 4.8421 | 2388 | 11563 |
| ARHGEF7 | 0.2974 | TJP2 | 0.4650 | EGR3 | 4.8315 | 2867 | 13852 |
| RABGAP1L | 0.2970 | TBC1D16 | 0.4649 | REREP3 | 4.8154 | 65 | 313 |
| WNT5A | 0.2968 | SEPT9 | 0.4645 | LRRC26 | 4.8120 | 734 | 3532 |
| PCDHGC5 | 0.2961 | PCDHGB1 | 0.4645 | TFAP2C | 4.8057 | 453 | 2177 |
| PAX8 | 0.2960 | SLC25A22 | 0.4640 | SATB2 | 4.8032 | 1387 | 6662 |
| ZNF667 | 0.2958 | FBLN1 | 0.4635 | CSMD3 | 4.8012 | 3089 | 14831 |
| MT1IP | 0.2957 | GRAMD1B | 0.4625 | SH3RF3-AS1 | 4.7997 | 599 | 2875 |
| SSH1 | 0.2956 | PPAP2C | 0.4616 | ITGB8 | 4.7960 | 1554 | 7453 |
| RIN2 | 0.2952 | USP44 | 0.4615 | NKX6-1 | 4.7954 | 2679 | 12847 |
| ARAP1 | 0.2950 | TBX5 | 0.4608 | GBX2 | 4.7885 | 3602 | 17248 |
| KIAA0930 | 0.2942 | GRM1 | 0.4600 | MYOD1 | 4.7754 | 276 | 1318 |
| CLU | 0.2938 | HES5 | 0.4597 | SPTBN4 | 4.7714 | 433 | 2066 |
| HPS4 | 0.2913 | PLXNB2 | 0.4596 | NUBP2 | 4.7703 | 1258 | 6001 |
| PLEC | 0.2910 | GATA3 | 0.4595 | LRRFIP1 | 4.7599 | 554 | 2637 |
| PLXNB2 | 0.2905 | MIR4726 | 0.4594 | HOXD11 | 4.7566 | 3254 | 15478 |
| KLHDC7B | 0.2901 | ANKLE1 | 0.4590 | DEF8 | 4.7547 | 852 | 4051 |
| ANKRD30B | 0.2900 | TBX5-AS1 | 0.4581 | SOCS2 | 4.7394 | 2840 | 13460 |
| COL16A1 | 0.2885 | MIR3147 | 0.4580 | DENND5A | 4.7270 | 1791 | 8466 |
| GRHL2 | 0.2680 | TMEM200B | 0.4575 | HIST1H2BO | 4.7160 | 1838 | 8668 |
| MT1DP | 0.2669 | KCNC1 | 0.4564 | MIR663A | 4.7048 | 1904 | 8958 |
| MOB3A | 0.2864 | PCDHGB2 | 0.4562 | DHODH | 4.6600 | 1938 | 9031 |
| MTUS1 | 0.2864 | LOC100505666 | 0.4562 | C1orf109 | 4.6354 | 2252 | 10439 |
| MIR3147 | 0.2853 | PCDHA13 | 0.4556 | PRR11 | 4.6332 | 856 | 3966 |
| RTN4 | 0.2651 | IAH1 | 0.4554 | KCNG3 | 4.6299 | 978 | 4528 |

| | | | | | | |
|---|---|---|---|---|---|---|
| DMPK | 0.2846 | DNASE1L2 | 0.4554 | PENK | 4.6271 | 295 | 1365 |
| SLFN12 | 0.2845 | BCAR1 | 0.4552 | CECR5-AS1 | 4.6259 | 1684 | 7790 |
| CCDC8 | 0.2845 | PCDHGA9 | 0.4550 | ZNF503 | 4.6258 | 1745 | 8072 |
| VSIG2 | 0.2845 | RGS12 | 0.4544 | C10orf76 | 4.6253 | 2605 | 12049 |
| LTBP3 | 0.2840 | PLAGL1 | 0.4540 | SKA2 | 4.6093 | 883 | 4070 |
| PAX6 | 0.2834 | PCDHGA8 | 0.4527 | DLX2 | 4.5940 | 3116 | 14315 |
| CD70 | 0.2829 | ZNF503-AS2 | 0.4526 | MIR4520-1 | 4.5921 | 277 | 1272 |
| ZMYND8 | 0.2829 | OTX1 | 0.4523 | ESRP2 | 4.5915 | 142 | 652 |
| PRDM7 | 0.2827 | TFAP2A | 0.4514 | DACT3-AS1 | 4.5915 | 2404 | 11038 |
| LBX2-AS1 | 0.2825 | IRX1 | 0.4507 | HIST1H2BJ | 4.5912 | 3826 | 17566 |
| MIMT1 | 0.2822 | WNT2B | 0.4506 | CLCN7 | 4.5875 | 1760 | 8074 |
| WNT5B | 0.2817 | DMRTA2 | 0.4504 | HCG25 | 4.5745 | 1993 | 9117 |
| PPP1R3B | 0.2806 | NLRP6 | 0.4504 | COL27A1 | 4.5682 | 2017 | 9214 |
| INPP5F | 0.2806 | IFITM1 | 0.4503 | SPSB3 | 4.5400 | 1289 | 5852 |
| SRCIN1 | 0.2801 | TCF4 | 0.4500 | CECR5 | 4.5350 | 1729 | 7841 |
| NDUFA4L2 | 0.2800 | TTC34 | 0.4489 | MRPL20 | 4.5281 | 1812 | 8205 |
| BCAR1 | 0.2799 | TYMP | 0.4468 | ELAVL2 | 4.4906 | 320 | 1437 |
| GRIN1 | 0.2793 | MIR3621 | 0.4465 | RUNX2 | 4.4854 | 2468 | 11070 |
| C9orf129 | 0.2788 | PLEC | 0.4459 | IER5L | 4.4794 | 461 | 2065 |
| SLC25A22 | 0.2782 | NODAL | 0.4454 | DKFZp686K1684 | 4.4565 | 230 | 1025 |
| PCDHB6 | 0.2782 | KCTD1 | 0.4450 | RCN1 | 4.4416 | 231 | 1026 |
| HLX | 0.2781 | DMRT3 | 0.4448 | SMPD3 | 4.4390 | 1155 | 5127 |
| TJP2 | 0.2776 | NSMF | 0.4445 | SLC30A4 | 4.4160 | 1435 | 6337 |
| ATP2B2 | 0.2775 | PLEKHA7 | 0.4443 | MIR4520-2 | 4.4076 | 314 | 1384 |
| MIR125B1 | 0.2773 | TREX1 | 0.4442 | CARKD | 4.4069 | 3303 | 14556 |
| STRA6 | 0.2770 | LINC01132 | 0.4435 | MIR124-2 | 4.4054 | 111 | 489 |
| RBFOX1 | 0.2768 | DKFZp686K1684 | 0.4431 | BMP7 | 4.3895 | 2262 | 9929 |
| LRRTM2 | 0.2767 | RCN1 | 0.4431 | ATP9B | 4.3776 | 2092 | 9158 |
| RFPL2 | 0.2765 | MOB2 | 0.4430 | FBP1 | 4.3750 | 40 | 175 |
| FLJ12825 | 0.2765 | MIR141 | 0.4422 | NR5A2 | 4.3502 | 691 | 3006 |
| ZFYVE28 | 0.2762 | KAZALD1 | 0.4411 | CCDC85A | 4.3052 | 639 | 2751 |
| ZNF398 | 0.2761 | DNAH10 | 0.4411 | CYP26B1 | 4.3000 | 260 | 1118 |
| MIR4472-2 | 0.2761 | MT1IP | 0.4407 | NME3 | 4.2986 | 1869 | 8034 |
| DGKZ | 0.2759 | WNT5B | 0.4406 | PIGZ | 4.2947 | 1062 | 4561 |
| ADGRG1 | 0.2757 | MSC-AS1 | 0.4400 | VTN | 4.2859 | 2249 | 9639 |
| MBNL2 | 0.2756 | LOC728613 | 0.4373 | UNCX | 4.2711 | 3567 | 15235 |
| OAF | 0.2754 | SCN4B | 0.4366 | NPEPL1 | 4.2626 | 1923 | 8197 |
| CECR1 | 0.2754 | C19orf83 | 0.4359 | RGS17 | 4.2578 | 3964 | 16878 |
| CIRBP-AS1 | 0.2749 | AGAP2-AS1 | 0.4356 | NOTCH3 | 4.2565 | 1848 | 7866 |
| SIX3 | 0.2735 | RIPK4 | 0.4353 | LHB | 4.2500 | 924 | 3927 |
| CTNNA1 | 0.2734 | ZIM2 | 0.4352 | CDCA8 | 4.2440 | 2520 | 10695 |
| DMRT3 | 0.2732 | PEG3 | 0.4352 | SEPT9 | 4.2418 | 182 | 772 |
| C8orf46 | 0.2731 | RNF44 | 0.4352 | RAI1 | 4.2414 | 3932 | 16677 |
| IAH1 | 0.2728 | CD14 | 0.4346 | PPP1R14C | 4.2352 | 2776 | 11757 |
| SLC5A8 | 0.2727 | ZNF492 | 0.4343 | DHRS3 | 4.2343 | 286 | 1211 |
| PCDHB19P | 0.2719 | SNTG2 | 0.4336 | ANKRD18DP | 4.2322 | 2054 | 8693 |
| MOB2 | 0.2712 | RBFOX1 | 0.4333 | ACAA1 | 4.2206 | 2167 | 9146 |
| NTM | 0.2712 | SFRP1 | 0.4324 | MYEOV2 | 4.2061 | 1640 | 6898 |
| PCOLCE | 0.2711 | CALY | 0.4324 | RIMBP2 | 4.1955 | 2747 | 11525 |
| WFDC1 | 0.2711 | ARHGEF25 | 0.4318 | HMX3 | 4.1943 | 4756 | 19948 |
| PCDHGA2 | 0.2706 | RFPL2 | 0.4316 | GRIN3A | 4.1883 | 579 | 2425 |
| SRGAP3 | 0.2700 | BHLHE22 | 0.4316 | TBC1D9B | 4.1682 | 1623 | 6765 |
| ELF3 | 0.2698 | ZNF439 | 0.4315 | THBD | 4.1620 | 747 | 3109 |
| BZRAP1 | 0.2694 | CIZ1 | 0.4314 | GGN | 4.1496 | 615 | 2552 |
| SORBS2 | 0.2689 | SIX2 | 0.4314 | PREX1 | 4.1439 | 980 | 4061 |
| CIZ1 | 0.2688 | SLFN12 | 0.4308 | STRADA | 4.1361 | 3585 | 14828 |
| LRRC2 | 0.2683 | CYP26B1 | 0.4307 | PAX7 | 4.1332 | 1569 | 6485 |
| SRPK2 | 0.2678 | SP9 | 0.4303 | ZFP36L2 | 4.1224 | 4991 | 20575 |
| MIR4708 | 0.2678 | CT62 | 0.4301 | THEM6 | 4.1000 | 420 | 1722 |
| FOLH1 | 0.2677 | LOC145845 | 0.4291 | TNPO2 | 4.0950 | 1095 | 4484 |
| PCDHA13 | 0.2673 | SOX10 | 0.4290 | ICAM1 | 4.0907 | 2217 | 9069 |
| NODAL | 0.2669 | FGR | 0.4284 | RPP25 | 4.0896 | 2635 | 10776 |
| FOXP1 | 0.2665 | MAP3K14-AS1 | 0.4282 | SOCS2-AS1 | 4.0852 | 5117 | 20904 |
| SCN4B | 0.2663 | SP8 | 0.4281 | MOB3A | 4.0816 | 49 | 200 |
| IGSF9B | 0.2663 | MIR124-2HG | 0.4275 | FOXE1 | 4.0803 | 4595 | 18749 |
| FRMD4A | 0.2656 | C19orf33 | 0.4274 | SYCE3 | 4.0787 | 788 | 3214 |
| LOC145845 | 0.2654 | TEF | 0.4274 | SEC61A2 | 4.0745 | 1410 | 5745 |
| KIF1A | 0.2649 | S100A10 | 0.4271 | MB21D1 | 4.0739 | 284 | 1157 |
| ZFAND5 | 0.2648 | RBM47 | 0.4270 | NGEF | 4.0727 | 454 | 1849 |

| | | | | | | | |
|---|---|---|---|---|---|---|---|
| PRSS3 | 0.2648 | DRD4 | 0.4269 | MEX3B | 4.0707 | 3054 | 12432 |
| RGS14 | 0.2647 | FAM131A | 0.4263 | TEX30 | 4.0604 | 2187 | 8880 |
| GATA4 | 0.2646 | SLC4A2 | 0.4260 | ARL4C | 4.0546 | 1172 | 4752 |
| MAFK | 0.2644 | MYOD1 | 0.4257 | MECOM | 4.0444 | 1620 | 6552 |
| MBNL1 | 0.2643 | MIR4520-1 | 0.4255 | PTRF | 4.0409 | 685 | 2768 |
| SMIM17 | 0.2641 | ZNF436-AS1 | 0.4250 | CCDC8 | 4.0392 | 51 | 206 |
| TFAP2E | 0.2637 | MT1DP | 0.4245 | C2CD4A | 4.0357 | 2773 | 11191 |
| CECR7 | 0.2637 | SNAPC2 | 0.4241 | KIAA1875 | 4.0350 | 486 | 1961 |
| CDX1 | 0.2636 | MRPS31P5 | 0.4240 | TIPIN | 4.0284 | 2147 | 8649 |
| NEAT1 | 0.2627 | KLHL35 | 0.4239 | FOXL1 | 4.0189 | 1854 | 7451 |
| LBX2 | 0.2623 | C7orf50 | 0.4238 | CCND1 | 4.0116 | 3444 | 13816 |
| SEC14L1 | 0.2622 | MB21D1 | 0.4237 | NIPA1 | 4.0067 | 2823 | 11311 |
| BZRAP1-AS1 | 0.2621 | MIR135B | 0.4228 | RFX4 | 3.9970 | 1015 | 4057 |
| RSRC2 | 0.2617 | DHRS3 | 0.4227 | ITPRIPL2 | 3.9781 | 778 | 3095 |
| C9orf172 | 0.2616 | FAM83F | 0.4224 | MIR3177 | 3.9740 | 1882 | 7479 |
| PALM2-AKAP2 | 0.2603 | PCDHGA2 | 0.4220 | DIABLO | 3.9724 | 2649 | 10523 |
| MIR21 | 0.2600 | FLNC | 0.4216 | ST8SIA5 | 3.9712 | 1667 | 6620 |
| EVA1B | 0.2597 | PCDHB18P | 0.4216 | C2orf61 | 3.9689 | 1799 | 7140 |
| KCNJ5 | 0.2590 | LOC648987 | 0.4211 | CALM2 | 3.9672 | 1800 | 7141 |
| DSCR9 | 0.2590 | COL16A1 | 0.4211 | LOC93622 | 3.9613 | 2479 | 9820 |
| PFN3 | 0.2589 | CECR1 | 0.4209 | MYD88 | 3.9557 | 1287 | 5091 |
| CACNA1B | 0.2586 | NEAT1 | 0.4207 | ZIC1 | 3.9552 | 3664 | 14492 |
| EIF4A2 | 0.2581 | PENK | 0.4205 | ZAP70 | 3.9507 | 527 | 2082 |
| ZSCAN10 | 0.2581 | CBFA2T3 | 0.4203 | SPTBN1 | 3.9419 | 878 | 3461 |
| FGR | 0.2580 | ZNF37A | 0.4202 | FERD3L | 3.9292 | 678 | 2664 |
| RAB25 | 0.2579 | MAP1LC3A | 0.4201 | TUBB3 | 3.9238 | 2204 | 8648 |
| PLXNB1 | 0.2579 | CDT1 | 0.4201 | PTPN18 | 3.9213 | 4004 | 15701 |
| C19orf33 | 0.2576 | SOWAHC | 0.4199 | MGC12916 | 3.9079 | 1693 | 6616 |
| HERPUD1 | 0.2575 | MTL5 | 0.4191 | TSR3 | 3.9019 | 2099 | 8190 |
| PCDHGA1 | 0.2575 | MIR4472-2 | 0.4185 | MIR193A | 3.9000 | 600 | 2340 |
| SCART1 | 0.2574 | RTKN | 0.4181 | MYLIP | 3.8999 | 2628 | 10249 |
| C9orf64 | 0.2570 | MAP3K6 | 0.4178 | GSC | 3.8980 | 1304 | 5083 |
| AKAP6 | 0.2570 | ERVMER34-1 | 0.4177 | GNPTG | 3.8946 | 2069 | 8058 |
| TRIML2 | 0.2565 | ZMYND8 | 0.4177 | LINC00925 | 3.8942 | 312 | 1215 |
| LTB4R2 | 0.2565 | LY6G5C | 0.4174 | ALDH2 | 3.8904 | 3366 | 13095 |
| MFN1 | 0.2564 | TBX3 | 0.4173 | KAZALD1 | 3.8803 | 234 | 908 |
| FOXK2 | 0.2559 | ZNF560 | 0.4171 | C4orf48 | 3.8734 | 3816 | 14781 |
| ABCD2 | 0.2559 | ANKRD30B | 0.4165 | FOXF1 | 3.8692 | 3876 | 14997 |
| ZNF208 | 0.2557 | UBC | 0.4164 | MIR4745 | 3.8656 | 677 | 2617 |
| TRIM58 | 0.2556 | LINC00925 | 0.4163 | ZFP90 | 3.8568 | 2793 | 10772 |
| REREP3 | 0.2554 | ZFP64 | 0.4154 | PDGFRA | 3.8548 | 1811 | 6981 |
| DDX5 | 0.2550 | MIR4520-2 | 0.4151 | ZNF263 | 3.8169 | 355 | 1355 |
| CACNB3 | 0.2549 | KCNJ5 | 0.4143 | PER3 | 3.8159 | 2396 | 9143 |
| HMGA1 | 0.2545 | ZNF729 | 0.4143 | TDRD6 | 3.8153 | 1792 | 6837 |
| LOC100130700 | 0.2544 | CIRBP-AS1 | 0.4131 | LINC00921 | 3.8123 | 357 | 1361 |
| GCNT2 | 0.2544 | ZSCAN10 | 0.4127 | PLEKHA7 | 3.7885 | 227 | 860 |
| UCKL1 | 0.2543 | TTYH1 | 0.4125 | GLUD1 | 3.7828 | 3536 | 13376 |
| LTBP1 | 0.2542 | ELAVL2 | 0.4115 | JMJD8 | 3.7780 | 1153 | 4356 |
| ZSCAN18 | 0.2538 | MIR302B | 0.4113 | MRI1 | 3.7665 | 1426 | 5371 |
| CTSF | 0.2537 | PCDHA5 | 0.4105 | CBS | 3.7636 | 2360 | 8882 |
| SLC26A10 | 0.2535 | FAM110A | 0.4101 | HS3ST3B1 | 3.7522 | 4783 | 17947 |
| LRP1 | 0.2531 | ELAVL4 | 0.4101 | C1QL1 | 3.7512 | 828 | 3106 |
| CCDC166 | 0.2528 | SNORA63 | 0.4097 | KATNB1 | 3.7389 | 2669 | 9979 |
| TRIM2 | 0.2527 | LHX6 | 0.4095 | ACTR1A | 3.7356 | 5102 | 19059 |
| CRCT1 | 0.2526 | SERPINB6 | 0.4094 | C20orf166-AS1 | 3.7355 | 518 | 1935 |
| NTRK2 | 0.2526 | MICALL2 | 0.4093 | FAM35A | 3.7255 | 3846 | 13583 |
| FAM102A | 0.2523 | CXCL12 | 0.4088 | MBP | 3.7113 | 426 | 1561 |
| GJC2 | 0.2521 | EPCAM | 0.4087 | FBXO6 | 3.6998 | 2861 | 10585 |
| WNT2B | 0.2518 | YBX3P1 | 0.4084 | LTBP4 | 3.6903 | 113 | 417 |
| PCDHA5 | 0.2518 | PROX1 | 0.4082 | GRM8 | 3.6881 | 3023 | 11149 |
| COL1A2 | 0.2516 | ZFYVE28 | 0.4079 | TMEM88 | 3.6871 | 163 | 601 |
| KCNC1 | 0.2514 | MIMT1 | 0.4079 | IRX2 | 3.6861 | 2673 | 9853 |
| ARNT2 | 0.2514 | NOTUM | 0.4075 | SUFU | 3.6853 | 3162 | 11653 |
| ELN | 0.2512 | LHX8 | 0.4071 | PCYT2 | 3.6787 | 2334 | 8586 |
| ZNF662 | 0.2511 | DMPK | 0.4069 | ASH2L | 3.6758 | 3538 | 13005 |
| UBC | 0.2511 | SEPT10 | 0.4069 | KLHL11 | 3.6724 | 2692 | 9886 |
| DLG2 | 0.2508 | SALL1 | 0.4062 | BLVRB | 3.6721 | 738 | 2710 |
| HPN | 0.2506 | TK2 | 0.4062 | MAFA | 3.6606 | 5316 | 19460 |
| ANXA3 | 0.2504 | HYLS1 | 0.4059 | MAP3K14-AS1 | 3.6579 | 266 | 973 |

| | | | | | | | |
|---|---|---|---|---|---|---|---|
| RAP1GAP | 0.2503 | LRRC4C | 0.4058 | C1QL2 | 3.6532 | 3065 | 11197 |
| TMEM121 | 0.2502 | SRGAP3 | 0.4058 | IZUMO4 | 3.6485 | 165 | 602 |
| ZNF649 | 0.2500 | AMN | 0.4055 | SCGB3A1 | 3.6415 | 491 | 1788 |
| PSPH | 0.2498 | LINC01124 | 0.4050 | NPR3 | 3.6382 | 2598 | 9452 |
| NUPR1L | 0.2498 | IRF2BP2 | 0.4047 | ULK2 | 3.6362 | 3752 | 13643 |
| SFRP1 | 0.2496 | NOTCH1 | 0.4047 | ABCA3 | 3.6346 | 104 | 378 |
| YBX3P1 | 0.2496 | GCGR | 0.4046 | MSC | 3.6311 | 366 | 1329 |
| RNF126P1 | 0.2490 | CECR7 | 0.4044 | ABCA17P | 3.6286 | 105 | 381 |
| FOXJ1 | 0.2489 | C9orf129 | 0.4044 | CCRN4L | 3.6267 | 1125 | 4080 |
| ARHGEF4 | 0.2488 | NUDT3 | 0.4039 | ZFP64 | 3.6262 | 313 | 1135 |
| WI2-2373I1.2 | 0.2483 | EZR | 0.4024 | FAM8A1 | 3.6215 | 3255 | 11788 |
| LTB4R | 0.2483 | TRIM67 | 0.4022 | LINC00221 | 3.6206 | 1186 | 4294 |
| CIDEB | 0.2476 | HTRA4 | 0.4022 | KIF3B | 3.6173 | 2694 | 9745 |
| PCOLCE-AS1 | 0.2476 | ZNF263 | 0.4020 | KCNK4 | 3.6134 | 3996 | 14439 |
| PPP2R1B | 0.2475 | CLMP | 0.4018 | IRX4 | 3.6102 | 5488 | 19813 |
| CACNG2 | 0.2469 | LINC00921 | 0.4017 | OTP | 3.6091 | 1540 | 5558 |
| LOC728613 | 0.2468 | ZBTB4 | 0.4015 | LRP8 | 3.6015 | 517 | 1862 |
| MEF2D | 0.2461 | RTP5 | 0.4015 | DLL4 | 3.6008 | 4156 | 14972 |
| MIR181A1HG | 0.2459 | KCNJ3 | 0.4014 | RPS6KA4 | 3.6004 | 4570 | 16454 |
| MT1A | 0.2459 | ADGRG1 | 0.4009 | NCLN | 3.5975 | 3031 | 10904 |
| RPS6KA1 | 0.2457 | RNF126P1 | 0.4005 | ZFAT | 3.5975 | 1662 | 5979 |
| ZNF727 | 0.2455 | EML2 | 0.4002 | PUSL1 | 3.5938 | 544 | 1955 |
| ZNF572 | 0.2453 | PITX1 | 0.4002 | POU4F3 | 3.5937 | 3638 | 13074 |
| MIR4710 | 0.2453 | LOC100130700 | 0.4000 | HIST1H3F | 3.5878 | 1601 | 5744 |
| TACR3 | 0.2452 | MSC | 0.4000 | RIPK4 | 3.5844 | 243 | 871 |
| TAOK3 | 0.2446 | CENPBD1P1 | 0.4000 | NPTX2 | 3.5727 | 3606 | 12883 |
| GALNT9 | 0.2446 | POMK | 0.3997 | LOC100288181 | 3.5706 | 496 | 1771 |
| MMP9 | 0.2445 | SPACA6P | 0.3993 | CDKN1A | 3.5696 | 1703 | 6079 |
| CTHRC1 | 0.2443 | MMEL1 | 0.3991 | TAL1 | 3.5659 | 417 | 1487 |
| SNAPC2 | 0.2441 | C5orf52 | 0.3989 | HYAL2 | 3.5641 | 1170 | 4170 |
| CNTNAP2 | 0.2441 | SOX30 | 0.3988 | ZCWPW1 | 3.5611 | 2333 | 8308 |
| TNFRSF14 | 0.2440 | CRYAB | 0.3986 | HCG11 | 3.5588 | 374 | 1331 |
| RTP5 | 0.2439 | HCG11 | 0.3985 | POU4F1 | 3.5571 | 4434 | 15772 |
| FAM46B | 0.2437 | MIR4641 | 0.3984 | CXCL12 | 3.5471 | 329 | 1167 |
| EPS15L1 | 0.2437 | MIR99AHG | 0.3982 | RSPO1 | 3.5408 | 2794 | 9893 |
| LOC100631378 | 0.2437 | MYT1 | 0.3976 | NETO2 | 3.5399 | 4942 | 17494 |
| ABCA3 | 0.2436 | SLC26A10 | 0.3974 | LRRC41 | 3.5390 | 2525 | 8936 |
| GALNT8 | 0.2434 | ZNF436 | 0.3971 | CNTFR | 3.5371 | 769 | 2720 |
| MIR183 | 0.2429 | NOL3 | 0.3968 | CENPB | 3.5365 | 643 | 2274 |
| ABCA17P | 0.2429 | VSIG2 | 0.3964 | RHOB | 3.5348 | 4774 | 16875 |
| ARAP2 | 0.2428 | MDGA1 | 0.3961 | LETM1 | 3.5212 | 4110 | 14472 |
| KIAA0195 | 0.2427 | TPTE | 0.3957 | C8orf58 | 3.5160 | 4089 | 14377 |
| FAM110A | 0.2426 | PIM3 | 0.3956 | CPEB2 | 3.5080 | 6201 | 21753 |
| CABP1 | 0.2422 | POLR2A | 0.3956 | INTS1 | 3.5059 | 2208 | 7741 |
| PTPRE | 0.2417 | PFN3 | 0.3955 | TMED2 | 3.5010 | 4024 | 14088 |
| ZADH2 | 0.2415 | KIF1A | 0.3949 | THSD1 | 3.5007 | 1534 | 5370 |
| L1TD1 | 0.2414 | ARHGEF4 | 0.3948 | MT2A | 3.5003 | 3712 | 12993 |
| FBLN1 | 0.2413 | LOC100631378 | 0.3943 | KISS1R | 3.4954 | 2386 | 8340 |
| KCNN3 | 0.2411 | UCKL1 | 0.3932 | ARHGAP20 | 3.4924 | 1836 | 6412 |
| PIK3R1 | 0.2403 | PIP5KL1 | 0.3932 | TM9SF1 | 3.4829 | 1901 | 6621 |
| GPR21 | 0.2403 | REM1 | 0.3926 | CHMP4A | 3.4816 | 1902 | 6622 |
| RABGAP1 | 0.2403 | A1BG-AS1 | 0.3919 | RFFL | 3.4678 | 3760 | 13039 |
| SLC4A2 | 0.2403 | TACSTD2 | 0.3917 | LOC100130370 | 3.4676 | 2915 | 10108 |
| HSPA8 | 0.2402 | ANKRD11 | 0.3916 | ADCY2 | 3.4662 | 918 | 3182 |
| CD177 | 0.2402 | DOK7 | 0.3915 | PXYLP1 | 3.4647 | 3546 | 12286 |
| ZNF280D | 0.2400 | LOC100287846 | 0.3911 | C5orf38 | 3.4642 | 3195 | 11068 |
| MKL2 | 0.2398 | TUSC1 | 0.3911 | PPP1R37 | 3.4641 | 2340 | 8106 |
| PPFIBP2 | 0.2395 | VAX2 | 0.3911 | WNK1 | 3.4614 | 3351 | 11599 |
| PCDHB16 | 0.2395 | CYP11A1 | 0.3910 | HNRNPL | 3.4586 | 1110 | 3839 |
| ADGRA1 | 0.2391 | ACOT2 | 0.3909 | CNNM1 | 3.4488 | 3213 | 11081 |
| TBX5-AS1 | 0.2391 | KIAA0930 | 0.3907 | PCDH10 | 3.4486 | 6037 | 20819 |
| ARHGAP23 | 0.2390 | RNF216 | 0.3906 | NOP14-AS1 | 3.4469 | 1271 | 4381 |
| DNAH10 | 0.2389 | FAM160A1 | 0.3899 | WDR27 | 3.4387 | 2904 | 9986 |
| MIR1180 | 0.2387 | EZR-AS1 | 0.3896 | FLOT1 | 3.4339 | 1429 | 4907 |
| PRKACB | 0.2386 | PCDHB19P | 0.3894 | MIB2 | 3.4316 | 1191 | 4087 |
| MAP4 | 0.2384 | OAF | 0.3889 | SYNM | 3.4310 | 478 | 1640 |
| CASC10 | 0.2383 | SLC51A | 0.3887 | ERCC6 | 3.4259 | 1646 | 5639 |
| CHD2 | 0.2381 | EPS8L2 | 0.3885 | SLC45A1 | 3.4223 | 1859 | 6362 |
| PCDHB13 | 0.2380 | LOC441666 | 0.3882 | CDC25B | 3.4178 | 766 | 2618 |

| | | | | | | | |
|---|---|---|---|---|---|---|---|
| AMPD2 | 0.2380 | FOXK2 | 0.3881 | RBX1 | 3.4150 | 4660 | 15914 |
| MIR4641 | 0.2380 | FGF8 | 0.3880 | HCN4 | 3.4097 | 3112 | 10611 |
| ANKRD28 | 0.2379 | ECHDC3 | 0.3877 | CRNDE | 3.4073 | 3847 | 13108 |
| RWDD2B | 0.2378 | TM6SF1 | 0.3870 | EPB41L4B | 3.4023 | 1894 | 6444 |
| RARRES3 | 0.2376 | TMEM179 | 0.3870 | C15orf65 | 3.3981 | 2974 | 10106 |
| HES5 | 0.2375 | SPHK1 | 0.3870 | CCPG1 | 3.3958 | 2979 | 10116 |
| LTBP4 | 0.2373 | TAL1 | 0.3868 | ABHD16B | 3.3919 | 4241 | 14385 |
| GPRC5B | 0.2367 | LOC440040 | 0.3867 | RAD9A | 3.3849 | 3149 | 10659 |
| FYN | 0.2363 | PLIN2 | 0.3867 | PCED1A | 3.3826 | 5319 | 17992 |
| CT62 | 0.2361 | THEM6 | 0.3866 | WNT3 | 3.3800 | 50 | 169 |
| NRXN1 | 0.2353 | SALL4 | 0.3864 | ISM1 | 3.3756 | 2391 | 8071 |
| TEKT4 | 0.2351 | LIN28B | 0.3861 | ISCA1 | 3.3735 | 5607 | 18915 |
| CELF2 | 0.2348 | BOLL | 0.3861 | DKK1 | 3.3693 | 2513 | 8467 |
| SPARCL1 | 0.2343 | VPS9D1-AS1 | 0.3860 | MLLT3 | 3.3688 | 5610 | 18899 |
| PCDHGA4 | 0.2340 | ZIC5 | 0.3860 | GPRIN1 | 3.3654 | 5184 | 17446 |
| MT1E | 0.2336 | MBP | 0.3859 | SSTR1 | 3.3603 | 2906 | 9765 |
| LGALS1 | 0.2334 | RABGAP1L | 0.3858 | FGF3 | 3.3592 | 5972 | 20061 |
| RBAKDN | 0.2332 | TEKT4 | 0.3857 | RNPS1 | 3.3582 | 1164 | 3909 |
| MYF6 | 0.2327 | SNORA81 | 0.3857 | ERCC6-PGBD3 | 3.3546 | 1647 | 5525 |
| ARHGEF25 | 0.2327 | CMAHP | 0.3854 | SMARCA4 | 3.3486 | 2312 | 7742 |
| TUSC1 | 0.2327 | ADGRB1 | 0.3854 | GGTA1P | 3.3462 | 1086 | 3634 |
| SASH1 | 0.2324 | PCOLCE-AS1 | 0.3853 | TRANK1 | 3.3448 | 4202 | 14055 |
| CASKIN2 | 0.2324 | SPTBN4 | 0.3853 | EPHB2 | 3.3426 | 2592 | 8664 |
| MCHR1 | 0.2323 | RAET1G | 0.3853 | FAM92B | 3.3406 | 3186 | 10643 |
| RNH1 | 0.2321 | ZNF471 | 0.3852 | ZNF555 | 3.3379 | 2619 | 8742 |
| BMF | 0.2319 | SNORA4 | 0.3850 | IER3 | 3.3344 | 945 | 3151 |
| ZNF300P1 | 0.2319 | ANXA3 | 0.3848 | CDK5R2 | 3.3332 | 5562 | 18539 |
| SH2B3 | 0.2319 | TRIM58 | 0.3847 | PRKG1-AS1 | 3.3201 | 2387 | 7925 |
| ZNF726 | 0.2318 | DPPA2 | 0.3846 | LOC648967 | 3.3127 | 291 | 964 |
| SYNE1 | 0.2317 | RAB25 | 0.3842 | BAHCC1 | 3.3079 | 2631 | 8703 |
| RTN4R | 0.2315 | LINC00982 | 0.3841 | KCNQ5 | 3.3057 | 3304 | 10922 |
| PCDHB14 | 0.2313 | TUBGCP6 | 0.3840 | HHEX | 3.2995 | 551 | 1818 |
| LOC730668 | 0.2309 | SSH1 | 0.3837 | HTR1A | 3.2961 | 635 | 2093 |
| DRD4 | 0.2308 | ANKRD63 | 0.3835 | SYT12 | 3.2943 | 1784 | 5877 |
| LINC00839 | 0.2307 | ELN | 0.3834 | GNAZ | 3.2801 | 2374 | 7787 |
| PDXK | 0.2302 | PRKAR1B | 0.3834 | VAX2 | 3.2782 | 399 | 1308 |
| PCDHGA10 | 0.2301 | PSMD5 | 0.3834 | DAZL | 3.2769 | 2203 | 7219 |
| RIPK3 | 0.2301 | PTPRE | 0.3832 | ISL2 | 3.2762 | 3342 | 10949 |
| RAET1G | 0.2301 | TSPO | 0.3826 | CERCAM | 3.2743 | 948 | 3104 |
| SLC22A16 | 0.2298 | EP400NL | 0.3825 | TNFRSF10D | 3.2660 | 1173 | 3831 |
| EML2 | 0.2297 | PCOLCE | 0.3825 | DBIL5P | 3.2650 | 1366 | 4460 |
| PTCHD3P1 | 0.2297 | C9orf64 | 0.3822 | GEMIN4 | 3.2649 | 1344 | 4388 |
| MIRLET7B | 0.2295 | TFAP2C | 0.3820 | MAL | 3.2632 | 19 | 62 |
| AATK-AS1 | 0.2295 | NGEF | 0.3820 | ANP32B | 3.2624 | 5655 | 18449 |
| SULT1A1 | 0.2294 | PSMD5-AS1 | 0.3819 | C9orf172 | 3.2614 | 88 | 287 |
| LY6G5C | 0.2290 | WFDC1 | 0.3816 | ERRFI1 | 3.2608 | 2784 | 9078 |
| FLNC | 0.2289 | ST3GAL5 | 0.3815 | TCF7 | 3.2575 | 1872 | 6098 |
| ENTPD1-AS1 | 0.2289 | DGKZ | 0.3815 | SCD5 | 3.2549 | 3993 | 12997 |
| GAS7 | 0.2289 | MAD2L2 | 0.3814 | NANS | 3.2490 | 3887 | 12629 |
| RHOJ | 0.2288 | DDR1 | 0.3813 | CPEB1-AS1 | 3.2444 | 1608 | 5217 |
| ZNF835 | 0.2283 | IER5L | 0.3813 | LINC00960 | 3.2368 | 3154 | 10209 |
| MIR124-2HG | 0.2283 | HSPB2 | 0.3812 | HYLS1 | 3.2346 | 341 | 1103 |
| CLDN3 | 0.2282 | HSPB2-C11orf52 | 0.3812 | KMT2A | 3.2311 | 6191 | 20004 |
| SLC17A7 | 0.2279 | GPRC5C | 0.3811 | MIR1-1HG | 3.2296 | 823 | 2658 |
| LRIG1 | 0.2274 | ZNF354C | 0.3811 | NR4A3 | 3.2215 | 4559 | 14687 |
| SLC25A25 | 0.2270 | DRAXIN | 0.3810 | TBX1 | 3.2214 | 1147 | 3695 |
| PNMAL2 | 0.2270 | SRD5A2 | 0.3809 | PLEKHH3 | 3.2183 | 4352 | 14006 |
| FTCD | 0.2269 | NKX6-3 | 0.3809 | RNF31 | 3.2156 | 4755 | 15290 |
| AMN | 0.2268 | PODXL2 | 0.3808 | HOXA9 | 3.2081 | 1033 | 3314 |
| LEFTY1 | 0.2268 | MX1 | 0.3808 | FAM21C | 3.2008 | 4716 | 15095 |
| PCDHB17P | 0.2266 | KCNN3 | 0.3805 | MAGI2-AS3 | 3.1993 | 4896 | 15664 |
| CALN1 | 0.2264 | HMG20B | 0.3804 | TUBA3D | 3.1970 | 3061 | 9786 |
| ZNF98 | 0.2264 | ADM | 0.3803 | ADCY8 | 3.1924 | 1476 | 4712 |
| TRIM6 | 0.2263 | APOB | 0.3797 | LOC100132111 | 3.1884 | 844 | 2691 |
| TRIM6-TRIM34 | 0.2263 | HTR2A | 0.3796 | HBQ1 | 3.1875 | 3088 | 9843 |
| SYNGR1 | 0.2263 | RASGRP2 | 0.3796 | ZNF354B | 3.1796 | 746 | 2372 |
| DDRGK1 | 0.2263 | TBX15 | 0.3795 | DENND6B | 3.1778 | 3290 | 10455 |
| S100A10 | 0.2260 | SYNM | 0.3794 | CDH1 | 3.1644 | 1685 | 5332 |
| ZNF728 | 0.2260 | CHRNB4 | 0.3793 | NKX2-2 | 3.1630 | 6664 | 21078 |

| Gene | Value | Gene | Value | Gene | Value | N1 | N2 |
|---|---|---|---|---|---|---|---|
| MAP3K6 | 0.2260 | HPN | 0.3791 | TRIL | 3.1603 | 1379 | 4358 |
| DOCK9 | 0.2260 | UHRF1 | 0.3790 | FZD10 | 3.1595 | 6058 | 19140 |
| SLK | 0.2259 | GCM2 | 0.3790 | LOC100289495 | 3.1579 | 3300 | 10421 |
| IGF1 | 0.2259 | GPX4 | 0.3785 | FOXB2 | 3.1578 | 5958 | 18814 |
| MIR1244-2 | 0.2257 | TGFBR3L | 0.3784 | RNF13 | 3.1567 | 3619 | 11424 |
| ZBTB18 | 0.2256 | FAIM2 | 0.3783 | GCM2 | 3.1556 | 482 | 1521 |
| PRKAR1B | 0.2256 | KIAA1875 | 0.3782 | SOX21 | 3.1511 | 5374 | 16934 |
| TPM3 | 0.2254 | PCDHB16 | 0.3781 | HLTF | 3.1498 | 3311 | 10429 |
| CDT1 | 0.2254 | LEFTY1 | 0.3780 | RAVER1 | 3.1498 | 1736 | 5468 |
| MIR124-2 | 0.2252 | KDM2B | 0.3776 | ZADH2 | 3.1463 | 123 | 387 |
| ZNF331 | 0.2252 | KRT19 | 0.3773 | SEPT5-GP1BB | 3.1413 | 927 | 2912 |
| ST3GAL5 | 0.2250 | SCGB3A1 | 0.3771 | DPP6 | 3.1368 | 2010 | 6305 |
| MIR4763 | 0.2250 | CTHRC1 | 0.3771 | MEGF8 | 3.1338 | 919 | 2880 |
| SPATA13 | 0.2247 | HPSE2 | 0.3769 | STOM | 3.1304 | 3774 | 11814 |
| PTMS | 0.2242 | PHYHIPL | 0.3769 | LDOC1L | 3.1281 | 1593 | 4983 |
| ASPDH | 0.2240 | RIPK3 | 0.3766 | CCDC79 | 3.1251 | 4013 | 12541 |
| LGI1 | 0.2240 | LOC100288181 | 0.3764 | FAM182B | 3.1250 | 24 | 75 |
| ANKRD11 | 0.2239 | LINC01305 | 0.3763 | BIVM | 3.1246 | 3621 | 11314 |
| RNF44 | 0.2239 | WSB1 | 0.3763 | FASN | 3.1203 | 2411 | 7523 |
| ACOT2 | 0.2238 | UTS2R | 0.3762 | IRAK2 | 3.1180 | 2865 | 8933 |
| CHRM1 | 0.2238 | SATB2-AS1 | 0.3762 | ZBTB12 | 3.1171 | 2563 | 7989 |
| CLASP2 | 0.2237 | RNH1 | 0.3761 | ANKRD39 | 3.1165 | 1434 | 4469 |
| MIR96 | 0.2236 | RAB34 | 0.3761 | ALX3 | 3.1148 | 915 | 2850 |
| PPP2R4 | 0.2233 | HIST1H3J | 0.3759 | C5orf66 | 3.1074 | 782 | 2430 |
| HESX1 | 0.2232 | SLK | 0.3758 | LOC100129518 | 3.1033 | 1288 | 3997 |
| ANK3 | 0.2232 | PTPN14 | 0.3758 | C19orf73 | 3.1031 | 2377 | 7376 |
| TBX5 | 0.2231 | NARR | 0.3756 | ATP8A2 | 3.1017 | 1249 | 3874 |
| C11orf45 | 0.2230 | MAP2K3 | 0.3754 | TBX2 | 3.1006 | 4114 | 12756 |
| CYP4F22 | 0.2229 | ZSCAN18 | 0.3750 | KMT2B | 3.0989 | 5663 | 17549 |
| TK2 | 0.2229 | GPM6A | 0.3747 | EMILIN3 | 3.0967 | 693 | 2146 |
| HHLA1 | 0.2224 | LIME1 | 0.3746 | PITPNC1 | 3.0953 | 4028 | 12468 |
| HSD17B7P2 | 0.2221 | PARD3 | 0.3741 | CACNA1B | 3.0947 | 95 | 294 |
| ATXN7L1 | 0.2221 | HEYL | 0.3740 | BMP4 | 3.0923 | 1821 | 5631 |
| SAMD13 | 0.2221 | MIR330 | 0.3736 | EME2 | 3.0893 | 3675 | 11353 |
| LINC01532 | 0.2221 | ZRANB2-AS1 | 0.3735 | MRPS34 | 3.0867 | 3749 | 11572 |
| SLC51A | 0.2220 | HIST1H3E | 0.3734 | KCNJ3 | 3.0833 | 360 | 1110 |
| KCNIP3 | 0.2220 | INPP5F | 0.3732 | CGB8 | 3.0825 | 1916 | 5906 |
| TGIF1 | 0.2219 | LRP8 | 0.3732 | CGB7 | 3.0814 | 1917 | 5907 |
| SERPINB6 | 0.2215 | C20orf166-AS1 | 0.3730 | LOC401463 | 3.0791 | 1947 | 5995 |
| PLEKHB1 | 0.2214 | PCDHGC3 | 0.3729 | LBX2 | 3.0761 | 92 | 283 |
| PPAP2C | 0.2212 | TNFRSF14 | 0.3727 | RNPEPL1 | 3.0756 | 2830 | 8704 |
| SIX2 | 0.2211 | PNMAL2 | 0.3727 | MPC1 | 3.0733 | 4009 | 12321 |
| NSMF | 0.2211 | DDRGK1 | 0.3725 | DLX1 | 3.0663 | 5850 | 17938 |
| MIR589 | 0.2209 | KAT2A | 0.3724 | TMEM127 | 3.0634 | 1104 | 3382 |
| CDH11 | 0.2208 | GPRC5B | 0.3723 | CIAO1 | 3.0597 | 1055 | 3228 |
| EXTL1 | 0.2207 | COL1A2 | 0.3718 | RAX | 3.0596 | 587 | 1796 |
| MIR497 | 0.2202 | PRR15 | 0.3718 | MIER3 | 3.0540 | 4665 | 14247 |
| HOPX | 0.2201 | ZAP70 | 0.3714 | TMEM179 | 3.0506 | 415 | 1266 |
| CYTH1 | 0.2201 | DNMT3B | 0.3713 | TCTEX1D2 | 3.0501 | 3011 | 9184 |
| LCN10 | 0.2200 | PCDHA2 | 0.3711 | LHX4 | 3.0467 | 6963 | 21214 |
| CYP11A1 | 0.2200 | ZNF441 | 0.3711 | WWTR1 | 3.0288 | 2153 | 6521 |
| LRIF1 | 0.2199 | MBNL2 | 0.3709 | PRR36 | 3.0270 | 3217 | 9738 |
| DOK7 | 0.2198 | FZD9 | 0.3708 | ERVMER34-1 | 3.0262 | 305 | 923 |
| ABHD14A | 0.2197 | CACNG2 | 0.3707 | ACAT2 | 3.0262 | 1376 | 4164 |
| ABHD14A-ACY1 | 0.2197 | MEGF1 | 0.3706 | TBX18 | 3.0196 | 4126 | 12459 |
| PRSS33 | 0.2197 | TMEM184B | 0.3703 | LOC100652758 | 3.0163 | 5044 | 15214 |
| LOC100506474 | 0.2196 | ARHGEF7 | 0.3702 | WWTR1-AS1 | 3.0162 | 2039 | 6150 |
| RBM39 | 0.2195 | SHISA3 | 0.3701 | RNF168 | 3.0156 | 4625 | 13947 |
| PCDHB11 | 0.2193 | RAB11FIP3 | 0.3697 | ONECUT3 | 3.0153 | 5679 | 17124 |
| PPARGC1A | 0.2193 | GPER1 | 0.3697 | DSP | 3.0148 | 3848 | 11601 |
| FLJ26650 | 0.2193 | SH3RF3 | 0.3697 | SPHKAP | 3.0146 | 2936 | 8851 |
| SARDH | 0.2193 | GRIN2D | 0.3694 | HLTF-AS1 | 3.0124 | 3627 | 10926 |
| LDLRAD4 | 0.2192 | GALNT8 | 0.3692 | FAM160A1 | 3.0124 | 404 | 1217 |
| CHMP7 | 0.2191 | FUT2 | 0.3691 | NDRG3 | 3.0108 | 4153 | 12504 |
| A1BG-AS1 | 0.2191 | PUSL1 | 0.3690 | CLDN3 | 3.0065 | 154 | 463 |
| OLIG2 | 0.2191 | PRR23C | 0.3689 | THRAP3 | 3.0049 | 2035 | 6115 |
| NCS1 | 0.2190 | ANK3 | 0.3688 | INSM1 | 3.0040 | 2021 | 6071 |
| PTGER1 | 0.2190 | TSSK6 | 0.3687 | FAM135B | 3.0026 | 1161 | 3486 |
| PDE4D | 0.2189 | CEP131 | 0.3687 | PLIN2 | 3.0024 | 419 | 1258 |

| | | | | | | | |
|---|---|---|---|---|---|---|---|
| DHX58 | 0.2188 | FAM69B | 0.3684 | KCNN1 | 2.9977 | 2595 | 7779 |
| MIR375 | 0.2187 | AATK-AS1 | 0.3683 | MAP1LC3A | 2.9899 | 298 | 891 |
| SORT1 | 0.2187 | HHEX | 0.3683 | DPYSL5 | 2.9897 | 6143 | 18366 |
| ADGRB1 | 0.2186 | CACNB3 | 0.3682 | UQCRH | 2.9746 | 5075 | 15096 |
| GPT | 0.2184 | PLEKHB1 | 0.3680 | CDCA3 | 2.9698 | 4565 | 13557 |
| MOBP | 0.2184 | LRRFIP1 | 0.3680 | FLI1 | 2.9697 | 1022 | 3035 |
| GRIA1 | 0.2184 | ACOT4 | 0.3679 | SIX1 | 2.9659 | 6388 | 18946 |
| DLEU2 | 0.2183 | LOC730668 | 0.3678 | ZGPAT | 2.9623 | 4223 | 12510 |
| KAT2A | 0.2183 | TGIF1 | 0.3676 | LHX3 | 2.9616 | 2681 | 7940 |
| AGAP2-AS1 | 0.2182 | LOXL2 | 0.3674 | NTF3 | 2.9615 | 3322 | 9838 |
| IGFBP6 | 0.2182 | GLTPD2 | 0.3674 | UBXN2A | 2.9571 | 2216 | 6553 |
| SERPINH1 | 0.2182 | PCDHB17P | 0.3673 | RNF165 | 2.9568 | 2130 | 6298 |
| MIR4526 | 0.2181 | ARAP1 | 0.3673 | C6orf203 | 2.9530 | 3298 | 9739 |
| MIR3675 | 0.2180 | SEC14L1 | 0.3672 | CPEB1 | 2.9527 | 1563 | 4615 |
| MNS1 | 0.2180 | CCDC114 | 0.3666 | PEG10 | 2.9502 | 4494 | 13258 |
| C1QTNF3 | 0.2176 | SPON2 | 0.3666 | ZMYND11 | 2.9497 | 3734 | 11014 |
| PRKAR1A | 0.2174 | MST1L | 0.3664 | FKBP3 | 2.9495 | 6016 | 17744 |
| FAM83F | 0.2169 | PCDHGC5 | 0.3663 | C2 | 2.9446 | 2346 | 6908 |
| MSRA | 0.2167 | IGSF9B | 0.3659 | USP5 | 2.9420 | 5487 | 16143 |
| TMBIM1 | 0.2165 | ANXA2 | 0.3659 | SGCE | 2.9405 | 4624 | 13597 |
| SOX10 | 0.2164 | SCART1 | 0.3658 | PHF20 | 2.9387 | 5565 | 16354 |
| RANBP3 | 0.2163 | COL22A1 | 0.3658 | WEE1 | 2.9369 | 824 | 2420 |
| LHPP | 0.2162 | MIR375 | 0.3657 | MIR3131 | 2.9360 | 2389 | 7014 |
| AKAP13 | 0.2162 | CRYBA2 | 0.3656 | PACS2 | 2.9333 | 1049 | 3077 |
| TLE2 | 0.2161 | RBAKDN | 0.3655 | FAM84A | 2.9325 | 3793 | 11123 |
| LINC01021 | 0.2158 | ZNF331 | 0.3655 | KLHL17 | 2.9315 | 4292 | 12582 |
| FGF8 | 0.2157 | NR2E1 | 0.3653 | DECR2 | 2.9314 | 816 | 2392 |
| BTBD3 | 0.2157 | DGCR8 | 0.3649 | BATF3 | 2.9290 | 3720 | 10896 |
| OLFM1 | 0.2156 | MAPK11 | 0.3649 | TUBA8 | 2.9281 | 1766 | 5171 |
| MCAM | 0.2155 | BAIAP2 | 0.3646 | TPTE | 2.9217 | 383 | 1119 |
| LOC100129931 | 0.2155 | GRIN3A | 0.3643 | ALOX15P1 | 2.9194 | 1328 | 3877 |
| ZNF532 | 0.2152 | SLC6A11 | 0.3641 | FZD9 | 2.9135 | 532 | 1550 |
| ATRIP | 0.2151 | CYP27C1 | 0.3640 | SFT2D1 | 2.9031 | 3737 | 10849 |
| RASA3 | 0.2150 | FAM102A | 0.3637 | FBRS | 2.9026 | 1437 | 4171 |
| CR1L | 0.2148 | PHOX2A | 0.3635 | MEF2BNB-MEF2B | 2.9003 | 3611 | 10473 |
| CX3CL1 | 0.2145 | OBSCN | 0.3635 | B3GNT2 | 2.9002 | 3326 | 9646 |
| ZACN | 0.2144 | PCDHGA4 | 0.3634 | MEF2BNB | 2.8998 | 3612 | 10474 |
| CYP4F2 | 0.2144 | CXXC5 | 0.3634 | DOK1 | 2.8998 | 1297 | 3761 |
| COMMD3 | 0.2143 | RAX | 0.3633 | MAU2 | 2.8961 | 3291 | 9531 |
| COMMD3-BMI1 | 0.2143 | TBX4 | 0.3632 | IFITM3 | 2.8956 | 5687 | 16467 |
| APELA | 0.2142 | GPT | 0.3630 | FGF5 | 2.8949 | 4948 | 14324 |
| CLMP | 0.2142 | LGALS1 | 0.3630 | SLC4A11 | 2.8906 | 2321 | 6709 |
| PSD3 | 0.2142 | EEF2 | 0.3628 | NRARP | 2.8886 | 6589 | 19033 |
| HTRA4 | 0.2142 | PIF1 | 0.3626 | SIM1 | 2.8883 | 4845 | 13994 |
| NOL3 | 0.2140 | HNF1A | 0.3625 | ATRN | 2.8878 | 3966 | 11453 |
| NFASC | 0.2139 | TPCN2 | 0.3622 | FBXO32 | 2.8818 | 3477 | 10020 |
| CHRNA4 | 0.2139 | PCDHGA10 | 0.3622 | GPR135 | 2.8810 | 2680 | 7721 |
| ARID5A | 0.2137 | RTN4R | 0.3621 | PPIL2 | 2.8789 | 2684 | 7727 |
| MIR100HG | 0.2137 | ESAM | 0.3621 | PIP5K1P1 | 2.8775 | 3943 | 11346 |
| TMEM184B | 0.2136 | CLUAP1 | 0.3620 | NR6A1 | 2.8764 | 5954 | 17126 |
| GAREM | 0.2135 | SH3RF3-AS1 | 0.3619 | NRBP2 | 2.8763 | 2466 | 7093 |
| PCDHA2 | 0.2134 | MIR193A | 0.3618 | CYP27B1 | 2.8753 | 1275 | 3666 |
| TMEM88 | 0.2131 | MFAP2 | 0.3617 | HIST1H2BN | 2.8748 | 5918 | 17013 |
| IZUMO4 | 0.2130 | LCN10 | 0.3615 | SLC25A30 | 2.8694 | 1432 | 4109 |
| ADGRB2 | 0.2130 | CLU | 0.3615 | ATP5J2-PTCD1 | 2.8684 | 4780 | 13711 |
| ABHD14B | 0.2130 | IFT140 | 0.3614 | ZNF436 | 2.8681 | 379 | 1087 |
| LOC100128239 | 0.2129 | DNAH17-AS1 | 0.3614 | ATP5J2 | 2.8680 | 4781 | 13712 |
| PSMG3 | 0.2126 | PSMG3 | 0.3613 | CNOT3 | 2.8602 | 3862 | 11046 |
| ATP6V0CP3 | 0.2124 | RASA3 | 0.3612 | GPX4 | 2.8551 | 483 | 1379 |
| PLEKHG3 | 0.2124 | AMH | 0.3612 | CRACR2B | 2.8542 | 624 | 1781 |
| PSMG3-AS1 | 0.2124 | MIR4522 | 0.3611 | TUBB4B | 2.8534 | 4337 | 12375 |
| FAIM2 | 0.2121 | GGCT | 0.3611 | ATE1 | 2.8506 | 2946 | 8398 |
| LOC100507346 | 0.2121 | NUDT16L1 | 0.3611 | AK4 | 2.8488 | 959 | 2732 |
| MICALL2 | 0.2121 | SLC6A5 | 0.3610 | SYT5 | 2.8471 | 4101 | 11676 |
| ZMIZ1 | 0.2119 | RPS6KA1 | 0.3607 | ADGRA1 | 2.8440 | 141 | 401 |
| SRD5A2 | 0.2118 | PSMG3-AS1 | 0.3606 | HIST1H3H | 2.8399 | 3498 | 9934 |
| MIR330 | 0.2118 | GGN | 0.3603 | GPR6 | 2.8381 | 914 | 2594 |
| APBB1 | 0.2117 | CTSF | 0.3603 | PNPLA2 | 2.8349 | 1042 | 2954 |
| ABHD17A | 0.2117 | PTGER3 | 0.3602 | LOXL1 | 2.8340 | 1602 | 4540 |

| | | | | | | | |
|---|---|---|---|---|---|---|---|
| RASGRP2 | 0.2116 | MYBBP1A | 0.3601 | MGA | 2.8328 | 6017 | 17045 |
| ZNF177 | 0.2116 | ASPDH | 0.3601 | SMAD4 | 2.8312 | 2210 | 6257 |
| ZNF471 | 0.2115 | ZNF649 | 0.3600 | WTIP | 2.8282 | 716 | 2025 |
| MFSD11 | 0.2114 | TRIM4 | 0.3600 | YPEL3 | 2.8250 | 623 | 1760 |
| CACNB1 | 0.2114 | GPR25 | 0.3596 | SHISA7 | 2.8221 | 1585 | 4473 |
| CA14 | 0.2113 | YPEL3 | 0.3596 | HPCAL4 | 2.8220 | 2455 | 6928 |
| PODXL2 | 0.2112 | CRACR2B | 0.3596 | SPATA33 | 2.8212 | 4044 | 11409 |
| PITX1 | 0.2109 | MFSD10 | 0.3595 | FKBP11 | 2.8203 | 1786 | 5037 |
| CLDN4 | 0.2108 | MCAM | 0.3594 | KCMF1 | 2.8198 | 7309 | 20610 |
| LINC00905 | 0.2107 | WNT7B | 0.3594 | GABBR2 | 2.8185 | 3190 | 8991 |
| BTBD19 | 0.2107 | SP5 | 0.3593 | C19orf83 | 2.8174 | 241 | 679 |
| C2orf70 | 0.2106 | FZD2 | 0.3590 | ASCL1 | 2.8160 | 2783 | 7837 |
| TRA2B | 0.2105 | EPHA4 | 0.3589 | HIST1H2AK | 2.8146 | 4287 | 12066 |
| DAB2IP | 0.2105 | SLC15A3 | 0.3589 | DDX19B | 2.8103 | 5276 | 14827 |
| ZIC5 | 0.2104 | SLC17A7 | 0.3585 | TTYH3 | 2.8102 | 1254 | 3524 |
| ZNF709 | 0.2104 | ZNF208 | 0.3584 | GATA6 | 2.8079 | 4445 | 12481 |
| COL22A1 | 0.2102 | MARVELD1 | 0.3582 | CPNE9 | 2.8067 | 5354 | 15027 |
| PCDHB12 | 0.2101 | HTR1A | 0.3581 | LINC00909 | 2.8049 | 4537 | 12726 |
| CACNB4 | 0.2100 | ARHGAP23 | 0.3579 | MAD2L2 | 2.8039 | 459 | 1287 |
| CMIP | 0.2100 | PKP3 | 0.3579 | ATG4B | 2.8031 | 2473 | 6932 |
| RNF112 | 0.2096 | PRDM7 | 0.3578 | LRRK1 | 2.8015 | 3975 | 11136 |
| NOTCH1 | 0.2095 | CCDC85A | 0.3577 | RSPRY1 | 2.8012 | 5688 | 15933 |
| SENP2 | 0.2094 | NR3C1 | 0.3577 | UCK1 | 2.8011 | 5053 | 14154 |
| ZBTB20 | 0.2093 | PFKP | 0.3574 | VAX1 | 2.7961 | 775 | 2167 |
| TMEM40 | 0.2093 | ARC | 0.3571 | ZNF318 | 2.7874 | 2888 | 8050 |
| CERKL | 0.2093 | CENPB | 0.3570 | WDR45B | 2.7856 | 3550 | 9889 |
| MAP7 | 0.2091 | DNAJB6 | 0.3569 | MAN1B1-AS1 | 2.7848 | 7114 | 19811 |
| MIR497HG | 0.2088 | MIRLET7B | 0.3566 | CARD14 | 2.7828 | 4125 | 11479 |
| GLTPD2 | 0.2087 | FBXL16 | 0.3563 | ZMYM5 | 2.7810 | 1498 | 4166 |
| SLC25A18 | 0.2087 | MARVELD2 | 0.3563 | PPAP2C | 2.7807 | 187 | 520 |
| FAM13A | 0.2086 | RGMA | 0.3559 | DNAJB1 | 2.7764 | 4124 | 11450 |
| PKD2L2 | 0.2086 | LVRN | 0.3558 | ADCK5 | 2.7764 | 3050 | 8468 |
| LIME1 | 0.2086 | TEX22 | 0.3558 | ZHX2 | 2.7725 | 5485 | 15207 |
| KIAA1522 | 0.2083 | SARDH | 0.3556 | WHSC1 | 2.7711 | 2250 | 6235 |
| ESRP2 | 0.2080 | LAPTM4B | 0.3551 | RBM8A | 2.7705 | 4205 | 11650 |
| APOL2 | 0.2080 | ASB6 | 0.3548 | SP6 | 2.7694 | 941 | 2606 |
| RALGDS | 0.2079 | PCDHB13 | 0.3545 | CAMSAP3 | 2.7668 | 3963 | 10965 |
| PCDHB8 | 0.2078 | TADA2B | 0.3544 | LBX2-AS1 | 2.7662 | 77 | 213 |
| ASIC4 | 0.2078 | PRSS3 | 0.3544 | LOXL1-AS1 | 2.7642 | 1514 | 4185 |
| TGIF2 | 0.2076 | ABHD17A | 0.3543 | PAX9 | 2.7618 | 5873 | 16220 |
| ABLIM2 | 0.2074 | OLIG3 | 0.3542 | CAST | 2.7611 | 1842 | 5086 |
| BCL2L10 | 0.2074 | MAPT-IT1 | 0.3542 | PCDHGB7 | 2.7605 | 3156 | 8712 |
| USP6 | 0.2073 | PCDHGA1 | 0.3540 | FGF14 | 2.7586 | 990 | 2731 |
| LOC399815 | 0.2073 | BRF1 | 0.3540 | PLA2G16 | 2.7583 | 4134 | 11403 |
| FAM24B-CUZD1 | 0.2073 | ZACN | 0.3535 | COLCA2 | 2.7490 | 1032 | 2837 |
| FAM24B | 0.2073 | ARHGAP22 | 0.3534 | MTL5 | 2.7475 | 301 | 827 |
| KLHL35 | 0.2073 | ABCC5 | 0.3532 | HOXC6 | 2.7461 | 2198 | 6036 |
| CYFIP2 | 0.2072 | TBCD | 0.3530 | HOXC5 | 2.7453 | 2199 | 6037 |
| ARC | 0.2070 | MOS | 0.3530 | PPP1R9B | 2.7453 | 7117 | 19538 |
| LHX6 | 0.2069 | MIR589 | 0.3528 | HOXC4 | 2.7445 | 2200 | 6038 |
| TACSTD2 | 0.2067 | LOC100130872 | 0.3527 | LOC146880 | 2.7414 | 58 | 159 |
| SPATA2 | 0.2067 | FYTTD1 | 0.3525 | BRF1 | 2.7398 | 661 | 1811 |
| GLUL | 0.2066 | TPPP3 | 0.3525 | VPS9D1-AS1 | 2.7382 | 424 | 1161 |
| CYB5R3 | 0.2066 | HSPA8 | 0.3523 | GATA5 | 2.7367 | 5196 | 14220 |
| MGST1 | 0.2066 | PCDHGB8P | 0.3522 | DGKG | 2.7352 | 2266 | 6198 |
| LRRC61 | 0.2066 | ELMO1 | 0.3522 | SLITRK5 | 2.7347 | 2914 | 7969 |
| SLC3A2 | 0.2066 | CD177 | 0.3520 | LINC01019 | 2.7335 | 2732 | 7468 |
| SNORD12 | 0.2066 | DNMBP | 0.3519 | CHMP1A | 2.7329 | 4219 | 11530 |
| ACTR3C | 0.2063 | SMIM17 | 0.3517 | TAS1R3 | 2.7326 | 1305 | 3566 |
| TM6SF1 | 0.2063 | MIR4745 | 0.3515 | CTNND1 | 2.7325 | 2329 | 6364 |
| NAMA | 0.2062 | FERD3L | 0.3515 | PRPF8 | 2.7317 | 3929 | 10733 |
| C19orf83 | 0.2062 | FGF19 | 0.3514 | TMTC1 | 2.7305 | 3815 | 10417 |
| FAM60A | 0.2060 | SLC4A8 | 0.3512 | WT1-AS | 2.7296 | 7299 | 19925 |
| RMST | 0.2060 | PSPH | 0.3511 | KLF14 | 2.7290 | 2723 | 7431 |
| PAPLN | 0.2060 | NUPR1L | 0.3511 | TSEN34 | 2.7272 | 5866 | 16003 |
| RASGRP3 | 0.2059 | BSG | 0.3510 | ITGA8 | 2.7245 | 3016 | 8217 |
| FLJ13224 | 0.2057 | MIR4763 | 0.3509 | HGS | 2.7244 | 2152 | 5863 |
| SALL4 | 0.2057 | PTRF | 0.3509 | C1orf159 | 2.7210 | 4588 | 12464 |
| CCDC172 | 0.2057 | GJC2 | 0.3509 | INSM2 | 2.7203 | 7557 | 20557 |

## SUPPLEMENTARY DATA 2

Supplementary Data 2 provides Gene Ontology (GO) annotation results for some genomic sample pairs using gene rankings based on the magnitude of the differential mean methylation level (dMML), the Jensen-Shannon distance (JSD), and the relative Jensen-Shannon distance (RJSD). Supplementary Data 2 as attached hereto includes a portion of the collective data set as a representative sample.

stem-VS-brain-1

Only process categories with b ≥ 5 are shown.

| dMML MAGNITUDE RANKING | | | | | | |
|---|---|---|---|---|---|---|
| PROCESS DESCRIPTION | FDR q-VALUE | ENRICHMENT | N | B | n | b |
| cellular response to zinc ion | 5.64E-03 | 16 | 17331 | 16 | 334 | 5 |
| negative regulation of androgen receptor signaling pathway | 1.21E-02 | 12 | 17331 | 13 | 539 | 5 |
| anterior/posterior axis specification | 2.96E-02 | 11 | 17331 | 35 | 216 | 5 |
| response to follicle-stimulating hormone | 1.55E-02 | 11 | 17331 | 12 | 646 | 5 |
| modulation of excitatory postsynaptic potential | 1.05E-02 | 11 | 17331 | 28 | 348 | 6 |
| cell fate specification | 1.07E-02 | 9 | 17331 | 67 | 202 | 7 |
| regulation of androgen receptor signaling pathway | 1.87E-02 | 9 | 17331 | 22 | 539 | 6 |
| cellular response to gonadotropin stimulus | 4.29E-03 | 9 | 17331 | 16 | 885 | 7 |
| protein kinase C-activating G-protein coupled receptor signaling pathway | 2.59E-02 | 9 | 17331 | 31 | 392 | 6 |
| calcium-dependent cell-cell adhesion via plasma membrane cell adhesion molecules | 2.34E-06 | 9 | 17331 | 26 | 1012 | 13 |
| long-term memory | 2.46E-02 | 9 | 17331 | 28 | 435 | 6 |
| cellular response to follicle-stimulating hormone stimulus | 2.45E-02 | 8 | 17331 | 8 | 1318 | 5 |
| negative regulation of stem cell differentiation | 5.30E-04 | 8 | 17331 | 43 | 501 | 10 |
| negative regulation of peptidyl-tyrosine phosphorylation | 1.42E-02 | 8 | 17331 | 40 | 378 | 7 |
| atrioventricular valve morphogenesis | 2.01E-02 | 8 | 17331 | 14 | 964 | 6 |
| female gonad development | 3.08E-02 | 7 | 17331 | 16 | 910 | 6 |
| homophilic cell adhesion via plasma membrane adhesion molecules | 1.72E-18 | 6 | 17331 | 148 | 852 | 46 |
| regulation of gluconeogenesis | 1.72E-02 | 6 | 17331 | 36 | 616 | 8 |
| male gonad development | 2.69E-02 | 6 | 17331 | 82 | 273 | 8 |
| negative regulation of epithelial to mesenchymal transition | 1.18E-02 | 6 | 17331 | 22 | 1023 | 8 |
| gonad development | 1.55E-02 | 6 | 17331 | 95 | 273 | 9 |
| positive regulation of neuroblast proliferation | 1.42E-02 | 6 | 17331 | 22 | 1064 | 8 |
| response to gonadotropin | 7.85E-03 | 6 | 17331 | 29 | 910 | 9 |
| positive regulation of organ growth | 1.10E-02 | 6 | 17331 | 37 | 723 | 9 |
| heart valve morphogenesis | 1.86E-02 | 6 | 17331 | 25 | 964 | 8 |
| axis specification | 1.90E-02 | 6 | 17331 | 74 | 502 | 12 |
| regulation of neuroblast proliferation | 7.45E-03 | 5 | 17331 | 31 | 1064 | 10 |
| regulation of epithelial to mesenchymal transition | 1.42E-02 | 5 | 17331 | 66 | 501 | 10 |
| cell-cell adhesion via plasma-membrane adhesion molecules | 7.31E-17 | 5 | 17331 | 197 | 865 | 51 |
| synapse assembly | 8.15E-04 | 5 | 17331 | 59 | 820 | 14 |
| negative regulation of focal adhesion assembly | 1.82E-02 | 5 | 17331 | 14 | 2014 | 8 |
| regulation of stem cell differentiation | 3.02E-04 | 5 | 17331 | 118 | 526 | 17 |
| endothelial cell development | 1.30E-02 | 5 | 17331 | 26 | 1437 | 10 |
| negative regulation of adherens junction organization | 2.91E-02 | 5 | 17331 | 15 | 2014 | 8 |
| synapse organization | 3.96E-06 | 4 | 17331 | 119 | 841 | 25 |
| regulation of embryonic development | 5.65E-03 | 4 | 17331 | 109 | 517 | 14 |
| purine nucleoside transmembrane transport | 2.58E-02 | 4 | 17331 | 6 | 4064 | 6 |
| positive regulation of neural precursor cell proliferation | 1.82E-02 | 4 | 17331 | 38 | 1422 | 12 |
| very-low-density lipoprotein particle assembly | 1.78E-02 | 4 | 17331 | 9 | 4078 | 8 |
| negative regulation of ERK1 and ERK2 cascade | 1.57E-02 | 4 | 17331 | 50 | 1197 | 13 |
| striated muscle tissue development | 3.66E-04 | 4 | 17331 | 86 | 1260 | 22 |
| regulation of peptidyl-tyrosine phosphorylation | 1.14E-02 | 3 | 17331 | 212 | 328 | 14 |
| muscle tissue development | 1.23E-04 | 3 | 17331 | 100 | 1260 | 25 |
| regulation of cell-substrate adhesion | 2.14E-02 | 3 | 17331 | 169 | 393 | 13 |
| negative regulation of cell-substrate adhesion | 1.33E-02 | 3 | 17331 | 50 | 1546 | 15 |
| positive regulation of muscle tissue development | 2.49E-02 | 3 | 17331 | 54 | 1344 | 14 |
| cyclic nucleotide metabolic process | 2.00E-02 | 3 | 17331 | 56 | 1425 | 15 |
| regulation of RNA splicing | 1.30E-02 | 3 | 17331 | 86 | 1061 | 17 |
| muscle cell fate commitment | 4.06E-03 | 3 | 17331 | 10 | 5400 | 10 |
| nervous system development | 2.31E-05 | 3 | 17331 | 245 | 698 | 31 |
| skeletal muscle tissue development | 1.22E-02 | 3 | 17331 | 54 | 1772 | 17 |
| regulation of carbohydrate biosynthetic process | 1.30E-02 | 3 | 17331 | 84 | 1208 | 18 |
| muscle structure development | 2.46E-03 | 3 | 17331 | 108 | 1226 | 23 |
| regulation of voltage-gated calcium channel activity | 2.41E-02 | 3 | 17331 | 22 | 3456 | 13 |
| muscle organ development | 4.52E-03 | 3 | 17331 | 105 | 1226 | 22 |
| positive regulation of developmental growth | 1.53E-04 | 3 | 17331 | 154 | 1072 | 28 |
| columnar/cuboidal epithelial cell differentiation | 3.02E-03 | 3 | 17331 | 69 | 1897 | 22 |
| regulation of dendrite morphogenesis | 1.78E-02 | 3 | 17331 | 66 | 1630 | 18 |
| maintenance of cell number | 9.98E-03 | 3 | 17331 | 142 | 765 | 18 |
| regulation of microtubule polymerization | 1.48E-02 | 3 | 17331 | 32 | 3033 | 16 |
| regulation of cell-matrix adhesion | 7.18E-03 | 3 | 17331 | 88 | 1546 | 22 |
| regulation of dendrite development | 5.31E-04 | 3 | 17331 | 110 | 1630 | 29 |
| ionotropic glutamate receptor signaling pathway | 1.23E-02 | 3 | 17331 | 23 | 4055 | 15 |
| negative regulation of cellular response to growth factor stimulus | 2.68E-02 | 3 | 17331 | 132 | 754 | 16 |
| regulation of neural precursor cell proliferation | 2.00E-02 | 3 | 17331 | 73 | 1774 | 20 |
| axonogenesis | 2.59E-02 | 3 | 17331 | 109 | 1815 | 29 |
| sensory organ development | 6.11E-03 | 3 | 17331 | 106 | 1756 | 27 |
| negative regulation of neural precursor cell proliferation | 2.13E-02 | 2 | 17331 | 24 | 4639 | 16 |
| negative regulation of cell morphogenesis involved in differentiation | 8.25E-04 | 2 | 17331 | 109 | 2106 | 33 |
| tube development | 2.60E-02 | 2 | 17331 | 182 | 767 | 20 |
| transmembrane receptor protein serine/threonine kinase signaling pathway | 2.89E-02 | 2 | 17331 | 198 | 707 | 20 |
| cardiac septum morphogenesis | 7.44E-03 | 2 | 17331 | 46 | 3565 | 23 |
| response to growth factor | 1.01E-02 | 2 | 17331 | 243 | 780 | 26 |
| multicellular organismal signaling | 9.64E-03 | 2 | 17331 | 124 | 1717 | 29 |
| regulation of locomotion | 1.38E-04 | 2 | 17331 | 728 | 457 | 45 |
| cell-cell adhesion | 2.77E-07 | 2 | 17331 | 579 | 872 | 68 |
| system development | 3.45E-06 | 2 | 17331 | 639 | 701 | 60 |
| glutamate receptor signaling pathway | 2.02E-02 | 2 | 17331 | 39 | 4055 | 21 |
| regulation of axonogenesis | 1.59E-03 | 2 | 17331 | 153 | 1635 | 33 |
| trans-synaptic signaling | 1.47E-03 | 2 | 17331 | 448 | 649 | 38 |
| synaptic transmission | 1.47E-03 | 2 | 17331 | 448 | 649 | 38 |
| synaptic signaling | 1.48E-03 | 2 | 17331 | 448 | 649 | 38 |

| | | | | | | |
|---|---|---|---|---|---|---|
| negative regulation of secretion | 1.85E-02 | 2 | 17331 | 188 | 1019 | 25 |
| regulation of epithelial cell migration | 2.93E-02 | 2 | 17331 | 158 | 1126 | 23 |
| regulation of cell morphogenesis involved in differentiation | 2.24E-06 | 2 | 17331 | 314 | 1635 | 65 |
| steroid hormone mediated signaling pathway | 6.56E-03 | 2 | 17331 | 59 | 3911 | 29 |
| negative regulation of cell motility | 3.01E-04 | 2 | 17331 | 202 | 1733 | 44 |
| cell morphogenesis involved in differentiation | 1.01E-02 | 2 | 17331 | 156 | 1482 | 29 |
| negative regulation of cell migration | 5.58E-04 | 2 | 17331 | 194 | 1733 | 42 |
| embryonic hindlimb morphogenesis | 2.65E-02 | 2 | 17331 | 30 | 5374 | 20 |
| negative regulation of phosphate metabolic process | 2.38E-02 | 2 | 17331 | 529 | 441 | 29 |
| negative regulation of phosphorus metabolic process | 2.39E-02 | 2 | 17331 | 529 | 441 | 29 |
| positive regulation of cell growth | 1.41E-02 | 2 | 17331 | 137 | 1725 | 29 |
| regulation of Rho protein signal transduction | 1.24E-04 | 2 | 17331 | 101 | 3797 | 47 |
| positive regulation of growth | 2.60E-04 | 2 | 17331 | 228 | 1725 | 48 |
| positive regulation of cell morphogenesis involved in differentiation | 8.85E-03 | 2 | 17331 | 150 | 1695 | 31 |
| positive regulation of nucleic acid-templated transcription | 8.24E-04 | 2 | 17331 | 1367 | 282 | 47 |
| positive regulation of transcription, DNA-templated | 8.29E-04 | 2 | 17331 | 1367 | 282 | 47 |
| positive regulation of RNA biosynthetic process | 6.67E-04 | 2 | 17331 | 1395 | 282 | 48 |
| renal system process | 1.87E-02 | 2 | 17331 | 93 | 2757 | 31 |
| negative regulation of locomotion | 1.55E-04 | 2 | 17331 | 249 | 1733 | 52 |
| neuron projection morphogenesis | 2.35E-06 | 2 | 17331 | 187 | 2877 | 65 |
| negative regulation of cellular component movement | 4.00E-04 | 2 | 17331 | 231 | 1733 | 48 |
| negative regulation of signal transduction | 8.17E-05 | 2 | 17331 | 1036 | 502 | 62 |
| protein targeting to plasma membrane | 1.71E-02 | 2 | 17331 | 23 | 6937 | 19 |
| cell morphogenesis | 6.81E-03 | 2 | 17331 | 205 | 1482 | 36 |
| positive regulation of neuron projection development | 1.36E-03 | 2 | 17331 | 213 | 1748 | 44 |
| regulation of developmental growth | 9.19E-05 | 2 | 17331 | 291 | 1725 | 59 |
| circadian regulation of gene expression | 1.68E-02 | 2 | 17331 | 57 | 4351 | 29 |
| cellular response to acid chemical | 2.45E-02 | 2 | 17331 | 172 | 1495 | 30 |
| cell fate commitment | 4.13E-05 | 2 | 17331 | 149 | 3245 | 56 |
| regulation of cell morphogenesis | 1.19E-07 | 2 | 17331 | 483 | 1699 | 95 |
| positive regulation of nervous system development | 4.76E-08 | 2 | 17331 | 410 | 2043 | 97 |
| striated muscle cell differentiation | 2.55E-02 | 2 | 17331 | 49 | 4796 | 27 |
| regulation of cell junction assembly | 2.41E-02 | 2 | 17331 | 65 | 4029 | 30 |
| negative regulation of cell development | 2.91E-05 | 2 | 17331 | 290 | 2015 | 67 |
| detection of external stimulus | 8.31E-04 | 2 | 17331 | 183 | 2292 | 48 |
| regulation of protein binding | 1.04E-02 | 2 | 17331 | 166 | 1912 | 36 |
| purine ribonucleotide metabolic process | 1.04E-02 | 2 | 17331 | 237 | 1465 | 39 |
| detection of abiotic stimulus | 1.33E-02 | 2 | 17331 | 187 | 2292 | 48 |
| cell projection morphogenesis | 1.02E-04 | 2 | 17331 | 246 | 2292 | 63 |
| negative regulation of signaling | 3.76E-04 | 2 | 17331 | 1145 | 502 | 64 |
| positive regulation of developmental process | 2.46E-09 | 2 | 17331 | 1100 | 1072 | 131 |
| cell-cell junction organization | 1.54E-03 | 2 | 17331 | 167 | 2604 | 48 |
| cell migration | 4.98E-03 | 2 | 17331 | 723 | 629 | 50 |
| negative regulation of cell communication | 5.17E-04 | 2 | 17331 | 1157 | 502 | 64 |
| ribonucleotide metabolic process | 1.44E-02 | 2 | 17331 | 250 | 1465 | 40 |
| positive regulation of protein phosphorylation | 2.65E-02 | 2 | 17331 | 966 | 380 | 40 |
| cell junction assembly | 1.75E-03 | 2 | 17331 | 165 | 2683 | 48 |
| regulation of binding | 6.83E-03 | 2 | 17331 | 277 | 1528 | 46 |
| cell junction organization | 3.77E-04 | 2 | 17331 | 193 | 2773 | 58 |
| positive regulation of neurogenesis | 1.19E-06 | 2 | 17331 | 362 | 2424 | 95 |
| regulation of anatomical structure morphogenesis | 7.11E-11 | 2 | 17331 | 881 | 1650 | 158 |
| positive regulation of macromolecule biosynthetic process | 1.04E-02 | 2 | 17331 | 1581 | 282 | 48 |
| positive regulation of phosphorylation | 2.86E-02 | 2 | 17331 | 1003 | 380 | 41 |
| purine-containing compound metabolic process | 6.93E-03 | 2 | 17331 | 306 | 1465 | 48 |
| muscle cell differentiation | 1.25E-04 | 2 | 17331 | 117 | 4868 | 61 |
| cell part morphogenesis | 1.25E-04 | 2 | 17331 | 266 | 2418 | 69 |
| modulation of synaptic transmission | 1.54E-03 | 2 | 17331 | 277 | 1892 | 56 |
| purine nucleotide metabolic process | 2.51E-02 | 2 | 17331 | 259 | 1465 | 40 |
| locomotory behavior | 1.82E-03 | 2 | 17331 | 183 | 2743 | 53 |
| regulation of secretion | 5.83E-03 | 2 | 17331 | 664 | 785 | 55 |
| positive regulation of neuron differentiation | 1.16E-04 | 2 | 17331 | 291 | 2414 | 74 |
| signaling | 3.77E-05 | 2 | 17331 | 801 | 1067 | 90 |
| positive regulation of hydrolase activity | 1.52E-07 | 2 | 17331 | 802 | 1455 | 123 |
| growth | 3.48E-03 | 2 | 17331 | 364 | 1464 | 58 |
| regulation of growth | 1.09E-06 | 2 | 17331 | 614 | 1725 | 111 |
| regulation of cellular carbohydrate catabolic process | 1.01E-02 | 2 | 17331 | 42 | 7055 | 31 |
| regulation of carbohydrate catabolic process | 1.01E-02 | 2 | 17331 | 42 | 7055 | 31 |
| negative regulation of cell adhesion | 3.07E-02 | 2 | 17331 | 220 | 1695 | 39 |
| ribose phosphate metabolic process | 3.06E-02 | 2 | 17331 | 262 | 1465 | 40 |
| regulation of protein secretion | 2.84E-02 | 2 | 17331 | 394 | 1019 | 42 |
| positive regulation of cellular biosynthetic process | 1.42E-02 | 2 | 17331 | 1701 | 282 | 50 |
| single organism signaling | 5.68E-05 | 2 | 17331 | 798 | 1067 | 89 |
| developmental growth | 7.61E-03 | 2 | 17331 | 283 | 1735 | 51 |
| positive regulation of cell projection organization | 3.03E-04 | 2 | 17331 | 284 | 2414 | 71 |
| positive regulation of multicellular organismal process | 1.05E-08 | 2 | 17331 | 1350 | 1074 | 150 |
| positive regulation of cell development | 6.41E-07 | 2 | 17331 | 455 | 2461 | 115 |
| regulation of nervous system development | 2.28E-10 | 2 | 17331 | 707 | 2337 | 170 |
| embryonic appendage morphogenesis | 1.68E-02 | 2 | 17331 | 86 | 4783 | 42 |
| embryonic limb morphogenesis | 1.69E-02 | 2 | 17331 | 86 | 4783 | 42 |
| regulation of small GTPase mediated signal transduction | 2.38E-06 | 2 | 17331 | 263 | 3647 | 98 |
| establishment of protein localization to plasma membrane | 2.92E-02 | 2 | 17331 | 87 | 4522 | 40 |
| regulation of synaptic plasticity | 9.80E-04 | 2 | 17331 | 142 | 4055 | 58 |
| regulation of synapse structure or activity | 7.49E-04 | 2 | 17331 | 144 | 4055 | 59 |
| cell-cell signaling | 1.28E-03 | 2 | 17331 | 711 | 1032 | 74 |
| cellular component morphogenesis | 2.35E-06 | 2 | 17331 | 455 | 2461 | 113 |
| regulation of cell projection organization | 1.06E-07 | 2 | 17331 | 496 | 2859 | 133 |

| | | | | | | |
|---|---|---|---|---|---|---|
| morphogenesis of a branching structure | 2.88E-02 | 2 | 17331 | 166 | 2461 | 41 |
| cardiac conduction | 1.75E-02 | 2 | 17331 | 109 | 4302 | 47 |
| regulation of neurogenesis | 2.41E-08 | 2 | 17331 | 630 | 2337 | 148 |
| response to starvation | 1.56E-02 | 2 | 17331 | 157 | 2938 | 46 |
| regulation of protein phosphorylation | 1.09E-02 | 2 | 17331 | 1341 | 457 | 61 |
| regulation of protein transport | 6.82E-03 | 2 | 17331 | 722 | 872 | 63 |
| regulation of apoptotic process | 3.25E-03 | 2 | 17331 | 1385 | 505 | 70 |
| regulation of catalytic activity | 4.00E-05 | 2 | 17331 | 2227 | 464 | 103 |
| regulation of programmed cell death | 3.86E-03 | 2 | 17331 | 1395 | 505 | 70 |
| regulation of muscle cell differentiation | 1.14E-02 | 2 | 17331 | 150 | 3245 | 48 |
| regulation of organ morphogenesis | 7.01E-03 | 2 | 17331 | 177 | 3027 | 53 |
| regulation of establishment of protein localization | 6.32E-03 | 2 | 17331 | 790 | 893 | 69 |
| regulation of cellular component movement | 1.16E-05 | 2 | 17331 | 725 | 1748 | 123 |
| regulation of phosphorylation | 1.66E-02 | 2 | 17331 | 1428 | 457 | 63 |
| response to organic cyclic compound | 1.89E-03 | 2 | 17331 | 773 | 1099 | 82 |
| regulation of cell migration | 9.20E-05 | 2 | 17331 | 635 | 1748 | 107 |
| regulation of cell development | 1.74E-10 | 2 | 17331 | 803 | 2669 | 207 |
| regulation of cation channel activity | 1.83E-02 | 2 | 17331 | 84 | 5853 | 47 |
| regulation of actin filament-based process | 1.37E-05 | 2 | 17331 | 295 | 3782 | 107 |
| regionalization | 2.35E-02 | 2 | 17331 | 238 | 2340 | 53 |
| amino acid transport | 1.85E-02 | 2 | 17331 | 119 | 4776 | 54 |
| negative regulation of transcription from RNA polymerase II promoter | 2.13E-03 | 2 | 17331 | 714 | 1250 | 85 |
| embryonic organ morphogenesis | 4.08E-03 | 2 | 17331 | 122 | 5459 | 63 |
| regulation of actin cytoskeleton organization | 4.96E-05 | 2 | 17331 | 262 | 3991 | 99 |
| regulation of cell motility | 1.25E-04 | 2 | 17331 | 670 | 1748 | 111 |
| ameboidal-type cell migration | 1.96E-02 | 2 | 17331 | 149 | 3645 | 51 |
| regulation of phosphate metabolic process | 1.30E-02 | 2 | 17331 | 1649 | 457 | 71 |
| epithelial cell differentiation | 4.29E-03 | 2 | 17331 | 323 | 2439 | 74 |
| transmembrane receptor protein tyrosine kinase signaling pathway | 5.33E-04 | 2 | 17331 | 746 | 1457 | 102 |
| cytoskeleton organization | 3.35E-04 | 2 | 17331 | 634 | 1766 | 105 |
| positive regulation of transcription from RNA polymerase II promoter | 9.20E-05 | 2 | 17331 | 984 | 1296 | 120 |
| positive regulation of GTPase activity | 3.55E-07 | 2 | 17331 | 482 | 3363 | 152 |
| regulation of protein polymerization | 2.29E-02 | 2 | 17331 | 167 | 3325 | 52 |
| regulation of cell death | 1.72E-02 | 2 | 17331 | 1481 | 505 | 70 |
| regulation of phosphorus metabolic process | 1.48E-02 | 2 | 17331 | 1660 | 457 | 71 |
| positive regulation of cellular component organization | 5.36E-03 | 2 | 17331 | 1062 | 824 | 82 |
| regulation of cell growth | 2.47E-06 | 2 | 17331 | 368 | 3836 | 132 |
| enzyme linked receptor protein signaling pathway | 4.35E-06 | 2 | 17331 | 939 | 1695 | 149 |
| negative regulation of cell projection organization | 9.41E-04 | 2 | 17331 | 133 | 5504 | 68 |
| pattern specification process | 2.10E-03 | 2 | 17331 | 385 | 2376 | 85 |
| regulation of hydrolase activity | 5.22E-06 | 2 | 17331 | 1204 | 1378 | 154 |
| regulation of protein modification process | 1.64E-02 | 2 | 17331 | 1711 | 469 | 74 |
| regulation of cytoskeleton organization | 4.70E-05 | 2 | 17331 | 396 | 3337 | 121 |
| tissue morphogenesis | 1.30E-02 | 2 | 17331 | 368 | 2177 | 73 |
| regulation of GTPase activity | 7.17E-07 | 2 | 17331 | 531 | 3363 | 163 |
| regulation of neurotransmitter levels | 3.22E-02 | 2 | 17331 | 145 | 3957 | 52 |
| negative regulation of neuron projection development | 2.54E-02 | 2 | 17331 | 114 | 5504 | 57 |
| regulation of protein localization | 2.74E-02 | 2 | 17331 | 921 | 893 | 74 |
| cell adhesion | 3.80E-05 | 2 | 17331 | 963 | 1747 | 151 |
| biological adhesion | 2.80E-05 | 2 | 17331 | 966 | 1747 | 152 |
| regulation of neuron differentiation | 2.06E-08 | 2 | 17331 | 522 | 4126 | 194 |
| positive regulation of cell differentiation | 9.36E-08 | 2 | 17331 | 800 | 2826 | 203 |
| establishment or maintenance of cell polarity | 1.22E-02 | 2 | 17331 | 111 | 6359 | 63 |
| gland development | 7.10E-03 | 2 | 17331 | 260 | 3360 | 78 |
| positive regulation of gene expression | 1.27E-05 | 2 | 17331 | 1681 | 1126 | 169 |
| learning | 6.57E-03 | 2 | 17331 | 137 | 5335 | 65 |
| calcium ion transmembrane transport | 1.27E-03 | 2 | 17331 | 157 | 5657 | 79 |
| negative regulation of developmental process | 1.57E-08 | 2 | 17331 | 772 | 3265 | 224 |
| regulation of Ras protein signal transduction | 7.59E-06 | 2 | 17331 | 176 | 7085 | 110 |
| regulation of system process | 4.78E-04 | 2 | 17331 | 489 | 2780 | 120 |
| regulation of multicellular organismal process | 6.70E-13 | 2 | 17331 | 2460 | 1782 | 387 |
| negative regulation of cell growth | 2.59E-03 | 2 | 17331 | 159 | 5509 | 77 |
| response to mechanical stimulus | 3.31E-03 | 2 | 17331 | 197 | 4743 | 82 |
| negative regulation of growth | 2.38E-04 | 2 | 17331 | 226 | 5238 | 104 |
| regulation of transcription from RNA polymerase II promoter | 7.54E-05 | 2 | 17331 | 1706 | 1078 | 161 |
| regulation of secretion by cell | 2.72E-02 | 2 | 17331 | 611 | 1519 | 81 |
| negative regulation of nucleobase-containing compound metabolic process | 6.57E-04 | 2 | 17331 | 164 | 6158 | 88 |
| regulation of calcium ion transport | 1.53E-04 | 2 | 17331 | 203 | 5940 | 105 |
| actin filament organization | 6.56E-04 | 2 | 17331 | 1329 | 1195 | 138 |
| negative regulation of nitrogen compound metabolic process | 1.25E-04 | 2 | 17331 | 1429 | 1260 | 157 |
| actin filament-based process | 1.36E-03 | 2 | 17331 | 313 | 3962 | 107 |
| cell projection organization | 8.98E-04 | 2 | 17331 | 646 | 2292 | 128 |
| positive regulation of catalytic activity | 1.87E-05 | 2 | 17331 | 1461 | 1499 | 189 |
| negative regulation of neuron differentiation | 2.75E-03 | 1 | 17331 | 182 | 5504 | 86 |
| multicellular organismal homeostasis | 2.38E-02 | 1 | 17331 | 106 | 7065 | 64 |
| regulation of heart contraction | 1.67E-03 | 1 | 17331 | 217 | 5236 | 97 |
| positive regulation of apoptotic process | 3.87E-03 | 1 | 17331 | 565 | 2360 | 114 |
| positive regulation of programmed cell death | 3.47E-03 | 1 | 17331 | 569 | 2360 | 115 |
| negative regulation of transcription, DNA-templated | 6.44E-04 | 1 | 17331 | 1113 | 1532 | 146 |
| negative regulation of cellular biosynthetic process | 6.58E-04 | 1 | 17331 | 1397 | 1260 | 150 |
| negative regulation of biosynthetic process | 4.63E-04 | 1 | 17331 | 1419 | 1260 | 153 |
| regulation of ion transmembrane transporter activity | 7.69E-03 | 1 | 17331 | 169 | 5516 | 79 |
| cellular response to organic cyclic compound | 2.02E-02 | 1 | 17331 | 325 | 3077 | 85 |
| negative regulation of nervous system development | 2.79E-03 | 1 | 17331 | 251 | 4643 | 99 |
| regulation of primary metabolic process | 2.59E-04 | 1 | 17331 | 5418 | 292 | 134 |
| regulation of transport | 8.53E-04 | 1 | 17331 | 1686 | 1033 | 148 |

| | | | | | | |
|---|---|---|---|---|---|---|
| cellular response to lipid | 2.10E-02 | 1 | 17331 | 326 | 3123 | 86 |
| regulation of cellular metabolic process | 3.54E-04 | 1 | 17331 | 5505 | 292 | 135 |
| negative regulation of cell proliferation | 9.79E-04 | 1 | 17331 | 632 | 2604 | 139 |
| neuron projection guidance | 4.34E-04 | 1 | 17331 | 538 | 3195 | 145 |
| positive regulation of RNA metabolic process | 6.37E-04 | 1 | 17331 | 1434 | 1328 | 160 |
| positive regulation of molecular function | 1.13E-05 | 1 | 17331 | 1708 | 1499 | 216 |
| regulation of muscle contraction | 1.21E-02 | 1 | 17331 | 145 | 6006 | 73 |
| axon guidance | 6.23E-04 | 1 | 17331 | 537 | 3195 | 144 |
| negative regulation of cellular macromolecule biosynthetic process | 6.85E-04 | 1 | 17331 | 1265 | 1532 | 162 |
| immune effector process | 1.40E-02 | 1 | 17331 | 429 | 2955 | 105 |
| regulation of blood circulation | 6.73E-05 | 1 | 17331 | 289 | 6006 | 144 |
| anatomical structure formation involved in morphogenesis | 1.63E-03 | 1 | 17331 | 799 | 2196 | 146 |
| cell communication | 8.22E-04 | 1 | 17331 | 916 | 2087 | 159 |
| negative regulation of nucleic acid-templated transcription | 4.33E-04 | 1 | 17331 | 1145 | 1781 | 170 |
| negative regulation of RNA biosynthetic process | 5.26E-04 | 1 | 17331 | 1158 | 1781 | 171 |
| behavior | 3.08E-05 | 1 | 17331 | 523 | 4154 | 181 |
| negative regulation of cell differentiation | 1.41E-05 | 1 | 17331 | 591 | 3984 | 196 |
| regulation of cellular localization | 2.94E-02 | 1 | 17331 | 1202 | 1077 | 107 |
| regulation of multicellular organismal development | 8.43E-10 | 1 | 17331 | 1542 | 3245 | 412 |
| regulation of blood pressure | 2.78E-02 | 1 | 17331 | 140 | 6086 | 70 |
| regulation of muscle system process | 7.42E-03 | 1 | 17331 | 185 | 6006 | 91 |
| negative regulation of neurogenesis | 3.76E-03 | 1 | 17331 | 232 | 5567 | 106 |
| calcium ion transport | 2.38E-04 | 1 | 17331 | 221 | 6918 | 125 |
| positive regulation of nucleobase-containing compound metabolic process | 5.29E-03 | 1 | 17331 | 1640 | 1078 | 145 |
| positive regulation of cellular component movement | 2.28E-02 | 1 | 17331 | 401 | 3225 | 105 |
| actin cytoskeleton organization | 5.96E-04 | 1 | 17331 | 262 | 6158 | 131 |
| tissue development | 3.62E-03 | 1 | 17331 | 567 | 3057 | 141 |
| negative regulation of RNA metabolic process | 1.05E-03 | 1 | 17331 | 1198 | 1781 | 174 |
| negative regulation of cellular metabolic process | 9.03E-04 | 1 | 17331 | 2250 | 964 | 177 |
| regulation of cell differentiation | 8.40E-10 | 1 | 17331 | 1441 | 3255 | 382 |
| adult behavior | 8.92E-03 | 1 | 17331 | 142 | 7252 | 83 |
| negative regulation of gene expression | 7.85E-03 | 1 | 17331 | 1455 | 1260 | 148 |
| positive regulation of nitrogen compound metabolic process | 4.52E-03 | 1 | 17331 | 1724 | 1126 | 157 |
| regulation of metal ion transport | 1.58E-05 | 1 | 17331 | 321 | 6705 | 174 |
| negative regulation of macromolecule biosynthetic process | 8.66E-04 | 1 | 17331 | 1339 | 1696 | 184 |
| regulation of transmembrane transporter activity | 8.43E-03 | 1 | 17331 | 173 | 6705 | 93 |
| regulation of developmental process | 5.79E-12 | 1 | 17331 | 2048 | 3287 | 538 |
| positive regulation of cell death | 4.08E-03 | 1 | 17331 | 601 | 3237 | 155 |
| transcription, DNA-templated | 8.66E-04 | 1 | 17331 | 2248 | 1148 | 205 |
| nucleic acid-templated transcription | 8.96E-04 | 1 | 17331 | 2249 | 1148 | 205 |
| regulation of neuron projection development | 2.34E-06 | 1 | 17331 | 373 | 7032 | 209 |
| positive regulation of macromolecule metabolic process | 1.23E-04 | 1 | 17331 | 2772 | 1080 | 238 |
| anatomical structure morphogenesis | 1.14E-08 | 1 | 17331 | 1319 | 3586 | 377 |
| regulation of cellular carbohydrate metabolic process | 1.78E-02 | 1 | 17331 | 156 | 7055 | 87 |
| regulation of cation transmembrane transport | 4.39E-03 | 1 | 17331 | 203 | 6771 | 109 |
| cellular developmental process | 4.99E-10 | 1 | 17331 | 2436 | 2467 | 474 |
| regulation of membrane potential | 2.68E-02 | 1 | 17331 | 346 | 4158 | 113 |
| positive regulation of biosynthetic process | 1.83E-02 | 1 | 17331 | 1730 | 1126 | 153 |
| regulation of cellular component biogenesis | 1.79E-04 | 1 | 17331 | 685 | 4068 | 219 |
| cell differentiation | 1.21E-05 | 1 | 17331 | 1687 | 2340 | 309 |
| learning or memory | 6.60E-03 | 1 | 17331 | 223 | 6876 | 119 |
| cognition | 1.69E-03 | 1 | 17331 | 253 | 6876 | 136 |
| divalent metal ion transport | 8.63E-04 | 1 | 17331 | 261 | 7229 | 147 |
| positive regulation of cellular protein metabolic process | 9.59E-03 | 1 | 17331 | 1414 | 1621 | 178 |
| regulation of localization | 2.42E-05 | 1 | 17331 | 2290 | 1717 | 306 |
| anion transport | 1.32E-02 | 1 | 17331 | 467 | 4110 | 148 |
| regulation of RNA metabolic process | 1.42E-05 | 1 | 17331 | 3408 | 1188 | 314 |
| positive regulation of ion transport | 5.41E-03 | 1 | 17331 | 222 | 7266 | 124 |
| muscle system process | 5.59E-03 | 1 | 17331 | 256 | 6701 | 132 |
| divalent inorganic cation transport | 1.26E-03 | 1 | 17331 | 264 | 7229 | 147 |
| apoptotic signaling pathway | 2.33E-03 | 1 | 17331 | 339 | 6199 | 161 |
| embryonic morphogenesis | 2.29E-03 | 1 | 17331 | 409 | 5400 | 170 |
| positive regulation of protein metabolic process | 1.10E-02 | 1 | 17331 | 1502 | 1621 | 187 |
| system process | 1.03E-04 | 1 | 17331 | 1306 | 2859 | 287 |
| negative regulation of multicellular organismal process | 2.81E-05 | 1 | 17331 | 962 | 3973 | 294 |
| single-multicellular organism process | 3.71E-08 | 1 | 17331 | 2567 | 2340 | 461 |
| neurological system process | 3.04E-02 | 1 | 17331 | 846 | 2414 | 156 |
| single-organism behavior | 3.75E-04 | 1 | 17331 | 393 | 6704 | 200 |
| cellular response to organic substance | 6.61E-03 | 1 | 17331 | 993 | 2687 | 203 |
| cell development | 1.25E-04 | 1 | 17331 | 578 | 5630 | 247 |
| positive regulation of metabolic process | 1.08E-07 | 1 | 17331 | 3433 | 1702 | 446 |
| regulation of cell communication | 1.08E-08 | 1 | 17331 | 2867 | 2310 | 505 |
| single-organism developmental process | 1.23E-09 | 1 | 17331 | 4195 | 1699 | 541 |
| developmental process | 1.43E-09 | 1 | 17331 | 4555 | 1699 | 590 |
| regulation of anatomical structure size | 1.05E-02 | 1 | 17331 | 348 | 5685 | 150 |
| regulation of ion transport | 1.02E-05 | 1 | 17331 | 566 | 6733 | 287 |
| regulation of gene expression | 2.02E-05 | 1 | 17331 | 3964 | 1176 | 352 |
| phospholipid biosynthetic process | 3.17E-02 | 1 | 17331 | 207 | 7150 | 111 |
| organic anion transport | 2.74E-02 | 1 | 17331 | 362 | 5241 | 142 |
| small GTPase mediated signal transduction | 9.96E-03 | 1 | 17331 | 759 | 3412 | 195 |
| RNA biosynthetic process | 9.87E-03 | 1 | 17331 | 2526 | 1148 | 218 |
| ion transmembrane transport | 7.45E-04 | 1 | 17331 | 754 | 4541 | 256 |
| regulation of cellular macromolecule biosynthetic process | 1.54E-04 | 1 | 17331 | 3615 | 1188 | 322 |
| regulation of nucleobase-containing compound metabolic process | 7.68E-05 | 1 | 17331 | 3715 | 1188 | 332 |
| regulation of Wnt signaling pathway | 9.91E-03 | 1 | 17331 | 306 | 6654 | 152 |
| inorganic cation transmembrane transport | 2.38E-02 | 1 | 17331 | 475 | 4537 | 161 |

| | | | | | | |
|---|---|---|---|---|---|---|
| inorganic ion transmembrane transport | 1.45E-02 | 1 | 17331 | 549 | 4537 | 185 |
| regulation of ion transmembrane transport | 1.73E-03 | 1 | 17331 | 392 | 6771 | 197 |
| cellular response to chemical stimulus | 4.34E-03 | 1 | 17331 | 1254 | 2892 | 251 |
| negative regulation of metabolic process | 1.45E-03 | 1 | 17331 | 2550 | 1548 | 293 |
| regulation of cellular biosynthetic process | 1.37E-04 | 1 | 17331 | 3889 | 1176 | 340 |
| regulation of biosynthetic process | 1.44E-04 | 1 | 17331 | 3931 | 1176 | 343 |
| Wnt signaling pathway | 2.82E-02 | 1 | 17331 | 245 | 6941 | 126 |
| organ morphogenesis | 1.42E-02 | 1 | 17331 | 442 | 5421 | 177 |
| negative regulation of macromolecule metabolic process | 6.95E-03 | 1 | 17331 | 2287 | 1548 | 261 |
| negative regulation of cellular component organization | 4.18E-04 | 1 | 17331 | 581 | 6172 | 264 |
| regulation of macromolecule biosynthetic process | 3.53E-04 | 1 | 17331 | 3716 | 1188 | 327 |
| regulation of nitrogen compound metabolic process | 1.65E-04 | 1 | 17331 | 3991 | 1176 | 347 |
| regulation of transcription, DNA-templated | 3.20E-05 | 1 | 17331 | 3275 | 1701 | 411 |
| regulation of nucleic acid-templated transcription | 2.34E-05 | 1 | 17331 | 3292 | 1701 | 414 |
| regulation of RNA biosynthetic process | 3.08E-05 | 1 | 17331 | 3310 | 1701 | 415 |
| nucleobase-containing compound biosynthetic process | 1.85E-02 | 1 | 17331 | 2798 | 1118 | 230 |
| developmental process involved in reproduction | 2.74E-03 | 1 | 17331 | 557 | 5651 | 231 |
| metal ion transport | 2.49E-03 | 1 | 17331 | 559 | 5676 | 233 |
| regulation of intracellular signal transduction | 1.64E-04 | 1 | 17331 | 1567 | 3195 | 368 |
| regulation of transmembrane transport | 5.09E-03 | 1 | 17331 | 407 | 6771 | 201 |
| cellular response to endogenous stimulus | 7.40E-03 | 1 | 17331 | 596 | 5266 | 228 |
| heterocycle biosynthetic process | 2.65E-02 | 1 | 17331 | 2863 | 1118 | 233 |
| response to hormone | 2.82E-03 | 1 | 17331 | 679 | 5292 | 261 |
| positive regulation of cellular metabolic process | 1.58E-03 | 1 | 17331 | 2790 | 1657 | 336 |
| multicellular organismal process | 1.16E-08 | 1 | 17331 | 3295 | 2880 | 688 |
| neurotrophin TRK receptor signaling pathway | 7.87E-03 | 1 | 17331 | 377 | 7248 | 197 |
| neurotrophin signaling pathway | 6.87E-03 | 1 | 17331 | 380 | 7248 | 199 |
| regulation of cell proliferation | 7.53E-03 | 1 | 17331 | 1455 | 2834 | 297 |
| response to external stimulus | 2.59E-03 | 1 | 17331 | 1320 | 3439 | 327 |
| regulation of macromolecule metabolic process | 2.36E-05 | 1 | 17331 | 5430 | 1176 | 459 |
| response to endogenous stimulus | 4.14E-05 | 1 | 17331 | 1064 | 5958 | 450 |
| negative regulation of response to stimulus | 3.65E-04 | 1 | 17331 | 1318 | 4882 | 452 |
| anatomical structure development | 1.07E-07 | 1 | 17331 | 2719 | 3958 | 759 |
| regulation of signal transduction | 8.71E-08 | 1 | 17331 | 2507 | 4300 | 760 |
| regulation of signaling | 4.85E-09 | 1 | 17331 | 2844 | 4313 | 864 |
| regulation of metabolic process | 1.68E-06 | 1 | 17331 | 6271 | 1702 | 736 |
| regulation of cellular component organization | 1.33E-09 | 1 | 17331 | 2063 | 6506 | 930 |
| positive regulation of biological process | 8.18E-10 | 1 | 17331 | 5174 | 3245 | 1165 |
| positive regulation of signal transduction | 6.78E-03 | 1 | 17331 | 1393 | 4300 | 413 |
| positive regulation of signaling | 3.98E-03 | 1 | 17331 | 1517 | 4300 | 449 |
| transmembrane transport | 8.37E-05 | 1 | 17331 | 1124 | 7174 | 552 |
| negative regulation of biological process | 1.56E-05 | 1 | 17331 | 4335 | 2514 | 748 |
| positive regulation of cellular process | 1.66E-08 | 1 | 17331 | 4458 | 3245 | 996 |
| cation transport | 1.28E-02 | 1 | 17331 | 753 | 6829 | 350 |
| cell motility | 4.26E-03 | 1 | 17331 | 799 | 7181 | 391 |
| positive regulation of cell communication | 8.04E-03 | 1 | 17331 | 1537 | 4300 | 451 |
| regulation of organelle organization | 1.04E-03 | 1 | 17331 | 1019 | 6826 | 474 |
| ion transport | 2.60E-04 | 1 | 17331 | 1176 | 6829 | 547 |
| negative regulation of cellular process | 6.45E-06 | 1 | 17331 | 3973 | 3070 | 833 |
| regulation of response to stimulus | 1.35E-07 | 1 | 17331 | 3497 | 4231 | 1008 |
| animal organ development | 9.27E-03 | 1 | 17331 | 1212 | 5519 | 452 |
| movement of cell or subcellular component | 1.42E-05 | 1 | 17331 | 1451 | 7181 | 706 |
| response to abiotic stimulus | 5.35E-03 | 1 | 17331 | 1121 | 6709 | 503 |
| response to organic substance | 4.01E-04 | 1 | 17331 | 1770 | 5958 | 705 |
| locomotion | 1.42E-02 | 1 | 17331 | 953 | 7188 | 455 |
| response to oxygen-containing compound | 2.03E-02 | 1 | 17331 | 1227 | 5965 | 485 |
| intracellular signal transduction | 3.43E-04 | 1 | 17331 | 1706 | 6727 | 760 |
| cell surface receptor signaling pathway | 4.68E-05 | 1 | 17331 | 2176 | 6669 | 956 |
| regulation of molecular function | 2.46E-05 | 1 | 17331 | 2675 | 6725 | 1171 |
| response to chemical | 9.45E-04 | 1 | 17331 | 2223 | 6671 | 960 |
| regulation of biological quality | 8.14E-05 | 1 | 17331 | 3194 | 6204 | 1278 |
| single-organism transport | 2.17E-04 | 1 | 17331 | 2767 | 6829 | 1214 |
| single-organism localization | 2.23E-04 | 1 | 17331 | 2940 | 6829 | 1285 |
| transport | 4.32E-03 | 1 | 17331 | 3398 | 6834 | 1455 |
| establishment of localization | 3.75E-03 | 1 | 17331 | 3531 | 6829 | 1509 |
| signal transduction | 2.46E-02 | 1 | 17331 | 4599 | 5301 | 1513 |
| localization | 1.91E-02 | 1 | 17331 | 3863 | 6845 | 1634 |
| cellular component organization | 1.25E-02 | 1 | 17331 | 4682 | 6727 | 1937 |
| cellular component organization or biogenesis | 1.50E-02 | 1 | 17331 | 4717 | 6727 | 1949 |
| regulation of cellular process | 3.77E-06 | 1 | 17331 | 9563 | 5590 | 3258 |
| single-organism cellular process | 7.04E-08 | 1 | 17331 | 9341 | 6402 | 3668 |
| regulation of biological process | 3.95E-05 | 1 | 17331 | 10079 | 5613 | 3436 |
| biological regulation | 9.55E-06 | 1 | 17331 | 10542 | 5590 | 3579 |
| single-organism process | 4.22E-07 | 1 | 17331 | 10744 | 7194 | 4665 |
| biological_process | 1.32E-02 | 1 | 17331 | 15522 | 964 | 901 |
| cellular process | 1.53E-04 | 1 | 17331 | 12389 | 7206 | 5306 |

| JSD RANKING | | | | | | |
|---|---|---|---|---|---|---|
| PROCESS DESCRIPTION | FDR q-VALUE | ENRICHMENT | N | B | n | b |
| dorsal/ventral axis specification | 2.00E-03 | 19 | 17331 | 15 | 301 | 5 |
| bone morphogenesis | 7.71E-03 | 15 | 17331 | 26 | 219 | 5 |
| anterior/posterior axis specification | 4.07E-04 | 15 | 17331 | 35 | 235 | 7 |
| cell fate specification | 9.29E-07 | 14 | 17331 | 67 | 199 | 11 |
| regulation of mesonephros development | 2.19E-03 | 14 | 17331 | 25 | 298 | 6 |
| lens development in camera-type eye | 6.41E-04 | 13 | 17331 | 32 | 282 | 7 |
| regulation of branching involved in ureteric bud morphogenesis | 1.15E-02 | 13 | 17331 | 22 | 298 | 5 |
| glandular epithelial cell differentiation | 3.92E-03 | 12 | 17331 | 24 | 353 | 6 |
| cell fate determination | 3.19E-04 | 12 | 17331 | 45 | 253 | 8 |
| modulation of excitatory postsynaptic potential | 1.80E-02 | 12 | 17331 | 28 | 257 | 5 |
| neural tube development | 2.01E-02 | 12 | 17331 | 28 | 266 | 5 |
| cellular response to metal ion | 1.28E-03 | 10 | 17331 | 136 | 86 | 7 |
| axis specification | 1.43E-06 | 10 | 17331 | 74 | 301 | 13 |
| cardiocyte differentiation | 1.23E-02 | 10 | 17331 | 39 | 266 | 6 |
| positive regulation of hormone metabolic process | 1.70E-02 | 10 | 17331 | 11 | 797 | 5 |
| somatic stem cell population maintenance | 4.32E-07 | 10 | 17331 | 72 | 341 | 14 |
| telencephalon regionalization | 4.84E-03 | 10 | 17331 | 6 | 1484 | 5 |
| negative regulation of cell proliferation involved in kidney development | 2.56E-03 | 9 | 17331 | 5 | 1862 | 5 |
| response to follicle-stimulating hormone | 2.44E-02 | 9 | 17331 | 12 | 797 | 5 |
| cellular response to inorganic substance | 2.53E-03 | 9 | 17331 | 156 | 86 | 7 |
| cytoskeletal anchoring at plasma membrane | 2.33E-02 | 9 | 17331 | 11 | 881 | 5 |
| cardiac muscle cell differentiation | 1.67E-02 | 9 | 17331 | 29 | 401 | 6 |
| negative regulation of stem cell differentiation | 2.21E-03 | 9 | 17331 | 43 | 365 | 8 |
| forebrain dorsal/ventral pattern formation | 1.12E-02 | 9 | 17331 | 7 | 1404 | 5 |
| regulation of morphogenesis of a branching structure | 2.67E-03 | 9 | 17331 | 53 | 298 | 8 |
| cellular response to zinc ion | 1.13E-02 | 9 | 17331 | 16 | 746 | 6 |
| cerebral cortex regionalization | 1.18E-02 | 9 | 17331 | 7 | 1425 | 5 |
| eye development | 1.07E-02 | 9 | 17331 | 79 | 178 | 7 |
| calcium-dependent cell-cell adhesion via plasma membrane cell adhesion molecules | 4.40E-06 | 9 | 17331 | 26 | 930 | 12 |
| atrioventricular valve morphogenesis | 1.04E-02 | 9 | 17331 | 14 | 869 | 6 |
| sensory organ development | 1.75E-03 | 8 | 17331 | 106 | 178 | 9 |
| muscle cell fate commitment | 7.16E-03 | 8 | 17331 | 10 | 1271 | 6 |
| epithelial cell morphogenesis | 1.24E-02 | 8 | 17331 | 46 | 331 | 7 |
| formation of anatomical boundary | 1.07E-02 | 8 | 17331 | 6 | 1818 | 5 |
| cellular response to gonadotropin stimulus | 1.22E-03 | 8 | 17331 | 16 | 1100 | 8 |
| stem cell population maintenance | 6.05E-10 | 8 | 17331 | 139 | 341 | 21 |
| maintenance of cell number | 8.52E-10 | 8 | 17331 | 142 | 341 | 21 |
| spongiotrophoblast layer development | 2.09E-02 | 7 | 17331 | 7 | 1676 | 5 |
| negative regulation of epithelial to mesenchymal transition | 1.15E-02 | 7 | 17331 | 22 | 765 | 7 |
| heart valve morphogenesis | 1.35E-03 | 7 | 17331 | 25 | 869 | 9 |
| cerebral cortex neuron differentiation | 1.82E-02 | 7 | 17331 | 11 | 1378 | 6 |
| neuronal signal transduction | 2.27E-02 | 6 | 17331 | 6 | 2244 | 5 |
| cardiac ventricle morphogenesis | 7.49E-03 | 6 | 17331 | 23 | 936 | 8 |
| regulation of cell division | 1.90E-02 | 6 | 17331 | 303 | 64 | 7 |
| gonad development | 7.20E-04 | 6 | 17331 | 95 | 352 | 12 |
| cardiac chamber morphogenesis | 4.31E-03 | 6 | 17331 | 27 | 936 | 9 |
| embryonic digestive tract morphogenesis | 8.83E-04 | 6 | 17331 | 19 | 1526 | 10 |
| response to gonadotropin | 1.45E-02 | 6 | 17331 | 29 | 806 | 8 |
| mesonephros development | 1.02E-02 | 6 | 17331 | 11 | 1862 | 7 |
| ventricular septum morphogenesis | 4.04E-03 | 6 | 17331 | 21 | 1271 | 9 |
| lung-associated mesenchyme development | 1.11E-02 | 6 | 17331 | 11 | 1891 | 7 |
| adrenal gland development | 1.32E-02 | 6 | 17331 | 22 | 1100 | 8 |
| negative regulation of cell morphogenesis involved in differentiation | 7.51E-04 | 6 | 17331 | 109 | 365 | 13 |
| negative regulation of glycolytic process | 1.55E-02 | 6 | 17331 | 12 | 1804 | 7 |
| homophilic cell adhesion via plasma membrane adhesion molecules | 1.71E-14 | 5 | 17331 | 148 | 930 | 43 |
| regulation of epithelial cell differentiation | 1.20E-02 | 5 | 17331 | 121 | 266 | 10 |
| negative regulation of renal sodium excretion | 2.27E-02 | 5 | 17331 | 5 | 3264 | 5 |
| negative regulation of kidney development | 4.63E-03 | 5 | 17331 | 16 | 1862 | 9 |
| tube development | 9.32E-05 | 5 | 17331 | 182 | 274 | 15 |
| telencephalon development | 1.69E-02 | 5 | 17331 | 18 | 1484 | 8 |
| positive regulation of neuroblast proliferation | 1.06E-02 | 5 | 17331 | 22 | 1396 | 9 |
| lung epithelium development | 1.20E-02 | 5 | 17331 | 9 | 2664 | 7 |
| negative regulation of cell fate specification | 2.13E-02 | 5 | 17331 | 7 | 2978 | 6 |
| embryo implantation | 2.29E-02 | 5 | 17331 | 42 | 749 | 9 |
| negative regulation of cellular response to growth factor stimulus | 6.54E-03 | 5 | 17331 | 132 | 272 | 10 |
| regulation of neuroblast proliferation | 1.76E-03 | 5 | 17331 | 31 | 1396 | 12 |
| negative regulation of nucleotide catabolic process | 2.04E-02 | 5 | 17331 | 16 | 1804 | 8 |
| cardiac ventricle formation | 7.09E-03 | 5 | 17331 | 10 | 2978 | 8 |
| positive regulation of skeletal muscle tissue development | 1.90E-02 | 5 | 17331 | 24 | 1396 | 9 |
| positive regulation of myotube differentiation | 2.43E-02 | 5 | 17331 | 29 | 1161 | 9 |
| negative regulation of ATP metabolic process | 7.56E-03 | 5 | 17331 | 21 | 1804 | 10 |
| negative regulation of nucleoside metabolic process | 7.58E-03 | 5 | 17331 | 21 | 1804 | 10 |
| positive regulation of neural precursor cell proliferation | 7.17E-04 | 5 | 17331 | 38 | 1396 | 14 |
| cell-cell adhesion via plasma-membrane adhesion molecules | 4.51E-13 | 4 | 17331 | 197 | 930 | 47 |
| regulation of organ morphogenesis | 1.31E-04 | 4 | 17331 | 177 | 377 | 17 |
| embryonic hindlimb morphogenesis | 6.18E-04 | 4 | 17331 | 30 | 1847 | 14 |
| multicellular organismal response to stress | 4.50E-03 | 4 | 17331 | 60 | 859 | 13 |
| cell morphogenesis | 1.90E-04 | 4 | 17331 | 205 | 333 | 17 |
| cell differentiation involved in embryonic placenta development | 1.37E-02 | 4 | 17331 | 24 | 1674 | 10 |

| | | | | | | |
|---|---|---|---|---|---|---|
| cardiac chamber formation | 1.71E-02 | 4 | 17331 | 11 | 2978 | 8 |
| embryonic eye morphogenesis | 6.67E-03 | 4 | 17331 | 23 | 1962 | 11 |
| outflow tract morphogenesis | 1.53E-02 | 4 | 17331 | 40 | 1134 | 11 |
| neuroblast proliferation | 8.11E-03 | 4 | 17331 | 16 | 2640 | 10 |
| regulation of transcription regulatory region DNA binding | 3.67E-03 | 4 | 17331 | 33 | 1674 | 13 |
| regulation of neural precursor cell proliferation | 3.44E-06 | 4 | 17331 | 73 | 1396 | 24 |
| ionotropic glutamate receptor signaling pathway | 2.05E-02 | 4 | 17331 | 23 | 1884 | 10 |
| spinal cord association neuron differentiation | 1.88E-02 | 4 | 17331 | 14 | 2833 | 9 |
| synapse assembly | 6.20E-03 | 4 | 17331 | 59 | 1056 | 14 |
| hindlimb morphogenesis | 8.84E-04 | 4 | 17331 | 39 | 1847 | 16 |
| negative regulation of Wnt signaling pathway | 1.29E-03 | 4 | 17331 | 191 | 377 | 16 |
| protein targeting to plasma membrane | 6.12E-03 | 4 | 17331 | 23 | 2360 | 12 |
| skeletal muscle cell differentiation | 1.35E-02 | 4 | 17331 | 51 | 1181 | 13 |
| epithelium development | 9.22E-03 | 4 | 17331 | 227 | 266 | 13 |
| columnar/cuboidal epithelial cell differentiation | 6.25E-05 | 4 | 17331 | 69 | 1482 | 22 |
| regulation of organ formation | 4.31E-03 | 4 | 17331 | 32 | 2076 | 14 |
| cell differentiation in spinal cord | 2.22E-02 | 4 | 17331 | 40 | 1431 | 12 |
| positive regulation of extrinsic apoptotic signaling pathway | 1.70E-02 | 4 | 17331 | 52 | 1194 | 13 |
| central nervous system neuron differentiation | 1.06E-05 | 4 | 17331 | 85 | 1468 | 26 |
| synapse organization | 1.55E-04 | 4 | 17331 | 119 | 930 | 23 |
| embryonic cranial skeleton morphogenesis | 9.88E-03 | 4 | 17331 | 31 | 2035 | 13 |
| regulation of cellular response to growth factor stimulus | 1.36E-02 | 4 | 17331 | 234 | 272 | 13 |
| regulation of myotube differentiation | 1.27E-02 | 4 | 17331 | 54 | 1271 | 14 |
| regulation of cell fate commitment | 1.66E-02 | 4 | 17331 | 28 | 2102 | 12 |
| negative regulation of cellular component movement | 9.06E-03 | 4 | 17331 | 231 | 298 | 14 |
| mesenchyme development | 9.28E-04 | 4 | 17331 | 47 | 1891 | 18 |
| negative regulation of transcription regulatory region DNA binding | 4.03E-03 | 3 | 17331 | 16 | 3784 | 12 |
| positive regulation of heart growth | 1.95E-02 | 3 | 17331 | 27 | 2252 | 12 |
| negative regulation of cell development | 3.00E-07 | 3 | 17331 | 290 | 615 | 35 |
| positive regulation of transcription from RNA polymerase II promoter | 1.02E-06 | 3 | 17331 | 984 | 172 | 33 |
| negative regulation of neuron differentiation | 8.82E-04 | 3 | 17331 | 182 | 576 | 20 |
| steroid hormone mediated signaling pathway | 4.89E-04 | 3 | 17331 | 59 | 1862 | 21 |
| lung vasculature development | 1.36E-02 | 3 | 17331 | 8 | 5274 | 8 |
| stem cell proliferation | 5.18E-04 | 3 | 17331 | 46 | 2290 | 20 |
| negative regulation of locomotion | 1.66E-02 | 3 | 17331 | 249 | 298 | 14 |
| detection of temperature stimulus involved in sensory perception of pain | 2.35E-02 | 3 | 17331 | 14 | 3792 | 10 |
| detection of temperature stimulus involved in sensory perception | 2.36E-02 | 3 | 17331 | 14 | 3792 | 10 |
| neuroepithelial cell differentiation | 1.95E-02 | 3 | 17331 | 45 | 1653 | 14 |
| negative regulation of gliogenesis | 8.12E-03 | 3 | 17331 | 36 | 2214 | 15 |
| regulation of peptidyl-tyrosine phosphorylation | 7.27E-03 | 3 | 17331 | 212 | 402 | 16 |
| negative regulation of protein kinase activity by regulation of protein phosphorylation | 1.60E-02 | 3 | 17331 | 8 | 5420 | 8 |
| dorsal/ventral pattern formation | 9.43E-03 | 3 | 17331 | 63 | 1484 | 17 |
| positive regulation of stem cell proliferation | 4.19E-04 | 3 | 17331 | 67 | 1891 | 23 |
| positive regulation of organ growth | 1.26E-02 | 3 | 17331 | 37 | 2252 | 15 |
| stem cell differentiation | 2.34E-05 | 3 | 17331 | 67 | 2341 | 28 |
| nervous system development | 6.35E-07 | 3 | 17331 | 245 | 874 | 38 |
| enamel mineralization | 2.27E-02 | 3 | 17331 | 10 | 5097 | 9 |
| embryonic pattern specification | 1.00E-02 | 3 | 17331 | 52 | 1862 | 17 |
| negative regulation of neurogenesis | 3.27E-04 | 3 | 17331 | 232 | 615 | 25 |
| chondrocyte differentiation | 1.17E-02 | 3 | 17331 | 41 | 2237 | 16 |
| positive regulation of muscle tissue development | 7.75E-04 | 3 | 17331 | 54 | 2338 | 22 |
| regulation of stem cell proliferation | 1.22E-05 | 3 | 17331 | 97 | 1891 | 32 |
| male gonad development | 8.25E-03 | 3 | 17331 | 82 | 1340 | 19 |
| positive regulation of striated muscle tissue development | 1.85E-03 | 3 | 17331 | 53 | 2338 | 21 |
| positive regulation of muscle organ development | 1.85E-03 | 3 | 17331 | 53 | 2338 | 21 |
| epithelial to mesenchymal transition | 1.38E-02 | 3 | 17331 | 50 | 2020 | 17 |
| regulation of dendrite development | 1.39E-02 | 3 | 17331 | 110 | 1025 | 19 |
| positive regulation of stem cell differentiation | 1.44E-02 | 3 | 17331 | 50 | 2029 | 17 |
| tooth mineralization | 1.49E-02 | 3 | 17331 | 13 | 5097 | 11 |
| neuron fate commitment | 2.58E-04 | 3 | 17331 | 40 | 3476 | 23 |
| embryonic appendage morphogenesis | 2.47E-07 | 3 | 17331 | 86 | 2827 | 40 |
| embryonic limb morphogenesis | 2.49E-07 | 3 | 17331 | 86 | 2827 | 40 |
| regulation of cell morphogenesis involved in differentiation | 3.88E-06 | 3 | 17331 | 314 | 792 | 40 |
| regulation of catenin import into nucleus | 1.36E-02 | 3 | 17331 | 25 | 3758 | 15 |
| formation of primary germ layer | 8.71E-03 | 3 | 17331 | 47 | 2543 | 19 |
| negative regulation of neural precursor cell proliferation | 1.37E-02 | 3 | 17331 | 24 | 3976 | 15 |
| striated muscle tissue development | 7.55E-03 | 3 | 17331 | 86 | 1628 | 22 |
| embryonic skeletal system morphogenesis | 1.27E-05 | 3 | 17331 | 81 | 2764 | 35 |
| epithelial tube morphogenesis | 2.00E-03 | 3 | 17331 | 97 | 1719 | 26 |
| transcription from RNA polymerase II promoter | 1.35E-03 | 3 | 17331 | 547 | 317 | 27 |
| inner ear morphogenesis | 3.83E-04 | 3 | 17331 | 58 | 3023 | 27 |
| axonogenesis | 1.30E-03 | 3 | 17331 | 109 | 1668 | 28 |
| muscle tissue development | 3.98E-03 | 3 | 17331 | 100 | 1628 | 25 |
| glutamate receptor signaling pathway | 5.24E-03 | 3 | 17331 | 39 | 3367 | 20 |
| canonical Wnt signaling pathway | 2.98E-04 | 3 | 17331 | 88 | 2328 | 31 |
| regulation of muscle tissue development | 1.08E-02 | 3 | 17331 | 103 | 1484 | 23 |
| regionalization | 8.93E-08 | 3 | 17331 | 238 | 1484 | 53 |
| regulation of epithelial to mesenchymal transition | 1.36E-02 | 3 | 17331 | 66 | 2132 | 21 |
| negative regulation of cell growth | 7.91E-04 | 3 | 17331 | 159 | 1177 | 28 |
| limb morphogenesis | 1.85E-06 | 3 | 17331 | 102 | 2827 | 43 |
| appendage morphogenesis | 1.86E-06 | 3 | 17331 | 102 | 2827 | 43 |
| forebrain development | 1.48E-03 | 3 | 17331 | 52 | 3258 | 25 |

| | | | | | | |
|---|---|---|---|---|---|---|
| positive regulation of synaptic transmission | 1.08E-02 | 3 | 17331 | 110 | 1481 | 24 |
| regulation of stem cell differentiation | 8.59E-05 | 3 | 17331 | 118 | 2132 | 37 |
| ephrin receptor signaling pathway | 9.10E-04 | 3 | 17331 | 92 | 2221 | 30 |
| embryonic morphogenesis | 2.67E-11 | 3 | 17331 | 409 | 1489 | 89 |
| pattern specification process | 1.56E-11 | 3 | 17331 | 385 | 1484 | 83 |
| forelimb morphogenesis | 1.72E-02 | 3 | 17331 | 41 | 3202 | 19 |
| regulation of chondrocyte differentiation | 2.26E-02 | 3 | 17331 | 47 | 2794 | 19 |
| skeletal system morphogenesis | 2.34E-06 | 3 | 17331 | 111 | 2794 | 45 |
| neuron migration | 1.34E-02 | 3 | 17331 | 108 | 1541 | 24 |
| positive regulation of neurogenesis | 2.37E-11 | 3 | 17331 | 362 | 1571 | 82 |
| regulation of muscle organ development | 2.44E-02 | 2 | 17331 | 103 | 1484 | 22 |
| tube formation | 1.03E-03 | 2 | 17331 | 117 | 1905 | 32 |
| positive regulation of striated muscle cell differentiation | 1.85E-03 | 2 | 17331 | 49 | 3559 | 25 |
| cellular response to acid chemical | 7.88E-04 | 2 | 17331 | 172 | 1272 | 31 |
| positive regulation of nervous system development | 6.21E-11 | 2 | 17331 | 410 | 1571 | 91 |
| embryonic organ morphogenesis | 2.69E-09 | 2 | 17331 | 122 | 3547 | 61 |
| cardiac septum morphogenesis | 1.77E-04 | 2 | 17331 | 46 | 4489 | 29 |
| palate development | 1.44E-03 | 2 | 17331 | 75 | 2892 | 30 |
| negative regulation of reproductive process | 2.17E-02 | 2 | 17331 | 53 | 2874 | 21 |
| regulation of glial cell differentiation | 1.45E-02 | 2 | 17331 | 59 | 2856 | 23 |
| negative regulation of purine nucleotide metabolic process | 1.23E-02 | 2 | 17331 | 64 | 2747 | 24 |
| neural tube closure | 4.18E-03 | 2 | 17331 | 76 | 2698 | 28 |
| regulation of cartilage development | 1.22E-02 | 2 | 17331 | 63 | 2794 | 24 |
| hormone-mediated signaling pathway | 2.36E-02 | 2 | 17331 | 95 | 1862 | 24 |
| cell morphogenesis involved in differentiation | 1.70E-05 | 2 | 17331 | 156 | 2076 | 44 |
| cell fate commitment | 2.82E-10 | 2 | 17331 | 149 | 3503 | 70 |
| cell-cell adhesion | 3.25E-08 | 2 | 17331 | 579 | 943 | 73 |
| tube closure | 6.54E-03 | 2 | 17331 | 78 | 2698 | 28 |
| negative regulation of nucleotide metabolic process | 1.87E-02 | 2 | 17331 | 66 | 2747 | 24 |
| odontogenesis of dentin-containing tooth | 1.32E-02 | 2 | 17331 | 69 | 2893 | 26 |
| regulation of gliogenesis | 2.01E-02 | 2 | 17331 | 90 | 2214 | 26 |
| positive regulation of macromolecule biosynthetic process | 2.08E-04 | 2 | 17331 | 1581 | 213 | 44 |
| gland development | 1.13E-05 | 2 | 17331 | 260 | 1532 | 52 |
| positive regulation of neuron projection development | 1.06E-04 | 2 | 17331 | 213 | 1647 | 45 |
| regulation of nervous system development | 4.25E-13 | 2 | 17331 | 707 | 1484 | 134 |
| regulation of embryonic development | 2.54E-03 | 2 | 17331 | 109 | 2621 | 36 |
| positive regulation of muscle cell differentiation | 5.43E-04 | 2 | 17331 | 83 | 3642 | 38 |
| odontogenesis | 3.86E-03 | 2 | 17331 | 94 | 2893 | 34 |
| positive regulation of cell morphogenesis involved in differentiation | 8.60E-04 | 2 | 17331 | 150 | 2029 | 38 |
| vasculature development | 5.89E-03 | 2 | 17331 | 32 | 5803 | 23 |
| neuron projection morphogenesis | 4.77E-05 | 2 | 17331 | 187 | 2164 | 50 |
| anterior/posterior pattern specification | 4.50E-03 | 2 | 17331 | 143 | 1823 | 32 |
| regulation of striated muscle cell differentiation | 5.30E-03 | 2 | 17331 | 84 | 3200 | 33 |
| regulation of neuron differentiation | 2.61E-11 | 2 | 17331 | 522 | 1765 | 113 |
| positive regulation of neuron differentiation | 3.53E-08 | 2 | 17331 | 291 | 2290 | 81 |
| smooth muscle cell differentiation | 2.76E-03 | 2 | 17331 | 26 | 6981 | 22 |
| positive regulation of cell development | 1.20E-10 | 2 | 17331 | 455 | 2137 | 118 |
| regulation of neurogenesis | 2.96E-11 | 2 | 17331 | 630 | 1704 | 130 |
| response to growth factor | 1.03E-03 | 2 | 17331 | 243 | 1518 | 44 |
| cyclic nucleotide metabolic process | 1.95E-02 | 2 | 17331 | 56 | 4064 | 27 |
| cell junction organization | 1.12E-02 | 2 | 17331 | 193 | 1399 | 32 |
| positive regulation of developmental growth | 1.12E-03 | 2 | 17331 | 154 | 2252 | 41 |
| regulation of reproductive process | 1.75E-03 | 2 | 17331 | 126 | 2892 | 43 |
| positive regulation of gene expression | 1.46E-08 | 2 | 17331 | 1681 | 495 | 98 |
| reproductive structure development | 1.47E-03 | 2 | 17331 | 253 | 1484 | 44 |
| regulation of cardiac muscle tissue growth | 1.44E-02 | 2 | 17331 | 39 | 5504 | 25 |
| positive regulation of cell projection organization | 4.06E-05 | 2 | 17331 | 284 | 1891 | 62 |
| negative regulation of canonical Wnt signaling pathway | 4.45E-03 | 2 | 17331 | 162 | 2133 | 39 |
| muscle cell differentiation | 1.86E-04 | 2 | 17331 | 117 | 4004 | 53 |
| negative regulation of cell motility | 1.49E-02 | 2 | 17331 | 202 | 1493 | 34 |
| neuromuscular process | 5.64E-03 | 2 | 17331 | 85 | 3966 | 38 |
| regulation of small GTPase mediated signal transduction | 8.25E-06 | 2 | 17331 | 263 | 2401 | 71 |
| regulation of neuron projection development | 1.07E-05 | 2 | 17331 | 373 | 1765 | 74 |
| neuron differentiation | 3.55E-08 | 2 | 17331 | 230 | 3503 | 90 |
| positive regulation of developmental process | 6.26E-13 | 2 | 17331 | 1100 | 1493 | 183 |
| response to acid chemical | 1.12E-02 | 2 | 17331 | 309 | 1169 | 40 |
| central nervous system neuron development | 1.34E-02 | 2 | 17331 | 31 | 7026 | 24 |
| regulation of establishment of planar polarity | 2.22E-02 | 2 | 17331 | 46 | 5576 | 28 |
| heart development | 2.19E-02 | 2 | 17331 | 192 | 1676 | 35 |
| regulation of cardiac muscle tissue development | 1.51E-02 | 2 | 17331 | 52 | 5504 | 31 |
| regulation of Ras protein signal transduction | 2.67E-04 | 2 | 17331 | 176 | 2998 | 57 |
| positive regulation of growth | 1.03E-03 | 2 | 17331 | 228 | 2252 | 55 |
| developmental process involved in reproduction | 5.03E-06 | 2 | 17331 | 557 | 1526 | 91 |
| organ morphogenesis | 1.42E-10 | 2 | 17331 | 442 | 2893 | 137 |
| cell-cell signaling | 1.29E-03 | 2 | 17331 | 711 | 803 | 61 |
| positive regulation of cell differentiation | 1.55E-11 | 2 | 17331 | 800 | 2137 | 182 |
| regulation of cell development | 1.20E-11 | 2 | 17331 | 803 | 2137 | 183 |
| transmembrane receptor protein serine/threonine kinase signaling pathway | 1.90E-02 | 2 | 17331 | 198 | 1862 | 39 |
| cell migration | 1.85E-03 | 2 | 17331 | 723 | 799 | 61 |
| regulation of Wnt signaling pathway | 2.80E-04 | 2 | 17331 | 306 | 2076 | 67 |
| system development | 1.38E-08 | 2 | 17331 | 639 | 1920 | 129 |
| plasma membrane organization | 1.30E-02 | 2 | 17331 | 162 | 2423 | 41 |

| | | | | | | |
|---|---|---|---|---|---|---|
| regulation of anatomical structure morphogenesis | 2.71E-11 | 2 | 17331 | 881 | 1843 | 170 |
| cell-cell junction organization | 2.44E-02 | 2 | 17331 | 167 | 2129 | 37 |
| regulation of ossification | 1.23E-02 | 2 | 17331 | 177 | 2284 | 42 |
| regulation of Rho protein signal transduction | 5.18E-04 | 2 | 17331 | 101 | 5238 | 55 |
| Wnt signaling pathway | 1.35E-03 | 2 | 17331 | 245 | 2328 | 59 |
| regulation of organ growth | 5.24E-03 | 2 | 17331 | 76 | 5504 | 43 |
| response to starvation | 2.53E-03 | 2 | 17331 | 157 | 3165 | 51 |
| regulation of muscle cell differentiation | 9.10E-04 | 2 | 17331 | 150 | 3642 | 56 |
| cell projection morphogenesis | 3.69E-03 | 2 | 17331 | 246 | 2188 | 55 |

| RJSD RANKING | | | | | | |
|---|---|---|---|---|---|---|
| PROCESS DESCRIPTION | FDR q-VALUE | ENRICHMENT | N | B | n | b |
| cell differentiation involved in embryonic placenta development | 1.27E-06 | 56 | 17305 | 24 | 77 | 6 |
| stem cell population maintenance | 1.28E-05 | 31 | 17305 | 139 | 24 | 6 |
| maintenance of cell number | 1.41E-05 | 30 | 17305 | 142 | 24 | 6 |
| regulation of glial cell differentiation | 8.67E-05 | 29 | 17305 | 59 | 60 | 6 |
| regulation of gliogenesis | 8.35E-08 | 29 | 17305 | 90 | 60 | 9 |
| developmental growth involved in morphogenesis | 2.34E-03 | 24 | 17305 | 89 | 41 | 5 |
| negative regulation of gliogenesis | 1.04E-04 | 18 | 17305 | 36 | 182 | 7 |
| regulation of DNA binding | 1.76E-03 | 17 | 17305 | 89 | 69 | 6 |
| tube formation | 1.26E-06 | 17 | 17305 | 117 | 88 | 10 |
| bone morphogenesis | 7.42E-03 | 16 | 17305 | 26 | 212 | 5 |
| neuron migration | 4.83E-03 | 14 | 17305 | 108 | 70 | 6 |
| neural tube closure | 2.17E-02 | 13 | 17305 | 76 | 88 | 5 |
| commitment of neuronal cell to specific neuron type in forebrain | 2.69E-03 | 13 | 17305 | 7 | 960 | 5 |
| tube closure | 2.40E-02 | 13 | 17305 | 78 | 88 | 5 |
| embryonic cranial skeleton morphogenesis | 8.46E-06 | 12 | 17305 | 31 | 470 | 10 |
| cellular response to fibroblast growth factor stimulus | 1.14E-03 | 12 | 17305 | 26 | 397 | 7 |
| proximal/distal pattern formation | 3.37E-04 | 11 | 17305 | 28 | 442 | 8 |
| mesonephros development | 1.18E-02 | 11 | 17305 | 11 | 704 | 5 |
| neuron fate specification | 3.44E-04 | 11 | 17305 | 24 | 535 | 8 |
| outflow tract septum morphogenesis | 4.09E-03 | 11 | 17305 | 14 | 692 | 6 |
| negative regulation of glial cell differentiation | 2.15E-03 | 11 | 17305 | 26 | 442 | 7 |
| developmental growth | 1.34E-03 | 10 | 17305 | 283 | 41 | 7 |
| pattern specification involved in kidney development | 9.60E-03 | 10 | 17305 | 8 | 1066 | 5 |
| reproductive structure development | 4.08E-04 | 10 | 17305 | 253 | 54 | 8 |
| negative regulation of embryonic development | 2.47E-03 | 10 | 17305 | 24 | 501 | 7 |
| renal system development | 5.23E-03 | 10 | 17305 | 13 | 803 | 6 |
| response to fibroblast growth factor | 3.56E-03 | 10 | 17305 | 31 | 397 | 7 |
| embryonic forelimb morphogenesis | 2.55E-06 | 10 | 17305 | 34 | 635 | 12 |
| forebrain neuron fate commitment | 5.27E-04 | 10 | 17305 | 10 | 1266 | 7 |
| embryonic skeletal system morphogenesis | 1.74E-11 | 10 | 17305 | 81 | 470 | 21 |
| in utero embryonic development | 7.79E-03 | 9 | 17305 | 204 | 54 | 6 |
| forelimb morphogenesis | 2.36E-07 | 9 | 17305 | 41 | 635 | 14 |
| chordate embryonic development | 8.49E-03 | 9 | 17305 | 208 | 54 | 6 |
| negative regulation of kidney development | 9.59E-03 | 9 | 17305 | 16 | 704 | 6 |
| embryo development ending in birth or egg hatching | 9.25E-03 | 9 | 17305 | 212 | 54 | 6 |
| positive regulation of myotube differentiation | 1.72E-02 | 9 | 17305 | 29 | 397 | 6 |
| forebrain development | 2.39E-04 | 9 | 17305 | 52 | 382 | 10 |
| regulation of smoothened signaling pathway | 1.22E-03 | 8 | 17305 | 64 | 287 | 9 |
| embryonic appendage morphogenesis | 1.53E-08 | 8 | 17305 | 86 | 435 | 18 |
| embryonic limb morphogenesis | 1.54E-08 | 8 | 17305 | 86 | 435 | 18 |
| growth | 5.68E-03 | 8 | 17305 | 364 | 41 | 7 |
| regulation of mechanoreceptor differentiation | 2.43E-03 | 8 | 17305 | 7 | 1834 | 6 |
| regulation of inner ear receptor cell differentiation | 2.44E-03 | 8 | 17305 | 7 | 1834 | 6 |
| regulation of cell proliferation involved in heart morphogenesis | 3.69E-03 | 8 | 17305 | 14 | 1074 | 7 |
| skeletal system morphogenesis | 1.75E-11 | 8 | 17305 | 111 | 470 | 24 |
| regulation of auditory receptor cell differentiation | 1.17E-02 | 8 | 17305 | 6 | 1834 | 5 |
| smooth muscle cell differentiation | 9.60E-03 | 8 | 17305 | 26 | 596 | 7 |
| limb morphogenesis | 4.14E-09 | 8 | 17305 | 102 | 435 | 20 |
| appendage morphogenesis | 4.18E-09 | 8 | 17305 | 102 | 435 | 20 |
| negative regulation of transcription regulatory region DNA binding | 1.89E-02 | 8 | 17305 | 16 | 835 | 6 |
| embryo development | 7.33E-03 | 8 | 17305 | 246 | 64 | 7 |
| odontogenesis | 8.91E-03 | 8 | 17305 | 94 | 196 | 8 |
| cartilage development | 2.13E-02 | 7 | 17305 | 73 | 222 | 7 |
| regulation of transcription involved in cell fate commitment | 2.87E-03 | 7 | 17305 | 20 | 931 | 8 |
| signal transduction involved in regulation of gene expression | 2.96E-03 | 7 | 17305 | 20 | 937 | 8 |
| positive regulation of ossification | 2.55E-02 | 7 | 17305 | 85 | 196 | 7 |
| regulation of binding | 3.86E-03 | 7 | 17305 | 277 | 69 | 8 |
| enteroendocrine cell differentiation | 8.57E-03 | 7 | 17305 | 8 | 1863 | 6 |
| negative regulation of smoothened signaling pathway | 1.69E-02 | 7 | 17305 | 25 | 702 | 7 |
| thyroid gland development | 1.70E-02 | 7 | 17305 | 25 | 704 | 7 |
| spinal cord association neuron differentiation | 9.50E-07 | 7 | 17305 | 14 | 2157 | 12 |
| type B pancreatic cell development | 1.94E-02 | 7 | 17305 | 11 | 1377 | 6 |
| cardiac chamber formation | 5.28E-03 | 7 | 17305 | 11 | 1623 | 7 |
| cell fate specification | 2.54E-05 | 7 | 17305 | 67 | 535 | 14 |
| positive regulation of stem cell proliferation | 3.16E-04 | 7 | 17305 | 67 | 470 | 12 |
| positive regulation of striated muscle cell differentiation | 1.47E-02 | 6 | 17305 | 49 | 435 | 8 |
| developmental process involved in reproduction | 1.07E-07 | 6 | 17305 | 556 | 92 | 19 |
| regulation of development, heterochronic | 7.58E-04 | 6 | 17305 | 14 | 1733 | 9 |
| cardiac ventricle formation | 2.18E-02 | 6 | 17305 | 10 | 1623 | 6 |
| hindlimb morphogenesis | 5.86E-03 | 6 | 17305 | 39 | 635 | 9 |
| regulation of somitogenesis | 8.48E-03 | 6 | 17305 | 11 | 1771 | 7 |
| embryonic organ morphogenesis | 9.55E-14 | 6 | 17305 | 122 | 782 | 34 |
| suckling behavior | 1.74E-02 | 6 | 17305 | 16 | 1231 | 7 |
| regulation of timing of cell differentiation | 3.68E-03 | 6 | 17305 | 13 | 1733 | 8 |
| cardiac septum morphogenesis | 1.54E-05 | 6 | 17305 | 46 | 933 | 15 |
| glandular epithelial cell development | 1.98E-02 | 6 | 17305 | 15 | 1377 | 7 |
| regulation of heart morphogenesis | 9.50E-06 | 6 | 17305 | 24 | 1733 | 14 |
| regulation of organ formation | 3.62E-04 | 6 | 17305 | 32 | 1121 | 12 |
| developmental induction | 6.23E-03 | 6 | 17305 | 25 | 1086 | 9 |

| | | | | | | |
|---|---|---|---|---|---|---|
| negative regulation of oligodendrocyte differentiation | 6.75E-03 | 6 | 17305 | 14 | 1733 | 8 |
| endoderm formation | 2.18E-03 | 6 | 17305 | 14 | 1989 | 9 |
| palate development | 7.40E-04 | 5 | 17305 | 75 | 546 | 13 |
| regulation of cell fate commitment | 2.25E-02 | 5 | 17305 | 28 | 902 | 8 |
| negative regulation of epidermal cell differentiation | 7.49E-03 | 5 | 17305 | 13 | 1961 | 8 |
| positive regulation of neural precursor cell proliferation | 3.42E-04 | 5 | 17305 | 38 | 1097 | 13 |
| enamel mineralization | 2.96E-03 | 5 | 17305 | 10 | 2574 | 8 |
| stem cell differentiation | 5.68E-05 | 5 | 17305 | 67 | 779 | 16 |
| cell differentiation in spinal cord | 1.58E-07 | 5 | 17305 | 40 | 1637 | 20 |
| central nervous system neuron differentiation | 1.22E-08 | 5 | 17305 | 85 | 962 | 25 |
| mesoderm formation | 1.27E-03 | 5 | 17305 | 35 | 1151 | 12 |
| negative regulation of cell fate commitment | 2.71E-02 | 5 | 17305 | 12 | 1996 | 7 |
| neurogenesis | 2.79E-03 | 5 | 17305 | 45 | 940 | 12 |
| regulation of cardiac muscle tissue development | 1.72E-02 | 5 | 17305 | 52 | 692 | 10 |
| hemopoiesis | 2.30E-02 | 5 | 17305 | 90 | 400 | 10 |
| embryonic digestive tract morphogenesis | 1.29E-02 | 5 | 17305 | 19 | 1726 | 9 |
| anterior/posterior axis specification | 1.62E-05 | 5 | 17305 | 35 | 1771 | 17 |
| regulation of striated muscle cell differentiation | 2.42E-02 | 5 | 17305 | 84 | 435 | 10 |
| inner ear morphogenesis | 2.47E-07 | 5 | 17305 | 58 | 1465 | 23 |
| tooth mineralization | 5.60E-03 | 5 | 17305 | 13 | 2574 | 9 |
| outflow tract morphogenesis | 3.68E-03 | 5 | 17305 | 40 | 1117 | 12 |
| regulation of neural precursor cell proliferation | 2.59E-06 | 5 | 17305 | 73 | 1151 | 22 |
| morphogenesis of an epithelial fold | 1.10E-02 | 4 | 17305 | 15 | 2314 | 9 |
| embryonic axis specification | 1.69E-03 | 4 | 17305 | 30 | 1724 | 13 |
| neuron fate determination | 2.35E-03 | 4 | 17305 | 10 | 3615 | 9 |
| neuron fate commitment | 8.83E-10 | 4 | 17305 | 40 | 2712 | 27 |
| cell fate commitment | 6.93E-13 | 4 | 17305 | 149 | 1290 | 46 |
| positive regulation of muscle organ development | 1.30E-02 | 4 | 17305 | 53 | 952 | 12 |
| positive regulation of striated muscle tissue development | 1.31E-02 | 4 | 17305 | 53 | 952 | 12 |
| epithelial tube branching involved in lung morphogenesis | 1.06E-02 | 4 | 17305 | 17 | 2502 | 10 |
| positive regulation of muscle tissue development | 1.58E-02 | 4 | 17305 | 54 | 952 | 12 |
| regulation of stem cell proliferation | 2.16E-06 | 4 | 17305 | 97 | 1168 | 26 |
| negative regulation of nervous system development | 4.32E-06 | 4 | 17305 | 251 | 442 | 25 |
| heart looping | 6.68E-03 | 4 | 17305 | 56 | 1121 | 14 |
| forebrain neuron differentiation | 4.87E-03 | 4 | 17305 | 16 | 3097 | 11 |
| negative regulation of epithelial cell differentiation | 8.41E-04 | 4 | 17305 | 37 | 1961 | 16 |
| neuron differentiation | 3.74E-13 | 4 | 17305 | 230 | 1074 | 54 |
| stem cell proliferation | 3.32E-03 | 4 | 17305 | 46 | 1498 | 15 |
| embryonic pattern specification | 1.08E-04 | 4 | 17305 | 52 | 1772 | 20 |
| positive regulation of oligodendrocyte differentiation | 2.22E-02 | 4 | 17305 | 13 | 3218 | 9 |
| regulation of striated muscle tissue development | 9.06E-03 | 4 | 17305 | 101 | 692 | 15 |
| morphogenesis of embryonic epithelium | 5.76E-05 | 4 | 17305 | 24 | 3306 | 17 |
| embryonic eye morphogenesis | 3.68E-03 | 4 | 17305 | 23 | 2647 | 13 |
| formation of primary germ layer | 9.65E-05 | 4 | 17305 | 47 | 1989 | 20 |
| axis specification | 7.99E-07 | 4 | 17305 | 74 | 1771 | 28 |
| negative regulation of cell proliferation | 1.82E-03 | 4 | 17305 | 630 | 127 | 17 |
| canonical Wnt signaling pathway | 2.92E-05 | 4 | 17305 | 88 | 1281 | 24 |
| single organism reproductive process | 9.16E-04 | 4 | 17305 | 1078 | 79 | 18 |
| regulation of muscle tissue development | 1.07E-02 | 4 | 17305 | 103 | 692 | 15 |
| regulation of muscle organ development | 1.07E-02 | 4 | 17305 | 103 | 692 | 15 |
| regulation of oligodendrocyte differentiation | 1.99E-03 | 4 | 17305 | 31 | 2305 | 15 |
| regulation of dendritic spine morphogenesis | 1.54E-02 | 4 | 17305 | 27 | 2146 | 12 |
| endocrine pancreas development | 2.69E-03 | 4 | 17305 | 42 | 1863 | 16 |
| regulation of epidermal cell differentiation | 1.34E-03 | 4 | 17305 | 42 | 1980 | 17 |
| mesenchymal cell development | 2.52E-02 | 4 | 17305 | 23 | 2354 | 11 |
| hematopoietic or lymphoid organ development | 5.68E-03 | 4 | 17305 | 185 | 400 | 15 |
| vasculature development | 2.52E-02 | 3 | 17305 | 32 | 1871 | 12 |
| mesenchyme development | 2.27E-03 | 3 | 17305 | 47 | 1809 | 17 |
| ureteric bud development | 1.12E-02 | 3 | 17305 | 39 | 1809 | 14 |
| mesonephric tubule development | 7.41E-03 | 3 | 17305 | 42 | 1809 | 15 |
| epithelial tube morphogenesis | 3.82E-07 | 3 | 17305 | 97 | 1733 | 33 |
| cranial nerve development | 5.90E-03 | 3 | 17305 | 21 | 3176 | 13 |
| mesonephric epithelium development | 9.47E-03 | 3 | 17305 | 43 | 1809 | 15 |
| cell fate determination | 1.00E-06 | 3 | 17305 | 45 | 3128 | 27 |
| columnar/cuboidal epithelial cell differentiation | 1.84E-05 | 3 | 17305 | 69 | 2087 | 27 |
| regulation of mesonephros development | 3.37E-03 | 3 | 17305 | 25 | 3229 | 15 |
| negative regulation of neurogenesis | 4.53E-05 | 3 | 17305 | 232 | 628 | 27 |
| regulation of branching involved in ureteric bud morphogenesis | 1.19E-02 | 3 | 17305 | 22 | 3229 | 13 |
| negative regulation of BMP signaling pathway | 1.78E-02 | 3 | 17305 | 43 | 1944 | 15 |
| odontogenesis of dentin-containing tooth | 2.95E-03 | 3 | 17305 | 69 | 1620 | 20 |
| pattern specification process | 6.47E-22 | 3 | 17305 | 385 | 1536 | 106 |
| kidney epithelium development | 4.05E-03 | 3 | 17305 | 59 | 1809 | 19 |
| embryonic heart tube morphogenesis | 4.78E-03 | 3 | 17305 | 62 | 1733 | 19 |
| BMP signaling pathway | 1.54E-03 | 3 | 17305 | 78 | 1596 | 22 |
| embryonic skeletal system development | 1.18E-04 | 3 | 17305 | 36 | 3490 | 22 |
| epithelium development | 8.63E-05 | 3 | 17305 | 227 | 704 | 28 |
| regulation of BMP signaling pathway | 2.20E-04 | 3 | 17305 | 77 | 1944 | 26 |
| embryonic morphogenesis | 4.05E-29 | 3 | 17305 | 409 | 1940 | 138 |
| neuroepithelial cell differentiation | 1.80E-02 | 3 | 17305 | 45 | 2087 | 16 |
| tube morphogenesis | 1.57E-12 | 3 | 17305 | 229 | 1809 | 70 |
| regulation of epidermis development | 7.56E-03 | 3 | 17305 | 62 | 1980 | 20 |
| positive regulation of multicellular organismal process | 9.09E-06 | 3 | 17305 | 1350 | 169 | 37 |

| | | | | | |
|---|---|---|---|---|---|
| dorsal/ventral pattern formation | 1.30E-04 | 3 | 17305 | 63 | 2759 | 28 |
| cardiac septum development | 9.68E-03 | 3 | 17305 | 50 | 2368 | 19 |
| camera-type eye development | 1.43E-03 | 3 | 17305 | 54 | 2647 | 23 |
| regulation of dendrite morphogenesis | 1.07E-02 | 3 | 17305 | 66 | 1895 | 20 |
| morphogenesis of an epithelium | 5.42E-15 | 3 | 17305 | 297 | 1913 | 90 |
| system development | 1.99E-11 | 3 | 17305 | 639 | 733 | 73 |
| anterior/posterior pattern specification | 1.79E-07 | 3 | 17305 | 143 | 2033 | 45 |
| positive regulation of glial cell differentiation | 2.30E-02 | 3 | 17305 | 32 | 3246 | 16 |
| morphogenesis of a branching structure | 2.40E-07 | 3 | 17305 | 166 | 1809 | 46 |
| regulation of epithelial cell differentiation | 1.60E-05 | 3 | 17305 | 121 | 2124 | 39 |
| negative regulation of Wnt signaling pathway | 9.48E-05 | 3 | 17305 | 191 | 1179 | 34 |
| negative regulation of transcription from RNA polymerase II promoter | 9.26E-18 | 3 | 17305 | 714 | 1138 | 122 |
| Wnt signaling pathway | 9.00E-07 | 3 | 17305 | 245 | 1281 | 47 |
| negative regulation of transcription, DNA-templated | 4.15E-16 | 3 | 17305 | 1113 | 692 | 115 |
| negative regulation of growth | 7.07E-03 | 3 | 17305 | 226 | 685 | 23 |
| tissue morphogenesis | 2.88E-15 | 3 | 17305 | 368 | 1913 | 104 |
| negative regulation of nucleic acid-templated transcription | 4.38E-16 | 3 | 17305 | 1145 | 692 | 117 |
| regulation of embryonic development | 5.07E-06 | 3 | 17305 | 108 | 2638 | 42 |
| organ morphogenesis | 7.55E-14 | 3 | 17305 | 442 | 1537 | 100 |
| negative regulation of RNA biosynthetic process | 3.76E-16 | 3 | 17305 | 1158 | 692 | 118 |
| negative regulation of canonical Wnt signaling pathway | 1.26E-03 | 3 | 17305 | 162 | 1179 | 28 |
| pituitary gland development | 9.74E-03 | 3 | 17305 | 25 | 4648 | 17 |
| blood vessel morphogenesis | 1.62E-02 | 3 | 17305 | 76 | 1979 | 22 |
| regionalization | 1.07E-14 | 3 | 17305 | 238 | 2790 | 96 |
| positive regulation of osteoblast differentiation | 1.95E-02 | 2 | 17305 | 60 | 2437 | 21 |
| embryonic digit morphogenesis | 1.23E-02 | 2 | 17305 | 58 | 2638 | 22 |
| reproductive process | 5.06E-05 | 2 | 17305 | 1238 | 230 | 41 |
| response to BMP | 9.22E-03 | 2 | 17305 | 31 | 4298 | 19 |
| cellular response to BMP stimulus | 9.25E-03 | 2 | 17305 | 31 | 4298 | 19 |
| regulation of stem cell differentiation | 7.14E-04 | 2 | 17305 | 118 | 2040 | 34 |
| animal organ development | 5.74E-12 | 2 | 17305 | 1211 | 609 | 103 |
| branching morphogenesis of an epithelial tube | 4.22E-06 | 2 | 17305 | 132 | 2396 | 44 |
| morphogenesis of a branching epithelium | 2.94E-07 | 2 | 17305 | 156 | 2396 | 52 |
| tube development | 2.69E-06 | 2 | 17305 | 182 | 1902 | 48 |
| negative regulation of cellular biosynthetic process | 3.65E-16 | 2 | 17305 | 1397 | 692 | 133 |
| negative regulation of stem cell differentiation | 2.41E-02 | 2 | 17305 | 43 | 3395 | 20 |
| eye morphogenesis | 1.93E-02 | 2 | 17305 | 44 | 3521 | 21 |
| kidney development | 7.84E-04 | 2 | 17305 | 128 | 1842 | 32 |
| regulation of Wnt signaling pathway | 1.14E-05 | 2 | 17305 | 306 | 1179 | 49 |
| regulation of osteoblast differentiation | 4.77E-03 | 2 | 17305 | 112 | 2067 | 31 |
| axon guidance | 2.20E-05 | 2 | 17305 | 537 | 694 | 50 |
| neuron projection guidance | 2.43E-05 | 2 | 17305 | 538 | 694 | 50 |
| negative regulation of gene expression | 1.53E-15 | 2 | 17305 | 1455 | 692 | 135 |
| sensory organ development | 7.13E-04 | 2 | 17305 | 106 | 2647 | 37 |
| regulation of organ morphogenesis | 1.53E-05 | 2 | 17305 | 177 | 2078 | 48 |
| transcription from RNA polymerase II promoter | 3.55E-11 | 2 | 17305 | 547 | 1581 | 111 |
| negative regulation of cell growth | 1.86E-02 | 2 | 17305 | 159 | 1232 | 25 |
| sensory organ morphogenesis | 1.37E-02 | 2 | 17305 | 50 | 3958 | 25 |
| gland development | 2.21E-07 | 2 | 17305 | 260 | 2078 | 68 |
| negative regulation of neuron differentiation | 1.04E-03 | 2 | 17305 | 182 | 1733 | 39 |
| negative regulation of developmental process | 3.86E-06 | 2 | 17305 | 771 | 704 | 67 |
| response to growth factor | 6.64E-05 | 2 | 17305 | 243 | 1702 | 51 |
| regulation of morphogenesis of a branching structure | 1.64E-02 | 2 | 17305 | 53 | 3995 | 26 |
| regulation of cell growth | 2.23E-02 | 2 | 17305 | 368 | 644 | 29 |
| DNA replication initiation | 1.65E-02 | 2 | 17305 | 27 | 6077 | 20 |
| negative regulation of RNA metabolic process | 1.33E-15 | 2 | 17305 | 1198 | 1138 | 166 |
| negative regulation of cell development | 9.43E-06 | 2 | 17305 | 290 | 1733 | 61 |
| positive regulation of cell proliferation | 7.63E-06 | 2 | 17305 | 800 | 692 | 67 |
| regulation of growth | 3.91E-04 | 2 | 17305 | 614 | 692 | 51 |
| negative regulation of cellular macromolecule biosynthetic process | 3.49E-16 | 2 | 17305 | 1265 | 1157 | 176 |
| regulation of transcription from RNA polymerase II promoter | 1.66E-23 | 2 | 17305 | 1706 | 1164 | 238 |
| ameboidal-type cell migration | 5.24E-03 | 2 | 17305 | 149 | 1913 | 34 |
| positive regulation of cell morphogenesis involved in differentiation | 4.46E-03 | 2 | 17305 | 150 | 1961 | 35 |
| positive regulation of neurogenesis | 3.34E-09 | 2 | 17305 | 362 | 2209 | 95 |
| negative regulation of nucleobase-containing compound metabolic process | 7.95E-16 | 2 | 17305 | 1329 | 1138 | 179 |
| negative regulation of macromolecule biosynthetic process | 7.02E-16 | 2 | 17305 | 1338 | 1138 | 180 |
| anatomical structure development | 2.12E-22 | 2 | 17305 | 2718 | 692 | 223 |
| regulation of canonical Wnt signaling pathway | 5.94E-04 | 2 | 17305 | 237 | 1683 | 47 |
| positive regulation of cell development | 1.22E-08 | 2 | 17305 | 455 | 1802 | 96 |
| positive regulation of neuron differentiation | 9.03E-07 | 2 | 17305 | 291 | 2209 | 75 |
| positive regulation of cellular biosynthetic process | 4.30E-08 | 2 | 17305 | 1701 | 473 | 94 |
| meiotic nuclear division | 2.14E-02 | 2 | 17305 | 71 | 3639 | 30 |
| neuron development | 8.71E-03 | 2 | 17305 | 122 | 2678 | 38 |
| negative regulation of biosynthetic process | 1.00E-15 | 2 | 17305 | 1418 | 1138 | 187 |
| negative regulation of nitrogen compound metabolic process | 4.43E-16 | 2 | 17305 | 1429 | 1138 | 189 |
| eye development | 4.42E-04 | 2 | 17305 | 79 | 4720 | 43 |
| heart development | 3.67E-03 | 2 | 17305 | 192 | 1809 | 40 |
| positive regulation of biosynthetic process | 1.01E-07 | 2 | 17305 | 1730 | 473 | 94 |
| regulation of organ growth | 2.30E-02 | 2 | 17305 | 76 | 3558 | 31 |
| regulation of ossification | 2.30E-03 | 2 | 17305 | 177 | 2078 | 42 |
| regulation of cell morphogenesis involved in differentiation | 5.10E-06 | 2 | 17305 | 314 | 2001 | 72 |
| regulation of double-strand break repair | 2.18E-02 | 2 | 17305 | 36 | 5842 | 24 |

| | | | | | | |
|---|---|---|---|---|---|---|
| epithelial cell differentiation | 4.05E-06 | 2 | 17305 | 323 | 2087 | 76 |
| regulation of neuron differentiation | 2.68E-10 | 2 | 17305 | 522 | 2078 | 122 |
| regulation of cell proliferation | 7.80E-09 | 2 | 17305 | 1453 | 692 | 113 |
| positive regulation of developmental growth | 1.99E-02 | 2 | 17305 | 154 | 1930 | 33 |
| regulation of axonogenesis | 1.70E-02 | 2 | 17305 | 153 | 2001 | 34 |
| positive regulation of nervous system development | 7.08E-08 | 2 | 17305 | 410 | 2209 | 100 |
| tissue development | 2.70E-09 | 2 | 17305 | 567 | 1945 | 121 |
| regulation of multicellular organismal process | 2.17E-08 | 2 | 17305 | 2458 | 409 | 110 |
| regulation of neurogenesis | 6.90E-12 | 2 | 17305 | 630 | 2223 | 152 |
| regulation of cellular response to growth factor stimulus | 5.93E-05 | 2 | 17305 | 234 | 2647 | 67 |
| positive regulation of nucleobase-containing compound metabolic process | 6.53E-10 | 2 | 17305 | 1640 | 780 | 138 |
| neural precursor cell proliferation | 2.55E-02 | 2 | 17305 | 52 | 5360 | 30 |
| positive regulation of neuron projection development | 5.50E-03 | 2 | 17305 | 213 | 1961 | 45 |
| nervous system development | 2.01E-03 | 2 | 17305 | 245 | 1979 | 52 |
| regulation of developmental growth | 3.17E-04 | 2 | 17305 | 291 | 2014 | 63 |
| brain development | 3.18E-04 | 2 | 17305 | 182 | 2979 | 58 |
| negative regulation of multicellular organismal process | 2.69E-04 | 2 | 17305 | 962 | 689 | 71 |
| anatomical structure morphogenesis | 5.70E-21 | 2 | 17305 | 1318 | 2001 | 282 |
| mesoderm development | 3.57E-03 | 2 | 17305 | 44 | 7083 | 33 |
| positive regulation of cell differentiation | 3.86E-10 | 2 | 17305 | 800 | 1756 | 148 |
| regulation of nervous system development | 1.03E-11 | 2 | 17305 | 707 | 2223 | 165 |
| negative regulation of cellular metabolic process | 3.96E-11 | 2 | 17305 | 2250 | 694 | 163 |
| regulation of cell development | 1.27E-10 | 2 | 17305 | 803 | 2083 | 175 |
| single-multicellular organism process | 4.54E-08 | 2 | 17305 | 2567 | 456 | 122 |
| regulation of developmental process | 1.37E-09 | 2 | 17305 | 2044 | 692 | 147 |
| negative regulation of biological process | 6.24E-13 | 2 | 17305 | 4331 | 401 | 181 |
| cell morphogenesis | 9.20E-03 | 2 | 17305 | 205 | 2168 | 46 |
| positive regulation of transcription from RNA polymerase II promoter | 1.90E-11 | 2 | 17305 | 984 | 1960 | 198 |
| regulation of transcription, DNA-templated | 1.21E-22 | 2 | 17305 | 3275 | 978 | 327 |
| regulation of neuron projection development | 3.12E-03 | 2 | 17305 | 373 | 1612 | 61 |
| regulation of nucleic acid-templated transcription | 1.38E-22 | 2 | 17305 | 3292 | 978 | 328 |
| regulation of RNA biosynthetic process | 1.60E-22 | 2 | 17305 | 3310 | 978 | 329 |
| anatomical structure formation involved in morphogenesis | 2.42E-10 | 2 | 17305 | 798 | 2067 | 167 |
| regulation of RNA metabolic process | 7.16E-23 | 2 | 17305 | 3408 | 978 | 337 |
| regulation of anatomical structure morphogenesis | 4.99E-06 | 2 | 17305 | 878 | 1255 | 111 |
| negative regulation of metabolic process | 4.96E-11 | 2 | 17305 | 2549 | 694 | 178 |
| regulation of epithelial cell proliferation | 1.22E-02 | 2 | 17305 | 276 | 1894 | 52 |
| angiogenesis | 1.63E-02 | 2 | 17305 | 245 | 2067 | 50 |
| skeletal system development | 2.10E-03 | 2 | 17305 | 161 | 3640 | 58 |
| cellular response to growth factor stimulus | 1.01E-04 | 2 | 17305 | 215 | 3867 | 81 |
| multicellular organismal process | 2.30E-05 | 2 | 17305 | 3295 | 321 | 103 |
| regulation of cellular macromolecule biosynthetic process | 7.10E-21 | 2 | 17305 | 3615 | 978 | 345 |
| mitochondrial respiratory chain complex I assembly | 1.90E-02 | 2 | 17305 | 52 | 7128 | 36 |
| NADH dehydrogenase complex assembly | 1.91E-02 | 2 | 17305 | 52 | 7128 | 36 |
| mitochondrial respiratory chain complex I biogenesis | 1.91E-02 | 2 | 17305 | 52 | 7128 | 36 |
| regulation of macromolecule biosynthetic process | 6.34E-21 | 2 | 17305 | 3715 | 978 | 352 |
| negative regulation of cell differentiation | 1.40E-05 | 2 | 17305 | 591 | 2040 | 116 |
| regulation of cellular biosynthetic process | 5.77E-21 | 2 | 17305 | 3889 | 978 | 364 |
| gene silencing | 1.17E-02 | 2 | 17305 | 188 | 3081 | 55 |
| regulation of transmembrane receptor protein serine/threonine kinase signaling pathway | 5.68E-04 | 2 | 17305 | 216 | 3820 | 78 |
| cell morphogenesis involved in differentiation | 4.40E-04 | 2 | 17305 | 156 | 4889 | 72 |
| negative regulation of cellular process | 3.32E-11 | 2 | 17305 | 3970 | 644 | 240 |
| regulation of cell projection organization | 8.62E-03 | 2 | 17305 | 494 | 1612 | 74 |
| cell development | 6.13E-07 | 2 | 17305 | 578 | 2788 | 150 |
| positive regulation of developmental process | 4.34E-09 | 2 | 17305 | 1097 | 1983 | 202 |
| negative regulation of protein phosphorylation | 2.08E-02 | 2 | 17305 | 372 | 1869 | 64 |
| regulation of multicellular organismal development | 5.85E-09 | 2 | 17305 | 1540 | 1502 | 212 |
| positive regulation of nucleic acid-templated transcription | 2.36E-11 | 2 | 17305 | 1367 | 2035 | 255 |
| positive regulation of transcription, DNA-templated | 2.39E-11 | 2 | 17305 | 1367 | 2035 | 255 |
| regulation of cell differentiation | 2.15E-10 | 2 | 17305 | 1441 | 2045 | 270 |
| nucleosome assembly | 1.63E-02 | 2 | 17305 | 119 | 5424 | 59 |
| negative regulation of phosphorylation | 1.55E-02 | 2 | 17305 | 410 | 1869 | 70 |
| cell differentiation | 1.33E-15 | 2 | 17305 | 1687 | 2354 | 363 |
| regulation of cell morphogenesis | 1.38E-02 | 2 | 17305 | 461 | 1724 | 75 |
| negative regulation of macromolecule metabolic process | 6.81E-10 | 2 | 17305 | 2286 | 1138 | 236 |
| positive regulation of RNA biosynthetic process | 1.34E-10 | 2 | 17305 | 1395 | 2035 | 256 |
| cellular component morphogenesis | 4.05E-03 | 2 | 17305 | 454 | 2182 | 89 |
| positive regulation of biological process | 1.91E-09 | 2 | 17305 | 5170 | 431 | 199 |
| positive regulation of macromolecule metabolic process | 2.48E-10 | 2 | 17305 | 2771 | 1015 | 252 |
| positive regulation of RNA metabolic process | 1.07E-10 | 2 | 17305 | 1434 | 2035 | 262 |
| positive regulation of gene expression | 9.19E-12 | 2 | 17305 | 1681 | 2041 | 303 |
| protein-DNA complex assembly | 5.36E-03 | 2 | 17305 | 142 | 5444 | 68 |
| osteoblast differentiation | 2.71E-02 | 2 | 17305 | 104 | 6647 | 60 |
| nucleosome organization | 8.18E-03 | 1 | 17305 | 145 | 5623 | 70 |
| positive regulation of cellular metabolic process | 4.14E-08 | 1 | 17305 | 2789 | 1007 | 242 |
| cellular developmental process | 1.42E-16 | 1 | 17305 | 2434 | 2262 | 475 |
| positive regulation of macromolecule biosynthetic process | 4.29E-09 | 1 | 17305 | 1581 | 2035 | 276 |
| positive regulation of nitrogen compound metabolic process | 4.29E-10 | 1 | 17305 | 1724 | 2035 | 301 |
| regulation of macromolecule metabolic process | 6.49E-18 | 1 | 17305 | 5428 | 969 | 450 |
| regulation of cellular metabolic process | 2.66E-18 | 1 | 17305 | 5504 | 969 | 456 |
| regulation of primary metabolic process | 1.01E-18 | 1 | 17305 | 5417 | 1008 | 467 |
| positive regulation of canonical Wnt signaling pathway | 1.12E-02 | 1 | 17305 | 128 | 6681 | 71 |

| | | | | | |
|---|---|---|---|---|---|
| negative regulation of programmed cell death | 1.46E-02 | 1 | 17305 | 816 | 1634 | 111 |
| negative regulation of cell death | 1.03E-02 | 1 | 17305 | 878 | 1634 | 119 |
| negative regulation of signal transduction | 4.44E-04 | 1 | 17305 | 1036 | 1886 | 163 |
| negative regulation of apoptotic process | 1.95E-02 | 1 | 17305 | 807 | 1634 | 109 |
| regulation of metabolic process | 1.56E-18 | 1 | 17305 | 6268 | 962 | 500 |
| nucleocytoplasmic transport | 1.68E-02 | 1 | 17305 | 211 | 4975 | 86 |
| nuclear transport | 1.45E-02 | 1 | 17305 | 216 | 4975 | 88 |
| response to ionizing radiation | 5.40E-03 | 1 | 17305 | 142 | 7179 | 83 |
| ribonucleoprotein complex assembly | 4.76E-03 | 1 | 17305 | 178 | 6408 | 93 |
| regulation of protein serine/threonine kinase activity | 2.46E-02 | 1 | 17305 | 586 | 2213 | 106 |
| negative regulation of cell communication | 6.47E-04 | 1 | 17305 | 1157 | 1886 | 178 |
| cell cycle G1/S phase transition | 1.58E-02 | 1 | 17305 | 144 | 6588 | 77 |
| G1/S transition of mitotic cell cycle | 1.58E-02 | 1 | 17305 | 144 | 6588 | 77 |
| tRNA processing | 1.12E-02 | 1 | 17305 | 139 | 7127 | 80 |
| rRNA processing | 7.97E-03 | 1 | 17305 | 141 | 7210 | 82 |
| single-organism developmental process | 5.19E-20 | 1 | 17305 | 4192 | 2001 | 681 |
| cellular component biogenesis | 1.98E-02 | 1 | 17305 | 128 | 7220 | 74 |
| cell proliferation | 1.10E-02 | 1 | 17305 | 643 | 2535 | 131 |
| negative regulation of response to stimulus | 3.78E-04 | 1 | 17305 | 1318 | 1886 | 200 |
| regulation of cell cycle G1/S phase transition | 2.22E-02 | 1 | 17305 | 148 | 6707 | 79 |
| rRNA metabolic process | 1.54E-02 | 1 | 17305 | 147 | 7095 | 83 |
| negative regulation of mitotic cell cycle phase transition | 1.70E-02 | 1 | 17305 | 157 | 6707 | 84 |
| ribonucleoprotein complex subunit organization | 9.21E-03 | 1 | 17305 | 188 | 6408 | 96 |
| negative regulation of signaling | 3.01E-03 | 1 | 17305 | 1145 | 1886 | 172 |
| movement of cell or subcellular component | 9.74E-04 | 1 | 17305 | 1451 | 1686 | 195 |
| developmental process | 4.16E-23 | 1 | 17305 | 4552 | 2236 | 813 |
| positive regulation of Wnt signaling pathway | 2.11E-02 | 1 | 17305 | 161 | 6681 | 85 |
| translational elongation | 5.86E-03 | 1 | 17305 | 183 | 7064 | 102 |
| ncRNA processing | 2.77E-05 | 1 | 17305 | 307 | 7127 | 173 |
| protein-DNA complex subunit organization | 9.29E-03 | 1 | 17305 | 168 | 7181 | 95 |
| tRNA metabolic process | 3.70E-03 | 1 | 17305 | 190 | 7214 | 108 |
| translational termination | 1.77E-02 | 1 | 17305 | 165 | 7064 | 91 |
| RNA splicing | 1.63E-02 | 1 | 17305 | 298 | 4929 | 115 |
| nuclear division | 3.24E-04 | 1 | 17305 | 295 | 6970 | 160 |
| cell division | 1.16E-04 | 1 | 17305 | 350 | 6775 | 184 |
| nuclear-transcribed mRNA catabolic process | 2.74E-02 | 1 | 17305 | 174 | 6949 | 93 |
| DNA recombination | 3.59E-03 | 1 | 17305 | 237 | 7196 | 131 |
| organelle fission | 3.18E-04 | 1 | 17305 | 320 | 6970 | 172 |
| regulation of signal transduction | 1.30E-03 | 1 | 17305 | 2506 | 1197 | 231 |
| ncRNA metabolic process | 3.11E-06 | 1 | 17305 | 437 | 7214 | 242 |
| negative regulation of cell cycle process | 1.27E-02 | 1 | 17305 | 237 | 6707 | 121 |
| mRNA processing | 2.07E-02 | 1 | 17305 | 354 | 4929 | 133 |
| mitotic cell cycle phase transition | 3.95E-03 | 1 | 17305 | 275 | 6649 | 140 |
| cell cycle phase transition | 5.79E-03 | 1 | 17305 | 279 | 6649 | 141 |
| regulation of mitotic cell cycle phase transition | 1.07E-02 | 1 | 17305 | 255 | 6741 | 130 |
| positive regulation of cell cycle | 2.97E-03 | 1 | 17305 | 324 | 6458 | 159 |
| chromosome organization | 2.62E-03 | 1 | 17305 | 309 | 6842 | 160 |
| positive regulation of metabolic process | 1.53E-09 | 1 | 17305 | 3432 | 2045 | 531 |
| translational initiation | 2.77E-02 | 1 | 17305 | 209 | 7064 | 111 |
| regulation of cell cycle phase transition | 1.27E-02 | 1 | 17305 | 276 | 6741 | 139 |
| regulation of cell cycle process | 6.42E-04 | 1 | 17305 | 529 | 5761 | 228 |
| mitotic cell cycle process | 3.17E-07 | 1 | 17305 | 693 | 6775 | 351 |
| translation | 5.62E-03 | 1 | 17305 | 322 | 7064 | 168 |
| cellular macromolecular complex assembly | 4.41E-06 | 1 | 17305 | 612 | 7131 | 322 |
| DNA metabolic process | 4.32E-08 | 1 | 17305 | 740 | 7196 | 395 |
| DNA repair | 2.49E-04 | 1 | 17305 | 455 | 7196 | 241 |
| RNA processing | 7.06E-07 | 1 | 17305 | 696 | 7148 | 366 |
| peptide biosynthetic process | 7.58E-03 | 1 | 17305 | 343 | 7064 | 177 |
| cellular response to DNA damage stimulus | 2.16E-06 | 1 | 17305 | 697 | 7196 | 366 |
| regulation of gene expression | 2.87E-21 | 1 | 17305 | 3964 | 4381 | 1268 |
| nucleic acid metabolic process | 1.06E-42 | 1 | 17305 | 3690 | 7181 | 1928 |
| gene expression | 1.37E-07 | 1 | 17305 | 921 | 7175 | 476 |
| positive regulation of cellular process | 8.59E-09 | 1 | 17305 | 4454 | 1999 | 645 |
| RNA metabolic process | 4.93E-33 | 1 | 17305 | 3185 | 7163 | 1650 |
| mitotic cell cycle | 1.19E-02 | 1 | 17305 | 418 | 6663 | 200 |
| regulation of cell cycle | 4.13E-05 | 1 | 17305 | 987 | 5789 | 409 |
| cell cycle process | 1.98E-07 | 1 | 17305 | 1020 | 6914 | 505 |
| RNA biosynthetic process | 5.69E-21 | 1 | 17305 | 2526 | 7163 | 1292 |
| nucleobase-containing compound metabolic process | 3.53E-41 | 1 | 17305 | 4107 | 7181 | 2108 |
| regulation of mitotic cell cycle | 5.12E-03 | 1 | 17305 | 469 | 6993 | 234 |
| cell cycle | 1.32E-03 | 1 | 17305 | 605 | 6702 | 289 |
| regulation of response to stimulus | 1.85E-02 | 1 | 17305 | 3496 | 1199 | 297 |
| regulation of cellular process | 1.36E-09 | 1 | 17305 | 9556 | 736 | 501 |
| transcription, DNA-templated | 3.01E-17 | 1 | 17305 | 2248 | 7161 | 1146 |
| nucleic acid-templated transcription | 2.40E-17 | 1 | 17305 | 2249 | 7161 | 1147 |
| cellular macromolecule biosynthetic process | 4.28E-23 | 1 | 17305 | 2827 | 7199 | 1446 |
| aromatic compound biosynthetic process | 1.52E-22 | 1 | 17305 | 2865 | 7186 | 1458 |
| macromolecule biosynthetic process | 2.48E-25 | 1 | 17305 | 3105 | 7199 | 1584 |
| heterocycle metabolic process | 5.88E-40 | 1 | 17305 | 4291 | 7181 | 2184 |
| cellular aromatic compound metabolic process | 3.64E-40 | 1 | 17305 | 4304 | 7181 | 2191 |
| negative regulation of cell cycle | 1.99E-02 | 1 | 17305 | 456 | 6930 | 222 |
| mRNA metabolic process | 5.48E-03 | 1 | 17305 | 521 | 7148 | 262 |

| | | | | | | |
|---|---|---|---|---|---|---|
| chromatin organization | 7.96E-04 | 1 | 17305 | 620 | 7196 | 315 |
| regulation of cell communication | 1.96E-03 | 1 | 17305 | 2866 | 1999 | 405 |
| nucleobase-containing compound biosynthetic process | 1.65E-21 | 1 | 17305 | 2798 | 7186 | 1422 |
| heterocycle biosynthetic process | 2.28E-22 | 1 | 17305 | 2863 | 7186 | 1456 |
| organic cyclic compound biosynthetic process | 1.13E-21 | 1 | 17305 | 2984 | 7186 | 1507 |
| cellular nitrogen compound biosynthetic process | 8.61E-25 | 1 | 17305 | 3177 | 7260 | 1625 |
| regulation of signaling | 4.05E-03 | 1 | 17305 | 2843 | 1999 | 399 |
| regulation of biological process | 2.17E-08 | 1 | 17305 | 10071 | 709 | 499 |
| organic cyclic compound metabolic process | 2.40E-35 | 1 | 17305 | 4530 | 7181 | 2268 |
| cellular nitrogen compound metabolic process | 8.32E-42 | 1 | 17305 | 4855 | 7243 | 2462 |
| regulation of nucleobase-containing compound metabolic process | 4.31E-23 | 1 | 17305 | 3715 | 7164 | 1838 |
| cellular biosynthetic process | 9.79E-27 | 1 | 17305 | 3948 | 7260 | 1984 |
| nitrogen compound metabolic process | 5.10E-41 | 1 | 17305 | 5176 | 7243 | 2600 |
| cellular macromolecule catabolic process | 1.29E-02 | 1 | 17305 | 633 | 6728 | 294 |
| regulation of nitrogen compound metabolic process | 2.42E-25 | 1 | 17305 | 3991 | 7164 | 1973 |
| organic substance biosynthetic process | 3.13E-24 | 1 | 17305 | 4080 | 7260 | 2029 |
| biosynthetic process | 3.76E-25 | 1 | 17305 | 4151 | 7260 | 2066 |
| biological regulation | 2.19E-07 | 1 | 17305 | 10534 | 736 | 530 |
| macromolecular complex assembly | 2.86E-04 | 1 | 17305 | 1237 | 6409 | 541 |
| cellular response to stress | 1.78E-06 | 1 | 17305 | 1415 | 7259 | 701 |
| regulation of biosynthetic process | 3.73E-21 | 1 | 17305 | 3930 | 7164 | 1921 |
| cellular macromolecule metabolic process | 2.09E-42 | 1 | 17305 | 6368 | 7181 | 3109 |
| organonitrogen compound biosynthetic process | 1.00E-02 | 1 | 17305 | 900 | 7234 | 435 |
| cellular component assembly | 4.04E-02 | 1 | 17305 | 1839 | 6369 | 784 |
| protein complex assembly | 2.77E-02 | 1 | 17305 | 1010 | 6310 | 424 |
| macromolecule metabolic process | 2.90E-38 | 1 | 17305 | 7048 | 7181 | 3374 |
| macromolecular complex subunit organization | 2.03E-05 | 1 | 17305 | 2110 | 7145 | 990 |
| cellular metabolic process | 6.71E-42 | 1 | 17305 | 8095 | 7256 | 3872 |
| primary metabolic process | 1.31E-34 | 1 | 17305 | 8241 | 7186 | 3858 |
| protein complex subunit organization | 1.31E-02 | 1 | 17305 | 1456 | 7077 | 668 |
| organic substance metabolic process | 5.60E-35 | 1 | 17305 | 8520 | 7245 | 4006 |
| single-organism organelle organization | 6.92E-03 | 1 | 17305 | 1868 | 6993 | 840 |
| cellular response to stimulus | 1.89E-03 | 1 | 17305 | 2357 | 6943 | 1048 |
| organelle organization | 3.00E-04 | 1 | 17305 | 2487 | 6993 | 1120 |
| cellular component organization or biogenesis | 9.02E-09 | 1 | 17305 | 4712 | 6924 | 2085 |
| metabolic process | 2.54E-36 | 1 | 17305 | 9396 | 7186 | 4346 |
| cellular component organization | 2.52E-08 | 1 | 17305 | 4677 | 6924 | 2066 |
| macromolecule modification | 3.05E-03 | 1 | 17305 | 2891 | 7046 | 1286 |

SUPPLEMENTARY DATA 3

Supplementary Data 3 provides a list of ranked genes based on a bistability score and its association with a list of imprinted genes (CPOE) as well as a list of genes exhibiting monoallelic expression (MAE). Supplementary Data 3 as attached hereto includes a portion of the collective data set as a representative sample.

EP 3 472 358 B1

| GENE | BISTABILITY SCORE | CPOE | MAE | FULL NAME |
|---|---|---|---|---|
| TULP2 | 0.27390 | | | tubby like protein 2 |
| NUCB1 | 0.27372 | | | nucleobindin 1 |
| SNRPN | 0.22569 | ✔ | ✔ | small nuclear ribonucleoprotein polypeptide N |
| SNURF | 0.17791 | ✔ | | SNRPN upstream reading frame |
| ALOX12P2 | 0.16653 | | | arachidonate 12-lipoxygenase pseudogene 2 |
| TAPBPL | 0.16147 | | | TAP binding protein-like |
| WDR81 | 0.15800 | | ✔ | WD repeat domain 81 |
| MEST | 0.15557 | ✔ | | mesoderm specific transcript |
| MESTIT1 | 0.15557 | ✔ | | MEST intronic transcript 1, antisense RNA |
| SERPINE1 | 0.15441 | | ✔ | serpin peptidase inhibitor, clade E (nexin, plasminogen activator inhibito |
| SNORD32A | 0.14900 | | | small nucleolar RNA, C/D box 32A |
| CSTF3 | 0.14716 | | | cleavage stimulation factor, 3' pre-RNA, subunit 3 |
| CSTF3-AS1 | 0.14654 | | | CSTF3 antisense RNA 1 (head to head) |
| MIR22HG | 0.14534 | | | MIR22 host gene |
| CD27-AS1 | 0.14462 | | | CD27 antisense RNA 1 |
| RXRA | 0.14199 | | | retinoid X receptor alpha |
| ENDOU | 0.13644 | | | endonuclease, poly(U) specific |
| RNF41 | 0.13109 | | | ring finger protein 41, E3 ubiquitin protein ligase |
| RAPGEF3 | 0.12819 | | | Rap guanine nucleotide exchange factor 3 |
| NLRP1 | 0.12728 | | | NLR family, pyrin domain containing 1 |
| ZIM2 | 0.12709 | ✔ | | zinc finger, imprinted 2 |
| PEG3 | 0.12709 | ✔ | | paternally expressed 3 |
| SMDT1 | 0.12709 | | | single-pass membrane protein with aspartate-rich tail 1 |
| MIMT1 | 0.12681 | ✔ | | MER1 repeat containing imprinted transcript 1 (non-protein coding) |
| PPP2R3C | 0.12657 | | | protein phosphatase 2 regulatory subunit B", gamma |
| FDFT1 | 0.12271 | | | farnesyl-diphosphate farnesyltransferase 1 |
| RPL13A | 0.12051 | | | ribosomal protein L13a |
| TSPAN32 | 0.11887 | | | TSPAN32 |
| CDC16 | 0.11832 | | | cell division cycle 16 |
| VTRNA2-1 | 0.11715 | | | vault RNA 2-1 |
| KIAA0391 | 0.11613 | | | KIAA0391 |
| FLAD1 | 0.11260 | | | flavin adenine dinucleotide synthetase 1 |
| ELF3 | 0.11204 | | | E74-like factor 3 (ets domain transcription factor, epithelial-specific ) |
| PPP2R1B | 0.11184 | | | protein phosphatase 2 regulatory subunit A, beta |
| C11orf21 | 0.11175 | | ✔ | chromosome 11 open reading frame 21 |
| UBAP1 | 0.11175 | | | ubiquitin associated protein 1 |
| FMN1 | 0.10960 | | ✔ | formin 1 |
| TAGAP | 0.10956 | | | T-cell activation RhoGTPase activating protein |
| TOLLIP | 0.10879 | | | toll interacting protein |
| PEG10 | 0.10813 | ✔ | ✔ | paternally expressed 10 |
| CCDC125 | 0.10787 | | | coiled-coil domain containing 125 |
| IL16 | 0.10737 | | ✔ | interleukin 16 |
| SEMA6B | 0.10737 | | | sema domain, transmembrane domain (TM), and cytoplasmic domain, ( |
| TMEM173 | 0.10737 | | | transmembrane protein 173 |
| KRBA2 | 0.10665 | | | KRAB-A domain containing 2 |
| WSB1 | 0.10518 | | | WD repeat and SOCS box containing 1 |
| MIR4522 | 0.10518 | | | microRNA 4522 |
| ACAP3 | 0.10438 | | ✔ | ArfGAP with coiled-coil, ankyrin repeat and PH domains 3 |
| SLC25A32 | 0.10375 | | | solute carrier family 25 (mitochondrial folate carrier), member 32 |
| FBXO46 | 0.10299 | | | F-box protein 46 |
| ZMYND8 | 0.10299 | | | zinc finger, MYND-type containing 8 |
| MYH9 | 0.10299 | | | myosin, heavy chain 9, non-muscle |
| ASIC1 | 0.10294 | | | acid sensing ion channel subunit 1 |
| RGS12 | 0.10232 | | ✔ | regulator of G-protein signaling 12 |

| Gene | Value | Col1 | Col2 | Description |
|---|---|---|---|---|
| ISG15 | 0.10079 | | ✔ | ISG15 ubiquitin-like modifier |
| ACAP1 | 0.10079 | | ✔ | ArfGAP with coiled-coil, ankyrin repeat and PH domains 1 |
| PHACTR3 | 0.10079 | | ✔ | phosphatase and actin regulator 3 |
| WSCD2 | 0.09860 | | ✔ | WSC domain containing 2 |
| IDH2 | 0.09860 | | ✔ | isocitrate dehydrogenase 2 (NADP+), mitochondrial |
| DHX37 | 0.09838 | | | DEAH-box helicase 37 |
| SGCE | 0.09746 | ✔ | ✔ | sarcoglycan epsilon |
| SUDS3 | 0.09744 | | | apolipoprotein L6 |
| ATAD5 | 0.09641 | | | ATPase family, AAA domain containing 5 |
| LINC00961 | 0.09641 | | | long intergenic non-protein coding RNA 961 |
| EPN1 | 0.09628 | | | epsin 1 |
| ZCCHC24 | 0.09613 | | ✔ | zinc finger, CCHC domain containing 24 |
| AP4E1 | 0.09522 | | | adaptor related protein complex 4 epsilon 1 subunit |
| TFEB | 0.09518 | | ✔ | transcription factor EB |
| HNRNPA3 | 0.09463 | | | heterogeneous nuclear ribonucleoprotein A3 |
| RPH3AL | 0.09422 | | | rabphilin 3A-like (without C2 domains) |
| AMER3 | 0.09422 | | | APC membrane recruitment protein 3 |
| EXOC4 | 0.09422 | | | exocyst complex component 4 |
| SYTL1 | 0.09375 | | ✔ | synaptotagmin like 1 |
| LOC100506178 | 0.09349 | | | uncharacterized LOC100506178 |
| APOL6 | 0.09306 | | | SDS3 homolog, SIN3A corepressor complex component |
| ZBP1 | 0.09234 | | ✔ | Z-DNA binding protein 1 |
| PLEKHB1 | 0.09203 | | ✔ | pleckstrin homology domain containing B1 |
| MYL6 | 0.09203 | | | myosin light chain 6 |
| MAGEL2 | 0.09203 | ✔ | | MAGE family member L2 |
| AKR1B15 | 0.09203 | | | aldo-keto reductase family 1, member B15 |
| FES | 0.09171 | | ✔ | FES proto-oncogene, tyrosine kinase |
| MIR4444-1 | 0.09087 | | | microRNA 4444-1 |
| HIVEP3 | 0.08984 | | ✔ | human immunodeficiency virus type I enhancer binding protein 3 |
| THBS3 | 0.08984 | | ✔ | thrombospondin 3 |
| TNFRSF1A | 0.08984 | | | tumor necrosis factor receptor superfamily member 1A |
| LOC100129083 | 0.08984 | | | uncharacterized LOC100129083 |
| FHL2 | 0.08984 | | ✔ | four and a half LIM domains 2 |
| L3MBTL1 | 0.08984 | ✔ | ✔ | l(3)mbt-like 1 (Drosophila) |
| IMPDH1 | 0.08984 | | ✔ | IMP (inosine 5'-monophosphate) dehydrogenase 1 |
| PDYN | 0.08909 | | | prodynorphin |
| KCNQ1DN | 0.08765 | ✔ | | KCNQ1 downstream neighbor (non-protein coding) |
| LOC644656 | 0.08765 | | | uncharacterized LOC644656 |
| BMF | 0.08765 | | ✔ | Bcl2 modifying factor |
| C15orf52 | 0.08765 | | | chromosome 15 open reading frame 52 |
| KLK8 | 0.08765 | | | kallikrein related peptidase 8 |
| C1D | 0.08765 | | | C1D nuclear receptor corepressor |
| C20orf203 | 0.08765 | | | chromosome 20 open reading frame 203 |
| C2CD2 | 0.08765 | | ✔ | C2 calcium-dependent domain containing 2 |
| CRYBB2P1 | 0.08765 | | | crystallin beta B2 pseudogene 1 |
| EIF4G1 | 0.08765 | | | eukaryotic translation initiation factor 4 gamma 1 |
| C4orf33 | 0.08765 | | ✔ | |
| FKBPL | 0.08765 | | | |
| GATA4 | 0.08765 | | | |
| PNOC | 0.08765 | | | |
| PHKG1 | 0.08750 | | | |
| SMAD7 | 0.08732 | | ✔ | |
| MYO1F | 0.08719 | | | |
| ZNF143 | 0.08715 | | | |
| RBM47 | 0.08682 | | ✔ | |

| Gene | Value | | |
|---|---|---|---|
| LCP2 | 0.08635 | | ✔ |
| ACSL1 | 0.08549 | | ✔ |
| RRP15 | 0.08546 | | |
| HDGF | 0.08546 | | |
| ZNF507 | 0.08546 | | |
| KIAA1683 | 0.08546 | | |
| MX2 | 0.08546 | | |
| KCNT1 | 0.08546 | | |
| NR4A1 | 0.08522 | | ✔ |
| PXT1 | 0.08491 | | |
| CA5A | 0.08475 | | |
| SCNN1A | 0.08469 | | |
| HIST1H2BE | 0.08441 | | |
| PRCC | 0.08403 | | |
| NR1D1 | 0.08351 | | ✔ |
| SDHB | 0.08326 | | |
| C14orf159 | 0.08326 | | |
| DMKN | 0.08326 | | ✔ |
| BIRC7 | 0.08326 | | ✔ |
| KCTD20 | 0.08326 | | |
| CEP63 | 0.08249 | | |
| TTN-AS1 | 0.08232 | | |
| ANAPC13 | 0.08203 | | |
| BCAR3 | 0.08165 | | ✔ |
| DIRAS3 | 0.08107 | ✔ | ✔ |
| LINC01354 | 0.08107 | | |
| LOC100132078 | 0.08107 | | |
| C14orf93 | 0.08107 | | |
| ZNF383 | 0.08107 | | |
| GNAS | 0.08107 | ✔ | ✔ |
| TRAPPC13 | 0.08107 | | |
| HLA-DOA | 0.08107 | | |
| TFR2 | 0.08107 | | |
| GINS4 | 0.08107 | | |
| SEMA4B | 0.08104 | | |
| KRTAP10-4 | 0.07912 | | |
| GUCY1B2 | 0.07888 | | |
| PRCD | 0.07888 | | |
| SP100 | 0.07888 | | ✔ |
| DLGAP4 | 0.07888 | | |
| RRP1B | 0.07888 | | |
| HSF2BP | 0.07888 | | |
| SYNPR | 0.07888 | | |
| RAET1E | 0.07888 | | |
| SMU1 | 0.07888 | | |
| LOC284454 | 0.07871 | | |
| TPCN1 | 0.07835 | | |
| DCAF13 | 0.07771 | | |
| PLEKHG5 | 0.07763 | | ✔ |
| MEF2D | 0.07681 | | |
| EIF2B3 | 0.07669 | | |
| PAQR6 | 0.07669 | | ✔ |
| NABP2 | 0.07669 | | |
| CLPX | 0.07669 | | |
| GPX4 | 0.07669 | | |

| | | | |
|---|---|---|---|
| CACNA1A | 0.07669 | | ✔ |
| IZUMO1 | 0.07669 | | |
| MCHR1 | 0.07669 | | |
| AIMP1 | 0.07669 | | |
| TBCK | 0.07669 | | |
| DIAPH1 | 0.07669 | | |
| REPIN1 | 0.07669 | | ✔ |
| RAPGEF6 | 0.07656 | | |
| USP32 | 0.07576 | | |
| DSCAML1 | 0.07516 | | |
| KCNIP3 | 0.07481 | | ✔ |
| MAB21L3 | 0.07450 | | |
| NRD1 | 0.07450 | | |
| SLC22A11 | 0.07450 | | |
| COL4A2-AS1 | 0.07450 | | |
| FAM57B | 0.07450 | | ✔ |
| MCEMP1 | 0.07450 | | |
| LILRB2 | 0.07450 | | |
| C21orf62-AS1 | 0.07450 | | |
| PAXBP1 | 0.07450 | | |
| RUNX1 | 0.07450 | | ✔ |
| COMT | 0.07450 | | ✔ |
| TBC1D5 | 0.07450 | | |
| MED28 | 0.07450 | | |
| COX7A2 | 0.07450 | | |
| ZUFSP | 0.07450 | | |
| LOC100506474 | 0.07434 | | |
| UFC1 | 0.07400 | | |
| ADH5 | 0.07331 | | |
| ZNF575 | 0.07306 | | |
| LOC100128239 | 0.07303 | | |
| TNPO2 | 0.07268 | | |
| DMPK | 0.07251 | | ✔ |
| PCDH12 | 0.07251 | | |
| TGDS | 0.07235 | | |
| C10orf10 | 0.07231 | | |
| DLG5 | 0.07231 | | |
| STARD13 | 0.07231 | | |
| HAUS4 | 0.07231 | | |
| MIR5093 | 0.07231 | | |
| SERPINF1 | 0.07231 | | ✔ |
| SNHG20 | 0.07231 | | |
| PPP1R15A | 0.07231 | | |
| TMEM190 | 0.07231 | | |
| LOC100507053 | 0.07231 | | |
| SNORD33 | 0.07197 | | |
| ZNF445 | 0.07196 | | |
| UBXN10 | 0.07119 | | |
| MPV17 | 0.07091 | | |
| IKZF1 | 0.07085 | | |
| LOC100131496 | 0.07078 | | |
| LOC100133669 | 0.07076 | | |
| CASP8 | 0.07051 | | |
| ARL5C | 0.07050 | | |
| CTSZ | 0.07044 | | ✔ |

| | | |
|---|---|---|
| MTHFR | 0.07012 | |
| DGKZ | 0.07012 | ✔ |
| ATP5B | 0.07012 | |
| STXBP6 | 0.07012 | ✔ |
| PTPN21 | 0.07012 | |
| PSTPIP1 | 0.07012 | ✔ |
| SLC12A6 | 0.07012 | |
| BAIAP3 | 0.07012 | |
| GPATCH8 | 0.07012 | |
| ZNF90 | 0.07012 | |
| COX6B1 | 0.07012 | |
| LTBP4 | 0.07012 | ✔ |
| LILRB5 | 0.07012 | |
| PARVG | 0.07012 | |
| HPS4 | 0.07012 | |
| MB | 0.07012 | |

SUPPLEMENTARY DATA 4

Supplementary Data 4 shows a matrix of A/B compartment switching frequencies among 34 genomic samples. Supplementary Data 4 is attached hereto in its entirety.

| PHENOTYPES | B-stem | B-colonnormal | B-livernormal-1 | B-livernormal-2 |
|---|---|---|---|---|
| A-stem | 0.00% | 22.53% | 21.95% | 22.21% |
| A-colonnormal | 22.29% | 0.00% | 8.58% | 8.50% |
| A-livernormal-1 | 21.75% | 8.60% | 0.00% | 5.53% |
| A-livernormal-2 | 21.74% | 8.19% | 5.19% | 0.00% |
| A-livernormal-3 | 21.76% | 8.36% | 5.33% | 5.70% |
| A-livernormal-4 | 22.55% | 9.17% | 7.12% | 7.26% |
| A-livernormal-5 | 21.51% | 9.04% | 6.93% | 7.45% |
| A-lungnormal-1 | 21.49% | 8.04% | 9.13% | 9.41% |
| A-lungnormal-2 | 21.75% | 8.74% | 9.94% | 10.03% |
| A-lungnormal-3 | 21.81% | 10.28% | 11.28% | 11.52% |
| A-coloncancer | 23.12% | 9.96% | 10.44% | 10.61% |
| A-livercancer-1 | 22.74% | 14.80% | 15.04% | 14.86% |
| A-livercancer-2 | 21.64% | 11.52% | 9.99% | 10.17% |
| A-livercancer-3 | 27.35% | 14.94% | 13.41% | 13.93% |
| A-lungcancer-1 | 24.60% | 10.56% | 10.94% | 11.29% |
| A-lungcancer-2 | 23.34% | 6.85% | 9.88% | 9.92% |
| A-lungcancer-3 | 23.24% | 12.06% | 12.44% | 12.47% |
| A-brain-1 | 22.96% | 12.42% | 13.50% | 13.38% |
| A-brain-2 | 21.59% | 12.14% | 13.49% | 13.38% |
| A-fibro-P4 | 25.71% | 15.52% | 15.05% | 15.37% |
| A-fibro-P7 | 21.27% | 11.04% | 10.49% | 10.74% |
| A-fibro-P10 | 21.14% | 11.35% | 10.67% | 10.99% |
| A-fibro-P31 | 21.87% | 12.27% | 12.08% | 12.31% |
| A-fibro-P33 | 21.81% | 12.36% | 12.18% | 12.47% |
| A-CD4-Y1 | 22.92% | 9.88% | 12.03% | 11.83% |
| A-CD4-Y2 | 22.79% | 8.98% | 11.39% | 11.40% |
| A-CD4-Y3 | 22.88% | 10.74% | 12.86% | 12.69% |
| A-CD4-O1 | 22.83% | 5.65% | 9.07% | 8.86% |
| A-CD4-O2 | 22.62% | 6.87% | 9.82% | 9.50% |
| A-CD4-O3 | 22.78% | 6.42% | 9.73% | 9.51% |
| A-ker-Y1 | 22.68% | 11.48% | 12.58% | 12.55% |
| A-ker-Y2 | 22.54% | 11.91% | 12.90% | 12.90% |
| A-ker-O1 | 22.63% | 10.16% | 10.83% | 10.76% |
| A-ker-O2 | 21.88% | 9.71% | 9.62% | 9.97% |

| |
|---|
| switching ≥ 10% |
| switching < 10% |
| switching = 0% |

| PHENOTYPES | B-livernormal-3 | B-livernormal-4 | B-livernormal-5 | B-lungnormal-1 |
|---|---|---|---|---|
| A-stem | 22.02% | 22.75% | 21.72% | 21.61% |
| A-colonnormal | 8.37% | 9.17% | 9.03% | 8.03% |
| A-livernormal-1 | 5.36% | 7.12% | 6.92% | 9.12% |
| A-livernormal-2 | 5.38% | 6.90% | 7.11% | 9.08% |
| A-livernormal-3 | 0.00% | 7.01% | 6.84% | 9.31% |
| A-livernormal-4 | 7.05% | 0.00% | 8.33% | 10.37% |
| A-livernormal-5 | 6.88% | 8.33% | 0.00% | 9.52% |
| A-lungnormal-1 | 9.36% | 10.39% | 9.52% | 0.00% |
| A-lungnormal-2 | 10.01% | 10.94% | 10.26% | 8.49% |
| A-lungnormal-3 | 11.34% | 12.22% | 11.78% | 9.82% |
| A-coloncancer | 10.62% | 11.66% | 11.32% | 11.58% |
| A-livercancer-1 | 14.88% | 15.89% | 16.03% | 15.86% |
| A-livercancer-2 | 10.17% | 11.48% | 11.42% | 12.59% |
| A-livercancer-3 | 13.30% | 14.44% | 14.50% | 15.75% |
| A-lungcancer-1 | 11.39% | 12.41% | 11.57% | 11.60% |
| A-lungcancer-2 | 10.04% | 10.97% | 10.48% | 9.08% |
| A-lungcancer-3 | 12.61% | 13.53% | 12.63% | 12.66% |
| A-brain-1 | 13.33% | 13.44% | 14.25% | 13.26% |
| A-brain-2 | 13.12% | 13.37% | 14.34% | 13.08% |
| A-fibro-P4 | 15.16% | 16.22% | 15.40% | 15.45% |
| A-fibro-P7 | 10.71% | 11.72% | 10.99% | 11.20% |
| A-fibro-P10 | 10.96% | 11.68% | 11.33% | 11.58% |
| A-fibro-P31 | 12.34% | 13.06% | 12.35% | 12.39% |
| A-fibro-P33 | 12.44% | 13.20% | 12.44% | 12.49% |
| A-CD4-Y1 | 11.79% | 11.96% | 12.98% | 11.81% |
| A-CD4-Y2 | 11.06% | 11.27% | 12.23% | 10.92% |
| A-CD4-Y3 | 12.56% | 12.70% | 13.74% | 12.66% |
| A-CD4-O1 | 8.83% | 9.38% | 9.81% | 8.60% |
| A-CD4-O2 | 9.42% | 9.96% | 10.48% | 9.29% |
| A-CD4-O3 | 9.47% | 9.87% | 10.37% | 9.10% |
| A-ker-Y1 | 12.48% | 12.83% | 13.36% | 12.45% |
| A-ker-Y2 | 12.83% | 13.19% | 13.68% | 12.68% |
| A-ker-O1 | 10.74% | 11.38% | 11.63% | 11.10% |
| A-ker-O2 | 9.77% | 10.64% | 10.56% | 10.70% |

| |
|---|
| switching ≥ 10% |
| switching < 10% |
| switching = 0% |

| PHENOTYPES | B-lungnormal-2 | B-lungnormal-3 | B-coloncancer | B-livercancer-1 |
|---|---|---|---|---|
| A-stem | 21.99% | 21.99% | 21.05% | 23.70% |
| A-colonnormal | 8.72% | 10.26% | 7.64% | 15.93% |
| A-livernormal-1 | 9.96% | 11.28% | 8.13% | 16.17% |
| A-livernormal-2 | 9.78% | 11.29% | 7.95% | 15.80% |
| A-livernormal-3 | 10.01% | 11.31% | 8.28% | 15.98% |
| A-livernormal-4 | 10.98% | 12.22% | 9.36% | 17.04% |
| A-livernormal-5 | 10.29% | 11.79% | 9.01% | 17.19% |
| A-lungnormal-1 | 8.52% | 9.83% | 9.28% | 17.05% |
| A-lungnormal-2 | 0.00% | 10.18% | 10.04% | 17.21% |
| A-lungnormal-3 | 10.19% | 0.00% | 11.40% | 17.89% |
| A-coloncancer | 12.37% | 13.71% | 0.00% | 17.85% |
| A-livercancer-1 | 16.18% | 16.81% | 14.37% | 0.00% |
| A-livercancer-2 | 12.96% | 13.99% | 10.64% | 11.06% |
| A-livercancer-3 | 16.46% | 17.47% | 14.50% | 23.73% |
| A-lungcancer-1 | 12.67% | 14.21% | 9.35% | 19.70% |
| A-lungcancer-2 | 9.72% | 11.57% | 9.03% | 17.72% |
| A-lungcancer-3 | 13.34% | 14.50% | 11.05% | 19.23% |
| A-brain-1 | 13.77% | 14.19% | 13.14% | 18.33% |
| A-brain-2 | 13.38% | 13.58% | 12.94% | 17.00% |
| A-fibro-P4 | 16.25% | 17.32% | 14.96% | 22.87% |
| A-fibro-P7 | 11.84% | 13.04% | 10.59% | 17.46% |
| A-fibro-P10 | 12.26% | 13.38% | 10.02% | 17.67% |
| A-fibro-P31 | 13.05% | 14.24% | 11.79% | 17.72% |
| A-fibro-P33 | 13.20% | 14.29% | 11.95% | 17.69% |
| A-CD4-Y1 | 12.24% | 13.12% | 11.33% | 17.38% |
| A-CD4-Y2 | 11.45% | 12.41% | 10.71% | 17.22% |
| A-CD4-Y3 | 13.06% | 13.54% | 11.92% | 17.78% |
| A-CD4-O1 | 9.29% | 10.79% | 8.10% | 16.13% |
| A-CD4-O2 | 10.02% | 11.09% | 8.98% | 16.48% |
| A-CD4-O3 | 9.75% | 11.07% | 8.84% | 16.38% |
| A-ker-Y1 | 13.09% | 13.55% | 12.09% | 18.06% |
| A-ker-Y2 | 13.25% | 13.71% | 12.20% | 18.29% |
| A-ker-O1 | 11.71% | 12.60% | 10.39% | 17.48% |
| A-ker-O2 | 11.22% | 12.25% | 9.35% | 17.34% |

| switching ≥ 10% |
|---|
| switching < 10% |
| switching = 0% |

| PHENOTYPES | B-livercancer-2 | B-livercancer-3 | B-lungcancer-1 | B-lungcancer-2 |
|---|---|---|---|---|
| A-stem | 24.26% | 16.65% | 19.71% | 21.15% |
| A-colonnormal | 14.06% | 4.01% | 5.50% | 4.41% |
| A-livernormal-1 | 12.50% | 2.49% | 5.90% | 7.47% |
| A-livernormal-2 | 12.51% | 2.66% | 5.87% | 7.21% |
| A-livernormal-3 | 12.70% | 2.36% | 6.32% | 7.61% |
| A-livernormal-4 | 13.98% | 3.53% | 7.36% | 8.57% |
| A-livernormal-5 | 13.95% | 3.61% | 6.54% | 8.08% |
| A-lungnormal-1 | 15.12% | 4.84% | 6.59% | 6.67% |
| A-lungnormal-2 | 15.47% | 5.50% | 7.59% | 7.28% |
| A-lungnormal-3 | 16.47% | 6.55% | 9.18% | 9.14% |
| A-coloncancer | 15.49% | 5.91% | 6.60% | 8.93% |
| A-livercancer-1 | 12.71% | 11.66% | 13.58% | 14.17% |
| A-livercancer-2 | 0.00% | 7.56% | 9.10% | 10.25% |
| A-livercancer-3 | 21.06% | 0.00% | 11.73% | 13.65% |
| A-lungcancer-1 | 16.72% | 5.87% | 0.00% | 8.08% |
| A-lungcancer-2 | 15.21% | 5.15% | 5.45% | 0.00% |
| A-lungcancer-3 | 16.80% | 7.48% | 7.60% | 10.14% |
| A-brain-1 | 17.91% | 8.53% | 11.74% | 11.94% |
| A-brain-2 | 16.89% | 8.89% | 11.85% | 11.84% |
| A-fibro-P4 | 20.94% | 8.87% | 11.72% | 13.80% |
| A-fibro-P7 | 15.37% | 6.08% | 7.84% | 9.54% |
| A-fibro-P10 | 15.58% | 6.10% | 7.64% | 9.86% |
| A-fibro-P31 | 15.62% | 7.75% | 8.63% | 10.60% |
| A-fibro-P33 | 15.63% | 7.85% | 8.73% | 10.69% |
| A-CD4-Y1 | 16.66% | 7.05% | 9.91% | 9.77% |
| A-CD4-Y2 | 16.37% | 6.25% | 8.83% | 8.66% |
| A-CD4-Y3 | 17.14% | 7.68% | 10.50% | 10.40% |
| A-CD4-O1 | 14.40% | 4.14% | 6.29% | 5.34% |
| A-CD4-O2 | 15.07% | 4.78% | 7.03% | 6.37% |
| A-CD4-O3 | 15.07% | 4.65% | 6.88% | 6.09% |
| A-ker-Y1 | 17.29% | 7.46% | 10.31% | 10.79% |
| A-ker-Y2 | 17.71% | 7.78% | 10.92% | 11.15% |
| A-ker-O1 | 16.18% | 5.99% | 8.58% | 9.38% |
| A-ker-O2 | 15.30% | 5.14% | 7.41% | 8.69% |

| |
|---|
| switching ≥ 10% |
| switching < 10% |
| switching = 0% |

| PHENOTYPES | B-lungcancer-3 | B-brain-1 | B-brain-2 | B-fibro-P4 |
|---|---|---|---|---|
| A-stem | 20.03% | 23.04% | 24.38% | 17.06% |
| A-colonnormal | 8.62% | 12.36% | 14.87% | 6.64% |
| A-livernormal-1 | 9.03% | 13.45% | 16.20% | 6.19% |
| A-livernormal-2 | 8.73% | 13.02% | 15.89% | 6.12% |
| A-livernormal-3 | 9.17% | 13.24% | 15.83% | 6.25% |
| A-livernormal-4 | 10.11% | 13.38% | 16.08% | 7.37% |
| A-livernormal-5 | 9.21% | 14.19% | 17.06% | 6.58% |
| A-lungnormal-1 | 9.28% | 13.23% | 15.80% | 6.65% |
| A-lungnormal-2 | 9.89% | 13.71% | 16.10% | 7.36% |
| A-lungnormal-3 | 11.10% | 14.15% | 16.29% | 8.48% |
| A-coloncancer | 9.93% | 15.38% | 17.98% | 8.40% |
| A-livercancer-1 | 14.89% | 17.16% | 18.73% | 12.90% |
| A-livercancer-2 | 10.87% | 15.36% | 17.15% | 9.56% |
| A-livercancer-3 | 15.00% | 19.39% | 22.55% | 10.91% |
| A-lungcancer-1 | 9.25% | 16.72% | 19.59% | 7.94% |
| A-lungcancer-2 | 9.14% | 14.31% | 16.99% | 7.34% |
| A-lungcancer-3 | 0.00% | 16.82% | 19.77% | 8.28% |
| A-brain-1 | 13.46% | 0.00% | 15.70% | 10.38% |
| A-brain-2 | 13.67% | 12.97% | 0.00% | 10.70% |
| A-fibro-P4 | 13.73% | 19.12% | 22.16% | 0.00% |
| A-fibro-P7 | 9.54% | 14.55% | 17.09% | 0.29% |
| A-fibro-P10 | 9.88% | 14.73% | 17.07% | 2.58% |
| A-fibro-P31 | 10.54% | 15.95% | 18.37% | 2.81% |
| A-fibro-P33 | 10.57% | 16.10% | 18.36% | 2.84% |
| A-CD4-Y1 | 12.38% | 13.39% | 15.56% | 9.57% |
| A-CD4-Y2 | 11.49% | 13.32% | 15.35% | 8.95% |
| A-CD4-Y3 | 12.59% | 13.89% | 15.76% | 9.71% |
| A-CD4-O1 | 9.38% | 12.20% | 14.65% | 6.92% |
| A-CD4-O2 | 10.03% | 12.50% | 14.68% | 7.43% |
| A-CD4-O3 | 9.97% | 12.56% | 14.81% | 7.35% |
| A-ker-Y1 | 12.60% | 14.18% | 16.28% | 9.26% |
| A-ker-Y2 | 12.95% | 14.04% | 16.26% | 9.83% |
| A-ker-O1 | 11.09% | 13.56% | 15.75% | 7.71% |
| A-ker-O2 | 9.67% | 13.88% | 16.36% | 6.55% |

| switching ≥ 10% |
|---|
| switching < 10% |
| switching = 0% |

| PHENOTYPES | B-fibro-P7 | B-fibro-P10 | B-fibro-P31 | B-fibro-P33 |
|---|---|---|---|---|
| A-stem | 21.31% | 22.48% | 21.93% | 21.86% |
| A-colonnormal | 10.91% | 12.53% | 12.12% | 12.21% |
| A-livernormal-1 | 10.37% | 11.84% | 11.95% | 12.05% |
| A-livernormal-2 | 10.25% | 11.83% | 11.82% | 11.99% |
| A-livernormal-3 | 10.54% | 12.09% | 12.14% | 12.25% |
| A-livernormal-4 | 11.62% | 12.88% | 12.94% | 13.07% |
| A-livernormal-5 | 10.90% | 12.53% | 12.24% | 12.34% |
| A-lungnormal-1 | 11.13% | 12.80% | 12.31% | 12.41% |
| A-lungnormal-2 | 11.69% | 13.40% | 12.87% | 13.02% |
| A-lungnormal-3 | 12.94% | 14.57% | 14.13% | 14.17% |
| A-coloncancer | 12.78% | 13.50% | 13.96% | 14.12% |
| A-livercancer-1 | 16.31% | 17.81% | 16.64% | 16.59% |
| A-livercancer-2 | 12.74% | 14.25% | 12.96% | 12.99% |
| A-livercancer-3 | 16.86% | 18.18% | 18.50% | 18.60% |
| A-lungcancer-1 | 12.77% | 13.87% | 13.57% | 13.66% |
| A-lungcancer-2 | 11.83% | 13.44% | 12.85% | 12.95% |
| A-lungcancer-3 | 12.83% | 14.45% | 13.82% | 13.86% |
| A-brain-1 | 14.51% | 16.00% | 15.91% | 16.06% |
| A-brain-2 | 14.34% | 15.62% | 15.62% | 15.61% |
| A-fibro-P4 | 9.00% | 12.59% | 11.51% | 11.54% |
| A-fibro-P7 | 0.00% | 6.86% | 5.38% | 5.43% |
| A-fibro-P10 | 5.56% | 0.00% | 6.83% | 6.86% |
| A-fibro-P31 | 5.39% | 8.14% | 0.00% | 1.92% |
| A-fibro-P33 | 5.43% | 8.16% | 1.92% | 0.00% |
| A-CD4-Y1 | 13.74% | 14.60% | 14.78% | 14.88% |
| A-CD4-Y2 | 13.21% | 14.36% | 14.26% | 14.30% |
| A-CD4-Y3 | 13.85% | 15.00% | 15.07% | 15.13% |
| A-CD4-O1 | 11.31% | 12.45% | 12.55% | 12.67% |
| A-CD4-O2 | 11.71% | 12.98% | 12.92% | 13.03% |
| A-CD4-O3 | 11.75% | 12.83% | 12.94% | 13.07% |
| A-ker-Y1 | 13.66% | 15.12% | 14.88% | 15.04% |
| A-ker-Y2 | 14.15% | 15.42% | 15.35% | 15.45% |
| A-ker-O1 | 12.06% | 13.56% | 13.60% | 13.65% |
| A-ker-O2 | 10.66% | 12.29% | 12.42% | 12.51% |

| switching ≥ 10% |
| switching < 10% |
| switching = 0% |

| PHENOTYPES | B-CD4-Y1 | B-CD4-Y2 | B-CD4-Y3 | B-CD4-O1 |
|---|---|---|---|---|
| A-stem | 23.02% | 22.91% | 23.02% | 22.95% |
| A-colonnormal | 9.83% | 8.94% | 10.68% | 5.60% |
| A-livernormal-1 | 12.00% | 11.34% | 12.82% | 9.02% |
| A-livernormal-2 | 11.49% | 11.01% | 12.30% | 8.44% |
| A-livernormal-3 | 11.70% | 10.98% | 12.47% | 8.74% |
| A-livernormal-4 | 11.92% | 11.25% | 12.65% | 9.33% |
| A-livernormal-5 | 12.93% | 12.18% | 13.69% | 9.77% |
| A-lungnormal-1 | 11.78% | 10.91% | 12.62% | 8.59% |
| A-lungnormal-2 | 12.15% | 11.37% | 13.00% | 9.21% |
| A-lungnormal-3 | 13.08% | 12.36% | 13.51% | 10.75% |
| A-coloncancer | 13.60% | 12.99% | 14.19% | 10.36% |
| A-livercancer-1 | 16.16% | 15.99% | 16.50% | 14.87% |
| A-livercancer-2 | 14.06% | 13.81% | 14.55% | 11.78% |
| A-livercancer-3 | 17.91% | 17.11% | 18.53% | 14.98% |
| A-lungcancer-1 | 14.92% | 13.82% | 15.49% | 11.28% |
| A-lungcancer-2 | 12.12% | 11.00% | 12.77% | 7.70% |
| A-lungcancer-3 | 15.76% | 14.86% | 15.97% | 12.73% |
| A-brain-1 | 13.39% | 13.33% | 13.90% | 12.20% |
| A-brain-2 | 12.81% | 12.61% | 13.00% | 11.91% |
| A-fibro-P4 | 18.31% | 17.71% | 18.48% | 15.70% |
| A-fibro-P7 | 13.76% | 13.26% | 13.91% | 11.36% |
| A-fibro-P10 | 13.34% | 13.11% | 13.75% | 11.20% |
| A-fibro-P31 | 14.82% | 14.32% | 15.13% | 12.60% |
| A-fibro-P33 | 14.94% | 14.36% | 15.20% | 12.72% |
| A-CD4-Y1 | 0.00% | 10.66% | 12.24% | 9.31% |
| A-CD4-Y2 | 10.67% | 0.00% | 11.69% | 8.39% |
| A-CD4-Y3 | 12.23% | 11.70% | 0.00% | 10.42% |
| A-CD4-O1 | 9.31% | 8.37% | 10.41% | 0.00% |
| A-CD4-O2 | 9.68% | 8.59% | 10.71% | 5.87% |
| A-CD4-O3 | 9.60% | 8.56% | 10.58% | 5.59% |
| A-ker-Y1 | 13.16% | 12.68% | 13.56% | 11.26% |
| A-ker-Y2 | 13.24% | 12.66% | 13.41% | 11.44% |
| A-ker-O1 | 12.18% | 11.87% | 12.71% | 10.04% |
| A-ker-O2 | 12.58% | 11.93% | 12.91% | 9.82% |

| |
|---|
| switching ≥ 10% |
| switching < 10% |
| switching = 0% |

| PHENOTYPES | B-CD4-O2 | B-CD4-O3 | B-ker-Y1 | B-ker-Y2 |
|---|---|---|---|---|
| A-stem | 22.75% | 22.93% | 22.79% | 22.65% |
| A-colonnormal | 6.82% | 6.37% | 11.43% | 11.82% |
| A-livernormal-1 | 9.77% | 9.70% | 12.53% | 12.86% |
| A-livernormal-2 | 9.08% | 9.11% | 12.21% | 12.55% |
| A-livernormal-3 | 9.34% | 9.40% | 12.43% | 12.76% |
| A-livernormal-4 | 9.92% | 9.83% | 12.80% | 13.13% |
| A-livernormal-5 | 10.43% | 10.33% | 13.32% | 13.60% |
| A-lungnormal-1 | 9.27% | 9.09% | 12.43% | 12.65% |
| A-lungnormal-2 | 9.95% | 9.71% | 13.02% | 13.18% |
| A-lungnormal-3 | 11.05% | 11.03% | 13.51% | 13.66% |
| A-coloncancer | 11.24% | 11.11% | 14.36% | 14.44% |
| A-livercancer-1 | 15.29% | 15.15% | 16.94% | 17.11% |
| A-livercancer-2 | 12.47% | 12.47% | 14.73% | 15.14% |
| A-livercancer-3 | 15.63% | 15.52% | 18.35% | 18.65% |
| A-lungcancer-1 | 12.02% | 11.88% | 15.30% | 15.91% |
| A-lungcancer-2 | 8.73% | 8.48% | 13.15% | 13.52% |
| A-lungcancer-3 | 13.40% | 13.36% | 15.96% | 16.31% |
| A-brain-1 | 12.50% | 12.56% | 14.17% | 14.03% |
| A-brain-2 | 11.94% | 12.06% | 13.54% | 13.49% |
| A-fibro-P4 | 16.20% | 16.14% | 18.04% | 18.59% |
| A-fibro-P7 | 11.75% | 11.81% | 13.72% | 14.18% |
| A-fibro-P10 | 11.72% | 11.59% | 13.89% | 14.16% |
| A-fibro-P31 | 12.97% | 13.02% | 14.95% | 15.40% |
| A-fibro-P33 | 13.08% | 13.15% | 15.10% | 15.50% |
| A-CD4-Y1 | 9.68% | 9.61% | 13.16% | 13.21% |
| A-CD4-Y2 | 8.59% | 8.58% | 12.69% | 12.65% |
| A-CD4-Y3 | 10.71% | 10.59% | 13.55% | 13.39% |
| A-CD4-O1 | 5.87% | 5.59% | 11.26% | 11.43% |
| A-CD4-O2 | 0.00% | 6.44% | 11.73% | 11.83% |
| A-CD4-O3 | 6.42% | 0.00% | 11.70% | 11.77% |
| A-ker-Y1 | 11.73% | 11.69% | 0.00% | 12.43% |
| A-ker-Y2 | 11.84% | 11.78% | 12.44% | 0.00% |
| A-ker-O1 | 10.38% | 10.32% | 11.31% | 11.60% |
| A-ker-O2 | 10.37% | 10.22% | 11.33% | 11.81% |

| switching ≥ 10% |
| switching < 10% |
| switching = 0% |

| PHENOTYPES | B-ker-O1 | B-ker-O2 |
|---|---|---|
| A-stem | 22.75% | 21.99% |
| A-colonnormal | 10.08% | 9.61% |
| A-livernormal-1 | 10.77% | 9.53% |
| A-livernormal-2 | 10.30% | 9.52% |
| A-livernormal-3 | 10.64% | 9.65% |
| A-livernormal-4 | 11.31% | 10.56% |
| A-livernormal-5 | 11.57% | 10.50% |
| A-lungnormal-1 | 11.05% | 10.67% |
| A-lungnormal-2 | 11.62% | 11.12% |
| A-lungnormal-3 | 12.53% | 12.18% |
| A-coloncancer | 12.64% | 11.58% |
| A-livercancer-1 | 16.19% | 16.08% |
| A-livercancer-2 | 13.55% | 12.69% |
| A-livercancer-3 | 16.83% | 15.97% |
| A-lungcancer-1 | 13.57% | 12.40% |
| A-lungcancer-2 | 11.72% | 11.02% |
| A-lungcancer-3 | 14.44% | 13.02% |
| A-brain-1 | 13.57% | 13.88% |
| A-brain-2 | 12.99% | 13.59% |
| A-fibro-P4 | 16.48% | 15.30% |
| A-fibro-P7 | 12.10% | 10.70% |
| A-fibro-P10 | 12.31% | 11.04% |
| A-fibro-P31 | 13.65% | 12.48% |
| A-fibro-P33 | 13.70% | 12.57% |
| A-CD4-Y1 | 12.16% | 12.57% |
| A-CD4-Y2 | 11.88% | 11.93% |
| A-CD4-Y3 | 12.70% | 12.90% |
| A-CD4-O1 | 10.01% | 9.78% |
| A-CD4-O2 | 10.38% | 10.36% |
| A-CD4-O3 | 10.30% | 10.18% |
| A-ker-Y1 | 11.30% | 11.32% |
| A-ker-Y2 | 11.59% | 11.81% |
| A-ker-O1 | 0.00% | 8.42% |
| A-ker-O2 | 8.43% | 0.00% |

| switching ≥ 10% |
|---|
| switching < 10% |
| switching = 0% |

SUPPLEMENTARY DATA 5

Supplementary Data 5 provides a list of gene rankings based on a decreasing differential entropic sensitivity index (dESI) when comparing colon normal to colon cancer. Supplementary Data 5 as attached hereto includes a portion of the collective data set as a representative sample.

Illustrative examples of the invention are attached herein as Supplementary Data 1-5.

coloncancer-VS-colonnormal

| dESI RANKING | |
|---|---|
| GENE | SCORE |
| QKI | 2.2750 |
| CAHM | 2.1461 |
| ANKRD33B | 1.7514 |
| LIMD2 | 1.7255 |
| LOC729683 | 1.7132 |
| FLI1 | 1.6580 |
| PHF21B | 1.6505 |
| HOXA9 | 1.6230 |
| FOXQ1 | 1.5885 |
| PREX1 | 1.5882 |
| POU3F1 | 1.5582 |
| FAT1 | 1.5214 |
| TENM4 | 1.5178 |
| CTBP2 | 1.5115 |
| CHST11 | 1.4625 |
| NDRG4 | 1.4450 |
| AUTS2 | 1.4237 |
| FOXA1 | 1.4107 |
| CHST15 | 1.4105 |
| TBCD | 1.3689 |
| VIM | 1.3611 |
| SOWAHC | 1.3521 |
| SEPT10 | 1.3465 |
| CBS | 1.3382 |
| TMEM178B | 1.3269 |
| PPP1R16B | 1.3217 |
| CRHR1 | 1.3167 |
| IKZF1 | 1.3159 |
| FAM110C | 1.3140 |
| EFNB2 | 1.3047 |
| ARHGAP21 | 1.3005 |
| NGFR | 1.2980 |
| NR2F2 | 1.2828 |
| KCNK12 | 1.2793 |
| BMP2 | 1.2750 |
| HOXD8 | 1.2641 |
| ZIC2 | 1.2577 |
| FAM84A | 1.2513 |
| MAFB | 1.2387 |
| ENOSF1 | 1.2386 |
| BCL2L11 | 1.2386 |
| LBH | 1.2367 |
| IRS2 | 1.2338 |
| CSMD2 | 1.2305 |
| WNT7A | 1.2295 |
| LOC101054525 | 1.2278 |
| PLXNC1 | 1.2196 |
| KLF4 | 1.2125 |

coloncancer-VS-colonnorma

| | |
|---|---|
| IGSF9B | 1.2069 |
| WNT3A | 1.2019 |
| CEBPA-AS1 | 1.1916 |
| CEBPA | 1.1888 |
| T | 1.1861 |
| LHX1 | 1.1841 |
| BRSK2 | 1.1824 |
| FAM19A5 | 1.1769 |
| ZMIZ1 | 1.1755 |
| ID4 | 1.1613 |
| RASSF10 | 1.1603 |
| SATB2 | 1.1567 |
| FZD8 | 1.1434 |
| ZNF570 | 1.1409 |
| SMOC2 | 1.1405 |
| TMEM132E | 1.1404 |
| NSG1 | 1.1387 |
| RAVER1 | 1.1360 |
| UST | 1.1330 |
| RGS20 | 1.1325 |
| CLDN5 | 1.1310 |
| MTCL1 | 1.1300 |
| PDE8A | 1.1139 |
| GNAQ | 1.1127 |
| MTA2 | 1.1102 |
| RASGRP2 | 1.1007 |
| PDE8B | 1.0989 |
| TIAM1 | 1.0966 |
| ZBTB46 | 1.0956 |
| ACTN1 | 1.0793 |
| POU4F1 | 1.0787 |
| JAG1 | 1.0771 |
| RSPO3 | 1.0757 |
| ZNRF3 | 1.0735 |
| GTF2IRD1 | 1.0717 |
| THRB | 1.0712 |
| ADAMTS1 | 1.0655 |
| KCNQ5 | 1.0642 |
| PAX6 | 1.0617 |
| NTRK3 | 1.0603 |
| NFIX | 1.0547 |
| ADAM23 | 1.0535 |
| CCDC85C | 1.0507 |
| GLB1L3 | 1.0461 |
| ZNF569 | 1.0458 |
| RUNX1 | 1.0446 |
| BHLHE22 | 1.0430 |
| THRB-AS1 | 1.0414 |
| B3GAT2 | 1.0391 |
| KBTBD11 | 1.0334 |
| PRDM12 | 1.0334 |

| | coloncancer-VS-colonnormal |
|---|---|
| PIK3CD | 1.0327 |
| SDC2 | 1.0324 |
| LOC285696 | 1.0319 |
| SH2D3C | 1.0303 |
| KIF5C | 1.0290 |
| PDE10A | 1.0280 |
| GFRA1 | 1.0279 |
| FAM20C | 1.0274 |
| KIF1A | 1.0267 |
| GUCY1A2 | 1.0265 |
| HSF4 | 1.0228 |
| JPH3 | 1.0222 |
| BASP1 | 1.0212 |
| NCOR2 | 1.0207 |
| SOX7 | 1.0205 |
| RNF220 | 1.0200 |
| PYDC1 | 1.0187 |
| LINGO1 | 1.0152 |
| GJC1 | 1.0136 |
| ACVR2A | 1.0129 |
| C2CD4C | 1.0102 |
| KIF26B | 1.0095 |
| PCDH9 | 1.0076 |
| MPPED2 | 1.0066 |
| FKBP1B | 1.0059 |
| APP | 1.0054 |
| AXIN2 | 1.0035 |
| BARX1 | 0.9994 |
| CASKIN1 | 0.9992 |
| TUSC1 | 0.9988 |
| MAPK11 | 0.9940 |
| PRICKLE1 | 0.9938 |
| ACTN1-AS1 | 0.9937 |
| RAB11FIP4 | 0.9911 |
| ROR2 | 0.9902 |
| LMX1B | 0.9873 |
| RTKN | 0.9809 |
| PAX5 | 0.9802 |
| GSG1L | 0.9787 |
| PLD5 | 0.9766 |
| PPIC | 0.9748 |
| TMEM163 | 0.9730 |
| PGR | 0.9728 |
| BMP6 | 0.9722 |
| SLC44A5 | 0.9717 |
| TCEA2 | 0.9715 |
| SOCS3 | 0.9692 |
| SMG1P2 | 0.9677 |
| SLC7A5P1 | 0.9677 |
| PRDM16 | 0.9671 |
| GS1-24F4.2 | 0.9669 |

| | coloncancer-VS-colonnormal |
|---|---|
| COL4A1 | 0.9665 |
| IGF2BP3 | 0.9649 |
| PPP2R2C | 0.9624 |
| CRIP2 | 0.9608 |
| NPTX1 | 0.9605 |
| C11orf96 | 0.9604 |
| PTPRS | 0.9603 |
| DACT1 | 0.9578 |
| SEMA5A | 0.9576 |
| GFPT2 | 0.9574 |
| RORB | 0.9574 |
| TRIP6 | 0.9563 |
| XKR5 | 0.9556 |
| SDK2 | 0.9545 |
| MIR193A | 0.9538 |
| COL4A2 | 0.9538 |
| HOXA7 | 0.9532 |
| MIR1469 | 0.9527 |
| FOXP2 | 0.9523 |
| GATA2 | 0.9520 |
| EN1 | 0.9520 |
| FBN1 | 0.9494 |
| SNHG18 | 0.9492 |
| FNBP1L | 0.9490 |
| SLC16A11 | 0.9489 |
| ANKRD9 | 0.9487 |
| CYP26A1 | 0.9456 |
| IRF4 | 0.9456 |
| CACNA1D | 0.9442 |
| VAV3-AS1 | 0.9428 |
| ARHGAP20 | 0.9410 |
| KIAA1024 | 0.9394 |
| GALNT14 | 0.9389 |
| ASCL2 | 0.9385 |
| VAV3 | 0.9385 |
| NAPRT | 0.9381 |
| STAC2 | 0.9355 |
| CHST1 | 0.9343 |
| EVA1C | 0.9336 |
| PXDC1 | 0.9327 |
| PRSS3 | 0.9327 |
| EPS8L2 | 0.9297 |
| CDH4 | 0.9297 |
| CHST2 | 0.9284 |
| ABO | 0.9279 |
| MATK | 0.9272 |
| PITX2 | 0.9259 |
| GLIS3 | 0.9258 |
| SATB2-AS1 | 0.9244 |
| LOC440461 | 0.9231 |
| ISLR2 | 0.9227 |

| | coloncancer-VS-colonnormal |
|---|---|
| FBLIM1 | 0.9213 |
| ANKRD34B | 0.9204 |
| SHC2 | 0.9202 |
| LTBP4 | 0.9192 |
| C5orf38 | 0.9167 |
| UNC5A | 0.9163 |
| FSTL4 | 0.9162 |
| NCKAP1 | 0.9154 |
| ZNF503 | 0.9144 |
| FZD7 | 0.9140 |
| LPAR1 | 0.9131 |
| NRG3 | 0.9127 |
| SLC35D3 | 0.9110 |
| PVRL3 | 0.9109 |
| CYS1 | 0.9101 |
| SOX8 | 0.9089 |
| SDK1 | 0.9084 |
| FAM189A1 | 0.9070 |
| LMF1 | 0.9066 |
| ZNF503-AS2 | 0.9059 |
| FGF5 | 0.9059 |
| MEX3B | 0.9056 |
| FAM84B | 0.9056 |
| PYGO1 | 0.9049 |
| BMP7 | 0.9041 |
| CLSTN2 | 0.9031 |
| ADAMTS17 | 0.9029 |
| FNDC1 | 0.9028 |
| GREB1L | 0.8998 |
| ZNF264 | 0.8993 |
| LOC401463 | 0.8987 |
| LTBP2 | 0.8976 |
| RIMBP2 | 0.8971 |
| ADD2 | 0.8970 |
| FLNC | 0.8964 |
| PCDH7 | 0.8953 |
| BAMBI | 0.8950 |
| AMZ1 | 0.8947 |
| ACKR3 | 0.8947 |
| GRM4 | 0.8944 |
| GDNF | 0.8934 |
| EFCC1 | 0.8923 |
| SFMBT2 | 0.8920 |
| FZD5 | 0.8901 |
| SMAD1 | 0.8898 |
| EPB41L3 | 0.8895 |
| CAMK2N2 | 0.8890 |
| LOC283731 | 0.8874 |
| RHOB | 0.8859 |
| KLF11 | 0.8854 |
| FGF3 | 0.8853 |

coloncancer-VS-colonnormal

| | |
|---|---|
| SCUBE1 | 0.8835 |
| SMAGP | 0.8834 |
| TMEFF2 | 0.8833 |
| PVRL2 | 0.8826 |
| SOX21 | 0.8823 |
| TNRC18 | 0.8814 |
| PTHLH | 0.8814 |
| FOXI3 | 0.8809 |
| KLF2 | 0.8768 |
| PRKCB | 0.8765 |
| CRMP1 | 0.8749 |
| SIRPA | 0.8741 |
| KDM2A | 0.8733 |
| ZNF141 | 0.8726 |
| GRK5 | 0.8718 |
| ZFPM2 | 0.8712 |
| NFATC1 | 0.8707 |
| NCAM1 | 0.8705 |
| LINC00261 | 0.8704 |
| AKNA | 0.8703 |

## Claims

1. A method for performing epigenetic analysis comprising calculating an epigenetic potential energy landscape (PEL), or the corresponding joint probability distribution, of a genomic region within one or more genomic samples, wherein the PEL is defined by

$$V_{\mathbf{X}}(\mathbf{x}) = \phi_0 - \log P_{\mathbf{X}}(\mathbf{x}),$$

wherein:

- $V_{\mathbf{x}}(\mathbf{x})$ is the PEL within a genomic region,
- $P_{\mathbf{x}}(\mathbf{x})$ is the joint probability of the random variable **X,** representing the methylation state of the modeled methylation sites, taking a value **x** within the genomic region, and
- $\Phi_0$ is a constant;

and wherein the joint probability distribution of a genomic region is computed by:

$$P_{\mathbf{X}}(\mathbf{x}) = \frac{1}{Z} \exp\{-V_{\mathbf{X}}(\mathbf{x})\},$$

wherein:

- $P_{\mathbf{x}}(\mathbf{x})$ is the joint probability of the random variable **X,** representing the methylation state of the modeled methylation sites, taking a value **x** within the genomic region,
- $V_{\mathbf{X}}(\mathbf{x})$ is the PEL within the genomic region, and
- $Z$ is the partition function computed by a recursive method;

wherein calculating the PEL comprises:

**a)** partitioning a genome into discrete genomic regions;
**b)** analyzing the methylation status within a genomic region by fitting a parametric statistical model (The Model) to methylation data that takes into account dependence among the methylation states at individual methylation sites and has the number of parameters growing slower than geometrically in the number of methylation sites inside the region; and
**c)** computing and analyzing a PEL, or the corresponding joint probability distribution, within the genomic region and/or its subregions and/or merged super-regions, thereby performing epigenetic analysis, wherein the PEL is calculated as follows:

$$V_{\mathbf{X}}(\mathbf{x}) = -\sum_{n=1}^{N} a_n (2x_n - 1) - \sum_{n=2}^{N} c_n (2x_n - 1)(2x_{n-1} - 1) ,$$

wherein:

- $V_X(\mathbf{x})$ is the PEL within a genomic region,
- $N$ is the number of modeled methylation sites within the genomic region, and
- $\{a_1,\dots,a_N\}$ and $\{c_2,\dots,c_N\}$ are parameters of the model, wherein the PEL parameters $\{a_1,\dots,a_N\}$ and $\{c_2,\dots,c_N\}$ are specified by setting $a_n = \alpha + \beta\rho_n$ and $c_n = \gamma / d_n$, wherein $\rho_n$ is the CpG density of the $n$-th modeled methylation site and $d_n$ is the distance of the $n$-th modeled methylation site from its "nearest-neighbor" modeled methylation site $n$ -1;

and wherein the method is implemented using a computer program operating on a computer system.

2. The method of claim 1, wherein each discrete genomic region is about 3000 base pairs in length and the subregions are about 150 base pairs in length.

3. The method of claim 1, wherein the parameters $\alpha$, $\beta$, $\gamma$ are estimated from methylation data using a maximum-likelihood approach.

4. The method of claim 1, further comprising comparing the PEL or its associated joint probability distribution, calculated for a genomic region of a first genome, with another PEL or its associated joint probability distribution, calculated for the corresponding genomic region of a second genome, optionally wherein PEL comparisons are performed for genomic regions across the entire first and second genome.

5. The method of claim 1, wherein analyzing the PEL further comprises quantifying the methylation level within genomic subregions.

6. The method of claim 5, wherein the methylation level within a genomic subregion is quantified using:

$$L = \frac{1}{N} \sum_{n=1}^{N} X_n ,$$

wherein:

- $L$ is the methylation level within a genomic subregion,
- $N$ is the number of modeled methylation sites within the genomic subregion, and
- $X_n$ is a random variable that takes value 0 if the $n$-th modeled methylation site of the genomic subregion is unmethylated and 1 if said site is methylated.

7. The method of claim 5, further comprising calculating a probability distribution for the methylation level within a genomic subregion.

8. The method of claim 7, wherein the probability distribution of the methylation level is computed as follows:

$$P_L(l) = \sum_{x \in S(Nl)} P_{\mathbf{X}}(\mathbf{x}),$$

wherein:

- $P_L(l)$ is the probability of the random variable $L$ for the methylation level taking a value $l$ within a genomic subregion,
- $P_{\mathbf{X}}(\mathbf{x})$ is the joint probability of the random variable $\mathbf{X}$, representing the methylation state of the modeled methylation sites, taking a value $\mathbf{x}$ within the genomic region, calculated by the method of claim 7,
- $S(lN)$ is the set of all methylation states within the genomic subregion with exactly $l \times N$ modeled methylation sites being methylated, and
- $N$ is the number of modeled methylation sites within the genomic subregion.

9.  The method of claim 1, further comprising annotating genomic features by analyzing the joint probability distribution or derivative summaries that overlap said genomic features, optionally wherein the genomic features are selected from the group consisting of genes, gene promoters, introns, exons, transcription start sites (TSSs), CpG islands (CGIs), CGI island shores, CGI shelves, differentially methylated regions (DMRs), entropy blocks (EBs), topologically associating domains (TADs), hypomethylated blocks, lamin-associated domains (LADs), large organized chromatin K9-modifications (LOCKs), imprinting control regions (ICRs), and transcription factor binding sites.

10. The method of claim 1, comprising acquiring methylation data from one or more techniques selected from the group consisting of whole genome bisulfite DNA sequencing, PCR-targeted bisulfite DNA sequencing, capture bisulfite sequencing, nanopore-based sequencing, single molecule real-time sequencing, bisulfite pyrosequencing, Gem-Code sequencing, 454 sequencing, insertion tagged sequencing, or other related methods.

11. A non-transitory computer readable storage medium encoded with a computer program, the program comprising instructions that when executed by one or more processors cause the one or more processors to perform operations to perform the method according to claims 1-10.

**Patentansprüche**

1.  Ein Verfahren zum Durchführen epigenetischer Analyse, beinhaltend das Berechnen einer epigenetischen Landschaft der potenziellen Energie (Potential Energy Landscape; PEL) oder der entsprechenden gemeinsamen Probabilitätsverteilung einer Genomregion innerhalb einer oder mehrerer genomischer Proben, wobei die PEL wie folgt definiert ist:

$$V_{\mathbf{X}}(\mathbf{x}) = \phi_0 - \log P_{\mathbf{X}}(\mathbf{x}),$$

wobei:

- $V_{\mathbf{X}}(\mathbf{x})$ die PEL innerhalb einer Genomregion ist,
- $P_{\mathbf{X}}(\mathbf{x})$ die gemeinsame Probabilität der Zufallsvariablen $\mathbf{X}$ ist, die den Methylierungszustand der modellierten Methylierungsstellen darstellt und innerhalb der Genomregion einen Wert $\mathbf{x}$ annimmt, und
- $\phi_0$ eine Konstante ist;

und wobei die gemeinsame Probabilitätsverteilung einer Genomregion wie folgt errechnet wird:

$$P_{\mathbf{X}}(\mathbf{x}) = \frac{1}{Z} \exp\{-V_{\mathbf{X}}(\mathbf{x})\},$$

wobei:

- $P_{\mathbf{X}}(\mathbf{x})$ die gemeinsame Probabilität der Zufallsvariablen $\mathbf{X}$ ist, die den Methylierungszustand der modellierten Methylierungsstellen darstellt und innerhalb der Genomregion einen Wert $\mathbf{x}$ annimmt,

- $V_x(\mathbf{x})$ die PEL innerhalb der Genomregion ist und
- $Z$ die durch ein rekursives Verfahren errechnete Partitionsfunktion ist;

wobei das Berechnen der PEL Folgendes beinhaltet:

a) Partitionieren eines Genoms in diskrete Genomregionen;
b) Analysieren des Methylierungszustands innerhalb einer Genomregion durch Anpassen eines parametrischen statistischen Modells (das Modell), das Abhängigkeit der Methylierungszustände an einzelnen Methylierungsstellen untereinander berücksichtigt und bei dem die Zahl von Parametern langsamer wächst als geometrisch bei der Zahl von Methylierungsstellen innerhalb der Region, an Methylierungsdaten; und
c) Errechnen und Analysieren einer PEL oder der entsprechenden gemeinsamen Probabilitätsverteilung innerhalb der Genomregion und/oder ihrer Teilregionen und/oder zusammengefasster Superregionen, wodurch eine epigenetische Analyse durchgeführt wird, wobei die PEL wie folgt berechnet wird:

$$V_{\mathbf{X}}(\mathbf{x}) = -\sum_{n=1}^{N} a_n (2x_n - 1) - \sum_{n=2}^{N} c_n (2x_n - 1)(2x_{n-1} - 1),$$

wobei:

- $V_x(\mathbf{x})$ die PEL innerhalb einer Genomregion ist,
- $N$ die Zahl modellierter Methylierungsstellen innerhalb der Genomregion ist und
- $\{a_1,...,a_N\}$ und $\{c_2,...,c_N\}$ Parameter des Modells sind, wobei die PEL-Parameter $\{a_1,...,a_N\}$ und $\{c_2,...,c_N\}$ durch Festsetzen von $a_n = \alpha + \beta\rho_n$ und $c_n = \gamma/d_n$ spezifiziert sind, wobei $\rho_n$ die CpG-Dichte der $n$-ten modellierten Methylierungsstelle ist und $d_n$ der Abstand der $n$-ten modellierten Methylierungsstelle von ihrer "nächsten benachbarten" modellierten Methylierungsstelle $n$ - 1 ist;

und wobei das Verfahren unter Verwendung eines auf einem Computersystem ablaufenden Computerprogramms umgesetzt wird.

2. Verfahren gemäß Anspruch 1, wobei jede diskrete Genomregion etwa 3000 Basenpaare lang ist und die Teilregionen etwa 150 Basenpaare lang sind.

3. Verfahren gemäß Anspruch 1, wobei die Parameter $\alpha, \beta, \gamma$ anhand von Methylierungsdaten unter Verwendung eines Ansatzes der maximalen Wahrscheinlichkeit abgeschätzt werden.

4. Verfahren gemäß Anspruch 1, ferner beinhaltend das Vergleichen der PEL oder ihrer assoziierten gemeinsamen Probabilitätsverteilung, berechnet für eine Genomregion eines ersten Genoms, mit einer anderen PEL oder ihrer assoziierten gemeinsamen Probabilitätsverteilung, berechnet für die entsprechende Genomregion eines zweiten Genoms, wobei optional PEL-Vergleiche für Genomregionen über das gesamte erste und zweite Genom hinweg durchgeführt werden.

5. Verfahren gemäß Anspruch 1, wobei das Analysieren der PEL ferner das Quantifizieren des Methylierungsgrades innerhalb von Genomteilregionen beinhaltet.

6. Verfahren gemäß Anspruch 5, wobei der Methylierungsgrad innerhalb einer Genomteilregion unter Verwendung der folgenden Formel quantifiziert wird:

$$L = \frac{1}{N} \sum_{n=1}^{N} X_n,$$

wobei:

- $L$ der Methylierungsgrad innerhalb einer Genomteilregion ist,
- $N$ die Zahl modellierter Methylierungsstellen innerhalb der Genomteilregion ist und
- $X_n$ eine Zufallsvariable ist, die den Wert 0 annimmt, wenn die n-te modellierte Methylierungsstelle der Genomteilregion unmethyliert ist, und 1, wenn die Stelle methyliert ist.

**7.** Verfahren gemäß Anspruch 5, ferner beinhaltend das Berechnen einer Probabilitätsverteilung für den Methylierungsgrad innerhalb einer Genomteilregion.

**8.** Verfahren gemäß Anspruch 7, wobei die Probabilitätsverteilung des Methylierungsgrades wie folgt errechnet wird:

$$P_L(l) = \sum_{x \in S(Nl)} P_{\mathbf{X}}(\mathbf{x}) \,,$$

wobei:

- $P_L(l)$ die Probabilität der Zufallsvariablen $L$ für den Methylierungsgrad ist, die innerhalb einer Genomteilregion einen Wert $l$ annimmt,
- $P_x(\mathbf{x})$ die gemeinsame Probabilität der Zufallsvariablen $\mathbf{X}$ ist, die den Methylierungszustand der modellierten Methylierungsstellen darstellt und innerhalb der Genomregion einen Wert $\mathbf{x}$ annimmt, berechnet mit dem Verfahren gemäß Anspruch 7,
- $S(lN)$ der Satz aller Methylierungszustände innerhalb der Genomteilregion mit exakt $l \times N$ modellierten Methylierungsstellen, die methyliert sind, ist und
- $N$ die Zahl modellierter Methylierungsstellen innerhalb der Genomteilregion ist.

**9.** Verfahren gemäß Anspruch 1, ferner beinhaltend das Annotieren von genomischen Merkmalen durch Analysieren der gemeinsamen Probabilitätsverteilung oder der Derivatzusammenfassungen, die die genomischen Merkmale überlappen, wobei die genomischen Merkmale optional aus der Gruppe ausgewählt sind, die aus Genen, Genepromotoren, Introns, Exons, Transkriptionsstartstellen (TSS), CpG-Inseln (CGI), CGI-Insel-Shores, CGI-Shelves, differenziell methylierten Regionen (DMR), Entropieblöcken (EB), topologisch assoziierten Domänen (TAD), hypomethylierten Blöcken, laminassoziierten Domänen (LAD), großen organisierten Chromatin-K9-Modifikationen (LOCK), Prägungssteuerungsregionen (ICR) und Transkriptionsfaktorbindungsstellen besteht.

**10.** Verfahren gemäß Anspruch 1, beinhaltend das Gewinnen von Methylierungsdaten aus einer oder mehreren Techniken, ausgewählt aus der Gruppe, bestehend aus Ganzgenom-Bisulfit-DNA-Sequenzierung, gezielter PCR-Bisulfit-DNA-Sequenzierung, Bisulfit-Einfangsequenzierung, auf Nanoporen basierender Sequenzierung, Einzelmolekül-Echtzeitsequenzierung, Bisulfit-Pyrosequenzierung, GemCode-Sequenzierung, 454-Sequenzierung, insertionsmarkierter Sequenzierung oder anderen verwandten Verfahren.

**11.** Ein nicht flüchtiges, computerlesbares Speichermedium, das mit einem Computerprogramm codiert ist, wobei das Programm Anweisungen beinhaltet, die bei Ausführung durch einen oder mehrere Prozessoren dazu führen, dass der eine oder die mehreren Prozessoren Abläufe durchführen, um das Verfahren gemäß den Ansprüchen 1-10 durchzuführen.

**Revendications**

**1.** Une méthode pour effectuer une analyse épigénétique comprenant le calcul d'un paysage d'énergie potentielle (PEL) épigénétique, ou de la distribution de probabilité conjointe correspondante, d'une région génomique au sein d'un ou de plusieurs échantillons génomiques, où le PEL est défini par

$$V_{\mathbf{X}}(\mathbf{x}) = \phi_0 - \log P_{\mathbf{X}}(\mathbf{x}),$$

où :

- $V_x(\mathbf{x})$ est le PEL au sein d'une région génomique,
- $P_x(\mathbf{x})$ est la probabilité conjointe que la variable aléatoire $\mathbf{X}$, représentant l'état de méthylation des sites de méthylation modélisés, prenne une valeur $\mathbf{x}$ au sein de la région génomique, et
- $\phi_0$ est une constante ;

et où la distribution de probabilité conjointe d'une région génomique est calculée informatiquement par :

$$P_{\mathbf{x}}(\mathbf{x}) = \frac{1}{Z}\exp\{-V_{\mathbf{X}}(\mathbf{x})\},$$

où :

- $P_{\mathrm{x}}(\mathbf{x})$ est la probabilité conjointe que la variable aléatoire **X,** représentant l'état de méthylation des sites de méthylation modélisés, prenne une valeur **x** au sein de la région génomique,
- $V_{\mathrm{x}}(\mathbf{x})$ est le PEL au sein de la région génomique, et
- $Z$ est la fonction de partition calculée informatiquement par une méthode récursive ;

où le calcul du PEL comprend :

a) le fait de partitionner un génome en régions génomiques discrètes ;
b) le fait d'analyser l'état de méthylation au sein d'une région génomique en ajustant un modèle statistique paramétrique (Le Modèle) aux données de méthylation qui prend en compte la dépendance entre les états de méthylation au niveau des sites de méthylation individuels et dont le nombre de paramètres croît plus lentement que géométriquement dans le nombre de sites de méthylation au sein de la région ; et
c) le fait de calculer informatiquement et d'analyser un PEL, ou la distribution de probabilité conjointe correspondante, au sein de la région génomique et/ou de ses sous-régions et/ou super-régions fusionnées, en effectuant ainsi une analyse épigénétique, où le PEL est calculé comme suit :

$$V_{\mathbf{X}}(\mathbf{x}) = -\sum_{n=1}^{N} a_n(2x_n - 1) - \sum_{n=2}^{N} c_n(2x_n - 1)(2x_{n-1} - 1),$$

où :

- $V_{\mathrm{x}}(\mathbf{x})$ est le PEL au sein d'une région génomique,
- $N$ est le nombre de sites de méthylation modélisés au sein de la région génomique, et
- $\{\alpha_1,...,\alpha_N\}$ et $\{c_2,...,c_N\}$ sont les paramètres du modèle, où les paramètres de PEL $\{\alpha_1,...,\alpha_N\}$ et $\{c_2,...,c_N\}$ sont spécifiés en fixant $\alpha_n = \alpha + \beta\rho_n$, et $c_n = \gamma / d_n$, où $\rho_n$ est la masse volumique de CpG du $n$-ième site de méthylation modélisé et $d_n$ est la distance du $n$-ième site de méthylation modélisé par rapport à son site de méthylation modélisé « voisin le plus proche » $n$-1 ;

et où la méthode est mise en œuvre à l'aide d'un programme informatique fonctionnant sur un système informatique.

2. La méthode de la revendication 1, où chaque région génomique discrète a une longueur d'environ 3 000 paires de bases et les sous-régions ont une longueur d'environ 150 paires de bases.

3. La méthode de la revendication 1, où les paramètres $\alpha$, $\beta$, $\gamma$ sont estimés à partir des données de méthylation à l'aide d'une approche de maximum de vraisemblance.

4. La méthode de la revendication 1, comprenant en outre la comparaison du PEL ou de sa distribution de probabilité conjointe associée, calculée pour une région génomique d'un premier génome, avec un autre PEL ou sa distribution de probabilité conjointe associée, calculée pour la région génomique correspondante d'un deuxième génome, facultativement où les comparaisons de PEL sont effectuées pour des régions génomiques sur l'ensemble du premier et du deuxième génome.

5. La méthode de la revendication 1, où l'analyse du PEL comprend en outre la quantification du niveau de méthylation au sein des sous-régions génomiques.

6. La méthode de la revendication 5, où le niveau de méthylation au sein d'une sous-région génomique est quantifié à l'aide de :

$$L = \frac{1}{N} \sum_{n=1}^{N} X_n \, ,$$

où :

- $L$ est le niveau de méthylation au sein d'une sous-région génomique,
- $N$ est le nombre de sites de méthylation modélisés au sein de la sous-région génomique, et
- $X_n$ est une variable aléatoire qui prend la valeur 0 si le $n$-ième site de méthylation modélisé de la sous-région génomique est non méthylé et 1 si ledit site est méthylé.

7. La méthode de la revendication 5, comprenant en outre le calcul d'une distribution de probabilité pour le niveau de méthylation au sein d'une sous-région génomique.

8. La méthode de la revendication 7, où la distribution de probabilité du niveau de méthylation est calculée informatiquement comme suit :

$$P_L(l) = \sum_{x \in S(Nl)} P_X(\mathbf{x}) \, ,$$

où :

- $P_L(l)$ est la probabilité que la variable aléatoire $L$ pour le niveau de méthylation prenne une valeur $l$ au sein d'une sous-région génomique,
- $P_X(\mathbf{x})$ est la probabilité conjointe que la variable aléatoire $\mathbf{X}$, représentant l'état de méthylation des sites de méthylation modélisés, prenne une valeur $\mathbf{x}$ au sein de la région génomique, calculée par la méthode de la revendication 7,
- $S(lN)$ est l'ensemble de tous les états de méthylation au sein de la sous-région génomique avec exactement $l \times N$ sites de méthylation modélisés méthylés, et
- $N$ est le nombre de sites de méthylation modélisés au sein de la sous-région génomique.

9. La méthode de la revendication 1, comprenant en outre l'annotation de caractéristiques génomiques en analysant la distribution de probabilité conjointe ou de sommes de dérivées qui se superposent auxdites caractéristiques génomiques, facultativement où les caractéristiques génomiques sont sélectionnées dans le groupe constitué de gènes, de promoteurs de gènes, d'introns, d'exons, de sites d'initiation de transcription (TSS), d'îlots CpG (CGI), de rivages d'îlots CGI, de bords CGI, de régions différentiellement méthylées (DMR), de blocs d'entropie (EB), de domaines topologiquement associés (TAD), de blocs hypométhylés, de domaines associés à la lamine (LAD), de modifications K9 de la chromatine organisée de grande envergure (LOCK), de régions de contrôle d'empreinte (ICR) et de sites de liaison de facteur de transcription.

10. La méthode de la revendication 1, comprenant l'acquisition de données de méthylation à partir d'une ou plusieurs techniques sélectionnées dans le groupe constitué de séquençage d'ADN au bisulfite du génome entier, de séquençage d'ADN au bisulfite ciblé par PCR, de séquençage au bisulfite par capture, de séquençage à base de nanopore, de séquençage en temps réel de molécule unique, de pyroséquençage au bisulfite, de séquençage GemCode, de séquençage 454, de séquençage marqué par insertion, ou autres méthodes apparentées.

11. Support de stockage lisible par ordinateur non transitoire codé avec un programme informatique, le programme comprenant des instructions qui, lorsqu'elles sont exécutées par un ou plusieurs processeurs, amènent ces un ou plusieurs processeurs à effectuer des opérations pour effectuer la méthode selon les revendications 1 à 10.

**FIGURE 1A**

**FIGURE 1B**

**FIGURE 1C**

**FIGURE 2A**

**FIGURE 2B**

**FIGURE 2C**

**FIGURE 3A**

**FIGURE 3B**

**FIGURE 3C**

**FIGURE 3D**

**FIGURE 4A**

**FIGURE 4B**

**FIGURE 5A**

**FIGURE 5B**

**FIGURE 5C**

**FIGURE 6**

**FIGURE 7A**

# EP 3 472 358 B1

**FIGURE 7B**

102

**FIGURE 7C**

**FIGURE 7D**

**FIGURE 7E**

**FIGURE 8**

**FIGURE 9A**

**FIGURE 9B**

**FIGURE 9C**

**FIGURE 9D**

**FIGURE 9E**

**FIGURE 10A**

**FIGURE 10B**

**FIGURE 11**

**FIGURE 12**

**FIGURE 13**

**FIGURE 14A**

**FIGURE 14B**

**FIGURE 15A**

**FIGURE 15B**

**FIGURE 15C**

**FIGURE 16**

**FIGURE 17**

**FIGURE 18A**

**FIGURE 18B**

**FIGURE 19A**

**FIGURE 19B**

**FIGURE 19C**

**FIGURE 19D**

**FIGURE 20A**

**FIGURE 20B**

**FIGURE 20C**

**FIGURE 21**

**FIGURE 22A**

**FIGURE 22B**

**FIGURE 22C**

**FIGURE 22D**

**FIGURE 22E**

**FIGURE 23**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5888737 A
- US 6175002 B
- US 5695934 A
- US 6140489 A
- US 5863722 A
- US 2007007991 A
- US 20090247414 A
- US 20100111768 A
- WO 2007123744 A

### Non-patent literature cited in the description

- **M. STEVENS et al.** *Genome Research,* vol. 23 (9), 1541-1553
- **ZILLER, M. J. et al.** *Nature,* 2013, vol. 500, 477-481
- **WU, H. et al.** *Biostatistics,* 2010, vol. 11, 499-514
- **TIMP, W. et al.** *Genome Med.,* 2014, vol. 6, 61
- **WEN, B. et al.** *BMC Genomics,* 2012, vol. 13, 566
- **GUELEN, L. et al.** *Nature,* 2008, vol. 453, 948-951
- **DIXON, J. R. et al.** *Nature,* 2015, vol. 518, 331-336
- **FORTIN, J. P ; HANSEN, K. D.** *Genome Biol.,* 2015, vol. 16, 180
- **BANDOPADHAYAY, P. et al.** MYB-QKI rearrangements in angiocentric glioma drive tumorigenicity through a tripartite mechanism. *Nat. Genet.,* 2016, vol. 48, 273-282
- Exactly Solved Models. **BAXTER, R. J.** Statistical Mechanics. Academic Press, 1982
- **BENNET, C. H.** The thermodynamics of computation - a review. *Int. J. Theor. Phys.,* 1982, vol. 21, 905-940
- **BERGMAN, Y. ; CEDAR, H.** DNA methylation dynamics in health and disease. *Nat. Struct. Mol. Biol.,* 2013, vol. 20, 274-281
- **BERMAN, B. P. et al.** Regions of focal DNA hypermethylation and long-range hypomethylation in colorectal cancer coincide with nuclear lamina-associated domains. *Nat. Genet.,* 2012, vol. 44, 40-46
- **BICKEL, P. J. ; DOKSUM, K. A.** Mathematical Statistics: Basic Ideas and Selected Topics. Prentice-Hall, 2007, vol. I
- **BOYES, J. ; BIRD, A.** Repression of genes by DNA methylation depends on CpG density and promoter strength: evidence for involvement of a methyl-CpG binding protein. *EMBO J.,* 1992, vol. 11, 327-333
- **COVER, T. M. ; THOMAS, J.A.** Elements of Information Theory. John Wiley & Sons, 2006
- **DE LA CRUZ, C. C. et al.** The polycomb group protein SUZ12 regulates histone H3 lysine 9 methylation and HP1 alpha distribution. *Chromosome Res.,* 2007, vol. 15, 299-314
- **DEBAUN, M. R. et al.** Epigenetic alterations of H19 and LIT1 distinguish patients with Beckwith-Wiedemann syndrome with cancer and birth defects. *Am. J. Hum. Genet.,* 2002, vol. 70, 604-611
- **DEKKER, J. ; MARTI-RENOM, M. A. ; MIRNY, L. A.** Exploring the three-dimensional organization of genomes: interpreting chromatin interaction data. *Nat. Rev. Genet.,* 2013, vol. 14, 390-403
- **DIXON, J. R. et al.** Topological domains in mammalian genomes identified by analysis of chromatin interactions. *Nature,* 2012, vol. 485, 376-380
- **DIXON, J. R. et al.** Chromatin architecture reorganization during stem cell differentiation. *Nature,* 2015, vol. 518, 331-336
- **EDEN, E. et al.** GOrilla: a tool for discovery and visualization of enriched GO terms in ranked gene lists. *BMC Bioinformatics,* 2009, vol. 10, 48
- **FENG, F. et al.** Genomic landscape of human allele-specific DNA methylation. *Proc. Natl. Acad. Sci. USA,* 2012, vol. 109, 7332-7337
- **FASHAMI, M. S. ; ATULASIMHA, J. ; BANDYO-PADHYAY, S.** Energy dissipation and error probability in fault-tolerant binary switching. *Sci. Rep.,* 2013, vol. 3, 3204
- **FAVOROV, A. et al.** Exploring massive, genome scale datasets with the GenometriCorr package. *PLoS Comput. Biol.,* 2012, vol. 8, e1002529
- **FORTIN, J. P. ; HANSEN, K. D.** Reconstructing A/B compartments as revealed by Hi-C using long-range correlations in epigenetic data. *Genome Biol.,* 2015, vol. 16, 180
- **FRIEL, N. ; RUE, H.** Recursive computing and simulation-free inference for general factorizable models. *Biometrika,* 2007, vol. 94, 661-672

- **FU, A. Q. et al.** Statistical inference of transmission fidelity of DNA methylation patterns over somatic cell divisions in mammals. *Ann. Appl. Stat.,* 2010, vol. 4, 871-892
- **FU, A. Q. et al.** Statistical inference of in vivo properties of human DNA methyltransferases from double-stranded methylation patterns. *PLoS One,* 2012, vol. 7, e32225
- **GENEREUX, D. P. et al.** A population-epigenetic model to infer site-specific methylation rates from double-stranded DNA methylation patterns. *P. Natl. Acad. Sci. USA,* 2005, vol. 102 (16), 5802-5807
- **GIBCUS, J. H. ; DEKKER, J.** The hierarchy of the 3D genome. *Mol. Cell,* 2013, vol. 49, 773-782
- **GUELEN, L. et al.** Domain organization of human chromosomes revealed by mapping of nuclear lamina interactions. *Nature,* 2008, vol. 453, 948-951
- **HANSEN, K. D. et al.** Increased methylation variation in epigenetic domains across cancer types. *Nat. Genet.,* 2011, vol. 43, 768-775
- **HANSEN, K. D. et al.** Large-scale hypomethylated blocks associated with Epstein-Barr virus-induced B-cell immortalization. *Genome Res.,* 2014, vol. 24, 177-184
- **HUANG, J. ; MARCO, E. ; PINELLO, L. ; YUAN, G. C.** Predicting chromatin organization using histone marks. *Genome Biol.,* 2015, vol. 16, 162
- **HUYER, W. ; NEUMAIER, A.** Global optimization by multilevel coordinate search. *J. Global Optim.,* 1999, vol. 14, 331-355
- **ILLINGWORTH, R. S. ; BIRD, A. P.** CpG islands - 'A rough guide. *FEBS Lett.,* 2009, vol. 583, 1713-1720
- **KANEDA, H. et al.** FOXQ1 is overexpressed in colorectal cancer and enhances tumorigenicity and tumor growth. *Cancer Res.,* 2010, vol. 70, 2053-2063
- **KOHLI, R. M. ; ZHANG, Y.** TET enzymes, TDG and the dynamics of DNA demethylation. *Nature,* 2013, vol. 502, 472-479
- **LACEY, M. R. ; EHRLICH, M.** Modeling dependence in methylation patterns with application to ovarian carcinomas. *Stat. Appl. Genet. M. B.,* 2009, vol. 8, 40
- **LANDAN, G. et al.** Epigenetic polymorphism and the stochastic formation of differentially methylated regions in normal and cancerous tissues. *Nat. Genet.,* 2012, vol. 44, 1207-1214
- **LANDAUER, R.** Uncertainty principle and minimal energy dissipation in the computer. *Int. J. Theor. Phys.,* 1982, vol. 21, 283-297
- **LEWIS, A. ; MURRELL, A.** Genomic imprinting: CTCF protects the boundaries. *Curr. Biol.,* 2004, vol. 14, R284-286
- **LI, S. et al.** Dynamic evolution of clonal epialleles revealed by methclone. *Genome Biol.,* 2014, vol. 15, 472
- **LIN, J.** Divergence measures based on the Shannon entropy. *IEEE Trans. Inform. Theory,* 1991, vol. 37, 145-151
- **MANNENS, M. et al.** Positional cloning of genes involved in the Beckwith-Wiedemann syndrome, hemihypertrophy, and associated childhood tumors. *Med. Pediatr. Oncol.,* 1996, vol. 27, 490-494
- **MARGUERON, R. ; REINBERG, D.** The Polycomb complex PRC2 and its mark in life. *Nature,* 2011, vol. 469, 343-349
- **MARVAN, M.** The energy dissipation, the error probability and the time of duration of a logical operation. *Kybernetika,* 1982, vol. 18, 345-355
- **MURTAGH, F. ; LEGENDRE, P.** Ward's hierarchical agglomerative clustering method: Which algorithms implement Ward's criterion?. *J. Classif.,* 2014, vol. 31, 274-295
- **NAKAMURA, T. et al.** Fusion of the nucleoporin gene NUP98 to HOXA9 by the chromosome translocation t(7;11)(p15;p15) in human myeloid leukaemia. *Nat. Genet.,* 1996, vol. 12, 154-158
- **NORA, E. P. et al.** Spatial partitioning of the regulatory landscape of the X-inactivation centre. *Nature,* 2012, vol. 485, 381-385
- **OGAWA, O. et al.** Relaxation of insulin-like growth factor II gene imprinting implicated in Wilms' tumour. *Nature,* 1993, vol. 362, 749-751
- **PENG, H. et al.** LIMD2 is a small LIM-only protein overexpressed in metastatic lesions that regulates cell motility and tumor progression by directly binding to and activating the integrin-linked kinase. *Cancer Res.,* 2014, vol. 74, 1390-1403
- **PETERS, M. J. et al.** The transcriptional landscape of age in human peripheral blood. *Nat Commun,* 2015, vol. 6, 8570
- **PFEIFER, G. P. et al.** Polymerase chain reaction-aided genomic sequencing of an X chromosome-linked CpG island: methylation patterns suggest clonal inheritance, CpG site autonomy, and an explanation of activity state stability. *Proc. Natl. Acad. Sci. USA,* 1990, vol. 87, 8252-8256
- Numerical Recipes. **PRESS, W. H. ; TEUKOLSKY, S.A. ; VETTERLING, W.T. ; FLANNERY, B.P.** The Art of Scientific Computing. Cambridge University Press, 2007
- **PUJADAS, E. ; FEINBERG, A. P.** Regulated noise in the epigenetic landscape of development and disease. *Cell,* 2012, vol. 148, 1123-1131
- **RAO, S. S. et al.** A 3D map of the human genome at kilobase resolution reveals principles of chromatin looping. *Cell,* 2014, vol. 159, 1665-1680
- **REEVES, R. ; PETTIT, A. N.** Efficient recursions for general factorisable models. *Biometrika,* 2004, vol. 91, 751-757

- **SCHLAEGER, T. M. et al.** A comparison of non-integrating reprogramming methods. *Nat. Biotechnol.,* 2015, vol. 33, 58-63
- **SHIPONY, Z. et al.** Dynamic and static maintenance of epigenetic memory in pluripotent and somatic cells. *Nature,* 2014, vol. 513, 115-119
- **SONTAG, L. B. ; LORINCZ, M. C. ; LUEBECK, E. G.** Dynamics, stability and inheritance of somatic DNA methylation imprints. *J. Theor. Biol.,* 2006, vol. 242, 890-899
- **STÖGER, R. et al.** Epigenetic variation illustrated by DNA methylation patterns of the fragile-X gene FMR1. *Hum. Mol. Genet.,* 1997, vol. 6, 1791-1801
- **STOREY, J. D. ; TIBSHIRANI, R.** Statistical significance for genomewide studies. *Proc. Natl. Acad. Sci. U. S. A.,* 2003, vol. 100, 9440-9445
- **TIMP, W. ; FEINBERG, A. P.** Cancer as a dysregulated epigenome allowing cellular growth advantage at the expense of the host. *Nat. Rev. Cancer,* 2013, vol. 13, 497-510
- **TIMP, W. et al.** Large hypomethylated blocks as a universal defining epigenetic alteration in human solid tumors. *Genome Med.,* 2014, vol. 6, 61
- **VANDIVER, A. R. et al.** Age and sun exposure-related widespread genomic blocks of hypomethylation in nonmalignant skin. *Genome Biol.,* 2015, vol. 16, 80
- **VISEL, A. ; MINOVITSKY, S. ; DUBCHAK, I. ; PENNACCHIO, L. A.** VISTA Enhancer Browser - a database of tissue-specific human enhancers. *Nucleic Acids Res.,* 2007, vol. 35, D88-92
- **WADDINGTON, C. H.** The strategy of genes. *Allen and Unwin,* 1957
- **WEN, B. et al.** Large histone H3 lysine 9 dimethylated chromatin blocks distinguish differentiated from embryonic stem cells. *Nat. Genet.,* 2009, vol. 41, 246-250
- **WEN, B. et al.** Euchromatin islands in large heterochromatin domains are enriched for CTCF binding and differentially DNA-methylated regions. *BMC Genomics,* 2012, vol. 13, 566
- **WU, H. et al.** Redefining CpG islands using hidden Markov models. *Biostatistics,* 2010, vol. 11, 499-514
- **YAMAMOTO, K. et al.** Polycomb group suppressor of zeste 12 links heterochromatin protein 1 alpha and enhancer of zeste 2. *J. Biol. Chem.,* 2004, vol. 279, 401-406
- **YANG, G. et al.** RNA-binding protein quaking, a critical regulator of colon epithelial differentiation and a suppressor of colon cancer. *Gastroenterology,* 2010, vol. 138, 231-240
- **YU, H. et al.** Tet3 regulates synaptic transmission and homeostatic plasticity via DNA oxidation and repair. *Nat. Neurosci.,* 2015, vol. 18, 836-843
- **ZILLER, M. J. et al.** Charting a dynamic DNA methylation landscape of the human genome. *Nature,* 2013, vol. 500, 477-481